# EUROPEAN PATENT SPECIFICATION

(11) **EP 1 409 686 B1**
(45) Date of publication and mention of the grant of the patent: **22.03.2006**
(21) Application number: 02750735.9
(22) Date of filing: 26.07.2002
(51) Int. Cl.: C12N 15/52, C12P 19/62, C12N 9/00, C12N 15/63

(54) **GENES AND PROTEINS FOR THE BIOSYNTHESIS OF ROSARAMICIN**
GENE UND PROTEINE ZUR ROSARAMICIN BIOSYNTHESE
GENES ET PROTEINES INTERVENANT DANS LA BIOSYNTHESE DE ROSARAMICINE

(30) Priority: 26.07.2001 US 307629 P
(43) Date of publication of application: 21.04.2004
(73) Proprietor: Ecopia Biosciences Inc., Saint-Laurent, Québec H4S 2A1 (CA)
(72) Inventor: FARNET, Chris, M., Outremont, Québec H2V 3S3 (CA); STAFFA, Alfredo, Saint-Laurent, Québec H4L 5M5 (CA); YANG, Xianshu, Belmont, MA 02478 (US)
(74) Representative: Vossius & Partner
(86) International application number: PCT/CA2002/001177
(87) International publication number: WO 2003/010193

(56) References cited:
- US-A- 4 234 690
- DATABASE EMBL [Online] Entry SF08223, 20 April 1994 (1994-04-20) MERSON-DAVIES L.A. ET AL.: "Streptomyces fradiae cytochrome P-450, dTDP-glucose synthase, dTDP-glucose dehydratase, thioesterase, TylCVI (tylCVI), and TylR (tylR) genes " Database accession no. U08223 XP002228882 & MERSON-DAVIES L.A. ET AL.: "Analysis of five tylosin biosynthetic genes from the tylIBA region of the Streptomyces fradiae genome" MOLECULAR MICROBIOLOGY, vol. 13, 1994, pages 349-355,
- DATABASE EMBL [Online] Entry SFTYLORF, 8 January 1997 (1997-01-08) GANDECHA A.R. : "Streptomyces fradiae tylMIII(orf1*), tylMII(orf2*), tylMI(orf3*) and ccr(orf4*) genes" Database accession no. X81885 XP002228883
- DATABASE SWALL [Online] Entry Q9ZBK1, 1 May 1999 (1999-05-01) SEEGER, K.J. ET AL.: "Crotonyl CoA reductase" Database accession no. Q9ZBK1 XP002228884 & BENTLEY, S.D. ET AL.: "A set of ordered cosmids and a detailed genetic and physical map for the 8 Mb Streptomyces coelicolor A3(2) chromosome" MOLECULAR MICROBIOLOGY, vol. 21, 1996, pages 77-96,

### SEQUENCE LISTING

<110> ECOPIA BIOSCIENCES INC.
   Farnet, Chris
   Yang, Xianshu
   Staffa, Alfredo
<120> GENES AND PROTEINS FOR THE BIOSYNTHESIS OF ROSARAMICIN
<130> 3016-3PCT.
<160> 39
<170> PatentIn version 3.0
<210> 1
   <211> 60196
   <212> DNA
   <213> micromonospora carbonacea subspecies aurantiaca
<400> 1
<210> 2
   <211> 560
   <212> PRT
   <213> micromonospora carbonacea subspecies aurantiaca
<400> 2
   Val Pro Val Pro Thr Gln Glu Ala Pro Leu Arg Asn Ser Pro Pro Pro
   1 5 10 15
   Ala His Ser Gln Leu Val Leu Ser Glu Val Thr Lys His Tyr Ala Glu
   20 25 30
   Arg Val Val Leu Asp Arg Val Ser Leu Thr Val Lys Pro Gly Glu Arg
   35 40 45
   Val Gly Val Ile Gly Glu Asn Gly Ser Gly Lys Ser Thr Leu Leu Arg
   50 55 60
   Leu Val Ala Gly Leu Glu Thr Pro Asp Asn Gly Glu Leu Thr Val Ser
   65 70 75 80
   Ala Pro Gly Gly Ile Gly Tyr Leu Ala Gln Arg Leu Arg Leu Pro Ala
   85 90 95
   Gly Gly Ser Thr Val Arg Asp Val Val Asp His Thr Leu Ala Asp Leu
   100 105 110
   Arg Asp Leu Glu Ala Arg Leu Arg Ala Ala Glu Ala Asp Leu Ala Thr
   115 120 125
   Ala Thr Pro Glu Gln Leu Asp Ala Tyr Gly Thr Leu Leu Thr Val Phe
   130 135 140
   Glu Ala Arg Gly Gly Tyr Gln Ala Asp Ala Arg Val Asp Ala Ala Leu
   145 150 155 160
   His Gly Leu Gly Leu Ala Glu Leu Asp Arg Asp Arg Asp Val Asp Thr
   165 170 175
   Leu Ser Gly Gly Glu Arg Ser Arg Leu Ala Leu Ala Ala Thr Leu Ala
   180 185 190
   Ala Ala Pro Glu Leu Leu Leu Leu Asp Glu Pro Thr Asn Asp Leu Asp
   195 200 205
   Ile Glu Ala Val Glu Trp Leu Glu Asp His Leu Arg Ser His Arg Gly
   210 215 220
   Thr Val Val Val Val Thr His Asp Arg Val Phe Leu Glu Ser Val Thr
   225 230 235 240
   Ser Thr Ile Leu Glu Val Asp Thr Asp Thr Arg Ala Val His Arg Tyr
   245 250 255
   Gly Asp Gly Tyr Ala Ser Tyr Leu Arg Ala Lys Ala Ala Leu Arg Glu
   260 265 270
   Ser Arg Glu Arg Ala Tyr Ala Glu Trp Val Ala Glu Val Glu Arg Gln
   275 280 285
   Ser Gln Leu Ala Glu Arg Ala Gly Thr Met Leu Arg Ser Ile Ser Arg
   290 295 300
   Lys Gly Pro Ala Ala Phe Ser Gly Ala Gly Ala His Arg Ser Arg Ser
   305 310 315 320
   Ser Ser Thr Ala Thr Ser Arg Lys Ala Arg Asn Ala Asn Glu Arg Leu
   325 330 335
   Arg Arg Leu Arg Glu Asn Pro Val Pro Arg Pro Ala Asp Pro Leu Arg
   340 345 350
   Phe Thr Ala Ser Val Ala Pro Asp Ala Thr Asp Ala Asp Thr Arg Arg
   355 360 365
   Val Glu Leu Thr Asp Val Arg Val Gly Arg Arg Leu His Val Pro Glu
   370 375 380
   Leu Thr Ile Gly Pro Ala Glu Arg Leu Leu Val Thr Gly Pro Asn Gly
   385 390 395 400
   Ala Gly Lys Ser Thr Leu Met Arg Val Leu Ala Gly Glu Leu Val Pro
   405 410 415
   Asp Gly Gly Thr Val Arg Leu Pro Ala Arg Ile Gly His Leu Arg Gln
   420 425 430
   Asp Val Thr Val Gly Gln Pro Gly Arg Ser Leu Leu Glu Thr Tyr Ala
   435 440 445
   Ser Gly Arg Pro Gly His Pro Glu Glu Tyr Ala Glu Glu Leu Leu Ala
   450 455 460
   Arg Gly Leu Phe Arg Pro Asp Asp Leu Arg Met Pro Val Gly Thr Leu
   465 470 475 480
   Ser Val Gly Gln Arg Arg Arg Ile Asp Leu Ala Arg Leu Val Ala Arg
   485 490 495
   Pro Ala Asp Leu Leu Leu Leu Asp Glu Pro Thr Asn His Phe Ala Pro
   500 505 510
   Leu Leu Val Glu Glu Leu Glu Gln Ala Leu Asp Gly Tyr Ala Gly Ala
   515 520 525
   Leu Val Val Val Thr His Asp Arg Arg Met Arg Ser Thr Phe Thr Gly
   530 535 540
   Ala Arg Leu Glu Leu His Gln Gly Val Ala Thr Gly Ala Ser Arg Ala
   545 550 555 560
<210> 3
   <211> 1683
   <212> DNA
   <213> micromonospora carbonacea subspecies aurantiaca
<400> 3
   gtgccagttc cgacacagga ggcccccttg cggaacagcc cgccgccagc ccattcgcag      60
   ctcgtcctga gcgaggtcac gaagcactac gccgagcggg tcgtcctgga ccgcgtttcg 120
   ctcaccgtca agccggggga gcgggtcggc gtcatcggcg agaacgggtc ggggaagtcg 180
   accctgctgc ggctcgtcgc ggggctggag acgccggaca acggcgagtt gaccgtctcg 240
   gcgcccgggg gcatcggcta tctcgcccag cggcttcggc tgccggccgg cggcagcacc 300
   gtacgggatg tggtggacca cacgctcgcc gacctgcgag acctggaggc gcggttgcgc 360
   gccgccgagg cggacctggc caccgccacg cccgagcagt tggacgccta cggcacgctg 420
   ctcactgtgt tcgaggcccg cggcggctac caggccgacg cccgggtgga cgccgccctg 480
   cacggtctcg gcctggccga gctcgaccgc gatcgcgacg tcgacacgct ctccggcggg 540
   gaacggtccc ggctcgcgct cgccgcgacc ctggccgccg cgccggaact gctgctgctc 600
   gacgagccca ccaacgacct cgacatcgag gccgtggagt ggctggagga tcacctgcgg 660
   tcgcaccggg gcaccgtcgt cgtggtcact cacgaccggg tgttcctgga gtcggtcacg 720
   tccaccatcc tcgaggtcga caccgacacc cgggccgtgc accggtacgg cgacggctat 780
   gccagctacc tgcgggccaa ggccgccctc cgggagagcc gggagcgcgc gtacgcggaa 840
   tgggtggccg aggtcgagcg gcagtcccaa ctcgcggagc gggccgggac gatgctccgg 900
   tcgatctccc gcaagggacc ggctgcgttc agcggggccg gtgcccaccg ctcccggtcg 960
   tcgtcgacgg cgacgtcacg caaggcccgc aacgccaacg agcggcttcg ccggctgcgg 1020
   gagaatccgg taccgcgacc cgccgacccg ttgcgcttca ccgcgtcggt cgccccggat 1080
   gccacggacg ccgatacccg ccgcgtcgag ttgaccgacg tccgggtggg ccgccgcctg 1140
   cacgtgcccg agctgaccat cggacccgcc gaacggttgc tggtgaccgg acccaacggc 1200
   gcgggtaaga gcaccctgat gcgggtgctc gccggggaac tcgtgcccga cggcggaacg 1260
   gtgcggctgc cggctcggat cggccacctg cgtcaggacg tgacggtcgg gcagcccggg 1320
   cgctctctgc tggagacgta cgcgtcgggt cggccggggc atcccgagga gtacgcggag 1380
   gagttgctcg cccgcggtct gttccggccc gatgacctgc gcatgccggt cgggacgctc 1440
   tccgtcgggc agcgccgccg gatcgacctg gcccggctgg tcgcccgccc ggccgacctg 1500
   ctgctgttgg acgagcccac caaccacttc gcgcccctgc tcgtggagga gctggaacag 1560
   gcgctggacg gctacgccgg agcgctggtc gtggtgacgc acgaccggcg gatgcggagc 1620
   accttcaccg gggctcggct ggaactgcac cagggcgtgg ccaccggggc gagccgggcc 1680
   tga 1683
<210> 4
   <211> 264
   <212> PRT
   <213> micromonospora carbonacea subspecies aurantiaca
<400> 4
   Met Ser Pro Ser Ala Asp Pro Ser Glu Leu Trp Leu Arg Arg Tyr Arg
   1 5 10 15
   Pro Val Asn Asp Pro Ala Val Arg Leu Phe Cys Phe Pro His Ala Gly
   20 25 30
   Gly Ala Ala Ser Ala Tyr Leu Pro Phe Ala Arg Arg Leu Ala Ala Asp
   35 40 45
   Val Asp Val Leu Ala Val Gln Tyr Pro Gly Arg Gln Asp Arg Arg Gly
   50 55 60
   Glu Pro Leu Ile Glu Ser Val Asp Ala Leu Val Asp Gly Leu Leu Pro
   65 70 75 80
   Ala Leu Leu Ala Trp Ala Asp Arg Pro Val Ala Phe Phe Gly His Ser
   85 90 95
   Met Gly Ala Thr Val Ala Phe Glu Ala Ala Arg Arg Leu Pro Pro Ala
   100 105 110
   Asp Ala Asp Arg Leu Val His Leu Phe Ala Ser Gly Arg Arg Ser Pro
   115 120 125
   Ser Val Gly Arg Arg Asp Arg Phe Tyr Arg Phe Asp Asp Glu Leu Ile
   130 135 140
   Asp Glu Ile Arg Arg Leu Gln Gly Thr Asp Ser Ser Leu Leu Asp Asp
   145 150 155 160
   Arg Glu Leu Leu Asp Met Leu Leu Pro Ala Ile Arg Asn Asp Tyr Arg
   165 170 175
   Ala Ala Ala Ala Tyr Glu Tyr Arg Pro Gly Pro Arg Leu Arg Cys Pro
   180 185 190
   Val Thr Val Leu Ala Gly Ala Ala Asp Thr His Val Thr Thr Asp Glu
   195 200 205
   Ala Ala Ala Trp Ala Glu Val Thr Ala Ala Ala Thr Met Val Arg Thr
   210 215 220
   Phe Pro Gly Gly His Phe Tyr Leu Asn Asp Gln Leu Asp Ala Val Cys
   225 230 235 240
   Ala Glu Val Thr Thr Thr Leu Ala Ala Val Ser Thr Thr Ala Leu Thr
   245 250 255
   Ala Val Pro Gly Ala Asp Pro Gly
   260
<210> 5
   <211> 795
   <212> DNA
   <213> micromonospora carbonacea subspecies aurantiaca
<400> 5
   atgtccccgt ccgccgatcc gtccgagctg tggctacgcc gctaccggcc cgtcaacgac 60
   cccgccgtcc ggctgttctg cttcccgcac gccgggggcg cggccagcgc gtacctgccg 120
   ttcgcccgcc ggctcgccgc cgacgtggac gtgctggcgg tccagtaccc gggccggcag 180
   gaccgccgcg gcgaaccctt gatcgagtcc gtcgacgccc tggtggacgg gctcctgccc 240
   gcactgctcg cctgggcgga ccgaccggtg gccttcttcg gtcacagcat gggcgccacg 300
   gtggccttcg aggccgcccg ccggctccca ccggccgacg ccgatcggct cgtgcacctc 360
   ttcgcctccg gccgccgtag cccgtccgtc gggcggcggg accggttcta ccggtttgac 420
   gacgagctga tcgacgagat ccgccggctc cagggcaccg attccagcct cctggacgac 480
   agggaactgc tggacatgct cctccccgcc atccgcaacg actaccgggc cgccgccgcc 540
   tacgaatacc ggccagggcc caggctgcgt tgcccggtca ccgtactcgc cggggccgcc 600
   gacacccacg tcaccaccga cgaggccgcg gcgtgggccg aggtgaccgc agcggccacg 660
   atggtccgca cgttcccggg cgggcacttc tatctcaacg atcagctcga cgctgtgtgc 720
   gccgaggtca cgaccaccct cgcagcggtg tccacgaccg ccctcacggc ggtgccgggt 780
   gccgaccccg gctga 795
<210> 6
   <211> 410
   <212> PRT
   <213> micromonospora carbonacea subspecies aurantiaca
<400> 6
   Met Thr Gln Thr Pro Asn Ala Pro Ala Gly Pro Ile Asp Leu Pro Lys
   1 5 10 15
   Gly Ala Asp Ala Gln Gly Leu Leu Asp Trp Phe Ala Tyr Met Arg Lys
   20 25 30
   Asn Trp Pro Val Ser Trp Asp Glu Thr Arg Gln Ala Trp His Val Phe
   35 40 45
   Ser Tyr Arg Asp Tyr Gln Thr Val Thr Thr Asn Pro Leu Ile Phe Ser
   50 55 60
   Ser Asp Phe Thr Ser Val Phe Pro Val Pro Ser Glu Leu Ala Leu Leu
   65 70 75 80
   Met Gly Pro Gly Thr Ile Gly Gly Ile Asp Pro Pro Arg His Ala Pro
   85 90 95
   Leu Arg Lys Leu Val Ser Gln Ala Phe Thr Pro Arg Arg Ile Ala Gln
   100 105 110
   Met Glu Leu Arg Ile Gly Gln Ile Thr Ala Asp Val Leu Asp Gln Val
   115 120 125
   Arg Asp Gln Asp Arg Ile Asp Ile Ala Ser Asp Leu Ala Tyr Pro Leu
   130 135 140
   Pro Val Thr Val Ile Ala Glu Leu Leu Gly Ile Pro Thr Lys Asp His
   145 150 155 160
   Glu Lys Phe Arg Glu Trp Val Asp Ile Ile Leu Ser Asn Glu Gly Leu
                   165                 170                 175
   Glu Tyr Pro Asn Leu Pro Asp Asp Phe Thr Glu Thr Val Gly Pro Ala
               180                 185                 190
   Ile Glu Glu Trp Ser Glu Phe Leu Tyr Ala Gln Ile Ala His Lys Arg
           195                 200                 205
   Ala Glu Pro Lys Asp Asp Leu Ile Ser Gly Leu Cys Ala Ala Glu Val
       210                 215                 220
   Asp Gly Arg Lys Leu Thr Asp Glu Glu Val Val Asn Ile Val Ala Leu
   225                 230                 235                 240
   Leu Leu Thr Ala Gly His Ile Ser Ser Ala Thr Leu Leu Ser Asn Leu
                   245                 250                 255
   Phe Leu Val Leu Glu Glu His Pro Gln Ala Gln Ala Ala Val Arg Ala
               260                 265                 270
   Asp Arg Ser Leu Val Pro Gly Val Ile Glu Glu Thr Leu Arg Tyr Arg
           275                 280                 285
   Ser Pro Phe Asn Cys Ile Phe Arg Ile Leu Asn Glu Asp Thr Asp Ile
       290                 295                 300
   Leu Gly His Pro Met Arg Lys Gly Gln Met Val Ile Ala Trp Ile Ala
   305                 310                 315                 320
   Ser Ala Asn Arg Asp Thr Glu Val Phe Thr Asp Pro Asp Thr Phe Asp
                   325                 330                 335
   Ile Arg Arg Glu Ser Asn Lys His Leu Ala Phe Gly His Gly Ile His
               340                 345                 350
   His Cys Leu Gly Ala Phe Leu Ala Arg Leu Glu Ala Lys Val Phe Leu
           355                 360                 365
   Asn Gln Thr Leu Asp Gln Phe Thr Glu Phe Arg Ile Asp His Val Gly
       370                 375                 380
   Val Glu Phe Tyr Asp Ala Asp Gln Leu Thr Ala Arg Arg Leu Pro Val
   385                 390                 395                 400
   Gln Val Val Arg Asp Gly Arg His Pro Lys
                   405                 410
<210> 7
   <211> 1233
   <212> DNA
   <213> micromonospora carbonacea subspecies aurantiaca
<400> 7
   atgacgcaga ccccgaacgc cccggcggga ccgatcgacc tgcccaaggg cgccgacgcc 60
   caggggctgc tggactggtt cgcgtacatg cggaagaact ggcccgtctc ctgggacgag 120
   acccgtcagg cctggcacgt gttctcctac cgggactacc agaccgtgac caccaacccg 180
   ctgatcttct cgtcggactt cacctcggtc tttcccgtac cgtcggagct ggccctgctg 240
   atgggccccg gcaccatcgg cggcatcgac ccgccgcggc acgcgccgct gcgcaagctg 300
   gtgagccagg cgttcacccc ccgccggatc gcccagatgg agctgcggat cgggcagatc 360
   accgccgacg tgctcgacca ggtacgcgac caggaccgga tcgacatcgc cagcgacctc 420
   gcgtacccgc tgccggtgac ggtcatcgcc gagctgctcg gcattcccac caaggatcac 480
   gagaagttcc gcgagtgggt ggacatcatc ctcagcaacg aagggctgga gtatcccaac 540
   ctcccggacg acttcaccga gacggtgggc cccgccatcg aggagtggtc cgaattcctg 600
   tacgcccaga tcgcccacaa gcgcgccgaa ccgaaggacg acctgatcag cggcctctgt 660
   gcggcggagg tcgacgggcg caagctgacc gacgaggaag tcgtcaacat cgtcgcgctg 720
   ctgctcaccg ccgggcacat ctccagcgcc acgctgctca gcaacctgtt cctggtgctg 780
   gaggagcacc cgcaggcaca ggccgcggtc cgcgccgacc gcagcctcgt gccgggcgtg 840
   atcgaggaga cgctgcgcta ccggtccccg ttcaactgca tcttccggat cctgaacgag 900
   gacaccgaca tcctcggcca ccccatgcgc aagggccaga tggtgatcgc ctggatcgcc 960
   tccgcgaacc gcgacaccga ggtgttcacg gacccggaca ccttcgacat ccgacgcgag 1020
   tcgaacaagc acctggcgtt cggccacggc atccaccact gcctgggcgc gttcctggcc 1080
   aggctggagg cgaaggtctt cctcaaccag acgctcgacc agttcaccga gttccggatc 1140
   gaccacgtcg gggtcgagtt ctacgacgcc gaccagctca ccgcgcgacg cctccccgtc 1200
   caggtggtac gcgacggacg gcacccgaag taa 1233
<210> 8
   <211> 402
   <212> PRT
   <213> micromonospora carbonacea subspecies aurantiaca
<400> 8
   Met Glu His Pro Val Thr Ala Gly Ser Cys Arg Phe Tyr Pro Phe Ser
   1 5 10 15
   Asp Arg Thr Asp Leu Asn Ile Asp Pro Thr Tyr Gly Glu Leu Arg Ser
   20 25 30
   Lys Glu Pro Val Ala Arg Val Arg Met Pro Tyr Gly Gly Asp Ala Trp
   35 40 45
   Leu Val Thr Arg His Ala Asp Ala Lys Lys Ala Leu Ser Asp Pro Arg
       50                  55                  60
   Leu Ser Ile Ala Ala Gly Ala Gly Arg Asp Val Pro Arg Ala Ser Pro
   65                  70                  75                  80
   Arg Leu Gln Glu Pro Asp Gly Leu Met Gly Leu Pro Pro Asp Ala His
                   85                  90                  95
   Ala Arg Leu Arg Arg Leu Val Ala Thr Ala Phe Thr Pro Lys Arg Val
               100                 105                 110
   Arg Asp Ile Ala Pro Arg Val Val Gln Leu Ala Asp Lys Leu Leu Asp
           115                 120                 125
   Asp Val Val Glu Thr Gly Pro Pro Ala Asp Leu Val Gln Gln Leu Ala
       130                 135                 140
   Leu Pro Leu Pro Val Met Ile Ile Cys Glu Met Met Gly Ile Gly Tyr
   145                 150                 155                 160
   Asp Glu Gln His Leu Phe Arg Ala Phe Ser Asp Ala Leu Met Ser Ser
                   165                 170                 175
   Thr Arg Tyr Thr Ala Asp Gln Val Asp Arg Ala Val Glu Asp Phe Val
               180                 185                 190
   Glu Tyr Leu Gly Gly Leu Leu Ala Gln Arg Arg Ala His Arg Thr Asp
           195                 200                 205
   Asp Leu Leu Gly Ala Leu Val Glu Ala Arg Asp Asp Gly Asp Arg Leu
       210                 215                 220
   Thr Glu Asp Glu Leu Val Met Leu Thr Gly Gly Leu Leu Val Gly Gly
   225                 230                 235                 240
   His Glu Thr Thr Ala Ser Gln Ile Ala Ser Gln Ile Phe Leu Leu Leu
                   245                 250                 255
   Arg Asp Arg Thr Arg Tyr Glu Gln Leu His Ala Arg Pro Glu Leu Ile
               260                 265                 270
   Pro Thr Ala Val Glu Glu Leu Leu Arg Val Ala Pro Leu Trp Ala Ser
           275                 280                 285
   Val Gly Pro Thr Arg Ile Ala Thr Glu Asp Leu Glu Leu Asn Gly Thr
       290                 295                 300
   Thr Ile Arg Ala Gly Asp Ala Val Val Phe Ser Leu Ala Ser Ala Asn
   305                 310                 315                 320
   Gln Asp Asp Asp Val Phe Ala Asn Ala Ala Asp Val Val Leu Asp Arg
                   325                 330                 335
   Asp Pro Asn Pro His Ile Ala Phe Gly His Gly Pro His Tyr Cys Ile
               340                 345                 350
   Gly Ala Ser Leu Ala Arg Leu Glu Ile Gln Ala Ala Ile Gly Ala Leu
   355 360 365
   Ala Arg Arg Leu Pro Gly Leu Arg Leu Ala Val Glu Glu Asn Glu Leu
   370 375 380
   Asp Trp Asn Lys Gly Met Met Val Arg Ser Leu Val Ser Leu Pro Val
   385 390 395 400
   Thr Trp
<210> 9
   <211> 1209
   <212> DNA
   <213> micromonospora carbonacea subspecies aurantiaca
<400> 9
   atggagcatc cagtaacggc cgggtcctgc aggttctacc ccttcagtga ccgtaccgac 60
   ctgaatatcg atcccacgta cggcgaactg cgctcgaaag agccggtcgc ccgcgtccgc 120
   atgccctacg gcggggacgc ctggctggtc acccggcacg ccgacgccaa gaaggccctc 180
   tctgaccccc gactcagcat tgcagccgga gccgggcggg acgtgccgcg cgcctccccc 240
   cgtctccagg aacccgacgg tctgatgggt cttccccccg acgcgcacgc ccgactgcgc 300
   aggctcgtcg ccacggcgtt cacgccgaag cgcgtacggg acatcgcccc gcgcgtcgtc 360
   cagctcgccg acaagcttct cgacgacgtg gtcgaaaccg ggccgccggc cgacctcgtg 420
   cagcagctcg cgcttcccct gccggtgatg atcatctgcg agatgatggg catcgggtac 480
   gacgagcagc acctgttccg tgccttcagc gatgccctga tgtcctccac ccgatacacg 540
   gccgaccagg tcgaccgcgc ggtagaggac ttcgtcgagt acctcggcgg cctcctcgcg 600
   cagcgccgtg cacaccgcac cgacgacctc ctcggcgccc tggtcgaggc gcgagacgac 660
   ggcgatcggc tgaccgagga cgaactcgtc atgctcaccg gcggcctgct cgtcggcggc 720
   cacgagacga ccgccagcca gatcgcctcg cagatcttcc tcctgctgcg cgaccggacc 780
   aggtacgagc aactccatgc ccgtccggag ttgatcccca cggcagtcga ggaactgctg 840
   cgggtggccc cgctctgggc ctcggtcggc cccacccgca tcgccaccga ggacctggaa 900
   ctcaacggga cgaccatccg ggccggcgac gccgtcgtct tctcgctggc gtccgccaat 960
   caggacgacg acgtcttcgc gaatgccgca gacgtcgtgc tcgaccgcga cccgaatccg 1020
   cacatcgcct tcgggcacgg gccccattac tgcatcgggg cgtcactggc cagactggaa 1080
   atacaggccg ccatcggcgc cttggccagg cggcttcccg gtctccgcct ggccgtcgag 1140
   gaaaacgaac ttgattggaa caagggaatg atggtacgca gcctcgtgtc ccttccggtg 1200
   acgtggtga 1209
<210> 10
   <211> 4471
   <212> PRT
   <213> micromonospora carbonacea subspecies aurantiaca
<400> 10
<210> 11
   <211> 13416
   <212> DNA
   <213> micromonospora carbonacea subspecies aurantiaca
<400> 11
   atgcgagttg tgggcgcaga cgcgtgcagc gcagccgtcc ccgccggacc gcggatgggc     60
   ttcccagcat cgttcttcga cccaggagac ctcatgaccg tgcagagtga cgtgttgcgc    120
   caccgcgata tcgccgtcat cgggatgtcc tgccggcttc ccggcgcgcc gagcatcgag    180
   gaattctggg acctgctgtg cagcgggcgg agcgcggtcg accgccagcc cgacggcggt    240
   tggcgggcgg tgatcgatgg gaagggagaa tccgacgccg cgttcttcgg catgtccccg    300
   cgccaggccg ccgcggtcga cccgcaacag cgcctgatgc tcgaactcgg ctgggaggca    360
   ctggagaacg cccgcatccg gcccgccgac ctgaagggct ccgacactgg cgtcttcgtg    420
   gggctcaccg ccgacgacta cgccaccttg ctgcgccgct ccggcacgcc catcagcggg    480
   cacaccgcga caggcctgaa ccgtagcctc acggccaacc gtctctcgta cctgctgggt    540
   ctgcgcggcc ccagcttcac cgtggactcc gcgcagtcgt catccctggt cgccgttcac    600
   ctggcgtgcg aaagcctgct gcggggcgag agcgcggtcg ccgtcgtcgg cggggtgagc    660
   ctcatcctgg cagaggagag caccgccgcc atggcgcgta tgggggcact ctctcctgac    720
   gggcgttgct tcaccttcga cgcccgggcc aacggctacg tccgtggcga gggtggcgtg    780
   gccatggtcc tcaagccgct gatccgcgcg atcgaggacg gcgaccaggt gcactgcgtc    840
   atccggggct gtgccgtcaa caacgacggc ggtggcccca gcctcaccca tcccgaccgg    900
   gaggcccagg aggcattgct gcgccgggcg tacgagcggg cgggggtggc ccccgaacac    960
   gtcgactacg tcgagctgca cggcaccggg acgaaggccg gcgaccccgt cgaggcggcg   1020
   gccctcgggg cggtgctggg tgtcgcccgc ggctgcgaca acccactcgc ggtcggatcg   1080
   gtcaagacca acgtcggcca cctggagggg gcggccggca tcacgggcct gctgaaggcg   1140
   gtgctgtgcg tacgtgaggg ggtgctgccg ccgagcctca acttccgtac gccgaacccg   1200
   gacatccgcc tcgacgagct gaacctccgg gttcagacgg aactgcagcc gtggccgggc   1260
   gacgggacgg gccgcccgcg tgtcgccgga gtgagttcct tcggcatggg cggtacgaat   1320
   gcgcatctga ttctcgagca ggctccggtg gcggctgagg aaacggctgt taccgatgcc   1380
   ggtgtcggtt cggttcgggt ggttccggtg gtggtgtcgg gtcgttcggt gggggctttg   1440
   cgggcgtatg cgggtcggtt gcgtgaggtg tgcgcggggt tgtctgacgg tggtggctcc   1500
   ggtggtggtt ctggtctggt ggatgtgggt tggtcgttgg tgtcgtcgcg gtcggtgttc   1560
   gagcatcggg cggtcgtgtt cggtgggggt gtcgccgagg tggtggcggg tttggatgcg   1620
   gtggcttctg gggcggtgag ttcgggttcg gtggtggtgg gttcggtggc gtcgggtgtt   1680
   gctggtggtg gtggtcgggt ggtgtttgtg tttccgggtc agggttggca gtgggtgggt   1740
   atgggtgcgg ctctgttgga cgagtcggag gtgtttgctg agtcgatggt ggagtgtggg   1800
   cgggcgttgt cggggtttgt ggattgggat ttgttggaag tggtccgcgg tggtgggggt   1860
   gacggatcgt ttggtcgggt tgatgtggtg cagccggtgt cgtgggcggt gatggtgtcg   1920
   ttggcgcggt tgtggatgtc ggtgggtgtg gtgccggatg cggtggtggg tcattcgcag   1980
   ggtgaggttg ctgcgccggt ggtggggggt gtgttgagtg tggctgatgg ggcgcgggtg   2040
   gtggcgttgc ggtcgcgggt gatcggtgag gtgttggcgg gtggtggtgc gatggtgtcg   2100
   gtggggttgc cggtggcggt tgtgttggat cggttggcgg ggtggggtgg tcggttgggt 2160
   gtggcggcgg tgaatggtcc gtcgttgacg gtggtgtcgg gggatgtgga tgctgctgtg 2220
   gggtttgttg gtgagtgtga gcgggatggg gtgtgggtgc ggcgggtggc ggtggattat 2280
   gcgtcgcatt cggcgcatgt ggaggcggtg gaggggatgc tgtcggggtt gttgggtggt 2340
   ttgtgtccgg ggcggggtgt ggtgccgttt tattcgtcgg tggtgggtgg tgtggttgat 2400
   ggggtgggtt tggatggtgg gtattggtat cggaatctgc gtgagcgggt gttgttttcg 2460
   gatgtggtgg ggcggcttgt tggggatggg ttttcggggt ttgtggagtg ttcggggcat 2520
   ccggtgttgg cgggtggggt gttggagtcg gtggcggtgg tggatccgga tgtgcggccg 2580
   gtggtggtgg ggtcgctgcg ccgtgatgat ggtgggtggg gccggttttt gacgtcggtg 2640
   ggtgaggcgt tcgtcggcgg gatgagtgtt gactggaagg gtgtgttcgc gggggcgggc 2700
   gcgcggttgg ttgacctgcc gacgtatccg ttccaacgac gccactactg ggcaccgaac 2760
   accgacggcg cgccagctcc gatcctcgat gatcacgcgg aggcggagaa cgaaccagcc 2820
   gaatccgagc cagggattcg ggccgagctt ctgacgttgg ccgagcccga gcaactgaac 2880
   cgactcttgg cgaccgttcg cgccagcacc gccgtcgttc tgggcctcga ctcggcgcag 2940
   gcggtcgatc cggagcgcac gttcaaggag catggattcg aatcggtcac cgccgtcgag 3000
   ctctgtaacc acctgcaacg cggcactggg ctgcgggttc ccgcctcgct tgtatacaac 3060
   catcccaccc cgatggccgc tgcccggaag ctgcaggaag aaattcaggg ccggcaaccg 3120
   gagaacgtcc ggcaggtcac ctccgctgct gctgtggatg atccggtggt ggtggtgggg 3180
   atgggttgtc gttttccggg tggggtggtg tgtgcggagg gtttgtggga tttggtgttg 3240
   gggggtgggg atgcggtgtc ggggtttccg gtggatcggg gttgggatgt ggaggggttg 3300
   tttgatccgg tgcggggtgt ggtggggaag tcgtatgtgc gggagggggg gtttgtgtat 3360
   gacgcgggga tgttcgatgc ggagtttttt ggtgtgtcgc cgcgtgaggc ggtggcgatg 3420
   gatccgcagc agcgtttgtt tttggaggtg tcgtgggagg cgttggagcg tgcggggatt 3480
   gatccgttgg gtttgcgggg ttcgcggacg ggtgtgtatg tgggggtgat gggtcaggag 3540
   tatgggccgc ggttggtgga gtcgggtggt gggtttgagg gttatttgtt gacggggacg 3600
   tcgccgagtg tggtgtcggg tcgtgtttcg tatgtgttgg ggttggaggg tccgtcgatt 3660
   tcggttgata cggcgtgttc gtcgtcgttg gtggcgttgc atttggcgtg tcaggggttg 3720
   cggttgggtg agtgtgatgt ggcgttggcg ggtggggtga cggtgattgc ggcgccgggg 3780
   ttgtttgtgg agttttctcg gcagggtggg ttgtcgggtg atgggcggtg tcgggcgttt 3840
   gcgggtggtg cggatgggac ggggtggggg gagggtgcgg gggtggtggt gttggagcgg 3900
   ttgtcggtgg cgcgggagcg tggtcatcgg gtgttggcgg tggtgcgggg ttctgcggtg 3960
   aatcaggatg gtgggtcgaa tggtttgacg gcgccgtcgg gggtggcgca gcgtcgggtg 4020
   attggtgcgg cgttggtggc ggcgggtttg ggtgtgtcgg atgtggatgt ggtggaggcg 4080
   catgggacgg ggactcggtt gggtgatccg attgaggctg aggcgttgtt ggggtcgtat 4140
   gggcggggtc gtgtgggtgg ggcgttgttg ttgggttcgg tgaagtcgaa tattggtcat 4200
   acgcaggcgg ctgcgggtgt ggcgggtgtg atcaagatgg tgatggcgtt gcgggcgggg 4260
   gtggtgccgg cgacgttgca tgtggatgtg ccgtcgccgt tggtggattg gtcttcgggt 4320
   ggggtggagt tggtgacgga ggcgcgggat tggccggtgg tgggtcgtgt gcgtcgtgcg 4380
   ggtgtgtcgg cgtttggggt gtcggggacg aatgcgcatc tgattttgga gcaggccccc 4440
   gaattcgacg atccggttgt taccgacacc gacaccgatg ctggtgtggg taggggtcta 4500
   tcggtggttc cggtggtggt ttcgggtcgt tcgacggcgg ctttgcgcgc ttatgcgggc 4560
   cggttgcgtg aggtgtgcgc gggtctttcc gatggtgccg gtctggtgaa tgtgggttgg 4620
   tcgttggtgt cgtcgcggtc ggtgttcgag catcgggcgg tcgtgtttgg tgggggtgtc 4680
   gccgaggtgg tggcgggttt ggatgcggtg gtttccgggg cggtggcttc gggttcggtg 4740
   gtggtgggtt cggtggcgtc gggtgttgct ggtggtggtg gtcgggtggt gtttgtgttt 4800
   ccgggtcagg gttggcagtg ggtgggtatg ggtgcggcgc tgctggacga gtcggaggtg 4860
   tttgctgagt cgatggtgga gtgtggtcgg gcgttgtcgg ggtttgtgga ttgggatttg 4920
   ttggaggtgg tgcggggtgg ggcgggtgag gggtgtgggg gtcgggttga tgtggtgcag 4980
   ccggtgtcgt gggcggtgat ggtgtcgttg gcgcggttgt ggatgtcggt gggtgtggtg 5040
   ccggatgcgg tggtgggtca ttcgcagggt gaggttgctg cggcggtggt ggggggtgtg 5100
   ttgagtgtgg ctgatggggc gcgggtggtg gcgttgcggt cgcgggtaat tggtgaggtg 5160
   ttggccggtg gtggtgcgat ggtgtcggtc ggactgccga tcgtggatgc gcaggaacgg 5220
   ttggcggggt ggggtggtcg gttgggtgtg gcggcggtga atggtccgtc gttgacggtg 5280
   gtgtcggggg atgtggatgc tgctgtgggg tttgttggtg agtgtgagcg ggatggggtg 5340
   tgggtgcggc gggtggcggt ggattatgcg tcgcattcgg cgcatgtgga ggcggtggag 5400
   gggatgctgt cggggttgtt gggtggtttg tgtccggggc ggggtgtggt gccgttttat 5460
   tcgtcggtgg tgggtggtgt ggttgatggg gtgggtttgg atggtgggta ttggtatcgg 5520
   aatctgcgtg agcgggtgtt gttttcggat gtggtggggc ggcttgttgg ggatgggttt 5580
   tcggggtttg tggagtgttc ggggcatccg gtgttggcgg gtggggtgtt ggagtcggtg 5640
   gcggtggtgg atccggatgt gcggccggtg gtggtggggt cgctgcgccg tgatgatggt 5700
   gggtggggcc ggtttttgac gtcggtgggt gaggcgttcg tcggcgggat gagtgttgac 5760
   tggaagggtg tgttcgcggg ggcgggcgcg cggttggttg acctgccgac gtatccgttc 5820
   caacgccgcc actactgggc accgactccc accaaccccg ccaccaaccc cgccacgggc 5880
   gacaccacca ccgccgaccc ggtgggtggc gtgcggtatc ggatcacctg gaaaccgttg 5940
   ccgacggacg acccccgacc cctcaccaac cgctggctac tcatcgccga cccggggacc 6000
   gccggctcgg agcttgccgc agacatcaca gcagcgctca ttcgcagggg cgccgaggtc 6060
   gagttgctgg ccgtggaccc gctcgcgggc cgggcccgga tcgccgaact gctcgccacc 6120
   acgacggctg ggccggtgcc gctgtcgggc gccgtgtctc ttctcgggct tgtgcaggac 6180
   gcgcatcctc aacacccctc catcggaatg ggcgtggtct cgtcgctggc gctggtgcag 6240
   gccatcggtg acgcgggagc cgagactcct ttgtggagcg tcacgcaggg ggcggtcgct 6300
   gtggtgcccc aggaggcgcc ggatgtgttc ggtgcgcagg tgtgggcgtt cgggcgggtg 6360
   gccgccctgg aactgccgga ccgctggggc ggcctggtcg accttccgtc cgtaccgaat 6420
   gcccggatgc tggaccagct cgccaacgcc ctcgccggag cggacggcga ggaccagatc 6480
   gcggtacgcg gctcggggat ctacgggcgt cgggtgacgc gcgcggcggg cactgcgcgc 6540
   cgggaatggc gccctcgcgg gaacatcctg gtgaccggag gtacgggaag tctgggtggc 6600
   cgggtggccc ggtggctcgc tcgcaacggt gccgaacacc tcgttctcac cagtcgtcgg 6660
   ggtgccgacg ccccgggggc ggcagaactg gaagctgatc ttcgcgcgct cggtgtcgag 6720
   gtgaccatgg ccgcctgcga tgtagcggac cgggctgcgc tgtccgacgt cctggcggcg 6780
   catccgccca ctgcggtctt ccacaccgcc ggagtcctgc acgacggtgt gatcgacacg 6840
   ctcgccgccg gacacatcga cgaggtcttc cgtccgaaga ccgctgccgc gctgctgctc 6900
   gacgaactca cccagcacca ggagctggac gccttcgtcc tcttctcatc ggttaccgga 6960
   gtctggggca acggcggcca ggcggcgtac gcggcggcga acgcatcgct ggacgccctg 7020
   gcggagcgac gtcgtgccgc aggtcttccc gccacctcca tagcttgggg actgtggggc 7080
   ggcggtggca tggcggaggg gatcggcgag cagaacctga accgccgtgg catcacggcc 7140
   ttggacccgg agctcggcat cgccgctctg cagcaggccc tcgaccgcga tgacgtgtct 7200
   gtcaccgtcg ccgacgtcga ctggacggtt ttcgctccgc gtcttgccga cctgcgctcg 7260
   gggcggctct tcgacggggt gcccgaggcc aggagcgcgc tcgatgcccg gaaagtggac 7320
   accgagtcgc cgagcgccgg ccttgcgcag cgggtggcgg ggatgcccga cgcggaacgg 7380
   cagcgggtcc tcctcgaaac ggtgcgggcg gcggccgcgg cggtcctgag gcacgagacg 7440
   gtggatgcgg tcgcgcccac ccgggccttc aaggacgccg gcttcgactc gctcacggcg 7500
   ctcgaactgc gcaaccacct caacagcacg accggtctga gtctgcctcc gacggtggtc 7560
   ttcgaccacc ccaccccgtc cacgttggcg aagttcctgg agggcgtcct cgtcggcgct 7620
   tctgccgagg aagtcccggt gactgccgca gccgtgcccg tcgacgagcc tattgccatc 7680
   gtcggcatgg cctgccgcta ccccggcgga gccgacactc ccgagaagct ctgggacctc 7740
   ctgctggccg gtgctgacgt catcggccca gcccccgacg accggggctg ggacgtggac 7800
   tccttctttg atcccgtgcc gggcgccgcg gggaagtcgt atgcgcggga gggggggttt 7860
   gtgtatgacg cggggatgtt cgatgcggag ttctttggtg tgtcgccgcg tgaggcggtg 7920
   gcgatggatc cgcagcagcg cttgttgttg gagacgtcgt gggaggcgtt ggagcgtgcg 7980
   ggaatcgatc cggcgggtct gcggggtagc cggaccggcg tgtactccgg cctgacccac 8040
   caggagtatg ccgcccgtct gcacgagcct ccgcaggaac tcgagggcta tctgctcacc 8100
   ggcaagtcgg tgagcgtcgc gtcgggtcgt gtttcgtatg tgttggggtt ggagggtccg 8160
   tcgatttcgg ttgatacggc gtgttcgtcg tcgttggtgg cgttgcattt ggcgtgtcag 8220
   gggttgcggt tgggtgagtg tgatgtggcg ttggcgggtg gggtgacggt gattgcggcg 8280
   ccggggttgt ttgtggagtt ttctcggcag ggtgggttgt cgggtgatgg gcggtgtcgg 8340
   gcgtttgcgg gtggtgcgga tgggacgggg tggggggagg gtgcgggggt ggtggtgttg 8400
   gagcggttgt cggtggcgcg ggagcgtggt catcgggtgt tggcggtggt gcggggttct 8460
   gcggtgaatc aggatggtgg gtcgaatggt ttgacggcgc cgtcgggggt ggcgcagcgt 8520
   cgggtgattg gtgcggcgtt ggtggcggcg ggtttgggtg tgtcggatgt ggatgtggtg 8580
   gaggcgcatg ggacggggac tcggttgggt gatccgattg aggctgaggc gttgttgggg 8640
   tcgtatgggc ggggtcgtgt gggtggggcg ttgttgttgg gttcggtgaa gtcgaatatt 8700
   ggtcatacgc aggcggctgc gggtgtggcg ggtgtgatca agatggtgat ggcgttgcgg 8760
   gcgggggtgg tgccggcgac gttgcatgtg gatgtgccgt cgccgttggt ggattggtct 8820
   tcgggtgggg tggagttggt gacggaggcg cgggattggc cggtggtggg tcgtgtgcgt 8880
   cgtgcgggtg tgtcggcgtt tggggtgtcg gggacgaatg cgcatctgat tttggagcag 8940
   gcccccgagt tcgacgatcc tgccgattcc gattccgatt ccgattccga ttccgatgcc 9000
   ggtgtcgtgg atggcggcga gggtggtgtt ggcaggagct tgtcggtggt tccggtggtg 9060
   gtgtcgggtc gttcggtggg ggctttgcgg gcgtatgcgg gtcggttgcg tgaggtgtgc 9120
   gcggggttgt ctgacggtgg tggctccggt ggtggttctg gtttggtgga tgtgggttgg 9180
   tcgttggtgt cgtcgcggtc ggtgtttgag catcgggcgg tcgtgttcgg tgggggtgtg 9240
   gaggaggttg ttgctggtct tggtgcggtg gcttctgggg cggtggcttc gggttcggtg 9300
   gtggtgggtt cggtggcgtc gggtgttgct ggtggtggtg gtcgggtggt gtttgtgttt 9360
   ccgggtcagg gttggcagtg ggtgggtatg ggtgcggcgc tgctggacga gtcggaggtg 9420
   ttcgccgagt cgatggtgga gtgtggtcgg gcgttgtcgg ggtttgtgga ttgggatttg 9480
   ttggaggtgg tgcgcggcgg ggcgggtgag gggtgtgggg gtcgggttga tgtggtgcag 9540
   ccggtgtcgt gggcggtgat ggtgtcgttg gcgcggttgt ggatgtcggt gggtgtggtg 9600
   ccggatgcgg tggtgggtca ttcgcagggt gaggttgctg cggcggtggt ggggggtgtg 9660
   ttgagtgtgg ctgatggggc gcgggtggtg gcgttgcggt cgcgggtgat cggtgaggtg 9720
   ttggccggtg gtggtgcgat ggtgtcggtc ggactgccga tcgtggatgt gcaggaacgg 9780
   ttggcggggt ggggtggtcg gttgggtgtg gcggcggtga atggtccgtc gttgacggtg 9840
   gtgtcggggg atgtggatgc tgctgtgggg tttgttggtg agtgtgagcg ggatggggtg 9900
   tgggtgcggc gggtggcggt ggattatgcg tcgcattcgg cgcatgtgga ggcggtggag 9960
   gggatgctgt cggggttgtt gggtggtttg tgtccggggc ggggtgtggt gccgttttat 10020
   tcgtcggtgg tgggtggtgt ggttgatggg gtgggtttgg atggtgggta ttggtatcgg 10080
   aatctgcgtg agcgggtgtt gttttcggat gtggtggggc ggcttgttgg ggatgggttt 10140
   tcggggtttg tggagtgttc ggggcatccg gtgttggcgg gtggggtgtt ggagtcggtg 10200
   gcggtggtgg atccggatgt gcggccggtg gtggtggggt cgctgcgccg tgatgatggt 10260
   gggtggggcc ggtttctgac gtcggtgggt gaggcgttcg tcggcgggat gagtgttgac 10320
   tggaagggtg tgttcgcggg ggcgggcgcg cggttggttg acctgccgac gtatccgttc 10380
   caacgacgcc actactgggc ccagacctcg cccgctggcg tcgggacggc cgcggcggcc 10440
   cggttcggca tggagtggga ggaccatccc ctgctcggcg gtgcgctgtc ggtcgggggc 10500
   tccaggagcc tgcttctggc cgggcatctg tcgctcgcct cgcacgcctg gctgaccgac 10560
   catgccgtct ccggcaccgt gctgctgccc ggtacggcct tcgtggaact cgccctgcac 10620
   gccgccgctg cggctggctg tccggaggtc gaggagctgc ggctggaggc tcccctggtg 10680
   gtgccggcca ggggcggggt gcggctccag gtgctcgtgg acgaccccga cgacggatcc 10740
   gaccgccgcg cggtaagcgt gttctcccgg gacgatgcgg cgccggccga gtccgcctgg 10800
   acgcggcacg cggtgggcgt cctggccgcg cggtcgcggc ctgcaccggc tgcgccctgg 10860
   cacaccgacg cctggccacc ttcgggcacg gagccggtcg acgtggccga cctgtatgag 10920
   cggttcgcgg cgctgggcta cgagtacggg gaggcgttcg ccgggctcca gggggtctgg 10980
   cggggggacg gcgaggtgtt cgccgaggtg cggctgcccg accgggtcag cgcggaggcc 11040
   attcgcttcg ggctgcatcc cgcgctgctc gacgccgccc tgcaggggtg gttggcgggc 11100
   gacctcgtcg gcgtccccga gggcagtgtg ctgctgccct tcgcctggca gggcgtcgtg 11160
   ctccacgcca ccggcgccga cactctgcgg gttcgcatcg gccggtccgg tgactcggcc 11220
   gtctgcctgc acgcggtgga cccggccggt gctccggtcc tctcgttgga cgccctggcc 11280
   ctgcgtccgc tcgtccggga acgcctcggg ctgcccgccg atgccggagc cggggcgttg 11340
   taccgggtcg gctggcggcg gcaggccgcc gttgccgggg cagccgaccg gcggtgggcg 11400
   gtcgtggccc cgaacggtgc cgaggcggac ggggccgccg agccgcaccg gtggccggtc 11460
   gccgccgtcg acgtgcacac cgacgtggac tcgctgcggg cggccctgga cgcgggcgcg 11520
   gaactgcccg ccgtcgtcct cgccgacttc cggagggccg ccggctggag cgtcgacagt 11580
   tcgctggccg ccggcccgtc gcccaacgac ggcgcggtgg gcgacggcgc ggtgggcgac 11640
   gcccgggccg gggccgtccg ggcggcgacc cgggccgggc tggatctgct gcaacgctgg 11700
   ctggccgacg agcggttcat cgcggccagg ctcgtggtgg tcaccgaacg ggccgtggcc 11760
   gccgggccgg acgaggacgt gccgggcctc gtccacgcgg gactgtgggg cctgctccgg 11820
   tcggcccaat cggagcaccc ggaccgcttc gtgctggtgg acgtcgacgc ggacgacagc 11880
   tcgctcgcgg cgctgccgtc ggccctcgcc atggacgcgc cccaactggt ggtgcgggcc 11940
   ggtcagatcc tgctgcccga gatcgagccg gtgcggcccg tacccgagcc ggagcaggcg 12000
   gaacccgaac cgggggccgt cctggacccc gacggcacgg tcctgctcac cggcgcgacc 12060
   ggcacgctcg gcgggctgct cgcccggcac ctggtgacca cccgtggtgc gcgccggctg 12120
   ctgctggtca gccgcagcgg tccggacgcc cccgatgccg gccggctgac cgaggagctg 12180
   accgggctcg gcgcccacgt gacgctggcc gcctgcgaca ccacggatcg cgccgcgctg 12240
   gccggcgtcc tgggcggcat ccccgccgag catccgctga ccgccgtggt gcacgtggcc 12300
   ggcgtactcg acgacggggc ggtgcaggcg ctcacccccg agcgggtcga cgcggtgctc 12360
   cggccgaagg tggacgcggc actgcacctg cacgaactga ccgcggggct gccgctggcc  12420
   gcgttcgtgc tgttctccgg ggcggcgggg atcctgggcc ggcccggcca ggccaactac  12480
   gcggcggcga acaccttcct ggacgccctg gcgcagcacc gacgggcccg gggcctgccc  12540
   ggcgtctccc tcgcctgggg cctgtggggg ctggccagcg acatgacggg ccacctgggc  12600
   gagcaggacc tgcggcggat gcggcgctcc ggcatcgccc cgatgaccgg cgaggagggc  12660
   ctcgcgctgt tcgacctggc cctcgacctg gcccgggacg aaccggtgct cgtaccggcc  12720
   cgactggacc cggcggcgct gcgccgggag tgggccgcca acggaccggg cgccgtcccg  12780
   gtcctgctgc ggggtctggt gccggcggct ccgctccgtc gcgcggcccc gtcgggcgcc  12840
   gccggcggtg cgcccgtgcc cgccgtcgcc gcgccgcagc aggcggacga gctgcgcggg  12900
   caactggccg ggaaggacgc gcaggcccag gtccggcagc tgctggatct ggtacgcgcc  12960
   catgtcgccg gggtgctcgc cctccgggaa gcggcggacg tggacccggg cagaccgttc  13020
   cgcgaggtcg gattcgactc gttgaccgca gtcgaactgc gcaaccggct gggctcggcg  13080
   accggcctgc ggttggcacc gagcctggtg ttcgaccatc cgaccccgtc ggccgtggcc  13140
   gagcacctcg tggaccgcct cgccgccgag ggggcggctg acgagggcgc ggcggcactg  13200
   accgggctcg acgcagtggc cgcggcgctc ggcgggatgc ggacggacga cgttcgccgg  13260
   gacatcgtcc gcaggcggct ggaggagatg ctcgccctgg tcggcgggcc acggtccggg  13320
   ccggcaggtg acgggctggt ggatgccacg gtcgccgagc gactggactc ggcttccgac  13380
   gacgaactct tcgccctgat cgaggagcag ctgtga                            13416
<210> 12
   <211> 1925
   <212> PRT
   <213> micromonospora carbonacea subspecies aurantiaca
<400> 12
   Val Thr Ala Asn Glu Asp Arg Met Arg Glu Tyr Leu Lys Arg Val Thr
   1               5                   10                  15
   Ala Glu Leu Ala Gly Thr Arg Arg Arg Leu Arg Glu Leu Glu Asp Ser
               20                  25                  30
   Ala Arg Glu Pro Ile Ala Ile Val Gly Met Ser Cys Arg Leu Pro Gly
           35                  40                  45
   Gly Val Ser Thr Pro Glu Asp Leu Trp Arg Leu Val Glu Ala Gly Thr
       50                  55                  60
   Asp Ala Ile Ser Gly Phe Pro Asp Asp Arg Gly Trp Asp Val Gly Arg
   65 70 75 80
   Leu Tyr Asp Pro Asp Pro Asp Ser Thr Gly Thr Ser Tyr Val Arg Glu
   85 90 95
   Gly Gly Phe Leu Tyr Asp Cys Ala Glu Phe Asp Pro Glu Phe Phe Thr
   100 105 110
   Val Ser Pro Arg Glu Ala Leu Ala Met Asp Pro Gln Gln Arg Leu Leu
   115 120 125
   Leu Glu Ala Ala Trp Glu Thr Phe Glu Arg Ala Gly Ile Ala Pro Asp
   130 135 140
   Ser Ala Arg Gly Thr Arg Thr Gly Val Tyr Val Gly Val Met Tyr Asp
   145 150 155 160
   Asp Tyr Gly Ser Arg Leu Ser Glu Val Pro Lys Asp Leu Glu Gly Tyr
   165 170 175
   Leu Val Asn Gly Ser Ala Gly Ser Val Ala Ser Gly Arg Ile Ala Tyr
   180 185 190
   Thr Leu Gly Leu Gln Gly Pro Ala Val Thr Val Asp Thr Ala Cys Ser
   195 200 205
   Ser Ser Leu Val Ala Leu His Leu Ala Val Gln Ala Leu Arg Ser Gly
   210 215 220
   Glu Cys Glu Leu Ala Leu Ala Gly Gly Ala Thr Val Leu Ala Thr Pro
   225 230 235 240
   Thr Met Phe Val Asp Phe Ala Arg Gln Arg Gly Leu Ala Glu Asp Gly
   245 250 255
   Arg Cys Lys Ala Phe Ala Asp Ala Ala Asp Gly Thr Gly Phe Gly Glu
   260 265 270
   Gly Val Gly Met Leu Leu Val Glu Arg Leu Ser Asp Ala Val Arg Asn
   275 280 285
   Arg Arg Gln Val Leu Ala Val Val Arg Gly Ser Ala Val Asn Gln Asp
   290 295 300
   Gly Ala Ser Asn Gly Leu Thr Ala Pro Asn Gly Thr Ala Gln Gln Leu
   305 310 315 320
   Val Ile Arg Gln Ala Leu Thr Asn Ala Gly Leu Ala Ala Asp Glu Val
   325 330 335
   Asp Ala Val Glu Ala His Gly Thr Gly Thr Arg Leu Gly Asp Pro Ile
   340 345 350
   Glu Ala Gln Ala Leu Leu Ala Thr Tyr Gly Gln Gly Arg Pro Ala Asp
   355 360 365
   Arg Pro Leu Leu Leu Gly Ser Leu Lys Ser Asn Ile Gly His Thr Gln
   370 375 380
   Ala Ala Ala Gly Val Ala Gly Val Ile Lys Thr Val Leu Ala Leu Arg
   385 390 395 400
   His Ala Arg Leu Pro Arg Thr Leu His Val Asp Arg Pro Ser Thr Arg
   405 410 415
   Val Asp Trp Ser Ser Gly Ala Val Arg Leu Leu Thr Glu Gly Arg Pro
   420 425 430
   Trp Pro Asp His Gly Asp Arg Pro Arg Arg Ala Gly Val Ser Ser Phe
   435 440 445
   Gly Ala Ser Gly Thr Asn Ala His Val Ile Leu Glu Ser Ala Pro Gly
   450 455 460
   Ala Ala Ala Gly Ala Thr Gly Ala Thr Asp Leu Ser Ala Pro Pro Ala
   465 470 475 480
   Ser Val Ala His His Pro Ala Thr Ala Thr Ala Thr Ala Pro Ala Ala
   485 490 495
   Thr Val Pro Thr Ala His Glu Pro Ala Gly Thr Ala Gly Asp Asp Pro
   500 505 510
   Val Trp Val Leu Ser Gly Arg Thr Glu Ala Ala Leu Arg Glu Gln Ala
   515 520 525
   Arg Arg Leu His Ala His Leu Thr Ser Arg Ala Arg Pro Glu Pro Ala
   530 535 540
   Asp Ala Val Ala Arg Ala Leu Ala Arg Ser Arg Thr Ala Phe Ala Tyr
   545 550 555 560
   Arg Ala Ala Val Leu Gly Arg Asp Asp Thr Ala Arg Leu Asp Gly Leu
   565 570 575
   His Ala Leu Ala Ala Gly Arg Ser Ala Ala Gly Leu Val Thr Gly Arg
   580 585 590
   Ala Val Pro Glu Arg Arg Val Ala Phe Leu Phe Thr Gly Gln Gly Ser
   595 600 605
   Gln Arg Pro Gly Ala Gly Arg Glu Leu Tyr Ala Arg His Pro Ala Phe
   610 615 620
   Ala Gln Ala Leu Asp Gly Val Leu Ala Glu Leu Asp Arg His Leu Asp
   625 630 635 640
   Arg Pro Leu Arg Ala Val Met Leu Ala Glu Pro Gly Thr Glu Ala Ala
   645 650 655
   Ala Leu Leu Asp Asp Thr Ala Tyr Thr Gln Pro Ala Leu Phe Ala Leu
   660 665 670
   Glu Val Ala Leu Phe Arg Leu Val Thr Ser Trp Gly Leu Arg Pro Asp
   675 680 685
   Ala Leu Leu Gly His Ser Val Gly Glu Ile Thr Ala Ala Tyr Val Ala
   690 695 700
   Gly Val Leu Thr Leu Pro Asp Ala Ala Arg Leu Val Ala Val Arg Gly
   705 710 715 720
   Arg Leu Met Ala Asp Leu Arg Ala Gly Gly Ala Met Ala Ala Leu Gln
   725 730 735
   Ala Ala Glu Ser Glu Val Asp Pro Leu Leu Ala Gly Arg Glu Gly Glu
   740 745 750
   Leu Ser Ile Ala Ala Val Asn Gly Pro Gln Ala Thr Val Ile Ala Gly
   755 760 765
   Asp Glu Ala Ala Val Glu Glu Gln Val Ala Leu Trp Arg Asp Arg Gly
   770 775 780
   Arg Arg Ala Arg Arg Leu Arg Val Gly His Ala Phe His Ser Val Arg
   785 790 795 800
   Met Asp Gly Met Leu Ala Glu Phe Glu Lys Ala Met Gly Asp Leu Arg
   805 810 815
   Ala Gly Glu Pro Thr Ile Pro Val Val Ala Asn Val Arg Gly Ala Ile
   820 825 830
   Ala Ser Gly Thr Asp Leu Arg Thr Ala Gly Tyr Trp Ile Arg His Ala
   835 840 845
   Arg Glu Pro Val Arg Phe Leu Asp Gly Met Arg Ala Leu Arg Ala Glu
   850 855 860
   Gly Val Asp Thr Phe Val Glu Leu Gly Pro Asp Gly Val Leu Thr Ala
   865 870 875 880
   Met Ala Arg Asp Cys Leu Ala Asp Pro Ala Asp Pro Val Asp Leu Ala
   885 890 895
   Asp Ala Ala Glu Pro Ala Gly Ala Ala Glu Pro Asp Arg Ser Leu Leu
   900 905 910
   Phe Leu Pro Thr Leu Arg Arg Asp Arg Asp Asp Ala Val Ala Val Arg
   915 920 925
   Glu Ala Leu Ala Ser Val His Val His Gly Leu Pro Val Asp Pro Val
   930 935 940
   Ala Pro Leu Gly Asp Gly Pro Leu Ala Thr Asp Leu Pro Thr Tyr Pro
   945 950 955 960
   Phe Gln Arg Ser Arg Tyr Trp Leu Asp Pro Arg Pro Gly Ala Arg Asp
   965 970 975
   Leu Thr Ala Val Gly Leu Asp Val Ala Gly His Pro Leu Leu Ala Val
   980 985 990
   Ala Val Asp Leu Pro Asp Gly Ala Gly Thr Val Trp Ser Gly Gln Leu
   995 1000 1005
   Cys Val Arg Thr His Pro Trp Leu Ala Asp His Ser Val Trp Gly
   1010 1015 1020
   Arg Thr Val Val Pro Gly Thr Ala Leu Leu Glu Ile Met His Arg
   1025 1030 1035
   Val Arg Ala Glu Val Gly Cys Thr Arg Val Ala Glu Leu Thr Phe
   1040 1045 1050
   Glu Ala Pro Met Val Leu Ala Asp Asp Gly Gly Val Arg Val Arg
   1055 1060 1065
   Val Val Val Asp Gly Pro Asp Ala Asp Gly Ala Arg Gln Val Arg
   1070 1075 1080
   Ile His Ser Ala Pro Val Gly Pro Glu Pro Pro His Trp Thr Arg
   1085 1090 1095
   His Ala Ser Gly Arg Val Asp Ser Ala Ala Pro Gly Pro Ala Ala
   1100 1105 1110
   Gly Pro Pro Ala Trp Asp Ala Gly Pro Gly Ser Asn Trp Pro Pro
   1115 1120 1125
   Glu Gly Ala Glu Pro Val Gly Val Glu Ser Glu Tyr Glu Arg Phe
   1130 1135 1140
   Ala Asp Asn Gly Ile Gly Tyr Gly Pro Ala Phe Arg Gly Leu Arg
   1145 1150 1155
   Ala Ala Trp Arg Arg Gly Asn Glu Thr Phe Ala Glu Val Arg Leu
   1160 1165 1170
   Pro Glu Gly Tyr Ala Ala Glu Ala Gly Asp Tyr Ala Val His Pro
   1175 1180 1185
   Ala Leu Leu Asp Ala Ala Leu His Ala Ile Val Phe Gly Asp Gln
   1190 1195 1200
   Phe Pro Gly Gly Ala His Gly Met Leu Pro Phe Ala Phe Thr Asp
   1205 1210 1215
   Val Arg Val Phe Ser Ser Gly Ala Asp Arg Leu Arg Val Arg Ile
   1220 1225 1230
   Ala Pro Ala Asp Ala Asp Ser Val Cys Val Thr Val Ala Asp Gly
   1235 1240 1245
   Asp Gly Thr Pro Val Leu Ala Ala Ala Thr Leu Ala Leu Arg Arg
   1250 1255 1260
   Val Ala Ala Asp Arg Ile Ala Ala Thr Val Thr Gly Gln Ala Pro
   1265 1270 1275
   Leu Tyr Arg Leu Glu Trp Ser Ala Val Arg Pro Ala Pro Val Ala
   1280 1285 1290
   Thr Gly Ala Arg Phe Ala Val Val Gly Ala Asp Ala Pro Leu Pro
   1295 1300 1305
   Ser Gly Ala Leu Gly Ala Gly Val Pro Val Gln Ala Tyr Pro Asp
   1310 1315 1320
   Leu Gly Ala Leu Ala Gly Ala Leu Ala Thr Asn Gly Ala Pro Gly
   1325 1330 1335
   His Val Leu Val Asp Phe Arg Arg Arg Ala Asp Gly Pro Ala Gly
   1340 1345 1350
   Arg Gln Pro Gly Asp Val Gly Ala Arg Thr Arg Arg Ala Leu Ala
   1355 1360 1365
   Val Val Gln Glu Trp Leu Ala Asp Asp Arg Phe Thr Gly Ser Arg
   1370 1375 1380
   Leu Val Val Leu Thr Ser Gly Ala Val Asp Ala Gly Thr Ala Val
   1385 1390 1395
   Thr Asp Pro Ala Ala Ala Gly Val Trp Gly Leu Leu Arg Val Ala
   1400 1405 1410
   Gln Thr Glu His Pro Asp Arg Phe Val Leu Val Asp Thr Asp Asp
   1415 1420 1425
   His Pro Asp Ser Leu Arg Ala Leu Pro Gly Ala Ile Val Ala Gly
   1430 1435 1440
   Glu Pro Gln Leu Ala Leu Arg Ala Gly Thr Ala Ser Val Pro Gly
   1445 1450 1455
   Leu Val Arg Val Pro Ala Gly Thr Gly Ala Ala Pro Pro Trp Ala
   1460 1465 1470
   Ala Ala Gly Thr Val Leu Val Thr Gly Gly Thr Gly Met Leu Gly
   1475 1480 1485
   Gly Ala Val Ala Arg His Leu Val Arg Arg His Gly Val Arg Arg
   1490 1495 1500
   Leu Leu Leu Val Gly Arg Arg Gly Pro Asp Ala Pro Gly Ala Ala
   1505 1510 1515
   Ala Leu Thr Arg Glu Leu Glu Glu Leu Gly Ala Ser Val Arg Val
   1520 1525 1530
   Ala Ala Cys Asp Val Gly Asp Arg Gly Ala Val Thr Arg Leu Leu
   1535 1540 1545
   Ala Gly Val Pro Ala Ala His Pro Leu Thr Ala Val Val His Ser
   1550 1555 1560
   Ala Gly Leu Pro Asp Asp Gly Val Leu Thr Ala Gln Thr Gly Glu
   1565 1570 1575
   Arg Val Ala Ala Val Leu Arg Ala Lys Ala Asp Ala Ala Val Asn
   1580 1585 1590
   Leu His Glu Leu Thr Arg His Leu Asp Leu Thr Ala Phe Val Leu
   1595 1600 1605
   Phe Ser Ser Val Ala Gly Thr Ile Gly Ser Ala Gly Gln Ala Gly
   1610 1615 1620
   Tyr Ala Ala Ala Asn Ala Phe Leu Asp Ala Phe Ala Ser Trp Arg
   1625 1630 1635
   Gln Gly Gln Gly Leu Pro Ala Thr Ala Leu Ala Trp Gly Pro Leu
   1640 1645 1650
   Asp Gly Gly Met Ala Ala Gly Leu Gly Thr Ala Asp Val Ala Arg
   1655 1660 1665
   Leu Arg Arg Ser Gly Leu Val Pro Leu Gly Val Asp Asp Ala Leu
   1670 1675 1680
   Val Leu Phe Asp Ala Ala Cys Ser Arg Pro Ala Ala Ala Tyr His
   1685 1690 1695
   Pro Val Arg Leu Asp Pro Ala Val Leu Arg Ser His Ala Ala Ala
   1700 1705 1710
   Asp Ser Ala Val Pro Ala Val Leu Leu Gly Pro Ser Arg Ala His
   1715 1720 1725
   Pro Arg Asp Gly Thr Pro Gly Lys Pro Ala Glu Ala Ala Leu Ala
   1730 1735 1740
   Ala Leu Leu Thr Gly Arg Ser Ala Ala Glu Arg Thr Ala Ile Leu
   1745 1750 1755
   Thr Asp Leu Val Arg Thr Glu Ala Ala Ala Val Leu Gly His Gly
   1760 1765 1770
   Glu Ala Ala Met Leu Ser Thr Gln Arg Ala Phe Arg Asp Ala Gly
   1775 1780 1785
   Phe Asp Ser Leu Thr Ala Val Asp Leu Arg Asn Arg Leu Gly Ala
   1790 1795 1800
   Ala Thr Gly Leu Ser Leu Pro Ala Ala Val Val Phe Asp His Pro
   1805 1810 1815
   Thr Pro Ala Ala Leu Ala Ala Tyr Leu Arg Thr Glu Leu Asp Arg
   1820 1825 1830
   Arg Ser Pro Thr Gly Gln Gln Phe Pro Thr Asp Ala Ala Gly Val
       1835                    1840                    1845
   Leu Ala  Met Leu Asp Arg Leu  Arg Asp Gly Ile Ala  Thr Val Val
       1850                    1855                    1860
   Arg Asp  Asp Ala Asp Arg Thr  Arg Ala Ala Asp Leu  Leu Arg Val
       1865                    1870                    1875
   Leu Leu  Ala Glu Val Gly Gly  Pro Gly Thr Gly Pro  Pro Arg Asp
       1880                    1885                    1890
   Thr Asp  Gly Gly Ser Gly Gly  Glu Val Ser Asp Arg  Leu Arg Thr
       1895                    1900                    1905
   Ala Ser  Asp Glu Glu Leu Phe  Asp Leu Leu Asp Ser  Asp Phe Arg
       1910                    1915                    1920
   Leu Ala
       1925
<210> 13
   <211> 5778
   <212> DNA
   <213> micromonospora carbonacea subspecies aurantiaca
<400> 13
   gtgaccgcga acgaggaccg gatgcgtgag tacctcaagc gggtcaccgc cgagctggcc 60
   gggacgcggc gacgcctgcg cgagctggag gacagcgcgc gtgagcccat cgcgatcgtg 120
   ggcatgagct gccggttgcc gggcggggtg agcacgcccg aggacctgtg gcggctggtc 180
   gaggccggta ccgacgcgat ctccggcttc cccgacgacc ggggctggga tgtcgggagg 240
   ctctacgacc cggatccgga ctcgaccgga acgagctacg tgcgcgaggg cggcttcctc 300
   tacgactgcg ccgagttcga cccggagttc ttcaccgtct cgccccgcga ggcgctggcc 360
   atggacccgc agcagcggct gctgctggag gccgcctggg agaccttcga acgggcgggg 420
   atcgcccccg actcggcccg cggcacccgc accggggtct acgtcggggt gatgtacgac 480
   gactacggca gccggctgtc ggaggtgccg aaggacctgg agggctacct ggtcaacggc 540
   agcgcgggca gtgtcgcgtc gggccggatc gcgtacacgc tggggttgca ggggccggcg 600
   gtgacggtcg acacggcctg ctcgtcgtcg ctggtcgcgt tgcacctggc cgtgcaggcg 660
   ctgcggtcgg gcgagtgtga gctggccctg gcgggcgggg cgacggtgct cgccacgccg 720
   acgatgttcg tcgacttcgc ccggcagcgc ggtctcgccg aggacggccg ttgcaaggcg 780
   ttcgcggacg ccgccgacgg gaccgggttc ggcgagggcg tggggatgct gctggtggaa 840
   cggctctcgg acgcggtccg caaccgtcgc caggtgctgg ccgtcgtgcg gggcagcgcg 900
   gtcaaccagg acggggcgag caacggcctg accgccccga acggtacggc ccagcaactg 960
   gtcatccggc aggcgttgac caacgcgggg ctggccgcgg acgaggtgga cgcggtggag 1020
   gcacacggca ccggcacccg gctgggcgat ccgatcgagg cgcaggcgct gctggcgacg 1080
   tacggccagg gccggccggc ggaccggccg ctcctgctgg gatccctgaa gtccaacatc 1140
   ggccacaccc aggccgccgc aggggtcgcc ggggtgatca agaccgtgct ggcgctgcgt 1200
   cacgcgcggc tgccccggac cctgcacgtc gatcgcccct cgacccgggt ggactggtcg 1260
   tcgggcgcgg tgcggctgct gaccgagggg cggccctggc ccgatcacgg cgaccggccc 1320
   cgccgggccg gggtctcctc gttcggcgcg agcggcacca acgcgcacgt catcctggag 1380
   agcgcccccg gtgcggcggc gggggcgacc ggggcgacgg acctctcggc cccgccggca 1440
   tccgtcgccc accatccggc cacggccacg gccacggccc cggcggcgac ggtgcccact 1500
   gcccacgaac cggcggggac ggccggcgac gaccccgtct gggtcctgtc cggccggacc 1560
   gaggcggccc tgcgcgagca ggcccggcgg ctacacgccc acctgacatc ccgggcgcgg 1620
   cccgagcccg ccgacgccgt ggcccgcgcg ctggcgcgct cccgcaccgc gttcgcgtac 1680
   cgggccgccg tgctgggccg ggacgacacc gcgcggctcg acggcctcca cgcgctcgcg 1740
   gcgggtcgca gcgccgcggg gctcgtcacc gggcgggccg tgccggagcg gcgcgtggcc 1800
   ttcctcttca ccgggcaggg cagccagcga ccgggcgcgg gccgggaact gtacgcccgg 1860
   catcccgcct tcgcacaggc cctggacggc gtcctcgcgg aactcgaccg gcacctggac 1920
   cggccgctgc gcgccgtcat gctcgccgag ccgggcaccg aggcggcggc gctgctggac 1980
   gacaccgcgt acacccagcc cgccctgttc gcgctggagg tggcgctgtt ccggctggtc 2040
   acgagctggg ggctgcggcc tgacgccctg ctgggccact cggtcgggga gatcaccgcg 2100
   gcgtacgtcg cgggcgtcct caccctgccg gacgccgccc ggctggtggc ggtgcgcggt 2160
   cgactcatgg cggacctgcg ggccggcggt gcgatggccg cgctccaggc cgccgagagc 2220
   gaggtcgacc ccctgttggc ggggcgggag ggcgaactgt cgatcgcagc ggtcaacggg 2280
   ccgcaggcaa ccgtgatcgc gggcgacgag gcggccgtcg aggagcaggt cgcgctgtgg 2340
   cgtgaccggg gtcgccgggc caggcgactg cgggtcggcc acgccttcca ctccgtacgg 2400
   atggacggga tgctcgccga gttcgagaag gcgatgggtg atctccgtgc cggcgagccg 2460
   acgatccccg tggtcgccaa cgtcaggggg gcgatcgcgt ccggcaccga cctccgtacg 2520
   gccgggtact ggatccggca cgcccgcgag ccggtgcgtt tcctcgacgg catgcgtgcg 2580
   ctgcgggccg agggcgtcga cacgttcgtg gaactcggcc ccgacggagt gctcacggcg 2640
   atggcgcgcg actgcctggc ggatcccgcc gacccggtgg atctcgcgga cgccgccgag 2700
   cccgccgggg ccgcggagcc cgaccgctcc ctgctgttcc tgcccaccct gcgccgggac 2760
   cgcgacgacg cagtggccgt gcgggaggcc ctggcatccg tccacgtgca cgggcttccc 2820
   gtcgacccgg tcgcgccgct cggcgacggc ccgctcgcca ccgacctgcc cacctacccg 2880
   ttccagcggt cccgctactg gctcgacccg cgtcccgggg cacgcgacct gaccgccgtg 2940
   ggcctcgacg tggccgggca cccgctgctc gccgtcgccg tggacctgcc cgacggcgcc 3000
   ggcacggtct ggagcggtca gctctgcgtg cggacgcatc cgtggctcgc cgaccacagc 3060
   gtgtgggggc gcacggtggt gccggggacc gcgctgctgg agatcatgca ccgagtgcgc 3120
   gccgaggtgg gctgcacccg ggtcgcggaa ctgaccttcg aggcgccgat ggtgctggcc 3180
   gacgacgggg gcgtccgcgt gcgggtcgtc gtcgacggac cagacgccga cggggcccgc 3240
   caggtccgga tccactccgc accggtgggg cccgagcctc cccactggac ccggcacgcc 3300
   tcgggccgcg tcgacagcgc cgcgccgggg ccggccgccg gcccacccgc gtgggacgcc 3360
   ggccctggca gcaactggcc gcccgagggg gcggagccgg tgggcgtcga gagcgagtac 3420
   gagcgcttcg ccgacaacgg catcggatac ggccccgcct tccgagggct gcgcgccgcg 3480
   tggcgtcgcg ggaacgagac gttcgccgag gtccggctcc ccgaggggta cgccgccgag 3540
   gcgggcgact acgccgtcca tccggcactg ctggacgcgg ccctgcacgc gatcgtcttc 3600
   ggtgaccagt ttcccggtgg ggcacacggg atgctgccgt tcgccttcac cgacgtgcgg 3660
   gtgttcagct ccggcgccga ccggctccgg gtgcgcatcg cgcccgccga tgccgactcg 3720
   gtctgcgtga ccgtcgccga cggcgacggg acgccggtcc tcgccgcagc caccctggcg 3780
   ttgcgccggg tcgccgccga ccggatcgcg gcgaccgtca ccggccaggc accgctgtac 3840
   cggttggagt ggtccgccgt gcggcccgcc ccggtggcca ccggggcgcg gttcgccgtc 3900
   gtcggcgcgg acgccccgct gccgtccggt gcgctggggg ccggggtgcc cgtccaggcg 3960
   tacccggacc tgggcgcgct ggccggcgcg ttggccacca acggggcacc gggccacgtg 4020
   ctcgtcgact tccgccgccg cgccgacggc ccggcagggc ggcagcccgg tgacgtgggt 4080
   gcacggaccc gacgggcgct ggccgtcgtc caggagtggc tcgccgacga ccgtttcacc 4140
   ggctcacggc tggtcgtgct caccagcgga gccgtggacg ccggaacagc cgtcaccgat 4200
   ccggccgccg ccggggtgtg gggcctgctg cgggtcgccc agaccgagca tccggaccgg 4260
   ttcgtcctcg tggacaccga cgaccacccg gattcgctgc gtgccctccc cggggcgatc 4320
   gttgcgggcg agccgcagct ggcactgcgg gccggcacgg ccagcgttcc gggcctggtg 4380
   cgggtgccgg ccggcaccgg tgccgccccg ccgtgggccg cagccggcac cgtcctcgtc   4440
   accgggggca ccggcatgct cggcggcgcg gtggcccggc acctggtccg ccggcacggg   4500
   gtccgccgcc tgctgctggt cggccggcgc gggccggacg cacccggcgc ggcggccctg   4560
   acccgggaac tggaggagct gggagcgtcc gtccgcgtcg ccgcctgcga cgtcggcgat   4620
   cgtggcgcgg tgacgcgcct gttggccggg gttcccgccg cgcatccgct caccgcggtg   4680
   gtgcactcgg ccggcctgcc cgacgacggc gtgctgaccg cacagaccgg cgagcgggtc   4740
   gcggcggtgc tccgcgccaa ggcggacgca gcggtcaacc tgcacgaact cacccggcat   4800
   ctcgacctca ccgccttcgt gctgttctcg tcggtagcgg ggacgatcgg cagcgccggg   4860
   caggccgggt acgccgccgc gaacgccttc ctcgacgcgt tcgcgagctg gcggcagggc   4920
   caggggctgc ccgccaccgc cctggcgtgg gggccgttgg acggcgggat ggccgccggc   4980
   ctcggcactg cggacgtggc acggctgcgc cggtccgggc tcgtgccgct cggcgtggac   5040
   gacgcgctcg ttctcttcga cgccgcctgc tcccgaccgg cggcggcgta ccaccccgtc   5100
   cgcctcgatc cggcggtgct gcggtcccac gccgccgccg acagcgcggt gcccgccgtc   5160
   ctgctcggtc cgagccgtgc gcacccgagg gacggtacgc cggggaagcc tgccgaagcc   5220
   gccctcgccg cgctgctgac cggcaggtcg gcggccgagc gtacggcgat cctgaccgac   5280
   ctggtgcgga cggaggccgc cgccgttctc gggcatggcg aggcggcgat gctgagcacg   5340
   cagcgggcct tccgcgacgc cggcttcgac tcgctcaccg ccgtggacct ccgcaaccgg   5400
   ctcggcgcgg ccacgggcct cagcctgccg gccgccgtcg tcttcgacca cccgaccccg   5460
   gcggccctgg ccgcctatct gcggaccgaa ctggaccgcc ggtcgcccac cgggcaacag   5520
   ttcccgacgg acgccgccgg tgttctggcc atgctcgacc gcctgcggga cggaatcgcg   5580
   acggtcgtca gggacgacgc cgaccggacc cgcgcagccg acctgttgcg tgtcctgctc   5640
   gccgaggtcg gcgggcccgg gacgggcccg ccccgcgaca ccgacggcgg ctccggcggc   5700
   gaggtcagcg accgcctccg gaccgcctcc gacgaggaac tgttcgacct gctcgacagc   5760
   gatttccgac tggcgtag                                                  5778
<210> 14
   <211> 3745
   <212> PRT
   <213> micromonospora carbonacea subspecies aurantiaca
<400> 14
   Val Ser Val Asn Asn Glu Asp Lys Leu Arg Glu Tyr Leu Arg Arg Ala
   1               5                   10                  15
   Met Ala Asp Leu His Glu Ser Arg Glu Arg Leu Arg Gln Tyr Glu Ser
               20                  25                  30
   Ala Ala Ala Val Asp Asp Pro Val Val Val Val Gly Met Gly Cys Arg
           35                  40                  45
   Phe Pro Gly Gly Val Val Cys Ala Glu Gly Leu Trp Asp Leu Val Leu
       50                  55                  60
   Gly Gly Gly Asp Ala Val Ser Gly Phe Pro Val Asp Arg Gly Trp Asp
   65                  70                  75                  80
   Val Glu Gly Leu Phe Asp Pro Val Arg Gly Val Val Gly Lys Ser Tyr
                   85                  90                  95
   Val Arg Glu Gly Gly Phe Val Tyr Asp Ala Gly Met Phe Asp Ala Glu
               100                 105                 110
   Phe Phe Gly Val Ser Pro Arg Glu Ala Val Ala Met Asp Pro Gln Gln
           115                 120                 125
   Arg Leu Phe Leu Glu Val Ser Trp Glu Ala Leu Glu Arg Ala Gly Ile
       130                 135                 140
   Asp Pro Leu Gly Leu Arg Gly Ser Arg Thr Gly Val Tyr Val Gly Val
   145                 150                 155                 160
   Met Gly Gln Glu Tyr Gly Pro Arg Leu Val Glu Ser Gly Gly Gly Phe
                   165                 170                 175
   Glu Gly Tyr Leu Leu Thr Gly Thr Ser Pro Ser Val Val Ser Gly Arg
               180                 185                 190
   Val Ser Tyr Val Leu Gly Leu Glu Gly Pro Ser Ile Ser Val Asp Thr
           195                 200                 205
   Ala Cys Ser Ser Ser Leu Val Ala Leu His Leu Ala Cys Gln Gly Leu
       210                 215                 220
   Arg Leu Gly Glu Cys Asp Val Ala Leu Ala Gly Gly Val Thr Val Ile
   225                 230                 235                 240
   Ala Ala Pro Gly Leu Phe Val Glu Phe Ser Arg Gln Gly Gly Leu Ser
                   245                 250                 255
   Gly Asp Gly Arg Cys Arg Ala Phe Ala Gly Gly Ala Asp Gly Thr Gly
               260                 265                 270
   Trp Gly Glu Gly Ala Gly Val Val Val Leu Glu Arg Leu Ser Val Ala
           275                 280                 285
   Arg Glu Arg Gly His Arg Val Leu Ala Val Val Arg Gly Ser Ala Val
       290                 295                 300
   Asn Gln Asp Gly Gly Ser Asn Gly Leu Thr Ala Pro Ser Gly Val Ala
   305 310 315 320
   Gln Arg Arg Val Ile Gly Ala Ala Leu Val Ala Ala Gly Leu Gly Val
   325 330 335
   Ser Asp Val Asp Val Val Glu Ala His Gly Thr Gly Thr Arg Leu Gly
   340 345 350
   Asp Pro Ile Glu Ala Glu Ala Leu Leu Gly Ser Tyr Gly Arg Gly Arg
   355 360 365
   Val Gly Gly Ala Leu Leu Leu Gly Ser Val Lys Ser Asn Ile Gly His
   370 375 380
   Thr Gln Ala Ala Ala Gly Val Ala Gly Val Ile Lys Met Val Met Ala
   385 390 395 400
   Leu Arg Ala Gly Val Val Pro Ala Thr Leu His Val Asp Val Pro Ser
   405 410 415
   Pro Leu Val Asp Trp Ser Ser Gly Gly Val Glu Leu Val Thr Glu Ala
   420 425 430
   Arg Asp Trp Pro Val Val Gly Arg Val Arg Arg Ala Gly Val Ser Ala
   435 440 445
   Phe Gly Val Ser Gly Thr Asn Ala His Leu Ile Leu Glu Gln Ala Pro
   450 455 460
   Glu Phe Asp Asp Pro Ala Asp Ser Asp Ser Asp Ser Asp Ser Asp Ala
   465 470 475 480
   Gly Val Val Asp Gly Gly Glu Gly Gly Val Gly Arg Ser Leu Ser Val
   485 490 495
   Val Pro Val Val Val Ser Gly Arg Ser Val Gly Ala Leu Arg Ala Tyr
   500 505 510
   Ala Gly Arg Leu Arg Glu Val Cys Ala Gly Leu Ser Asp Gly Gly Gly
   515 520 525
   Ser Gly Gly Gly Ser Gly Leu Val Asp Val Gly Trp Ser Leu Val Ser
   530 535 540
   Ser Arg Ser Val Phe Glu His Arg Ala Val Val Phe Gly Gly Gly Val
   545 550 555 560
   Glu Glu Val Val Ala Gly Leu Gly Ala Val Ala Ser Gly Ala Val Ala
   565 570 575
   Ser Gly Ser Val Val Val Gly Ser Val Ala Ser Gly Val Ala Gly Gly
   580 585 590
   Gly Gly Arg Val Val Phe Val Phe Pro Gly Gln Gly Trp Gln Trp Val
   595 600 605
   Gly Met Gly Ala Ala Leu Leu Asp Glu Ser Glu Val Phe Ala Glu Ser
       610                 615                 620
   Met Val Glu Cys Gly Arg Ala Leu Ser Gly Phe Val Asp Trp Asp Leu
   625                 630                 635                 640
   Leu Glu Val Val Arg Gly Gly Ala Gly Glu Gly Val Trp Gly Arg Val
                   645                 650                 655
   Asp Val Val Gln Pro Val Ser Trp Ala Val Met Val Ser Leu Ala Arg
               660                 665                 670
   Leu Trp Met Ser Val Gly Val Val Pro Asp Ala Val Val Gly His Ser
           675                 680                 685
   Gln Gly Glu Val Ala Ala Ala Val Val Gly Gly Val Leu Ser Val Ala
       690                 695                 700
   Asp Gly Ala Arg Val Val Ala Leu Arg Ser Arg Val Ile Gly Glu Val
   705                 710                 715                 720
   Leu Ala Gly Gly Gly Ala Met Val Ser Val Gly Leu Pro Ile Val Asp
                   725                 730                 735
   Val Gln Glu Arg Leu Ala Gly Trp Gly Gly Arg Leu Gly Val Ala Ala
               740                 745                 750
   Val Asn Gly Pro Ser Leu Thr Val Val Ser Gly Asp Val Asp Ala Ala
           755                 760                 765
   Val Gly Phe Val Gly Glu Cys Glu Arg Asp Gly Val Trp Val Arg Arg
       770                 775                 780
   Val Ala Val Asp Tyr Ala Ser His Ser Ala His Val Glu Ala Val Glu
   785                 790                 795                 800
   Gly Met Leu Ser Gly Leu Leu Gly Gly Leu Cys Pro Gly Arg Gly Val
                   805                 810                 815
   Val Pro Phe Tyr Ser Ser Val Val Gly Gly Val Val Asp Gly Val Gly
               820                 825                 830
   Leu Asp Gly Gly Tyr Trp Tyr Arg Asn Leu Arg Glu Arg Val Leu Phe
           835                 840                 845
   Ser Asp Val Val Gly Arg Leu Val Gly Asp Gly Phe Ser Gly Phe Val
       850                 855                 860
   Glu Cys Ser Gly His Pro Val Leu Ala Gly Gly Val Leu Glu Ser Val
   865                 870                 875                 880
   Ala Val Val Asp Pro Asp Val Arg Pro Val Val Val Gly Ser Leu Arg
                   885                 890                 895
   Arg Asp Asp Gly Gly Trp Gly Arg Phe Leu Thr Ser Val Gly Glu Ala
               900                 905                 910
   Phe Val Gly Gly Met Ser Val Asp Trp Lys Gly Val Phe Ala Gly Ala
   915 920 925
   Gly Ala Arg Leu Val Asp Leu Pro Thr Tyr Pro Phe Gln Arg Arg His
   930 935 940
   Tyr Trp Ala Pro Thr Pro Thr Asn Pro Ala Thr Asn Pro Ala Thr Asn
   945 950 955 960
   Pro Ala Thr Asn Pro Ala Thr Gly Asp Thr Thr Thr Ala Asp Pro Ala
   965 970 975
   Gly Asp Leu Arg Tyr Arg Ile Thr Trp Lys Pro Leu Pro Thr Asp Asp
   980 985 990
   Pro Arg Pro Leu Thr Asn Arg Trp Leu Leu Met Val Pro Glu Ala Leu
   995 1000 1005
   Ala Gly Asp Gly Val Val Ala Gly Val Arg Gln Ala Leu Ala Ala
   1010 1015 1020
   Arg Gly Ala Ser Val Glu Leu Leu Thr Val Gly Thr Ala Asp Arg
   1025 1030 1035
   Ala Gly Leu Ala Ala Leu Leu Thr Ser Ala Ala Pro Gly Asp Pro
   1040 1045 1050
   Glu Ala Ala Gly Pro Ala Gly Val Val Ser Leu Leu Ala Leu Ala
   1055 1060 1065
   Glu Gly Ala Asp Ala Arg His Pro Ala Val Pro Leu Gly Leu Thr
   1070 1075 1080
   Ala Ser Leu Ala Leu Ile Gln Ala Leu Ala Asp Ala Gly Thr Gln
   1085 1090 1095
   Ala Arg Leu Trp Ala Val Thr Arg Gly Ala Val Ala Val Ser Ser
   1100 1105 1110
   Gly Glu Val Pro Asp Ala Gly Gln Ala Gln Val Trp Gly Leu Gly
   1115 1120 1125
   Arg Val Ala Ala Leu Glu Leu Pro Asp Arg Trp Gly Gly Leu Val
   1130 1135 1140
   Asp Leu Pro Ala Leu Thr Gly Glu Arg Ala Phe Ala Gln Leu Ala
   1145 1150 1155
   Asp Val Val Gly Gly Ser Asn Gly Glu Asp Gln Val Ala Val Arg
   1160 1165 1170
   Ala Ser Gly Val Tyr Gly Arg Arg Leu Val Arg Ser Arg Ala Thr
   1175 1180 1185
   Val Thr Ser Gly Asp Trp Pro Ala Arg Gly Thr Ile Leu Val Val
   1190 1195 1200
   Gly Asp Thr Gly Pro Val Ala Ala Leu Leu Ala Gly Arg Leu Leu
   1205 1210 1215
   Gly Asp Gly Ala Ala His Val Val Leu Ala Gly Pro Ala Ala Ala
   1220 1225 1230
   Ser Thr Val Gly Leu Thr Gly Gly Ala Asp Arg Val Ala Leu Ile
   1235 1240 1245
   Asp Cys Asp Pro Ser Asp Arg Asp Ala Leu Ala Gly Leu Leu Gly
   1250 1255 1260
   Ala Tyr Arg Pro Thr Thr Ile Val Val Ala Pro Pro Ala Val Ala
   1265 1270 1275
   Leu Thr Ala Leu Ala Glu Thr Thr Pro Glu Asp Phe Val Ala Ala
   1280 1285 1290
   Val Ala Ala Lys Thr Thr Thr Ala Val His Leu Asp Ala Leu Ala
   1295 1300 1305
   Ala Glu Ala Glu Leu Glu Leu Asp Ala Phe Val Val Phe Ser Ser
   1310 1315 1320
   Val Ser Gly Thr Trp Gly Gly Ala Gly His Gly Gly Tyr Ala Ala
   1325 1330 1335
   Gly Thr Ala Arg Leu Asp Ala Leu Val Glu Glu Arg Arg Ala Arg
   1340 1345 1350
   Gly Leu Pro Ala Thr Ala Ile Ala Trp Thr Pro Trp Ala Asp Ala
   1355 1360 1365
   Thr Thr Ala Ala Gly Gly Gln Ala Pro Asp Ala Ser Ala Gly Gly
   1370 1375 1380
   His Glu Pro Asp Thr Arg Ala Gly Gly Pro Asp Arg Glu Leu Leu
   1385 1390 1395
   Arg Arg Gly Gly Leu Thr Pro Leu Asp Pro Gly Ala Ala Leu Asp
   1400 1405 1410
   Val Leu Arg Gly Ala Val Ala Arg Gly Glu Gly Leu Val Thr Val
   1415 1420 1425
   Ala Asp Val Asp Trp Ala Arg Phe Val Ala Ser Tyr Thr Ala Ala
   1430 1435 1440
   Arg Pro Thr Thr Leu Phe Asp Glu Leu Pro Glu Leu Arg Ala Thr
   1445 1450 1455
   Arg Glu Ala Glu His Thr Pro Ala Glu Asp Ser Ser Ala Gly Gly
   1460 1465 1470
   Glu Leu Val Arg Ala Leu Ser Gly Arg Pro Ala Ala Asp Gln His
   1475 1480 1485
   Arg Thr Leu Leu Arg Leu Val Arg Ala His Val Ala Ala Val Leu
   1490 1495 1500
   Gly His Asp Glu Ala Glu Ala Ala Asp Pro Asp Arg Ala Phe Arg
   1505 1510 1515
   Glu Leu Gly Phe Thr Ser Val Thr Ala Val Asp Leu Arg Asn Arg
   1520 1525 1530
   Leu Asn Ala Ala Thr Gly Leu Asn Leu Pro Ala Ser Val Val Phe
   1535 1540 1545
   Asp His Pro Ser Ala Arg Val Leu Ala Ala Tyr Leu Arg Ala Glu
   1550 1555 1560
   Leu Leu Gly Pro Glu Ala Asp Glu Asp Thr Ala Glu Ala Val Ala
   1565 1570 1575
   Pro Pro Ser Ala Pro Ala Gly Ala Gly Asp Asp Glu Pro Ile Ala
   1580 1585 1590
   Val Ile Gly Met Ala Cys Arg Phe Pro Gly Gly Val Asp Ala Pro
   1595 1600 1605
   Asp Asp Leu Trp Asp Leu Leu Ala Lys Gly Arg Asp Ala Ile Ser
   1610 1615 1620
   Arg Phe Pro Thr Asn Arg Gly Trp Asp Val Asp Gly Leu Tyr Asp
   1625 1630 1635
   Pro Asp Pro Glu Ala Pro Gly Arg Thr Tyr Val Arg Glu Gly Gly
   1640 1645 1650
   Phe Leu His Asp Ala Pro Asp Phe Asp Ala Ala Phe Phe Gly Ile
   1655 1660 1665
   Ser Pro Arg Glu Ala Leu Ala Met Asp Pro Gln Gln Arg Leu Leu
   1670 1675 1680
   Leu Glu Thr Thr Trp Glu Ser Leu Glu Arg Ala Gly Leu Asp Pro
   1685 1690 1695
   Thr Ala Leu Arg Gly Thr Arg Thr Gly Val Phe Val Gly Thr Asn
   1700 1705 1710
   Gly Gln His Tyr Met Pro Leu Leu Arg Asp Gly Ala Asp Asp Phe
   1715 1720 1725
   Asp Gly Tyr Leu Gly Thr Gly Asn Ser Ala Ser Val Met Ser Gly
   1730 1735 1740
   Arg Leu Ser Tyr Val Phe Gly Leu Glu Gly Pro Ala Val Thr Val
   1745 1750 1755
   Asp Thr Ala Cys Ser Ala Ser Leu Val Ala Leu His Leu Ala Val
   1760 1765 1770
   Gln Ala Leu Arg Arg Gly Glu Cys Thr Leu Ala Leu Val Gly Gly
   1775 1780 1785
   Ala Thr Val Met Ser Thr Pro Asp Met Leu Val Glu Phe Ser Arg
   1790 1795 1800
   Gln Arg Ala Met Ser Pro Asp Gly Arg Ser Lys Ala Phe Ala Ala
   1805 1810 1815
   Ala Ala Asp Gly Val Ala Leu Ser Glu Gly Ala Ala Met Met Val
   1820 1825 1830
   Val Gln Arg Leu Ala Asp Ala Glu Ala Ala Gly His Glu Ile Leu
   1835 1840 1845
   Ala Val Val Lys Gly Ser Ala Val Asn Gln Asp Gly Ala Ser Asn
   1850 1855 1860
   Gly Leu Thr Ala Pro Asn Gly Pro Ser Gln Glu Arg Val Ile Arg
   1865 1870 1875
   Gln Ala Leu Ala Asp Ala Gly Leu Arg Pro Asp Gln Val Asp Ala
   1880 1885 1890
   Val Glu Ala His Gly Thr Gly Thr Ala Leu Gly Asp Pro Ile Glu
   1895 1900 1905
   Ala Gln Ala Leu Leu Ala Thr Tyr Gly Arg Asp Arg Pro Ala Gly
   1910 1915 1920
   Arg Pro Leu Trp Leu Gly Ser Leu Lys Ser Asn Ile Gly His Thr
   1925 1930 1935
   Gln Ala Ala Ala Gly Ile Ala Gly Val Met Lys Val Ile Leu Ala
   1940 1945 1950
   Leu Arg His Asp Thr Leu Pro Arg Thr Leu His Val Asp Arg Pro
   1955 1960 1965
   Thr Pro Arg Val Asp Trp Ala Ser Gly Ala Val Ser Leu Leu Thr
   1970 1975 1980
   Glu Pro Val Pro Trp Pro Gln Gly Asp Glu Pro Arg Arg Ala Ala
   1985 1990 1995
   Val Ser Ser Phe Gly Ile Ser Gly Thr Asn Ala His Val Ile Val
   2000 2005 2010
   Glu Gln Ala Pro Pro Val Val Arg Glu Pro Ile Asp His Glu Ala
   2015 2020 2025
   Asp Glu Val Thr Val Pro Leu Phe Leu Ser Ala Arg Gly Ser Ala
   2030 2035 2040
   Ala Leu Cys Ala Gln Ala Ala Arg Leu Arg Ala Arg Leu Ile Glu
   2045 2050 2055
   Glu Pro Asp Leu Asp Ile Ala Glu Val Gly Tyr Thr Leu Ala Ala
   2060 2065 2070
   Thr Arg Ala Arg Phe Glu His Arg Ala Val Val Ile Gly Glu Ser
   2075 2080 2085
   Arg Ala Glu Val Gly Asp Ala Leu Ala Ala Leu Ala Arg Gly Glu
   2090 2095 2100
   Glu His Pro Ser Leu Leu Arg Gly Arg Ala Gly Ala Ser Asp Arg
   2105 2110 2115
   Val Ala Phe Val Phe Pro Gly Gln Gly Ser Gln Trp Ala Glu Met
   2120 2125 2130
   Ala Asp Gly Leu Leu Asp Arg Ser Pro Ala Phe Arg Ala Ser Ala
   2135 2140 2145
   Ser Ala Cys Asp Glu Ala Leu Arg Ala His Leu Asp Trp Ser Val
   2150 2155 2160
   Leu Asp Val Leu Arg Arg Val Pro Asp Ala Pro Ala Leu Ser Arg
   2165 2170 2175
   Val Asp Val Val Gln Pro Val Leu Phe Thr Met Met Val Ser Leu
   2180 2185 2190
   Ala Ala Ala Trp Arg Ala Leu Gly Val His Pro Ser Ala Val Val
   2195 2200 2205
   Gly His Ser Gln Gly Glu Ile Ala Ala Ala His Val Ala Gly Gly
   2210 2215 2220
   Leu Ser Leu Asp Asp Ala Ala Arg Ile Val Ala Leu Arg Ser Gln
   2225 2230 2235
   Ala Trp Leu Arg Leu Ala Gly Gln Gly Gly Met Val Ala Val Ser
   2240 2245 2250
   Leu Pro Val Asp Ala Leu Arg Ala Arg Leu Ala Arg Phe Gly Asp
   2255 2260 2265
   Arg Leu Ser Val Ala Ala Val Asn Ser Pro Gly Thr Ala Ala Val
   2270 2275 2280
   Ser Gly Tyr Pro Asp Ala Leu Ala Glu Leu Val Asp Glu Leu Thr
   2285 2290 2295
   Ala Glu Gly Val His Ala Lys Ala Ile Pro Gly Val Asp Thr Ala
   2300 2305 2310
   Gly His Ser Ala Gln Val Glu Val Leu Lys Asp His Leu Met Ala
   2315 2320 2325
   Ala Leu Ala Pro Val Ser Pro Arg Ser Ser Gln Ile Pro Phe Tyr
   2330 2335 2340
   Ser Thr Val Thr Gly Gly Leu Leu Asp Thr Ala Leu Leu Asp Ala
   2345 2350 2355
   Ala Tyr Trp Tyr Arg Asn Met Arg Asp Pro Val Glu Phe Glu Gln
   2360 2365 2370
   Ala Thr Arg Ala Met Leu Ala Asp Gly His Glu Gly Phe Leu Glu
   2375 2380 2385
   Pro Ser Pro His Pro Met Leu Ser Val Ser Leu Gln Gly Thr Ala
   2390 2395 2400
   Ala Asp Ala Gly Val Ala Ala Thr Val Leu Gly Thr Leu Arg Arg
   2405 2410 2415
   Gly Lys Gly Gly Ala Arg Trp Phe Gly Met Ala Leu Gly Leu Ala
   2420 2425 2430
   His Ala His Gly Ile Glu Ile Asp Ala Ser Val Leu Phe Gly Thr
   2435 2440 2445
   Asp Ser Arg Arg Val Asp Leu Pro Thr Tyr Pro Phe Gln Arg Glu
   2450 2455 2460
   Arg Phe Trp Tyr His Pro Pro Ala Ala Arg Gly Asp Val Ala Ser
   2465 2470 2475
   Ala Gly Leu Ser Gly Ala Asp His Pro Leu Leu Gly Gly Ala Val
   2480 2485 2490
   Glu Leu Pro Asp Arg Gly Gly His Val Tyr Pro Ala Arg Leu Gly
   2495 2500 2505
   Val Arg His His Pro Trp Leu Gly Glu His Ala Leu Leu Gly Ala
   2510 2515 2520
   Ala Ile Leu Pro Gly Ala Ala Tyr Ala Glu Leu Ala Leu Trp Ala
   2525 2530 2535
   Gly Arg Arg Asp Gly Ala Gly Arg Ile Glu Glu Leu Thr Leu Asp
   2540 2545 2550
   Ala Pro Leu Val Val Ala Asp Glu Ser Ala Ala Gln Leu Arg Leu
   2555 2560 2565
   Val Val Gly Pro Ala Asp Ala Glu Gly Arg Arg Gln Leu Thr Val
   2570 2575 2580
   His Ser Arg Ala Asp Gly Ala Asp Ala Asp Thr Ala Trp Thr Arg
   2585 2590 2595
   His Ala Gln Gly Thr Leu Val Pro Ala Asp Ala Asp Ala Ala Gly
   2600 2605 2610
   Ser Gly Asp Pro Gly Ala Pro Trp Pro Pro Ala Gly Ala Glu Pro
   2615 2620 2625
   Val Glu Val Ala Gly Leu Tyr Asp Arg Phe Ala Asp Arg Gly Tyr
   2630 2635 2640
   Gln Tyr Gly Pro Ser Phe Arg Gly Val Arg Ala Ala Trp Arg Ala
   2645 2650 2655
   Gly Asp Thr Val Tyr Ala Glu Val Ala Leu Pro Val Pro Gln Pro
   2660 2665 2670
   Gly Ser Pro Arg Phe Gly Val His Pro Ala Leu Leu Asp Ala Ala
   2675 2680 2685
   Phe Gln Ala Met Ser Leu Gly Ala Phe Phe Pro Glu Asp Gly Gln
   2690 2695 2700
   Val Arg Met Pro Phe Ala Leu Arg Gly Val Ser Ser Ser Gly Val
   2705 2710 2715
   Gly Ala Asp Arg Leu Arg Val Thr Ile Ser Pro Ala Gly Ala Glu
   2720 2725 2730
   Ala Val Arg Ile Ala Cys Val Asp Glu Arg Gly Asn Pro Val Val
   2735 2740 2745
   Val Ile Asp Ser Leu Val Ala Arg Ala Val Pro Val Glu Ala Leu
   2750 2755 2760
   Thr Pro Gly Thr Pro Gly Thr Gly Asp Gly Ala Leu His His Val
   2765 2770 2775
   Ala Trp Thr Ala Arg Pro Glu Pro Gly Val Ala Ala Val Gln Arg
   2780 2785 2790
   Trp Ala Val Val Gly Ala Ala Asp Pro Gly Leu Ala Gly Gly Leu
   2795 2800 2805
   Asp Arg Ala Gly Gly Leu Cys Gly Ala Tyr Pro Asp Leu Ala Gly
   2810 2815 2820
   Leu Val Ala Ala Val Ala Glu Gly Ala Ala Leu Pro Asp Val Val
   2825 2830 2835
   Ala Val Pro Val Pro Ser Gly Ala Pro Val Gly Pro Asp Ala Val
   2840 2845 2850
   Arg Ala Thr Val Leu Gly Ala Leu Asp Leu Ile Arg Ala Trp Leu
   2855 2860 2865
   Ala Val Glu Gly Arg Leu Gly Leu Ala Arg Leu Ala Phe Val Thr
   2870 2875 2880
   Thr Ser Ala Val Ala Val Gly Asp Gly Thr Glu His Val Asp Pro
   2885 2890 2895
   Val Ser Ala Ala Leu Trp Gly Leu Val Arg Ser Ala Gln Ser Glu
   2900 2905 2910
   Glu Pro Gly Arg Phe Val Leu Val Asp Leu Asp Ala Asp Pro Ala
   2915 2920 2925
   Ser Ala Ser Ala Leu Pro Ala Ala Leu Ala Ala Gly Glu Pro Gln
   2930 2935 2940
   Leu Ala Val Arg Ala Gly Ala Val His Val Pro Arg Leu Val Arg
   2945 2950 2955
   His Arg Pro Arg Pro Asp Gly Pro Leu Thr Pro Pro Ala Gly Ala
   2960 2965 2970
   Ala Trp Arg Leu Ala Ala Gly Gly Gln Gly Thr Leu Glu Gly Leu
   2975 2980 2985
   Ala Leu Val Pro Ala Pro Asp Ala Leu Ala Pro Leu Ala Pro Gly
   2990 2995 3000
   Gln Val Arg Val Ala Val Arg Ala Ala Gly Val Asn Phe Arg Asp
   3005 3010 3015
   Thr Leu Ile Ala Leu Gly Met Tyr Pro Gly Thr Pro Val Leu Gly
   3020 3025 3030
   Ala Glu Gly Ala Gly Val Ile Thr Glu Val Ala Pro Asp Val Ala
   3035 3040 3045
   Gly Phe Ala Pro Gly Asp Arg Val Leu Gly Met Trp Thr Gly Gly
   3050 3055 3060
   Leu Gly Pro Val Ala Val Ala Asp Ala Arg Met Leu Ala Arg Val
   3065 3070 3075
   Pro Arg Gly Trp Ser Tyr Ala Glu Ala Ala Ser Val Pro Ala Val
   3080 3085 3090
   Phe Leu Thr Ala His Tyr Ala Leu Thr Arg Leu Ala Gly Ile Arg
   3095 3100 3105
   Pro Gly Gln Ser Leu Leu Val His Ala Gly Ala Gly Gly Val Gly
   3110 3115 3120
   Met Ala Thr Leu Gln Leu Ala Arg His Leu Gly Val Glu Val Tyr
   3125 3130 3135
   Ala Thr Ala Ser Arg Gly Lys Trp Asp Thr Leu Arg Gly Leu Gly
   3140 3145 3150
   Leu Asp Asp Ala His Ile Ala Asp Ser Arg Ser Leu Asp Phe Ala
   3155 3160 3165
   Gly Arg Phe Leu Ala Ala Thr Gly Gly Arg Gly Val Asp Val Val
   3170 3175 3180
   Leu Asn Ser Leu Ala Gly Asp Phe Val Asp Ala Ser Leu Arg Leu
   3185 3190 3195
   Leu Pro Arg Gly Gly His Phe Leu Glu Leu Gly Lys Ala Asp Val
   3200 3205 3210
   Arg Asp Pro Asp Arg Ile Ala Ala Asp His Pro Gly Val Gly Tyr
   3215 3220 3225
   Arg Ala Phe Asp Leu Val Glu Ala Gly Pro Glu Leu Val Gly Gln
   3230 3235 3240
   Leu Leu Gly Glu Leu Met Glu Leu Phe Ala Ala Gly Val Leu Ser
   3245 3250 3255
   Pro Leu Pro Leu Thr Val Arg Asp Val Arg Arg Ala Arg Glu Ala
   3260 3265 3270
   Phe Arg Leu Ile Ser Gln Ala Arg His Val Gly Lys Val Val Leu
   3275 3280 3285
   Thr Met Pro Pro Ala Phe Gly Ala Tyr Gly Thr Val Leu Val Thr
   3290 3295 3300
   Gly Gly Thr Gly Thr Leu Gly Gly Ala Val Ala Arg His Leu Val
   3305 3310 3315
   Ala Arg His Gly Val Arg His Leu Val Leu Thr Gly Arg Ser Gly
   3320 3325 3330
   Pro Ala Ala Asp Gly Ala Ser Ala Leu Val Asp Glu Leu Thr Ala
   3335 3340 3345
   Ser Gly Ala Ser Val Thr Val Val Ala Cys Asp Ala Ala Asp Arg
   3350 3355 3360
   Val Ala Leu Arg Arg Leu Leu Asp Gly Ile Pro Ala Ala His Pro
   3365 3370 3375
   Leu Thr Ala Val Val His Ala Ala Gly Val Leu Asp Asp Ala Thr
   3380 3385 3390
   Ile Thr Ala Leu Thr Ala Gly Gln Val Asp Ala Val Leu Arg Pro
   3395 3400 3405
   Lys Ala Asp Ala Val Ile Asn Leu His Glu Leu Thr Arg Asp Arg
   3410 3415 3420
   Glu Leu Ser Ala Phe Val Leu Phe Ser Ser Ala Ala Ala Leu Phe
   3425 3430 3435
   Gly Ser Pro Gly Gln Gly Asn Tyr Ser Ala Ala Asn Gly Phe Val
   3440 3445 3450
   Asp Ala Phe Ala Gln Tyr Arg Arg Ala Gln Gly Leu His Ala Val
   3455 3460 3465
   Ser Leu Ala Trp Gly Leu Trp Ala Asp Ser Ser Arg Met Ala Gly
   3470 3475 3480
   His Leu Asp Gln Glu Gly Met Arg Arg Arg Met Ala Arg Gly Gly
   3485 3490 3495
   Val Leu Pro Leu Thr Thr Asp Gln Gly Leu Ala Leu Phe Asp Ala
   3500 3505 3510
   Ala Gln Leu Val Asp Glu Ala Leu Gln Val Pro Ile Arg Leu Asn
   3515 3520 3525
   Val Gly Ala Leu Arg Ala Ala Gly Arg Val Pro Ala Leu Leu Ala
   3530 3535 3540
   Asp Leu Val Pro Ala Ala Ala Ser Gly Ala Pro Ala Ala Thr Pro
   3545 3550 3555
   Thr Arg Asp Asp Ala Asp Arg Thr Leu Ala Asp Arg Leu Ala Gly
   3560 3565 3570
   Leu Thr Val Ala Glu Gln Arg Glu Leu Val Leu Glu Ser Val Arg
   3575 3580 3585
   Gly His Ala Ala Ala Val Leu Gly His Ala Asp Pro Gln Ala Val
   3590 3595 3600
   Asp Ala Asp Arg Ala Phe Arg Glu Leu Gly Phe Asp Ser Leu Thr
   3605 3610 3615
   Ala Val Glu Leu Arg Asn Arg Leu Ala Thr Ala Ser Gly Leu Arg
   3620 3625 3630
   Leu Pro Ala Thr Leu Val Phe Asp His Pro Thr Pro Glu Ala Leu
   3635 3640 3645
   Ala Glu His Leu Leu Ala Gly Leu Ala Pro Glu Gln Ala Arg Ala
   3650 3655 3660
   Glu Leu Pro Leu Leu Ala Glu Leu Gly Arg Leu Glu Ala Ala Leu
   3665 3670 3675
   Ala Ala Thr Asp Gly Ala Ala Leu Asp Gly Leu Asp Asp Leu Val
   3680 3685 3690
   Arg Arg Glu Val Gly Val Arg Ile Ala Ala Leu Ala Ala Arg Trp
   3695 3700 3705
   Gly Ala Ala Gly Asp Asp Val Ala Gly Ser Asp Gly Gly Gly Thr
   3710 3715 3720
   Ala Asp Ala Leu Glu Ser Ala Asp Asp Asp Glu Ile Phe Ala Phe
   3725 3730 3735
   Ile Asp Glu Arg Phe Arg Ala
   3740 3745
<210> 15
   <211> 11238
   <212> DNA
   <213> micromonospora carbonacea subspecies aurantiaca
<400> 15
<210> 16
   <211> 1574
   <212> PRT
   <213> micromonospora carbonacea subspecies aurantiaca
<400> 16
   Met Ser Asn Glu Gln Lys Leu Arg Glu Tyr Leu Arg Leu Thr Thr Thr
   1               5                   10                  15
   Glu Leu Ala Arg Ala Thr Asp Arg Leu Arg Ala Val Glu Ala Arg Ala
               20                  25                  30
   His Glu Pro Ile Ala Ile Val Gly Met Ala Cys Arg Tyr Pro Gly Gly
           35                  40                  45
   Val Gly Ser Pro Glu Glu Leu Trp Glu Leu Val Ala Ser Gly Thr Asp
       50                  55                  60
   Ala Ile Ser Pro Phe Pro Asp Asp His Gly Trp Asp Gly Asp Ala Leu
   65 70 75 80
   Tyr Asp Pro Asp Pro Glu Ala Ala Gly Arg Thr Tyr Cys Arg Glu Gly
   85 90 95
   Gly Phe Leu Ala Gly Val Gly Asp Phe Asp Ala Ala Phe Phe Gly Ile
   100 105 110
   Ser Pro Arg Glu Ala Leu Ala Met Asp Pro Gln Gln Arg Leu Leu Leu
   115 120 125
   Glu Thr Ser Trp Glu Ala Leu Glu Arg Ala Gly Ile Pro Pro Asp Ser
   130 135 140
   Leu Arg Gly Ser Arg Thr Gly Val Cys Val Gly Ala Trp His Gly Gly
   145 150 155 160
   Tyr Thr Asp Val Val Gly Gln Pro Pro Ala Glu Leu Glu Gly His Leu
   165 170 175
   Leu Thr Gly Gly Val Val Ser Phe Thr Ser Gly Arg Ile Ser Tyr Ala
   180 185 190
   Leu Gly Leu Glu Gly Pro Ala Leu Thr Val Asp Thr Ala Cys Ser Ser
   195 200 205
   Ser Leu Val Ala Leu His Leu Ala Val Arg Ala Leu Arg Gln Gly Glu
   210 215 220
   Cys Asp Leu Ala Leu Ala Gly Gly Ala Thr Val Leu Ala Ser Pro Ala
   225 230 235 240
   Val Phe Val Gln Phe Ser Arg Gln Arg Gly Leu Ala Pro Asp Gly Arg
   245 250 255
   Cys Lys Ala Phe Ala Asp Ser Ala Asp Gly Phe Gly Pro Ala Glu Gly
   260 265 270
   Val Gly Met Leu Val Val Glu Arg Leu Ser Asp Ala Val Arg His Gly
   275 280 285
   Arg Arg Val Leu Ala Leu Val Thr Gly Thr Ala Val Asn Gln Asp Gly
   290 295 300
   Ala Ser Asn Gly Leu Thr Ala Pro Ser Gly Pro Ala Gln Glu Lys Val
   305 310 315 320
   Leu Arg Gln Ala Leu Val Asp Ala Arg Val Thr Ala Ala Asp Val Asp
   325 330 335
   Ala Val Glu Ala His Gly Thr Gly Thr Arg Leu Gly Asp Pro Ile Glu
   340 345 350
   Val Arg Ala Leu Met Asn Val Tyr Gly Ala Gly Arg Pro Ala Asp Arg
   355 360 365
   Pro Leu Trp Leu Gly Ser Leu Lys Ser Asn Ile Gly His Thr Gln Ala
   370 375 380
   Ala Ala Gly Val Gly Gly Val Ile Lys Thr Val Leu Ala Met Arg His
   385 390 395 400
   Gly Val Leu Pro Pro Thr Leu His Val Asp Ala Pro Thr Thr Glu Val
   405 410 415
   Asp Trp Ser Ala Gly Gln Val Ala Leu Leu Arg Ala Glu Thr Pro Trp
   420 425 430
   Pro Asp Thr Gly Arg Pro Arg Arg Ala Gly Val Ser Ser Phe Gly Val
   435 440 445
   Ser Gly Thr Asn Ala His Val Val Leu Glu Gln Ala Pro Gly Pro Ala
   450 455 460
   Ala Ala Pro Ala Gly Asp Ala Pro Pro Ala Glu Thr Arg Pro Val Gly
   465 470 475 480
   Asp Pro Pro Pro Val Val Pro Leu Val Leu Ser Ala Arg Ser Gln Pro
   485 490 495
   Ala Leu Ala Gly Gln Ala Arg Arg Leu Arg Asp Leu Leu Ala Ala Ala
   500 505 510
   Pro Glu Thr Asp Leu Ala Ser Ala Gly Leu Ala Leu Ala Thr Ala Arg
   515 520 525
   Ser Val Phe Asp His Arg Ala Val Val Thr Ala Ala Gly Arg Pro Gln
   530 535 540
   Ala Leu Asp Ala Leu Asp Leu Leu Ala Gly Gly Glu Pro Gly Pro Ala
   545 550 555 560
   Val Thr Thr Gly Val Ala Ala Pro Thr Gly Arg Thr Val Phe Val Phe
   565 570 575
   Pro Gly Gln Gly Thr His Trp Ala Gly Met Gly Ala Asp Leu Leu Asp
   580 585 590
   Gln Ser Pro Val Phe Ala Glu Ser Met Arg Arg Cys Glu Gln Ala Leu
   595 600 605
   Ser Ala His Thr Asp Trp Lys Leu Gly Glu Val Ile Arg Gly Ala Ala
   610 615 620
   Gly Ser Pro Pro Leu Asp Arg Val Asp Val Leu Gln Pro Val Ser Trp
   625 630 635 640
   Ala Val Met Val Ser Leu Ala Gln Val Trp Arg Ser Leu Gly Val Glu
   645 650 655
   Pro Asp Ala Val Val Gly His Ser Gln Gly Glu Ile Ala Ala Ala Val
   660 665 670
   Val Cys Gly Ala Leu Thr Leu Pro Asp Ala Ala Arg Val Val Ala Leu
   675 680 685
   Arg Ser Gln Val Ile Gly Arg Val Leu Ser Gly Arg Gly Gly Met Ala
   690 695 700
   Ser Val Gln Leu Pro Ala Arg Glu Val Ala Gly Arg Leu Ala Ala Trp
   705 710 715 720
   Ala Gly Arg Leu Asp Val Ala Ala Val Asn Gly Pro Gln Ser Thr Val
   725 730 735
   Val Ser Gly Ala Ala Asp Ala Val Thr Glu Leu Val Glu Ala Phe Ala
   740 745 750
   Ala Glu Asp Val Arg Val Arg Arg Ile Pro Val Asp Tyr Ala Ser His
   755 760 765
   Ser Thr Gln Val Asp Arg Leu Arg Ala Glu Leu Leu Thr Val Leu Gly
   770 775 780
   Pro Val Asp Ala Arg Pro Ala Gln Val Pro Phe Tyr Ser Thr Val Gln
   785 790 795 800
   Gly Gly Arg Val Asp Thr Ala Gly Leu Asp Ala Gly Tyr Trp Tyr Arg
   805 810 815
   Asn Leu Arg Gly Gln Val Arg Phe Glu Glu Thr Val Arg Val Leu Leu
   820 825 830
   Asp Asp Gly His Arg Ala Phe Val Glu Ala Ala Ala His Ala Val Leu
   835 840 845
   Val Pro Ala Ile Gln Glu Leu Gly Asp Ser Ala Gly Val Arg Val Val
   850 855 860
   Ala Val Gly Ser Leu Arg Arg Glu Ala Gly Gly Leu Asp Arg Leu Leu
   865 870 875 880
   Ala Ser Ala Ala Glu Ala Phe Thr Gln Gly Val Ala Val Asp Trp Ser
   885 890 895
   Arg Ala Leu Ala Gly Ala Ala Arg Val Ala Val Asp Leu Pro Thr Tyr
   900 905 910
   Ala Phe Gln Arg Gln Arg Tyr Trp Leu Glu Pro Ala Ala Gln Ala Asp
   915 920 925
   Ser Gly Pro Ala Gly Asp Gly Trp Arg Tyr Arg Val Gly Trp Arg Arg
   930 935 940
   Leu Gln Arg Thr Gly Ala Ala Pro Ala Asp Arg Trp Leu Leu Val Thr
   945 950 955 960
   Gly Pro Glu Gln Pro Ala Glu Leu Val Glu Ala Val Arg Asp Ala Leu
   965 970 975
   Thr Ala Arg Gly Ala Glu Val Arg Leu Val Thr Val Glu Pro Thr Ser
   980 985 990
   Thr Asp Arg Ala Ala Cys Ala Ala Leu Leu Thr Ala Ala Gly Ala Gly
   995 1000 1005
   Gly Ala Thr Arg Val Leu Ser Leu Leu Gly Thr Asp Arg Arg Pro
   1010 1015 1020
   His Pro Asp His Pro Ala Val Ser Val Gly Ala Ala Ala Thr Leu
   1025 1030 1035
   Leu Leu Thr Gln Ala Val Ala Asp Ala Leu Pro Ala Ala Arg Leu
   1040 1045 1050
   Trp Val Val Thr Arg Gly Ala Val Ser Val Gly Pro Gly Glu Thr
   1055 1060 1065
   Ala Asp Glu Arg Gln Ala Gln Val Trp Gly Phe Gly Arg Val Ala
   1070 1075 1080
   Ala Leu Glu Leu Pro Arg Thr Trp Gly Gly Leu Val Asp Leu Pro
   1085 1090 1095
   Ala Asp Ala Asp Gly Pro Val Trp Glu Ala Phe Val Asp Val Leu
   1100 1105 1110
   Ala Gly Asp Glu Asp Gln Val Ala Leu Arg Gly Pro Val Gly Tyr
   1115 1120 1125
   Gly Arg Arg Leu Arg Arg Ala Pro Ala Leu Pro Ala Lys Arg Arg
   1130 1135 1140
   Tyr Arg Pro Arg Gly Thr Val Leu Val Thr Gly Gly Thr Gly Ala
   1145 1150 1155
   Leu Gly Ala His Val Ala Arg Arg Leu Ala Ala Gly Gly Ala Ala
   1160 1165 1170
   His Leu Val Leu Thr Ser Arg Arg Gly Ala Asp Ala Pro Gly Ala
   1175 1180 1185
   Ala Gly Leu Val Gly Glu Leu Arg Ala Leu Gly Ala Glu Val Thr
   1190 1195 1200
   Val Ala Val Cys Asp Val Ala Asp Arg Ala Ala Val Ala Ala Leu
   1205 1210 1215
   Leu Ala Gly Leu Pro Ala Asp Ala Pro Leu Ser Ala Val Phe His
   1220 1225 1230
   Thr Ala Gly Val Ala His Ser Met Pro Ile Gly Glu Thr Gly Leu
   1235 1240 1245
   Thr Asp Val Ala Glu Val Phe Ala Gly Lys Val Ala Gly Ala Arg
   1250 1255 1260
   His Leu Asp Glu Leu Thr Arg Gly His Asp Leu Asp Ala Phe Val
   1265 1270 1275
   Leu Tyr Ser Ser Asn Ala Gly Val Trp Gly Ser Ser Gly Gln Ser
   1280 1285 1290
   Ala Tyr Gly Ala Ala Asn Ala Ala Leu Asp Ala Leu Ala Glu Arg
   1295 1300 1305
   Arg Arg Ala Ala Gly Leu Thr Ala Thr Ser Val Ala Trp Gly Leu
   1310 1315 1320
   Trp Gly Ser Gly Gly Met Gly Glu Gly Asp Ala Glu Glu Tyr Leu
   1325 1330 1335
   Ser Arg Arg Gly Leu Arg Pro Met Pro Pro Glu Arg Gly Val Asp
   1340 1345 1350
   Ala Leu Leu Ala Ala Leu Asp Arg Asp Glu Thr Phe Val Ala Val
   1355 1360 1365
   Ala Asp Val Asp Trp Thr Leu Phe Thr Ala Gly Phe Thr Ala Phe
   1370 1375 1380
   Arg Pro Ser Pro Leu Leu Gly Asp Leu Pro Glu Ala Arg Ala Thr
   1385 1390 1395
   Leu Ala Asp Ala Gly Pro Ala Gly Ser Asp Leu Pro Ala Trp His
   1400 1405 1410
   Ala Ala Ala Ser Pro Asp Glu Arg Arg Arg Gly Leu Leu Asp Leu
   1415 1420 1425
   Val Arg Arg Gln Val Ala Ala Val Leu Gly His Pro Gly Pro Glu
   1430 1435 1440
   His Val Gly Pro Asp Ala Ala Phe Arg Glu Ile Gly Phe Asp Ser
   1445 1450 1455
   Leu Thr Ala Val Asp Leu Ala Lys Arg Leu Arg Ala Ala Val Gly
   1460 1465 1470
   Val Pro Leu Ser Ala Thr Leu Val Phe Asp His Pro Thr Ala Thr
   1475 1480 1485
   Ala Val Ala Glu His Leu Ala Gly Leu Leu Gly Pro Ala Pro Ala
   1490 1495 1500
   Gly Gly Asp Pro Arg Glu Ala Glu Val Arg Arg Ala Leu Ala Asp
   1505 1510 1515
   Leu Pro Leu Ala Arg Leu Arg Asp Ala Gly Leu Leu Asp Gly Leu
   1520 1525 1530
   Leu Ala Leu Ala Gly Leu Asp Ala Asp Ala Val Pro Asp Gly Pro
   1535 1540 1545
   Glu Pro Ala Pro Gly Asp Ala Ile Asp Glu Leu Asp Pro Glu Glu
   1550 1555 1560
   Leu Val Arg Arg Val Leu Asp Asn Ala Ser Ser
   1565 1570
<210> 17
   <211> 4725
   <212> DNA
   <213> micromonospora carbonacea subspecies aurantiaca
<400> 17
   atgtcgaacg agcagaagct ccgcgagtac ctgcggttga ccaccaccga gctggccagg 60
   gccaccgacc ggctgcgcgc ggtcgaggcg cgggcgcacg agccgatcgc gatcgtcggc 120
   atggcctgcc ggtaccccgg cggggtcggc tcaccggagg aactgtggga gctggtcgcc 180
   tcgggcacgg acgcgatctc cccgttcccc gacgaccacg gctgggacgg cgacgcgctg 240
   tacgacccgg acccggaggc ggcgggccgc acctactgcc gcgagggcgg gttcctcgcc 300
   ggggtcggcg acttcgacgc cgcgttcttc ggcatctcgc cccgcgaggc gctggccatg 360
   gacccgcagc agcgcctgct gctggagacg tcctgggagg cgctggagcg ggccgggatc 420
   cccccggact cgctgcgcgg cagccgtacc ggggtgtgcg tcggggcgtg gcacggcggc 480
   tacaccgacg tcgtcgggca gcccccggcg gaactggagg gccacctgct gaccggcggg 540
   gtggtcagct tcacctcggg gcggatctcg tacgcgctgg gcctggaggg gcccgcgttg 600
   acggtggaca ccgcctgctc gtcctcgctg gtggccctgc acctggcggt gcgggccctg 660
   cggcagggcg agtgcgacct ggcgttggcc ggcggggcga cggtgctggc cagcccggcg 720
   gtgttcgtgc agttctcgcg gcagcggggg ctggccccgg acggccggtg caaggcgttc 780
   gccgactcgg cggacgggtt cgggccggcc gagggggtcg gcatgctggt cgtggagcgg 840
   ctgtcggacg ccgtccgcca cgggcgccgg gtgctggccc tggtcaccgg cacggcggtc 900
   aaccaggacg gggcgagcaa cggcctcacc gcccccagcg gcccggcgca ggagaaggtg 960
   ctgcgccagg cgctcgtgga cgcccgggtg acggccgccg acgtcgacgc ggtcgaggcg 1020
   cacggcaccg gcacccggct cggcgacccg atcgaggtgc gggccctgat gaacgtgtac 1080
   ggtgccggcc ggcccgccga ccgtccgctc tggctcggtt cgctgaagtc caacatcggc 1140
   cacacccagg cggcggccgg ggtcggcggg gtcatcaaga cggtgctggc gatgcggcac 1200
   ggcgtcctgc cgcccaccct gcacgtggac gccccgacca ccgaggtcga ctggtccgcc 1260
   ggccaggtgg ccctgctgcg ggcagagaca ccgtggccgg acacgggtcg cccgcgccgc 1320
   gccggggtct cctccttcgg ggtgagcggc accaacgcgc acgtggtgct ggagcaggcc 1380
   cctgggcccg ccgccgcccc ggcgggtgac gccccgcccg ccgagacccg gcccgtcggc 1440
   gacccgccgc cggtcgtacc gctggtgttg tccgccaggt cgcagccggc gctggccggg 1500
   caggcccgcc ggctgcgcga cctgctggcc gcagcgccgg agaccgacct cgccagcgcc 1560
   ggactcgccc tggccaccgc gcggtcggtg ttcgaccacc gggcggtggt gacggccgcc 1620
   gggcgaccgc aggcgctcga cgcgctcgac ctgctggccg gcggcgaacc cggaccggcg 1680
   gtcacgaccg gcgtcgccgc ccccaccggg cgcaccgtgt tcgtctttcc cgggcagggg 1740
   acgcactggg ccggcatggg tgccgacctg ctcgaccagt caccggtgtt cgccgagtcg 1800
   atgcgacggt gcgagcaggc gctgtcggcg cacaccgact ggaagctcgg cgaggtgatc 1860
   cggggcgcgg ccggcagccc gccgctggac cgcgtggacg tgctccagcc cgtctcctgg 1920
   gcggtgatgg tgtcgctggc gcaggtgtgg cggtcgctcg gcgtcgagcc ggacgcggtg 1980
   gtcggccatt cccagggcga gatcgccgcc gcggtggtct gcggcgcgct gaccctgccg 2040
   gacgcggccc gggtggtcgc gctgcggtcc caggtcatcg gtcgggtgct ctccggtcgc 2100
   ggcggcatgg cgtccgtcca gctgccggcc cgggaggtcg cggggcggct ggccgcctgg 2160
   gcgggccggc tcgacgtcgc ggccgtcaac gggccacagt cgaccgtcgt gtccggtgcc 2220
   gccgacgcgg tcaccgaact ggtcgaggcg ttcgcggccg aggacgtccg ggtgcggcgg 2280
   atcccggtgg actacgcgtc ccactcgacg caggtggacc ggctgcgcgc cgagctgctc 2340
   accgtcctgg gcccggtcga cgcccgtccg gcgcaggtgc ccttctactc gacggtgcag 2400
   ggcgggcgcg tcgacactgc cggcctggac gccggctact ggtaccgcaa cctgcggggg 2460
   caggtccgct tcgaggagac cgtgcgggtg ctgctcgacg acgggcaccg cgccttcgtc 2520
   gaggccgccg cgcacgccgt cctcgtaccc gcgatccagg agctggggga cagcgccggc 2580
   gtccgggtgg tggccgtggg gtcgctgcgc cgggaggcgg gcggcctgga ccggctcctg 2640
   gcctcggcgg ccgaggcgtt cacccagggg gtggccgtgg actggccccg ggctctggcc 2700
   ggggccgcgc gcgtcgccgt ggacctgccc acgtacgcgt tccagcggca acgctactgg 2760
   ctggagcccg ccgcgcaggc ggactccggc ccggccgggg acggctggcg ctaccgggtc 2820
   ggctggcggc ggcttcagcg caccggcgcc gcgccggccg accggtggct gctggtgacc 2880
   ggcccggagc agccggcgga gctggtcgag gcggtgcgcg acgcgctcac cgcgcggggc 2940
   gccgaggtgc gcctggtgac cgtcgagccg accagcaccg accgggccgc gtgcgcggcg 3000
   ttgctcaccg cggccggtgc gggcggggcg acccgggtgc tgtcgctgct cggcaccgat 3060
   cgtcgcccgc accccgacca cccggccgtg tccgtcggcg ccgccgcgac gttgctgctg 3120
   acccaggccg tcgccgacgc cctgccggcc gcccggctgt gggtcgtcac ccggggcgcg 3180
   gtctccgtcg ggcccggcga gaccgccgac gagcgccagg cgcaggtctg ggggttcggc 3240
   cgggtcgcgg ccctcgaact gccccgcacg tggggcgggc tcgtcgacct gcccgccgac 3300
   gcggacggcc cggtgtggga ggcgttcgtg gacgtgctgg ccggggacga ggaccaggtc 3360
   gcgctgcgcg gcccggtcgg gtacggtcgc cggctccggc gcgcccccgc gctacccgcg 3420
   aagcggcggt accggcccag gggcaccgtc ctggtcaccg gcggcaccgg cgcgctcggc 3480
   gcgcacgtgg cccggcggtt ggccgccggc ggggccgcgc acctcgtgct caccagccgg 3540
   cgcggggccg acgcccccgg tgcggccggg ctggtcgggg aactccgggc gctgggcgcc 3600
   gaggtgaccg tcgcggtctg cgacgtcgcc gaccgggccg ccgtggcggc gctgctcgcc 3660
   gggctgcccg ccgacgcgcc gctgagcgcg gtcttccaca ccgcgggcgt ggcgcactcg 3720
   atgccgatcg gcgagaccgg gctcaccgac gtcgccgagg tgttcgccgg gaaggtcgcc 3780
   ggagcccgcc acctcgacga actcacccgg gggcacgacc tggacgcgtt cgtcctgtac 3840
   tcgtcgaacg cgggcgtgtg gggcagcagc gggcagagcg cgtacggggc ggccaacgcg 3900
   gccctcgacg cgctcgccga acggcggcgc gccgccgggc tgaccgccac ctccgtcgcc 3960
   tggggcctgt ggggctccgg gggcatgggc gagggcgacg ccgaggagta cctgagccgc 4020
   cggggcctgc ggccgatgcc tcccgagcgt ggcgtggacg ccctcctggc cgccctggac 4080
   cgggacgaga ccttcgtcgc cgtcgccgac gtggactgga cgctgttcac ggccgggttc 4140
   accgcgttcc ggcccagccc gctgctcggc gacctcccgg aggcccgcgc gacgctggcc 4200
   gacgccggac ccgcgggctc cgacctgccg gcctggcacg ccgccgcgag ccccgacgaa 4260
   cgccgccggg gcctgctcga cctggtacgc cggcaggtcg ccgccgtcct cggccacccg 4320
   gggcccgagc acgtcggccc cgacgccgcg ttccgggaga tcggattcga ctcgctgacc 4380
   gccgtcgacc tggccaagcg gctcagggcg gcggtcggcg tgccgctgtc cgccaccctc 4440
   gtcttcgacc accccaccgc gacggcggtc gccgagcacc tggccgggct gctcggtccc 4500
   gcgccggccg gcggcgaccc gcgcgaggcc gaggtgcgcc gggccctggc cgacctgccg 4560
   ctggcccggc tgcgggacgc cggcctactg gacggcctgc ttgcgcttgc ggggctggac 4620
   gccgacgcgg tgccggacgg gcccgagccg gctcccggcg acgccatcga cgaactcgat 4680
   ccagaggagc tggtgcgccg ggtgctggac aacgccagct cctga 4725
<210> 18
   <211> 1784
   <212> PRT
   <213> micromonospora carbonacea subspecies aurantiaca
<400> 18
   Met Val Met Pro Pro Asp Lys Val Ile Glu Ala Leu Arg Val Ser Val
   1               5                   10                  15
   Lys Glu Thr Glu Arg Leu Arg Arg Gln Asn His Glu Leu Leu Ala Ala
               20                  25                  30
   Leu His Gly Pro Ile Ala Val Val Gly Met Ala Cys Arg Tyr Pro Gly
           35                  40                  45
   Gly Val Ser Ser Pro Glu Asp Leu Trp Arg Leu Val Glu Thr Gly Thr
       50                  55                  60
   Asp Ala Ile Gly Gly Phe Pro Thr Asp Arg Gly Trp Asp Val Asp Ala
   65                  70                  75                  80
   Val Tyr Asp Pro Asp Pro Glu Ser Arg Asn Thr Thr Tyr Cys Arg Glu
                   85                  90                  95
   Gly Gly Phe Leu Ala Gly Ala Gly Asp Phe Asp Ala Ala Phe Phe Gly
               100                 105                 110
   Val Ser Pro His Glu Ala Val Val Met Asp Pro Gln Gln Arg Leu Leu
           115                 120                 125
   Leu Glu Val Ser Trp Glu Ala Leu Glu Arg Ser Gly Thr Asp Pro His
       130                 135                 140
   Ser Leu Arg Gly Ser Arg Thr Gly Val Tyr Val Gly Ala Ala His Gln
   145                 150                 155                 160
   Gly Tyr Ala Val Asp Ala Gly Gln Val Pro Glu Gly Ala Glu Gly Phe
                   165                 170                 175
   Arg Leu Thr Gly Ser Ala Asp Ala Val Leu Ser Gly Arg Ile Ser Tyr
               180                 185                 190
   Leu Leu Gly Leu Glu Gly Pro Ala Leu Thr Val Glu Thr Ala Cys Ser
           195                 200                 205
   Ser Ser Leu Val Ala Val His Leu Ala Val Gln Ala Leu Arg Arg Gly
       210                 215                 220
   Glu Cys Gly Leu Ala Leu Ala Gly Gly Val Ala Val Met Pro Asp Pro
   225                 230                 235                 240
   Ala Ala Phe Val Glu Phe Ser Arg Gln Arg Gly Leu Ala Ala Asp Gly
                   245                 250                 255
   Arg Cys Arg Ala Phe Gly Ala Gly Ala Asp Gly Thr Gly Trp Ala Glu
               260                 265                 270
   Gly Val Gly Val Leu Val Leu Gln Arg Leu Ser Asp Ala Val Arg Asp
   275 280 285
   Gly Arg Trp Val Leu Gly Val Ile Arg Gly Ser Ala Val Asn Gln Asp
   290 295 300
   Gly Ala Ser Asn Gly Leu Thr Ala Pro Ser Gly Pro Ala Gln Gln Arg
   305 310 315 320
   Val Ile Arg Gln Ala Leu Thr Asp Ala Arg Leu Gly Ala Asp Gln Ile
   325 330 335
   Asp Ala Val Glu Ala His Gly Thr Gly Thr Arg Leu Gly Asp Pro Ile
   340 345 350
   Glu Ala Gln Ala Leu Ile Ala Ala Tyr Gly Ala Asp Arg Thr Pro Asp
   355 360 365
   Arg Pro Leu Trp Leu Gly Ser Leu Lys Ser Asn Ile Gly His Ala Gln
   370 375 380
   Ala Ala Ala Gly Val Gly Gly Leu Ile Lys Met Leu Leu Ala Met Arg
   385 390 395 400
   Ala Gly Thr Leu Pro Pro Thr Leu His Ala Asp Val Pro Thr Pro Leu
   405 410 415
   Val Asp Trp Ser Ala Gly Val Val Arg Leu Ser Thr Gly Val Val Pro
   420 425 430
   Trp Pro Ala Leu Pro Gly Ala Pro Arg Arg Ala Gly Ile Ser Ala Phe
   435 440 445
   Gly Val Ser Gly Thr Asn Ala His Val Ile Val Glu Gln Pro Pro Pro
   450 455 460
   Val Pro Val Asp Asp Pro Ala Pro Pro Thr Arg Thr Leu Pro Leu Val
   465 470 475 480
   Pro Trp Val Leu Ser Gly Arg Thr Glu Ala Ala Leu Arg Ala Gln Ala
   485 490 495
   Asp Arg Leu Arg Thr His Leu Ala Ala His Pro Asp Ala Asp Pro Leu
   500 505 510
   Asp Val Gly Phe Ser Leu Ala Thr Ser Arg Ala Ala Leu Glu His Arg
   515 520 525
   Ala Val Leu Val Ala Ala Asp Arg Asp Gly Leu Leu Arg Leu Val Asp
   530 535 540
   Ala Leu Ala Ala Gly Glu Pro Ala Ala Gly Leu Ile Arg Gly Thr Val
   545 550 555 560
   Arg His Asp Arg Arg Thr Gly Phe Leu Phe Ala Gly Gln Gly Gly Gln
   565 570 575
   Arg Val Gly Met Ala Arg Glu Leu Tyr Glu Ala Phe Pro Ala Phe Ala
   580 585 590
   Asp Ala Leu Asp Gln Leu Ala Ala Arg Leu Asp Arg His Leu Asp Arg
   595 600 605
   Pro Leu Leu Arg Val Leu Phe Ala Glu Pro Gly Ser Asp Asp Ala Arg
   610 615 620
   Leu Leu Asp Gly Thr Arg Tyr Ala Gln Ala Ala Leu Phe Ala Val Glu
   625 630 635 640
   Val Ala Leu Phe Arg Leu Val His Gly Trp Gly Val Arg Pro Asp Val
   645 650 655
   Leu Leu Gly His Ser Val Gly Glu Leu Ala Ala Ala His Val Ala Gly
   660 665 670
   Val Leu Asp Val Asp Asp Ala Cys Glu Leu Val Ala Ala Arg Gly Arg
   675 680 685
   Leu Met Gly Glu Leu Pro Ser Gly Gly Ala Met Val Ala Val Arg Ala
   690 695 700
   Thr Glu Glu Glu Val Gly Pro Leu Leu Asp Gly Gln Arg Val Ala Val
   705 710 715 720
   Ala Ala Val Asn Gly Pro Arg Ser Val Val Val Ser Gly Asp Glu Glu
   725 730 735
   Ala Val Leu Ala Val Ala Ala Arg Cys Ala Ala Leu Gly His Arg Thr
   740 745 750
   Arg Arg Leu Asn Val Ser His Ala Phe His Ser Pro His Val Glu Ala
   755 760 765
   Met Leu Glu Pro Phe Arg Arg Val Ala Arg Gly Leu Thr Tyr His Ala
   770 775 780
   Pro Thr Ile Pro Val Val Ser Asn Ala Thr Gly Arg Leu Ala Thr Ala
   785 790 795 800
   Asp Ala Leu Arg Asp Pro Gly Tyr Trp Val Arg His Val Arg Gln Pro
   805 810 815
   Val Arg Phe Arg Asp Gly Val Arg Ala Ala Arg Asp Gln Gly Ala Thr
   820 825 830
   Ala Phe Val Gly Leu Gly Pro Asp Gly Val Leu Cys Ala Leu Ala Glu
   835 840 845
   Glu Cys Leu Gly Pro Thr Gly Asp Val Leu Leu Leu Pro Val Leu Arg
   850 855 860
   Pro Gly Arg Pro Glu Pro Ala Thr Leu Leu Ala Ala Leu Ala Gly Ala
   865 870 875 880
   Tyr Ala Gly Gly Ala Glu Met Asp Trp Ser Arg Val Phe Ala Gly Thr
                   885                 890                 895
   Gly Ala Arg Arg Val Glu Leu Pro Thr Tyr Ala Phe Gln His Arg Arg
               900                 905                 910
   Tyr Trp Leu Ala Pro Gly Pro Pro Ser Ala Arg Arg Asp Asp Ala Trp
           915                 920                 925
   Arg Tyr Arg Ile Ala Trp Arg Pro Leu Pro Thr Val Pro Ala Ala Ala
       930                 935                 940
   Gly Thr Glu Thr Val Ala Gly Ala Trp Leu Leu Val Val Pro Ala His
   945                 950                 955                 960
   Asp Gly Val Ala Ser Leu Ala Asp Ala Ala Glu Arg Ala Val His Arg
                   965                 970                 975
   Gly Gly Ala Thr Val Thr Arg Leu Thr Val Asp Ala Ala Asp Val Asp
               980                 985                 990
   Arg Asp Thr Leu Ala Ala Val Leu Thr Glu Ala Ala Ala Asp Ala Asp
           995                 1000                1005
   Gly Gly Pro Asp Gly Val Leu Cys Leu Leu Gly Leu Asp Asp Arg
       1010                1015                1020
   Ala His Pro Arg Ser Ala Ser Val Pro Arg Gly Val Leu Ala Thr
       1025                1030                1035
   Leu Ser Leu Ala Gln Ala Leu Thr Asp Leu Gly Ala Ser Ala Arg
       1040                1045                1050
   Leu Trp Cys Val Thr Arg Gly Ala Val Ala Val Thr Pro Gly Glu
       1055                1060                1065
   Ser Pro Ser Val Ala Gly Ala Gln Leu Trp Gly Phe Gly Arg Val
       1070                1075                1080
   Ala Ala Leu Glu Leu Pro Arg Ser Trp Gly Gly Leu Val Asp Leu
       1085                1090                1095
   Pro Val Asp Pro Asp Asp Arg Asp Trp Asp Leu Leu Arg Arg Ala
       1100                1105                1110
   Leu Arg Gly Pro Glu Asp Gln Val Ala Val Arg Gly Ala Val Gly
       1115                1120                1125
   Tyr Ala Arg Arg Leu Val Pro Ala Pro Ala Pro Arg Ala Glu Arg
       1130                1135                1140
   Ala Trp Arg Pro Arg Gly Thr Val Leu Val Thr Gly Gly Thr Gly
       1145                1150                1155
   Ala Leu Gly Ala His Thr Ala Arg Trp Leu Ala Arg Asn Gly Ala
       1160                1165                1170
   Thr His Leu Val Leu Thr Ser Arg Arg Gly Gly Asn Ala Pro Gly
   1175 1180 1185
   Val Ala Ala Leu Arg Ala Glu Leu Val Thr Leu Gly Ala Glu Val
   1190 1195 1200
   Thr Val Val Ala Cys Asp Val Ala Asp Arg Glu Ala Val Ala Gly
   1205 1210 1215
   Leu Leu Ala Gly Ile Pro Arg Ala Ala Pro Leu Thr Ala Val Phe
   1220 1225 1230
   His Ala Ala Gly Val Pro Gln Val Thr Pro Leu His Glu Thr Thr
   1235 1240 1245
   Pro Glu Leu Phe Ala Gln Val Cys Ala Gly Lys Val Ala Gly Ala
   1250 1255 1260
   Val His Leu His Glu Leu Ala Gly Asp Leu Asp Ala Phe Val Thr
   1265 1270 1275
   Phe Ala Ser Ala Ala Gly Val Trp Gly Ser Gly Gly Gln Cys Ala
   1280 1285 1290
   Tyr Ala Ala Ala Asn Ala Ala Leu Asp Ala Leu Ala Glu Arg Arg
   1295 1300 1305
   Arg Ala Ala Gly Leu Pro Ala Thr Ser Val Ala Trp Gly Val Trp
   1310 1315 1320
   Gly Gly Pro Gly Met Gly Ala Gly Ala Gly Glu Glu Tyr Leu Arg
   1325 1330 1335
   Arg Arg Gly Val Arg Ala Met Pro Pro Ala Ala Ala Leu Ala Ala
   1340 1345 1350
   Leu Gly Arg Ile Leu Asp Ala Asp Glu Thr Gly Val Thr Val Ser
   1355 1360 1365
   Asp Thr Glu Trp Gly Arg Phe Ala Ser Gly Phe Ala Ala Ala Arg
   1370 1375 1380
   Pro Ala Pro Leu Leu Ala Glu Leu Pro Gly Gly Asp Val Asp Pro
   1385 1390 1395
   Ala Gly Pro Ala His Arg Ala Gln Pro Pro Val Pro Arg Pro Ala
   1400 1405 1410
   Pro Ala Ala Thr Asp Arg Pro Gly Leu Leu Ala Leu Val Arg Ala
   1415 1420 1425
   Glu Ala Ala Gly Val Leu Gly His Asp Gly Ala Asp Asp Val Pro
   1430 1435 1440
   Ala Asp Ala Glu Phe Ser Ala Leu Gly Phe Asp Ser Leu Ala Ala
   1445 1450 1455
   Val Gln Leu Arg Arg Arg Leu Ala Glu Ala Thr Gly Leu Ser Leu
   1460 1465 1470
   Ser Ala Pro Val Leu Phe Asp His Arg Thr Pro Asp Ala Leu Ala
   1475 1480 1485
   Ala His Leu His Gly Leu Leu Thr Gly Ala Ala Gly Gly Pro Pro
   1490 1495 1500
   Ala Pro Ala Ala Gly Ser Ala Leu Val Glu Met Tyr Arg Arg Ala
   1505 1510 1515
   Val Ala Thr Gly Arg Ala Ala Glu Ala Val Glu Val Leu Gly Thr
   1520 1525 1530
   Val Ala Thr Phe Arg Pro Val Phe Arg Ser Pro Asp Glu Leu Gly
   1535 1540 1545
   Glu Pro Pro Ala Leu Val Pro Leu Gly Thr Gly Ala Gly Gly Pro
   1550 1555 1560
   Ala Leu Val Cys Cys Ala Gly Thr Ala Ala Ala Ser Gly Pro Arg
   1565 1570 1575
   Glu Phe Thr Ala Phe Ala Ala Ala Leu Ala Gly Leu Arg Asp Val
   1580 1585 1590
   Thr Val Leu Pro Gln Thr Gly Phe Leu Pro Gly Glu Pro Leu Pro
   1595 1600 1605
   Ala Gly Leu Asp Val Leu Leu Asp Ala Gln Ala Asp Ala Val Leu
   1610 1615 1620
   Ala His Cys Ala Gly Gly Pro Phe Val Leu Val Gly His Ser Ala
   1625 1630 1635
   Gly Ala Asn Met Ala His Ala Leu Thr Val Arg Leu Glu Ala Arg
   1640 1645 1650
   Gly Ala Asp Pro Ala Ala Leu Val Leu Met Asp Ile Tyr Thr Pro
   1655 1660 1665
   Ala Ala Pro Gly Ala Met Gly Val Trp Arg Glu Glu Met Leu Ala
   1670 1675 1680
   Trp Val Ala Glu Arg Ser Val Val Pro Val Asp Asp Thr Arg Leu
   1685 1690 1695
   Thr Ala Met Gly Ala Tyr His Arg Leu Leu Leu Asp Trp Ala Pro
   1700 1705 1710
   Arg Pro Thr Arg Ala Pro Val Leu His Leu Tyr Ala Gly Glu Pro
   1715 1720 1725
   Ala Gly Ala Trp Pro Asp Pro Arg Gln Asp Trp Arg Ser Arg Phe
   1730 1735 1740
   Asp Gly Ala His Thr Ser Ala Glu Val Pro Gly Thr His Phe Ser
   1745 1750 1755
   Met Met Thr Glu His Ala Pro Val Thr Ala Ala Thr Val His Lys
   1760 1765 1770
   Trp Leu Asp Glu Val Cys Pro Pro Arg Val Pro
   1775 1780
<210> 19
   <211> 5355
   <212> DNA
   <213> micromonospora carbonacea subspecies aurantiaca
<400> 19
   atggtcatgc cccccgacaa ggtgatcgag gcgctgcgtg tctccgtcaa ggagacggag 60
   cggctgcgcc ggcagaacca cgagctgctc gccgccctgc acgggccgat cgccgtcgtg 120
   ggcatggcct gccgctaccc gggcggggtg tcctctccgg aggacctgtg gcggctggtc 180
   gagacgggca cggacgcgat cggcggcttc cccaccgacc gtggctggga cgtcgacgcc 240
   gtgtacgacc cggatcctga gtcgcggaac accacctact gccgggaggg cgggttcctg 300
   gccggggcag gagacttcga cgccgcgttc ttcggggtgt cgccgcacga ggccgtggtc 360
   atggaccccc agcagcggct gcttctggag gtgtcctggg aggcgctgga gcggtccggg 420
   accgacccgc acagcctgcg cggctcgcgc accggggtct acgtcggtgc ggcccaccag 480
   gggtacgcgg tcgacgccgg tcaggtgccg gagggcgcgg aggggttccg gctgaccggc 540
   agcgccgacg ccgtcctgtc cggacggatc tcgtacctgc tcgggctgga gggtccggcc 600
   ctgaccgtcg agacggcctg ctcgtcctcg ctggtggcgg tgcacctcgc ggtgcaggcg 660
   ctgcgccggg gcgagtgcgg gctggcactg gccggcgggg tcgccgtgat gcccgacccg 720
   gcggcattcg tggagttctc ccggcagcgg ggcctcgcgg cggacgggcg ctgccgggcg 780
   ttcggggcgg gcgcggacgg caccggctgg gcggagggcg tcggtgtgct ggtcctgcaa 840
   cggctctccg acgcggtgcg cgacggccgc tgggtgctgg gcgtgatccg gggttcggcc 900
   gtcaaccagg acggggccag caacgggctg accgccccga gcggccccgc ccagcagcgg 960
   gtcatccggc aggcgctgac cgacgcccgg ctcggcgccg accagatcga cgcggtcgag 1020
   gcgcacggca cgggcacccg gctcggcgac ccgatcgagg cgcaggcgct gatcgccgcc 1080
   tacggcgccg accggacccc ggaccggccg ctctggctcg gctcgttgaa gtcgaacatc 1140
   gggcacgccc aggcggcggc cggcgtcggc ggcctgatca agatgctcct ggcgatgcgg 1200
   gccgggacgc tcccacccac cctgcacgcc gacgtcccga ccccgctggt cgactggtcc 1260
   gccggtgtcg tccggctgtc gaccggggtg gtgccctggc ccgcgttgcc cggggcgccc 1320
   cgcagggccg ggatctccgc gttcggggtg agcggcacca acgcgcacgt gatcgtcgag 1380
   cagccgccgc cggtcccggt cgacgacccg gcgccaccca cgaggaccct gccgctggtg 1440
   ccgtgggtgc tctccggccg gacggaggcg gcgctgcgcg cccaggcgga ccggttgcgt 1500
   acgcacctgg cggcgcaccc cgacgcggac ccgctggacg tgggattctc cctggccacc 1560
   agccgggccg cgctggagca ccgggccgtg ctggtggccg ccgaccgcga cggcctgctc 1620
   cgcctcgtcg acgcgctggc cgccggcgag ccggcggcgg gcctgatccg gggcacggta 1680
   cgtcacgatc gccggaccgg gttcctcttc gccgggcagg gcggccagcg cgtcgggatg 1740
   gcgcgcgaac tgtacgaggc gttccccgcc ttcgccgacg ccctggacca gctcgccgcc 1800
   cggctggacc ggcacctcga tcgtccgctg ctgcgggtgc tgttcgccga gccggggtcg 1860
   gacgacgccc ggctgctcga cggcacccgg tacgcgcagg ccgccctctt cgccgtcgag 1920
   gtggcgttgt tccgactggt ccacggctgg ggggtccggc ccgacgtgct gctcggccac 1980
   tcggtgggcg agctggcggc cgcgcacgtg gccggcgtac tcgacgtgga cgacgcgtgc 2040
   gagctggtcg cggcgcgggg ccggctgatg ggggagctgc cgtcgggcgg cgcgatggtg 2100
   gcggtccggg ccaccgagga ggaggtcggg cccctgctcg acgggcagcg ggtcgcggtg 2160
   gcggcggtca acggcccgcg ctcggtcgtg gtctccggcg acgaggaggc ggtgctggcc 2220
   gtggccgccc ggtgcgccgc cctcggccac cggacgcgac gcctcaacgt cagccacgcg 2280
   ttccactccc cgcacgtgga ggcgatgctg gagccgttcc ggcgggtggc gcggggcctg 2340
   acgtaccatg ccccgacgat cccggtggtg tcgaacgcga cgggccggct cgccaccgcc 2400
   gacgcgctgc gcgaccccgg ttactgggtc cggcacgtcc gccagcccgt ccggttccgg 2460
   gacggggtgc gggccgcccg cgaccagggg gccaccgcct tcgtcgggct cggcccggac 2520
   ggggtgctgt gcgcgttggc cgaggagtgc ctcgggccca ccggcgacgt gctgctgctg 2580
   ccggtgctgc gccccggtcg gccggagccc gccaccctgc tggccgccct ggccggggcg 2640
   tacgccggcg gcgcggaaat ggactggtcc cgggtgttcg cgggcaccgg cgcgcgcagg 2700
   gtcgagctgc ccacgtacgc cttccagcac cggcgctact ggctggcgcc gggcccgccg 2760
   tcggcccgcc gcgacgacgc ctggcggtac cggatcgcct ggcggcccct gccgaccgtg 2820
   cccgccgccg ccgggaccga gacggtggcc ggggcgtggt tgctggtggt ccccgcccac 2880
   gacggcgtcg cgtcgctcgc cgacgccgcc gagcgggccg tgcaccgggg cggggccacg 2940
   gtcacccggc tgacggtgga cgccgccgac gtggaccggg acaccctcgc cgccgtgctg 3000
   accgaggccg ccgccgacgc ggacggcggg ccggacgggg tgctctgcct gctgggcctc 3060
   gacgaccggg cacatccccg gtccgcctcg gtgccccgcg gggtgctggc gaccctgtcc 3120
   ctcgcccagg ccctgaccga cctgggggcc tccgcgcggc tgtggtgcgt gacccggggg 3180
   gcggtcgccg tgacgcccgg cgagtccccg tcggtcgccg gagcccagtt gtggggcttc 3240
   ggccgcgtgg ccgcgctcga actcccccgg tcctggggcg gcctggtgga cctgccggtc 3300
   gacccggacg accgggactg ggacctgctg cggcgcgcgc tgcgcggccc ggaggaccag 3360
   gtcgcggtcc ggggggcggt cgggtacgcc cggcggctgg tccccgcgcc cgcgccccgg 3420
   gccgagcggg cctggcgtcc gcgcggcacg gtcctggtga ccggcggtac gggcgcgctc 3480
   ggcgcgcaca cggcccgctg gctggcgcgc aacggcgcca cgcacctcgt cctcaccagc 3540
   cgccggggcg ggaacgcccc cggggtcgcc gcgctgcggg cggaactggt cacgctcggt 3600
   gccgaggtga ccgtggtcgc ctgcgacgtc gccgaccggg aggccgtggc cggcctgctc 3660
   gccgggattc cccgcgccgc tccgctcacc gccgtgttcc acgcggcggg cgtgccccag 3720
   gtgacgccgc ttcacgagac gaccccggag ttgttcgcgc aggtctgcgc aggcaaggtc 3780
   gccggggcgg tgcacctgca cgagttggcc ggtgacctgg acgccttcgt caccttcgcc 3840
   tccgccgccg gggtgtgggg cagcggcggg cagtgcgcgt acgctgcggc caacgccgcc 3900
   ctcgacgcgc tcgccgagcg tcgtcgcgcc gcagggctgc ccgcgacctc cgtcgcctgg 3960
   ggggtctggg gcgggcccgg catgggggcg ggcgcggggg aggagtacct gcgccgccgg 4020
   ggcgtccggg cgatgccccc ggcagccgcc ctcgccgccc tcgggcggat cctggacgcc 4080
   gacgagaccg gggtgacggt ctccgacacc gagtggggcc ggttcgcgtc cggcttcgcc 4140
   gccgcgcgtc ccgccccgct gctcgccgag ctgccgggcg gggacgtcga tccggccggc 4200
   ccggcgcacc gggcgcagcc gcccgtcccc cgaccggccc cggcagccac cgaccgcccc 4260
   gggctgctgg cgctggtccg cgccgaggcc gccggggtgc tggggcacga cggtgccgac 4320
   gacgttccgg ccgacgcgga gttctccgcc ctcggcttcg actcgctcgc cgccgtccag 4380
   ctgcgccgcc ggctcgccga ggccaccggc ctgagcctct cggccccggt tctgttcgac 4440
   caccgcaccc ctgacgcgct cgccgcgcac ctgcacggcc tgctcaccgg cgcggcgggc 4500
   gggccacccg cgccggccgc cgggagcgcc ctggtcgaga tgtaccggcg ggccgtcgcc 4560
   accggccgcg ccgccgaggc ggtggaggtg ctcggcaccg tcgccacgtt ccggccggtg 4620
   ttccggtccc cggacgaact gggcgagcca ccggccctcg tcccgctcgg caccggggcg 4680
   gggggacccg cgctggtctg ctgcgcgggc acggccgcgg cgtccggccc ccgcgagttc 4740
   acggcgttcg ccgccgcgct ggccggtctc cgggacgtca ccgtccttcc gcagaccggc 4800
   ttcctgcccg gcgagccgct gcccgccggg ctggacgtgc tgctcgacgc ccaggccgac 4860
   gccgtcctgg cccactgcgc cgggggaccc ttcgtcctgg tcggccactc ggccggggcg 4920
   aacatggcgc acgcgctgac ggtccgcctg gaggcgcggg gcgcggaccc cgccgcgctg 4980
   gtgctgatgg acatctacac gcccgccgcc ccgggggcga tgggggtgtg gcgcgaggag 5040
   atgctggcct gggtcgccga gcggtccgtc gtccccgtcg acgacacgcg gctgaccgcg 5100
   atgggcgcct atcaccggct gctcctggac tgggcgcccc ggccgacccg ggcacccgtg 5160
   ctgcacctgt atgccggtga accggcgggc gcctggccgg atccccggca ggactggcgt 5220
   tcgcgcttcg acggcgcgca caccagcgcc gaggtgcccg gcacccactt ctcgatgatg 5280
   accgagcacg cccccgtcac cgccgcgacc gtgcacaagt ggctcgacga ggtgtgcccg 5340
   ccccgcgttc cgtga 5355
<210> 20
   <211> 464
   <212> PRT
   <213> micromonospora carbonacea subspecies aurantiaca
<400> 20
   Val Thr Arg Thr Pro Gly Pro Ser Arg Arg Val Arg Arg Gln Gln Glu
   1 5 10 15
   Arg Lys Arg Met Ile Thr Val Pro Pro Asp Gly Asp Pro Ala Thr Trp
   20 25 30
   Ala Arg Arg Leu Gln Leu Thr Arg Ala Ala Gln Trp Phe Ala Gly Asn
   35 40 45
   His Gly Asp Pro Tyr Ala Leu Ile Leu Arg Ala Glu Thr Asp Asp Pro
   50 55 60
   Thr Pro Tyr Glu Gln Arg Val Ala Ala Gln Pro Leu Phe Arg Ser Glu
   65 70 75 80
   Gln Leu Asp Thr Trp Val Thr Gly Asp Ala Ala Leu Ala Arg Glu Val
   85 90 95
   Leu Thr Asp Asp Arg Phe Gly Trp Leu Thr Arg Ala Gly Gln Arg Pro
   100 105 110
   Ala Glu Arg Thr Leu Pro Leu Ala Gly Thr Ala Leu Asp His Gly Pro
   115 120 125
   Glu Ala Arg Arg Arg Leu Asp Ala Leu Ala Gly Phe Gly Gly Pro Val
       130                 135                 140
   Leu Arg Ala Asp Ala Ala Gly Ala Arg Thr Arg Val Val Glu Thr Thr
   145                 150                 155                 160
   Ala Val Leu Leu Asp Gly Ile Gly Glu Arg Phe Asp Leu Ala Val Leu
                   165                 170                 175
   Ala Arg Arg Leu Val Ala Ala Val Leu Ala Asp Leu Leu Gly Val Pro
               180                 185                 190
   Ala Ala Arg Arg Gly Arg Phe Ala Glu Ala Leu Ala Ala Ala Gly Arg
           195                 200                 205
   Thr Leu Asp Ser Arg Leu Cys Pro Gln Thr Val Ala Thr Ala Leu Ala
       210                 215                 220
   Thr Val Ala Ala Thr Ala Glu Leu Thr Asp Leu Leu Gly Glu Val Pro
   225                 230                 235                 240
   Pro Pro Pro Ser Leu Ser Pro Ser Ala Ala Gly Ser Gly Pro Pro Arg
                   245                 250                 255
   Pro Ser Ala Ala Gly Ser Trp Pro Pro Leu Pro Ala Asp Asp Arg Thr
               260                 265                 270
   Ala Ala Ala Leu Ala Leu Ala Val Gly Thr Ala Glu Pro Ala Ile Thr
           275                 280                 285
   Leu Leu Cys Asn Ala Val Gly Ala Leu Leu Asp Arg Pro Gly Gln Trp
       290                 295                 300
   Ala Leu Leu Gly Gly Asp Leu Asp Arg Ser Ala Ala Val Val Glu Glu
   305                 310                 315                 320
   Thr Leu Arg Cys Leu Pro Pro Val Arg Leu Glu Ser Arg Val Ala Gln
                   325                 330                 335
   Gln Asp Val Thr Leu Gly Gly Gln Phe Leu Pro Ala Asp Ser His Leu
               340                 345                 350
   Val Val Leu Val Ala Met Ala Asn Arg Gly Pro Arg Ala Ala Thr Ala
           355                 360                 365
   Pro Ser Pro Asp Ala Phe Asp Pro Gly Gly Ser Arg Val Pro Ala Arg
       370                 375                 380
   Asp Val Val Gly Leu Pro Gln Leu Ala Gly Ala Gly Pro Leu Ile Arg
   385                 390                 395                 400
   Leu Val Val Thr Thr Ala Leu Arg Thr Leu Ala Glu Ala Leu Pro Thr
                   405                 410                 415
   Leu Arg Arg Ala Ser Gly Gly Val Arg Trp Arg Arg Ser Pro Val Leu
               420                 425                 430
   Leu Gly His Ala Arg Phe Pro Val Ala Arg Ala Glu Ser Gly Glu Gln
           435                 440                 445
   Arg Ser Asp Asp Arg Pro Ala Leu Glu Glu Ala Ile Arg Cys Ala Ser
       450                 455                 460
<210> 21
   <211> 1395
   <212> DNA
   <213> micromonospora carbonacea subspecies aurantiaca
<400> 21
   gtgacccgta cgccgggtcc gtcccggcga gtccgacgac agcaggagag gaagcgcatg     60
   atcacagtcc cgcccgacgg ggatcccgcg acctgggccc gccggctgca actgacccgc    120
   gccgcgcagt ggttcgccgg caaccacggc gacccgtacg cgctgatcct gcgcgcggag    180
   accgacgacc cgaccccgta cgagcagcgg gtggccgccc agccgctgtt ccgcagcgag    240
   cagttggaca cctgggtgac cggggacgcc gcgctggccc gggaggtgtt gaccgacgac    300
   cggttcggct ggctgacccg ggctgggcag cggcccgccg agcggaccct gccgctggcc    360
   ggcacggcac tggaccacgg gccggaggcc cggcgtcggc tggacgcgct cgccgggttc    420
   ggcgggccgg tcctgcgggc cgacgccgca ggggcgcgta cccgggtcgt ggagaccacc    480
   gcggtcctgc tcgacgggat cggggagcgg ttcgacctgg ccgtgctcgc ccggcggctg    540
   gtcgctgcgg tgctggccga cctgctgggg gtgcccgccg cgcggcgggg ccgcttcgcc    600
   gaggcactcg ccgccgccgg ccgtacgctg gacagccggc tgtgcccgca gaccgtggcg    660
   accgctctcg ccaccgtcgc cgccaccgcc gagctgaccg acctgctggg cgaggtgccg    720
   cccccgccgt cgctgtcccc gtccgccgcc ggctccgggc cgccgcgtcc gtccgcagcc    780
   ggttcctggc cgccgctgcc ggctgacgac cggacggccg ccgcgctcgc gctggcggtc    840
   ggcacggccg aaccggcgat caccctgctc tgcaacgcgg tcggtgcgct gctcgaccgc    900
   cccgggcagt gggccctgct cggtggggac ctcgaccggt ccgccgccgt cgtcgaggag    960
   accctgcgct gccttccgcc ggtgcgcctg gagagccgcg tcgcgcagca ggacgtcacc   1020
   ctgggcgggc agttcctccc ggcggacagc cacctggtcg tgctggtcgc catggcgaac   1080
   cggggtccgc gcgcggcgac cgccccgagc ccggacgcgt tcgaccctgg cgggtcgcgc   1140
   gtcccggccc gcgacgtggt gggcctgccg cagcttgccg gcgccgggcc gctgatcaga   1200
   ctcgtcgtca cgaccgccct gcggaccctc gccgaggcgc tgcccacgct gcggcgggcg   1260
   tccggcggcg tccggtggcg acgctcgccc gtcctgctcg gccacgcccg ctttcccgtc   1320
   gcacgggcgg agagcggcga acagcggtcc gacgaccgcc cggcgctgga ggaggcgatc   1380
   cgatgcgcgt cctga 1395
<210> 22
   <211> 429
   <212> PRT
   <213> micromonospora carbonacea subspecies aurantiaca
<400> 22
   Met Thr Ser Phe Ala His Asn Thr His Tyr Tyr Ser Leu Val Pro Leu
   1               5                   10                  15
   Ala Trp Ala Leu Arg Ala Ala Gly His Glu Val Arg Val Ala Ser Gln
               20                  25                  30
   Pro Ser Leu Thr Asp Thr Ile Val Arg Ser Gly Leu Thr Ala Val Pro
           35                  40                  45
   Val Gly Asp Asp Gln Ala Ile Ile Asp Leu Leu Ala Glu Val Gly Gly
       50                  55                  60
   Asp Leu Val Pro Tyr Gln Arg Gly Leu Asp Phe Thr Glu Ala Arg Pro
   65                  70                  75                  80
   Glu Val Leu Thr Trp Glu Tyr Leu Leu Gly Gln Gln Thr Met Leu Thr
                   85                  90                  95
   Ala Leu Cys Phe Ala Pro Leu Asn Gly Val Ser Thr Met Asp Asp Met
               100                 105                 110
   Val Ala Leu Ala Arg Ser Trp Gln Pro Glu Leu Val Ile Trp Glu Pro
           115                 120                 125
   Phe Thr Tyr Ala Gly Pro Val Ala Ala Arg Val Val Gly Ala Thr His
       130                 135                 140
   Ala Arg Leu Leu Trp Gly Pro Asp Val Val Gly Asn Ala Arg Arg Leu
   145                 150                 155                 160
   Phe Thr Glu Ser Leu Ala Arg Gln Pro Asp Glu Gln Arg Glu Asp Pro
                   165                 170                 175
   Met Ala Glu Trp Leu Arg Cys Thr Leu His Arg Tyr Gly Cys Glu Leu
               180                 185                 190
   Gly Asp Asp Glu Val Glu Thr Leu Val Thr Gly Gly Trp Thr Ile Asp
           195                 200                 205
   Pro Thr Ala Asp Ser Thr Arg Leu Pro Val Pro Gly Arg Arg Val Ala
       210                 215                 220
   Met Arg Tyr Thr Pro Tyr Asn Ser Pro Ser Val Val Pro Glu Trp Val
   225                 230                 235                 240
   Ala Lys Ala Asp Arg Pro Arg Val Cys Leu Thr Leu Gly Val Ser Ser
                   245                 250                 255
   Arg Glu Thr Tyr Gly Arg Asp Val Val Ser Phe Gln Glu Leu Leu Gly
               260                 265                 270
   Ala Leu Gly Asp Leu Asp Val Glu Val Val Ala Thr Leu Ser Asp Ala
           275                 280                 285
   Gln Arg Glu Asp Leu Gly Asp Leu Pro Asp Asn Val Arg Val Cys Asp
       290                 295                 300
   Phe Val Pro Leu Asp Val Leu Leu Pro Thr Cys Ala Ala Ile Ile His
   305                 310                 315                 320
   His Gly Gly Ala Gly Thr Trp Ser Thr Ala Met Leu Tyr Gly Val Pro
                   325                 330                 335
   Gln Ile Met Ile Ala Ser Leu Trp Asp Ala Pro Leu Lys Ala Gln Gln
               340                 345                 350
   Ala Glu Arg Leu Gly Thr Gly Ile Ser Ile Pro Pro Glu Arg Leu Asp
           355                 360                 365
   Ala Pro Thr Leu Arg Ala Ala Val Val Arg Ile Leu Asp Asp Pro Ser
       370                 375                 380
   Ile Ala Ala Ala Ala Arg Arg Gln Arg Asp Glu Leu Arg Ala Ala Pro
   385                 390                 395                 400
   Ser Pro Ala Glu Val Val Arg Ile Leu Glu Arg Leu Val Ala Asp Asp
                   405                 410                 415
   Arg Pro Gly Arg Pro Ala Gly Thr Ala Thr Asp His Ser
               420                 425
<210> 23
   <211> 1290
   <212> DNA
   <213> micromonospora carbonacea subspecies aurantiaca
<400> 23
   atgacgtcct tcgcgcacaa cacccactac tacagcctgg tgccgttggc ctgggcgctg    60
   cgcgcggccg gccacgaggt acgggtggcg agccagccct cgctcaccga caccatcgtg   120
   cggtcggggc tgaccgcggt gccggtcggc gacgaccagg cgatcatcga cctgctcgcc   180
   gaggtcggcg gcgacctggt gccgtaccag cggggactgg acttcaccga ggcccgtccc   240
   gaagtgctga cctgggagta tctgctcggg cagcagacca tgctcaccgc gctgtgcttc   300
   gcgccgctca acggcgtctc cacgatggac gacatggtcg ccctggcccg gtcctggcag   360
   cccgagctgg tgatctggga gccgttcacc tacgccgggc cggtcgcggc gcgggtcgtc   420
   ggtgcgacgc acgcccggct gctctggggg ccggacgtgg tcggcaacgc ccggcggctg   480
   ttcaccgaga gcctggcgcg gcagccggat gagcagcgcg aggacccgat ggccgagtgg 540
   ttgcgctgca ccctgcaccg gtacggctgc gagctcggcg acgacgaggt ggagaccctg 600
   gtcaccggcg ggtggaccat cgatcccacc gccgacagca cccggcttcc cgtccccggg 660
   cgtcgggtgg ccatgcggta caccccgtac aacagcccgt ccgtggtgcc ggagtgggtg 720
   gccaaggccg accggccccg cgtctgcctc accctcggcg tgtcgagccg ggagacgtac 780
   ggcagggacg tggtctcctt ccaggagctg ctcggcgccc tcggcgacct ggacgtcgag 840
   gtcgtcgcga cgctcagcga cgcccagcgc gaggacctgg gtgacctgcc ggacaacgtc 900
   cgggtgtgcg acttcgtgcc gctggacgtg ctgctgccga cctgtgccgc gatcatccac 960
   cacggcgggg cgggcacgtg gtcgacggcc atgctctacg gggtgccgca gatcatgatc 1020
   gcgtcgctgt gggacgcccc gctcaaggcg cagcaggcgg agcgactcgg cacggggatc 1080
   tcgatcccgc cggagcggct cgacgccccg acgctgcggg cggccgtcgt ccggatcctc 1140
   gacgacccgt cgatcgccgc cgccgcccgc cgtcagcgcg acgagctgcg tgccgcgccg 1200
   tcgccggccg aggtggtccg catcctggaa cgcctcgtcg cggacgaccg gcccggccgg 1260
   ccggccggaa ccgccaccga ccactcctga 1290
<210> 24
   <211> 240
   <212> PRT
   <213> micromonospora carbonacea subspecies aurantiaca
<400> 24
   Met Ser Met Met Tyr Ala Asp Ala Ile Ala Glu Val Tyr Asp Leu Ile
   1 5 10 15
   Tyr Gln Gly Lys Gly Lys Asp Tyr Ala Ala Glu Ala Ala Glu Leu Glu
   20 25 30
   Ala Leu Ala Arg Ala Arg Arg Pro His Ala Arg Thr Leu Leu Asp Val
   35 40 45
   Ala Cys Gly Thr Gly Leu His Leu Arg His Leu Ala Gly Leu Phe Asp
   50 55 60
   Asp Val Gly Gly Ile Glu Leu Ala Pro Asp Met Leu Ser Ile Ala Gln
   65 70 75 80
   Gln Arg Asn Pro Gly Ala Ala Leu His Leu Gly Asp Met Arg Thr Phe
   85 90 95
   Asp Leu Gly His Arg Tyr Asp Val Ile Thr Cys Met Phe Ser Ser Val
   100 105 110
   Gly His Leu Ala Thr Thr Ala Glu Leu Asp Ala Thr Leu Ala Arg Phe
   115 120 125
   Ala Ala His Leu Ser Pro Gly Gly Val Ala Ile Val Glu Pro Trp Trp
   130 135 140
   Phe Pro Glu Thr Phe Thr Pro Gly Tyr Val Gly Ala Ser Leu Val Glu
   145 150 155 160
   Val Asp Gly Arg Thr Ile Ser Arg Val Ser His Ser Val Arg Glu Gly
   165 170 175
   Gly Ala Thr Arg Ile Thr Val His Tyr Leu Val Ala Ser Pro Gly Gly
   180 185 190
   Gly Val Arg His Phe Asp Glu Ser His Leu Ile Thr Leu Phe Glu Arg
   195 200 205
   Ser Asp Tyr Glu Arg Ala Phe Ala Arg Ala Gly Phe Thr Thr Glu Tyr
   210 215 220
   Leu Thr Pro Gly Pro Ser Gly Arg Gly Leu Phe Val Gly Val His Pro
   225 230 235 240
<210> 25
   <211> 723
   <212> DNA
   <213> micromonospora carbonacea subspecies aurantiaca
<400> 25
   atgtccatga tgtacgcgga cgccatcgcc gaggtctacg acctgatcta ccagggcaag 60
   ggcaaggact acgcggcgga ggcggcggag ctggaggcgc tggcccgggc ccgtcggccg 120
   cacgcccgga cgctgctgga cgtggcgtgc ggcacggggc tgcacctgcg gcacctggcg 180
   gggctcttcg acgacgtggg cggcatcgag ctggcaccgg acatgctgag catcgcccag 240
   cagcgaaacc ccggggcggc cctgcacctc ggcgacatgc ggaccttcga cctggggcac 300
   cgctacgacg tcatcacctg catgttcagt tcggtgggcc acctggccac cacggccgag 360
   ctggacgcga cgttggcccg gttcgccgcg cacctgtccc ccgggggagt ggcgatcgtc 420
   gagccgtggt ggttcccgga gaccttcacc cccgggtacg tgggcgcgag cctggtggag 480
   gtcgacggcc gtaccatctc gcgggtctcc cattcggtgc gcgagggcgg cgcgacccgg 540
   atcaccgtgc actacctcgt ggccagcccc ggcgggggag tccggcactt cgacgagagc 600
   cacctgatca ccctcttcga acggtccgac tacgaacgtg ccttcgcccg ggcgggtttc 660
   acgacggagt acctgacgcc cggcccgtcc ggccgcggtc tgttcgtcgg cgtccacccc 720
   tga 723
<210> 26
   <211> 1811
   <212> PRT
   <213> micromonospora carbonacea subspecies aurantiaca
<400> 26
   Met Pro Asp Thr Pro Glu Leu Asn Arg Ile Leu Asp Ala Ile Leu Ala
   1               5                   10                  15
   Gln Glu Thr Asp Ala Arg Glu Leu Ala Ala Leu Pro Leu Pro Ser Ser
               20                  25                  30
   Tyr Arg Ala Val Thr Val His Lys Asp Glu Thr Gly Met Phe Leu Gly
           35                  40                  45
   Leu Pro Arg Gln Glu Lys Asp Pro Arg Lys Ser Leu His Thr Glu Glu
       50                  55                  60
   Val Pro Val Pro Glu Leu Gly Pro Gly Glu Ala Leu Val Ala Val Leu
   65                  70                  75                  80
   Ala Ser Ser Val Asn Tyr Asn Thr Val Trp Ser Ser Leu Phe Glu Pro
                   85                  90                  95
   Leu Pro Thr Phe Gly Phe Leu Glu Arg Tyr Gly Arg Leu Ser Glu Leu
               100                 105                 110
   Ala Arg Arg His Asp Leu Pro Tyr His Ile Leu Gly Ser Asp Leu Ala
           115                 120                 125
   Gly Val Val Leu Arg Val Gly Pro Gly Val Asn Arg Trp Arg Pro Gly
       130                 135                 140
   Asp Glu Val Val Ala His Cys Leu Ser Val Glu Leu Glu Ser Ala Asp
   145                 150                 155                 160
   Gly His Gly Asp Thr Met Leu Asp Pro Glu Gln Arg Ile Trp Gly Phe
                   165                 170                 175
   Glu Thr Asn Phe Gly Gly Leu Ala Glu Ile Ala Leu Val Lys Ala Asn
               180                 185                 190
   Gln Leu Met Pro Lys Pro Asp His Leu Thr Trp Glu Glu Ala Ala Ala
           195                 200                 205
   Pro Gly Leu Val Asn Ser Thr Ala Tyr Arg Gln Leu Val Ser Gly Asn
       210                 215                 220
   Gly Ala Arg Met Lys Gln Gly Asp Asn Val Leu Val Trp Gly Ala Ser
   225                 230                 235                 240
   Gly Gly Leu Gly Ala Phe Ala Thr Gln Leu Val Leu Ala Gly Gly Ala
                   245                 250                 255
   Asn Pro Val Cys Val Val Ser Ser Pro Arg Lys Ala Asp Ile Cys Arg
               260                 265                 270
   Arg Met Gly Ala Glu Ala Val Ile Asp Arg Val Ala Glu Asp Tyr Arg
   275 280 285
   Phe Trp Ser Asp Glu Arg Thr Gln Asn Pro Arg Glu Trp Lys Arg Phe
   290 295 300
   Gly Ala Arg Ile Arg Glu Leu Thr Gly Gly Glu Asp Val Asp Ile Val
   305 310 315 320
   Phe Glu His Pro Gly Arg Glu Thr Phe Gly Ala Ser Val Tyr Val Thr
   325 330 335
   Arg Lys Gly Gly Thr Val Val Thr Cys Ala Ser Thr Ser Gly Phe Glu
   340 345 350
   His Val Tyr Asp Asn Arg Tyr Leu Trp Met Ser Leu Lys Arg Ile Val
   355 360 365
   Gly Thr His Phe Ala Asn Tyr Arg Glu Ala Trp Glu Ala Asn Arg Leu
   370 375 380
   Val Val Lys Gly Lys Ile His Pro Thr Leu Ser Arg Cys Tyr Pro Leu
   385 390 395 400
   Glu Glu Val Gly Gln Ala Val Tyr Asp Val His His Asn Leu His Gln
   405 410 415
   Gly Lys Val Gly Val Leu Ala Leu Ala Pro Arg Glu Gly Leu Gly Val
   420 425 430
   Arg Asn Pro Glu Leu Arg Glu Cys His Leu Ala Ala Ile Asn Arg Phe
   435 440 445
   Arg Val Pro Ala Ala Thr Gly Cys Cys Ala Gly Ala Cys Ala Cys Cys
   450 455 460
   Cys Cys Cys Gly Ala Gly Cys Thr Gly Ala Ala Cys Cys Gly Gly Ala
   465 470 475 480
   Thr Ala Cys Thr Cys Gly Ala Cys Gly Cys Gly Ala Thr Cys Cys Thr
   485 490 495
   Cys Gly Cys Cys Cys Ala Gly Gly Ala Gly Ala Cys Cys Gly Ala Cys
   500 505 510
   Gly Cys Gly Cys Gly Gly Gly Ala Gly Cys Thr Gly Gly Cys Gly Gly
   515 520 525
   Cys Cys Cys Thr Gly Cys Cys Gly Cys Thr Gly Cys Cys Cys Thr Cys
   530 535 540
   Cys Thr Cys Cys Thr Ala Cys Cys Gly Gly Gly Cys Cys Gly Thr Gly
   545 550 555 560
   Ala Cys Gly Gly Thr Gly Cys Ala Cys Ala Ala Gly Gly Ala Cys Gly
   565 570 575
   Ala Gly Ala Cys Gly Gly Gly Gly Ala Thr Gly Thr Thr Cys Cys Thr
   580 585 590
   Gly Gly Gly Cys Cys Thr Thr Cys Cys Cys Cys Gly Cys Cys Ala Gly
   595 600 605
   Gly Ala Gly Ala Ala Gly Gly Ala Cys Cys Cys Gly Cys Gly Cys Ala
   610 615 620
   Ala Gly Thr Cys Gly Cys Thr Gly Cys Ala Cys Ala Cys Gly Gly Ala
   625 630 635 640
   Gly Gly Ala Gly Gly Thr Gly Cys Cys Gly Gly Thr Gly Cys Cys Cys
   645 650 655
   Gly Ala Gly Cys Thr Gly Gly Gly Cys Cys Cys Cys Gly Gly Gly Gly
   660 665 670
   Ala Gly Gly Cys Cys Cys Thr Cys Gly Thr Cys Gly Cys Gly Gly Thr
   675 680 685
   Cys Cys Thr Gly Gly Cys Cys Ala Gly Cys Thr Cys Gly Gly Thr Cys
   690 695 700
   Ala Ala Cys Thr Ala Cys Ala Ala Cys Ala Cys Gly Gly Thr Cys Thr
   705 710 715 720
   Gly Gly Thr Cys Gly Thr Cys Gly Thr Thr Gly Thr Thr Cys Gly Ala
   725 730 735
   Gly Cys Cys Gly Cys Thr Gly Cys Cys Cys Ala Cys Cys Thr Thr Cys
   740 745 750
   Gly Gly Cys Thr Thr Cys Cys Thr Gly Gly Ala Gly Cys Gly Cys Thr
   755 760 765
   Ala Cys Gly Gly Cys Cys Gly Gly Cys Thr Cys Thr Cys Cys Gly Ala
   770 775 780
   Gly Cys Thr Gly Gly Cys Cys Cys Gly Gly Cys Gly Gly Cys Ala Cys
   785 790 795 800
   Gly Ala Cys Cys Thr Gly Cys Cys Gly Thr Ala Cys Cys Ala Cys Ala
   805 810 815
   Thr Cys Cys Thr Cys Gly Gly Cys Thr Cys Gly Gly Ala Cys Cys Thr
   820 825 830
   Gly Gly Cys Cys Gly Gly Cys Gly Thr Gly Gly Thr Gly Cys Thr Gly
   835 840 845
   Ala Gly Gly Gly Thr Cys Gly Gly Gly Cys Cys Cys Gly Gly Cys Gly
   850 855 860
   Thr Cys Ala Ala Cys Cys Gly Cys Thr Gly Gly Cys Gly Gly Cys Cys
   865 870 875 880
   Gly Gly Gly Thr Gly Ala Cys Gly Ala Gly Gly Thr Cys Gly Thr Gly
   885 890 895
   Gly Cys Gly Cys Ala Cys Thr Gly Cys Cys Thr Cys Thr Cys Gly Gly
   900 905 910
   Thr Gly Gly Ala Gly Cys Thr Gly Gly Ala Gly Thr Cys Cys Gly Cys
   915 920 925
   Cys Gly Ala Cys Gly Gly Cys Cys Ala Cys Gly Gly Cys Gly Ala Cys
   930 935 940
   Ala Cys Cys Ala Thr Gly Cys Thr Cys Gly Ala Cys Cys Cys Gly Gly
   945 950 955 960
   Ala Ala Cys Ala Gly Cys Gly Gly Ala Thr Cys Thr Gly Gly Gly Gly
   965 970 975
   Cys Thr Thr Cys Gly Ala Gly Ala Cys Cys Ala Ala Cys Thr Thr Cys
   980 985 990
   Gly Gly Cys Gly Gly Cys Cys Thr Cys Gly Cys Cys Gly Ala Gly Ala
   995 1000 1005
   Thr Cys Gly Cys Gly Thr Thr Gly Gly Thr Cys Ala Ala Gly Gly
   1010 1015 1020
   Cys Gly Ala Ala Cys Cys Ala Gly Cys Thr Gly Ala Thr Gly Cys
   1025 1030 1035
   Cys Cys Ala Ala Ala Cys Cys Cys Gly Ala Cys Cys Ala Cys Cys
   1040 1045 1050
   Thr Gly Ala Cys Cys Thr Gly Gly Gly Ala Gly Gly Ala Gly Gly
   1055 1060 1065
   Cys Cys Gly Cys Cys Gly Cys Gly Cys Cys Gly Gly Gly Ala Cys
   1070 1075 1080
   Thr Gly Gly Thr Cys Ala Ala Cys Thr Cys Cys Ala Cys Cys Gly
   1085 1090 1095
   Cys Cys Thr Ala Cys Cys Gly Cys Cys Ala Gly Cys Thr Gly Gly
   1100 1105 1110
   Thr Cys Thr Cys Cys Gly Gly Cys Ala Ala Cys Gly Gly Gly Gly
   1115 1120 1125
   Cys Cys Cys Gly Gly Ala Thr Gly Ala Ala Gly Cys Ala Gly Gly
   1130 1135 1140
   Gly Cys Gly Ala Cys Ala Ala Cys Gly Thr Cys Cys Thr Cys Gly
   1145 1150 1155
   Thr Cys Thr Gly Gly Gly Gly Gly Gly Cys Cys Ala Gly Cys Gly
   1160 1165 1170
   Gly Cys Gly Gly Thr Cys Thr Cys Gly Gly Cys Gly Cys Gly Thr
   1175 1180 1185
   Thr Cys Gly Cys Cys Ala Cys Cys Cys Ala Gly Cys Thr Cys Gly
   1190 1195 1200
   Thr Gly Cys Thr Gly Gly Cys Cys Gly Gly Cys Gly Gly Gly Gly
   1205 1210 1215
   Cys Cys Ala Ala Thr Cys Cys Cys Gly Thr Cys Thr Gly Cys Gly
   1220 1225 1230
   Thr Gly Gly Thr Cys Thr Cys Cys Ala Gly Cys Cys Cys Gly Cys
   1235 1240 1245
   Gly Cys Ala Ala Gly Gly Cys Cys Gly Ala Cys Ala Thr Cys Thr
   1250 1255 1260
   Gly Cys Cys Gly Thr Cys Gly Gly Ala Thr Gly Gly Gly Cys Gly
   1265 1270 1275
   Cys Cys Gly Ala Gly Gly Cys Cys Gly Thr Cys Ala Thr Cys Gly
   1280 1285 1290
   Ala Cys Cys Gly Gly Gly Thr Cys Gly Cys Cys Gly Ala Gly Gly
   1295 1300 1305
   Ala Cys Thr Ala Cys Cys Gly Cys Thr Thr Cys Thr Gly Gly Thr
   1310 1315 1320
   Cys Cys Gly Ala Cys Gly Ala Gly Cys Gly Cys Ala Cys Cys Cys
   1325 1330 1335
   Ala Gly Ala Ala Thr Cys Cys Cys Cys Gly Gly Gly Ala Gly Thr
   1340 1345 1350
   Gly Gly Ala Ala Gly Cys Gly Cys Thr Thr Cys Gly Gly Cys Gly
   1355 1360 1365
   Cys Ala Cys Gly Cys Ala Thr Thr Cys Gly Gly Gly Ala Gly Cys
   1370 1375 1380
   Thr Gly Ala Cys Cys Gly Gly Ala Gly Gly Cys Gly Ala Gly Gly
   1385 1390 1395
   Ala Cys Gly Thr Cys Gly Ala Cys Ala Thr Cys Gly Thr Cys Thr
   1400 1405 1410
   Thr Cys Gly Ala Gly Cys Ala Cys Cys Cys Cys Gly Gly Cys Cys
   1415 1420 1425
   Gly Gly Gly Ala Gly Ala Cys Gly Thr Thr Cys Gly Gly Cys Gly
   1430 1435 1440
   Cys Cys Thr Cys Gly Gly Thr Cys Thr Ala Cys Gly Thr Gly Ala
   1445 1450 1455
   Cys Cys Cys Gly Cys Ala Ala Ala Gly Gly Ala Gly Gly Cys Ala
   1460 1465 1470
   Cys Cys Gly Thr Gly Gly Thr Cys Ala Cys Cys Thr Gly Cys Gly
   1475 1480 1485
   Cys Cys Thr Cys Gly Ala Cys Gly Ala Gly Cys Gly Gly Thr Thr
   1490 1495 1500
   Thr Cys Gly Ala Gly Cys Ala Cys Gly Thr Cys Thr Ala Cys Gly
   1505 1510 1515
   Ala Cys Ala Ala Cys Cys Gly Thr Thr Ala Cys Cys Thr Gly Thr
   1520 1525 1530
   Gly Gly Ala Thr Gly Thr Cys Cys Cys Thr Gly Ala Ala Gly Cys
   1535 1540 1545
   Gly Cys Ala Thr Cys Gly Thr Cys Gly Gly Cys Ala Cys Gly Cys
   1550 1555 1560
   Ala Cys Thr Thr Cys Gly Cys Cys Ala Ala Thr Thr Ala Cys Cys
   1565 1570 1575
   Gly Gly Gly Ala Gly Gly Cys Gly Thr Gly Gly Gly Ala Ala Gly
   1580 1585 1590
   Cys Cys Ala Ala Cys Cys Gly Gly Thr Thr Gly Gly Thr Gly Gly
   1595 1600 1605
   Thr Cys Ala Ala Gly Gly Gly Cys Ala Ala Gly Ala Thr Cys Cys
   1610 1615 1620
   Ala Cys Cys Cys Gly Ala Cys Gly Cys Thr Gly Thr Cys Gly Cys
   1625 1630 1635
   Gly Cys Thr Gly Cys Thr Ala Cys Cys Cys Gly Cys Thr Gly Gly
   1640 1645 1650
   Ala Gly Gly Ala Gly Gly Thr Cys Gly Gly Cys Cys Ala Gly Gly
   1655 1660 1665
   Cys Gly Gly Thr Cys Thr Ala Cys Gly Ala Cys Gly Thr Cys Cys
   1670 1675 1680
   Ala Thr Cys Ala Cys Ala Ala Cys Cys Thr Gly Cys Ala Cys Cys
   1685 1690 1695
   Ala Gly Gly Gly Cys Ala Ala Gly Gly Thr Cys Gly Gly Cys Gly
   1700 1705 1710
   Thr Gly Cys Thr Cys Gly Cys Gly Cys Thr Cys Gly Cys Gly Cys
   1715 1720 1725
   Cys Gly Cys Gly Cys Gly Ala Gly Gly Gly Gly Cys Thr Cys Gly
   1730 1735 1740
   Gly Gly Gly Thr Cys Cys Gly Gly Ala Ala Cys Cys Cys Gly Gly
   1745 1750 1755
   Ala Gly Cys Thr Gly Cys Gly Gly Gly Ala Ala Thr Gly Cys Cys
   1760 1765 1770
   Ala Thr Cys Thr Thr Gly Cys Cys Gly Cys Gly Ala Thr Cys Ala
   1775 1780 1785
   Ala Cys Cys Gly Cys Thr Thr Cys Cys Gly Gly Gly Thr Gly Cys
   1790 1795 1800
   Cys Gly Gly Cys Cys Thr Gly Ala
   1805 1810
<210> 27
   <211> 1359
   <212> DNA
   <213> micromonospora carbonacea subspecies aurantiaca
<400> 27
   atgccagaca cccccgagct gaaccggata ctcgacgcga tcctcgccca ggagaccgac 60
   gcgcgggagc tggcggccct gccgctgccc tcctcctacc gggccgtgac ggtgcacaag 120
   gacgagacgg ggatgttcct gggccttccc cgccaggaga aggacccgcg caagtcgctg 180
   cacacggagg aggtgccggt gcccgagctg ggcccggggg aggccctcgt cgcggtcctg 240
   gccagctcgg tcaactacaa cacggtctgg tcgtcgttgt tcgagccgct gcccaccttc 300
   ggcttcctgg agcgctacgg ccggctctcc gagctggccc ggcggcacga cctgccgtac 360
   cacatcctcg gctcggacct ggccggcgtg gtgctgaggg tcgggcccgg cgtcaaccgc 420
   tggcggccgg gtgacgaggt cgtggcgcac tgcctctcgg tggagctgga gtccgccgac 480
   ggccacggcg acaccatgct cgacccggaa cagcggatct ggggcttcga gaccaacttc 540
   ggcggcctcg ccgagatcgc gttggtcaag gcgaaccagc tgatgcccaa acccgaccac 600
   ctgacctggg aggaggccgc cgcgccggga ctggtcaact ccaccgccta ccgccagctg 660
   gtctccggca acggggcccg gatgaagcag ggcgacaacg tcctcgtctg gggggccagc 720
   ggcggtctcg gcgcgttcgc cacccagctc gtgctggccg gcggggccaa tcccgtctgc 780
   gtggtctcca gcccgcgcaa ggccgacatc tgccgtcgga tgggcgccga ggccgtcatc 840
   gaccgggtcg ccgaggacta ccgcttctgg tccgacgagc gcacccagaa tccccgggag 900
   tggaagcgct tcggcgcacg cattcgggag ctgaccggag gcgaggacgt cgacatcgtc 960
   ttcgagcacc ccggccggga gacgttcggc gcctcggtct acgtgacccg caaaggaggc 1020
   accgtggtca cctgcgcctc gacgagcggt ttcgagcacg tctacgacaa ccgttacctg 1080
   tggatgtccc tgaagcgcat cgtcggcacg cacttcgcca attaccggga ggcgtgggaa 1140
   gccaaccggt tggtggtcaa gggcaagatc cacccgacgc tgtcgcgctg ctacccgctg 1200
   gaggaggtcg gccaggcggt ctacgacgtc catcacaacc tgcaccaggg caaggtcggc 1260
   gtgctcgcgc tcgcgccgcg cgaggggctc ggggtccgga acccggagct gcgggaatgc 1320
   catcttgccg cgatcaaccg cttccgggtg ccggcctga 1359
<210> 28
   <211> 636
   <212> PRT
   <213> micromonospora carbonacea subspecies aurantiaca
<400> 28
   Val His Gln Ala His Arg Asp Gly Val Asp Gln Ala Thr Leu Asp Arg
   1 5 10 15
   Val Met Ile Ala Lys Arg Leu Ala Leu Glu Leu Arg Glu Val Ile Gly
   20 25 30
   Arg Arg Cys Gln Arg Gln Ala Glu Leu Ala Ala Leu Val Asp Thr Ala
   35 40 45
   Arg Asp Leu Ala Gly Ala Thr Asn Leu Glu Ala Gly Leu Gln Leu Val
   50 55 60
   Val Arg Arg Thr Gln Leu Leu Leu Ala Gly Asp Val Ala Phe Val Ser
   65 70 75 80
   Leu Val Asp Asp Ala Thr Gly Glu Ser Tyr Val Ala Ser Ala Val Gly
   85 90 95
   Ala Ala Thr Ala Leu Thr Ser Gly Tyr Arg Leu Pro Trp Arg Asp Gly
   100 105 110
   Leu Val Val Ala Ala Ala Pro Arg Glu Pro Leu Ser Trp Thr Ala Asp
   115 120 125
   His Leu Ala Asp Glu Arg Leu Glu Arg His Pro Ala Ala Asp Gly Leu
   130 135 140
   Val Arg Ala Glu Gly Leu His Ala Val Leu Ser Val Val Leu Ser Val
   145 150 155 160
   Glu Gly Arg His Leu Gly Asn Leu His Val Gly His Arg Gln Val Arg
   165 170 175
   His Phe Ala Pro Asp Glu Val Ala Ser Leu Arg Leu Leu Ala Asp Leu
   180 185 190
   Ala Ala Thr Ala Val Glu Arg Ile Met Leu Leu Asp Asp Thr Trp Ala
   195 200 205
   Glu Leu Lys Gln Ala Gln Gln Glu Ala Ala Arg Ala Arg Ala Glu Leu
   210 215 220
   Asn Ala Val Arg Met Ala Asp Arg Leu Gln Pro Glu Leu Val Gln Leu
   225 230 235 240
   Ile Leu Asp Gly Gly Glu Leu Asp Asp Leu Val Gly Ser Ala Val Arg
   245 250 255
   Arg Leu Gly Gly Ala Leu His Val Arg Asp Arg Ala Asn Gly Val Leu
   260 265 270
   Ala Ala Ala Gly Glu Ile Pro Val Pro Asn Glu Arg Glu Leu Ala Arg
   275 280 285
   Val Arg Leu Asn Ala His Ala Thr Gly Arg Pro Gly Arg Leu Thr Thr
   290 295 300
   Gly Ser Trp Val Val Pro Leu Ala Ala Arg Ala Gly Asp Leu Gly Cys
   305 310 315 320
   Val Leu Phe His Ala Asp Glu Pro Ser Asp Asp Glu Arg Met Ala Ala
   325 330 335
   Leu Pro Ala Val Ala Gln Thr Val Ala Leu Leu Met Thr Arg Asn Gly
   340 345 350
   Gly Ser His Gly Gln Pro Gly Asp Gly Leu Leu Glu Asp Leu Leu Gly
   355 360 365
   Pro Trp Pro Asp Leu Glu Arg Gly Gly Lys Arg Arg Arg Tyr Thr Pro
   370 375 380
   Val Glu Phe Asp Arg Pro Tyr Val Val Val Val Ala Arg Pro Glu Gly
   385 390 395 400
   Ala Thr Ser Pro Arg Val Phe Glu Arg Ala Val Ser Val Ala His Gly
   405 410 415
   Leu Asn Gly Met Lys Ala Ile Arg Asp Gly Gln Ala Val Leu Leu Leu
   420 425 430
   Pro Gly Asp Asp Pro Gly Ala Arg Ala Arg Asp Val Thr Arg Glu Leu
   435 440 445
   Ser Gly Leu Leu Gly Leu Pro Val Thr Ala Gly Gly Ala Gly Pro Val
   450 455 460
   Arg Thr Ala Asp Ser Val Ser Arg Thr Tyr Gln Glu Ala Ala Arg Cys
   465 470 475 480
   Val Asp Ala Leu Ala Ala Leu Asp Ala Lys Gly Arg Ala Ala Cys Ser
   485 490 495
   Arg Asp Leu Gly Phe Leu Gly Leu Leu Val Ala Gly Gly His Asp Val
   500 505 510
   Thr Gly Phe Val Asp Arg Val Ile Gly Pro Val Leu Ser Tyr Asp Ala
           515                 520                 525
   Arg Arg Leu Thr Asn Leu Arg Glu Thr Leu Gln Thr Tyr Phe Asp Ser
       530                 535                 540
   Ala Gly Ser Arg Thr Arg Ala Ala Glu Met Leu His Leu His Pro Asn
   545                 550                 555                 560
   Thr Val Ser Arg Arg Leu Asp Arg Ile Ser Gln Leu Leu Gly Arg Asp
                   565                 570                 575
   Trp Arg Gln Pro Asp Arg Ala Leu Asp Thr Gln Leu Ala Leu Arg Leu
               580                 585                 590
   His Arg Ile Arg Gly Leu Leu Cys Gln Glu Arg Gly Tyr Pro Gly Pro
           595                 600                 605
   Ser Gln Glu Pro Asp Gln Pro Ala Arg Pro Ile Arg Arg His Arg Pro
       610                 615                 620
   Pro Ala Ser Ala Gly Arg Ala Pro Arg Thr Pro Arg
   625                 630                 635
<210> 29
   <211> 1911
   <212> DNA
   <213> micromonospora carbonacea subspecies aurantiaca
<400> 29
   gtgcaccagg cgcaccggga cggagtggac caggccacgc tcgaccgggt gatgatcgcc     60
   aagcgactcg cgttggagct tcgagaggtc atcgggaggc ggtgtcagcg gcaggcggag    120
   ctggccgccc tcgtcgacac cgcccgtgac ctcgccgggg cgacgaacct ggaggccggg    180
   ctgcagctgg tggtgcggcg gacccaactg ctgctcgccg gggacgtggc gttcgtcagc    240
   ctcgtcgacg acgcgaccgg cgaatcctac gtcgcctcgg ccgtcggggc ggccaccgcg    300
   ctgaccagcg gctaccggct gccctggcgc gacgggctgg tcgtggccgc cgcaccgcgc    360
   gagccactct cctggacggc ggaccacctc gccgacgagc gcctcgaacg acacccggcc    420
   gccgacggcc tggtccgcgc ggaagggctg cacgcggtgc tgtccgtggt tctgagcgtc    480
   gagggccggc acctcggcaa cctgcacgtc ggccaccggc aggtccgcca cttcgccccg    540
   gacgaggtcg cgtcgctgcg cctgctcgcc gatctcgcgg cgacggcagt ggagcggatc    600
   atgctgctcg acgacacgtg ggccgaactc aagcaggccc agcaggaggc ggccagggcc    660
   cgagccgagc tgaacgcggt ccgcatggcc gaccgcctgc aacccgaact cgtccagctc    720
   atcctcgacg gcggcgaact cgacgacctg gtgggcagcg ccgtgcggcg actgggcggc    780
   gccctgcacg tgcgtgaccg ggccaacggc gtgctggcgg cggccggtga aatccctgtc 840
   ccgaacgagc gggaactggc ccgagtgcgg ctgaacgccc acgccaccgg ccgacccggc 900
   cgcctgacca ccggttcctg ggtggtgccc ctggcggccc gcgccggtga cctcggctgt 960
   gtgttgttcc acgccgacga gccgtccgac gacgagcgga tggcggccct gccggcggtc 1020
   gcgcagaccg tggcgctgct gatgaccagg aacggcggga gccacggcca gccgggcgac 1080
   gggctcctgg aggacctgct cggcccgtgg ccggacctgg agcggggcgg gaagcgccgt 1140
   cggtacacac ctgtcgagtt cgaccggccc tacgtcgtcg tggtcgcccg ccccgagggc 1200
   gccacctcgc cccgggtgtt cgaacgggcg gtctccgtcg cccacggcct gaacggcatg 1260
   aaggccatcc gggacggcca ggcggtgctg ctgctgcccg gtgacgaccc gggggcccgg 1320
   gcccgggacg tgacgcggga actgagcggg ctgctcggcc taccggtcac ggccggaggc 1380
   gccggaccgg tgcgcacggc ggactcggtc agccgcacct accaggaggc ggcccggtgc 1440
   gtcgacgccc tggccgcgct ggacgcgaag gggcgggcgg cctgctcacg ggacctgggc 1500
   ttcctcgggc tgctggtcgc cggcggccac gacgtcaccg gtttcgtcga ccgggtcatc 1560
   ggacccgtgc tgagctacga cgcgcgccgg ctcacgaatc tcagggagac cctccagacc 1620
   tacttcgact cggcgggcag ccgtacccgg gcggcggaga tgctgcatct gcatccgaac 1680
   accgtgtccc gccggctgga ccgcatctcc cagctgctcg gccgggactg gcggcagccg 1740
   gaccgggccc tcgacacgca gctcgctctg cgcctgcacc ggatccgtgg cctgctctgc 1800
   caggaacggg gctacccggg cccatcgcag gagccggacc aacccgcgcg gcctatccgg 1860
   cggcaccgcc ctccagcatc cgcagggcgt gcgccacgga cgccaaggtg a 1911
<210> 30
   <211> 403
   <212> PRT
   <213> micromonospora carbonacea subspecies aurantiaca
<400> 30
   Met Val Pro Thr Leu Asp Val Arg Glu Glu Val Thr Ala Ala Arg Ser
   1 5 10 15
   Asp Pro Asp Thr Val Ser Arg Phe Cys Ala Ala Leu Leu Ala Ser Leu
   20 25 30
   Pro Arg Ala Asp Gln Arg Arg Lys Gly Glu Leu Tyr Val Arg Gly Leu
   35 40 45
   Leu Thr Ala Ser Gly Arg Lys Thr Met Arg Asn Leu Ala Ala Ile Ala
   50 55 60
   Asp Asp Pro Ala Ala Ala Gln Ser Met His His Phe Ile Ser Cys Ser
   65 70 75 80
   Thr Trp Asp Trp Glu Thr Val Arg Ala Ala Leu Ala Gly His Leu Asp
   85 90 95
   Arg Thr Leu Ser Pro Arg Ala Trp Val Val Arg Ser Met Leu Val Pro
   100 105 110
   Lys Thr Gly Arg His Ser Val Gly Val Glu Arg Arg Tyr Val Pro Ala
   115 120 125
   Leu Gly Glu Thr Val Asn Ser Gln Gln Ser Tyr Gly Leu Trp Leu Ala
   130 135 140
   Ser Glu Thr Val Ala Ala Pro Ile Asn Trp Gln Leu Ser Ile Gly Lys
   145 150 155 160
   Gly Trp Leu Gln Asp Asn Arg Ala Arg Ala Ser Val Pro Ala Asp Glu
   165 170 175
   Asp Gly Thr Thr Ser Asp Gly Ala Ala Val Gln Ala Val Leu Lys Ala
   180 185 190
   Ala Ala Trp Gly Ile Gly Pro Arg Pro Val Val Met Asp Ala Arg His
   195 200 205
   Ser Ala Leu Pro Pro Leu Ile Glu Ala Phe Thr Thr Ala Gly Leu Pro
   210 215 220
   Phe Leu Leu Arg Ile Asn Ser Gly Cys Thr Leu Leu Ala Ala Gly Pro
   225 230 235 240
   Gly Pro Arg Glu Asn Arg Val Ala Ala Ala Ser Ala Glu His Leu Leu
   245 250 255
   Ser Leu Thr Arg Ala Gln Arg Arg Pro Val Glu Trp Ile Asp Pro Ala
   260 265 270
   Ser Pro Gly Ala Arg Arg Thr Ser Leu Val Ala Pro Leu Gln Val Tyr
   275 280 285
   Trp Pro Gly Leu Ser Gly Ala Arg Pro Pro Gly Pro Ser Ala Pro Ala
   290 295 300
   Pro Pro Gly Ala Ala Arg Ala Ala Ala Pro Gly Leu Pro Leu Thr Leu
   305 310 315 320
   Leu Gly Lys Trp Gln Thr Tyr Glu Arg Gly Val Arg Gln Met Trp Leu
   325 330 335
   Thr Asn Met Thr Asp Ala Gly Tyr Gly Pro Leu Leu Arg Leu Ser Lys
   340 345 350
   Leu Thr Arg Arg Val Glu Thr Asp Phe Ser Gln Val Ser Leu Asp Val
   355 360 365
   Gly Ile Gln Asp Phe Glu Gly Arg Ser Tyr Gln Gly Trp His Arg His
   370 375 380
   Val Thr Leu Ala Ser Val Ala His Ala Leu Arg Met Leu Glu Gly Gly
   385 390 395 400
   Ala Ala Gly
<210> 31
   <211> 1212
   <212> DNA
   <213> micromonospora carbonacea subspecies aurantiaca
<400> 31
   atggtgccga cgctcgacgt ccgcgaggag gtgaccgcgg caaggtccga tccggacacc 60
   gtgtcccggt tctgcgccgc cctgctggcc tcgctgcccc gcgccgacca gcgacgcaag 120
   ggcgaactgt acgtccgggg gctgctgacc gcctccggcc gcaagaccat gcgcaacctg 180
   gccgccatcg ccgacgatcc ggcggcggca cagagcatgc accacttcat cagttgctcc 240
   acctgggact gggagaccgt ccgtgccgcg ctcgccggcc acctggaccg gacgctgtcg 300
   ccccgggcct gggtggtgcg gtcgatgctg gtgccgaaga ccggccggca ctcggtcggc 360
   gtggaacgcc ggtacgtgcc cgcgctgggc gagacggtca acagccagca gagctacggc 420
   ctctggctgg cctcggagac cgtcgccgcg cccatcaact ggcagttgtc catcggtaag 480
   ggttggctcc aggacaaccg cgcccgcgcg agcgtaccgg cggacgagga cggcacgacc 540
   agcgacggcg cggcggtgca ggcggtgctg aaggccgcgg cctggggaat cggccctcgc 600
   ccggtggtaa tggacgcccg gcactcggcg ctgcccccgc tgatcgaggc gttcaccacg 660
   gcgggtctgc ccttcctgct acggatcaac agcggctgca ccctgctggc cgccgggccc 720
   ggcccgcgcg agaaccgggt cgcggcggcc tccgccgagc acctgctcag cctgacgcgg 780
   gcccagcgcc gtccggtgga gtggatcgac ccggccagcc ccggcgcacg gcgcacgagc 840
   ctggtcgcac cgctacaggt ctattggccg ggcctgtccg gtgcccgccc gcccggtccg 900
   tccgccccgg ccccgccggg ggcggcgcgc gccgccgcgc ccgggctgcc cctgacactg 960
   ctcggcaagt ggcagaccta cgagcgcggc gtacggcaga tgtggctgac caacatgacc 1020
   gacgccgggt acggcccact gctgcggctg agcaagctca cccggcgggt cgagaccgac 1080
   ttctcccagg tcagcctcga cgtcggcatc caagacttcg agggtcggtc ataccaaggc 1140
   tggcaccggc acgtcacctt ggcgtccgtg gcgcacgccc tgcggatgct ggagggcggt 1200
   gccgccggat ag 1212
<210> 32
   <211> 481
   <212> PRT
   <213> micramonospora carbonacea subspecies aurantiaca
<400> 32
   Met Thr Ser Ala Ala His His Ser Pro His Pro Ala Lys Ala Asp Ala
   1               5                   10                  15
   Leu Met Asp Asp Ala His Ala Asp Ile Gly Ala Asp Ala Glu Ala Asp
               20                  25                  30
   Gly Arg Arg Leu Asp Arg Ala Ala Leu Arg Arg Val Ala Gly Leu Ser
           35                  40                  45
   Thr Glu Arg Ala Asp Val Thr Glu Val Glu Tyr Arg Gln Val Arg Leu
       50                  55                  60
   Glu Arg Val Val Leu Val Gly Val Trp Thr Ser Gly Thr Ala Asp Glu
   65                  70                  75                  80
   Ala Glu Arg Ser Leu Ala Glu Leu Ala Ala Leu Ala Glu Thr Ala Gly
                   85                  90                  95
   Ala Val Val Leu Asp Gly Val Ile Gln Arg Arg Asp Arg Pro Asp Pro
               100                 105                 110
   Ala Thr Tyr Ile Gly Ser Gly Lys Ala Arg Glu Leu Arg Asp Ile Val
           115                 120                 125
   Gln Glu Val Gly Ala Asp Thr Val Ile Cys Asp Gly Glu Leu Ser Pro
       130                 135                 140
   Ala Gln Leu Val Arg Leu Glu Glu Val Val Asp Ala Lys Val Val Asp
   145                 150                 155                 160
   Arg Thr Ala Leu Ile Leu Asp Ile Phe Ala Gln His Ala Thr Ser Arg
                   165                 170                 175
   Glu Gly Lys Ala Gln Val Ala Leu Ala Gln Met Gln Tyr Met Leu Pro
               180                 185                 190
   Arg Leu Arg Gly Trp Gly Gln Ser Leu Ser Arg Gln Met Gly Gly Gly
           195                 200                 205
   Ala Gly Gly Gly Gly Met Ala Thr Arg Gly Pro Gly Glu Thr Lys Ile
       210                 215                 220
   Glu Thr Asp Arg Arg Arg Ile His Glu Arg Met Ala Arg Leu Arg Arg
   225                 230                 235                 240
   Glu Ile Ala Glu Met Lys Ser Gly Arg Glu Leu Lys Arg Arg Asp Arg
                   245                 250                 255
   Arg Arg Asn Ser Val Pro Ser Val Ala Ile Ala Gly Tyr Thr Asn Ala
   260 265 270
   Gly Lys Ser Ser Leu Leu Asn Arg Leu Thr Gly Ala Ser Val Leu Val
   275 280 285
   Gln Asn Ala Leu Phe Ala Thr Leu Asp Pro Thr Val Arg Arg Ala Thr
   290 295 300
   Thr Pro Ser Gly Arg Ser Tyr Thr Ile Thr Asp Thr Val Gly Phe Val
   305 310 315 320
   Arg His Leu Pro His His Leu Val Glu Ala Phe Arg Ser Thr Leu Glu
   325 330 335
   Glu Val Ala Glu Ala Asp Leu Leu Leu His Val Val Asp Gly Ala His
   340 345 350
   Pro Ala Pro Leu Glu Gln Leu Ala Ser Val Arg Ala Val Ile Arg Asp
   355 360 365
   Val Asp Ala Ala Gly Val Pro Glu Leu Val Val Ile Asn Lys Ala Asp
   370 375 380
   Ala Ala Thr Pro Ala Ala Leu Ala Ala Leu Ala Glu Ala Glu Pro His
   385 390 395 400
   His Val Val Val Ser Ala Arg Thr Gly Gln Gly Ile Asp Thr Leu Arg
   405 410 415
   Gln Leu Leu Glu Ala Ala Leu Pro His Arg Glu Val Arg Val Asp Val
   420 425 430
   Leu Ile Pro Tyr Val Ala Gly Ser Leu Val Ala Arg Val His Ala Asp
   435 440 445
   Gly Glu Val Leu Ala Glu Glu His Thr Ala Asp Gly Thr Leu Leu Gln
   450 455 460
   Ala Arg Val Ala Pro Asp Leu Ala Ala Glu Leu Ser Ala Tyr Ala Arg
   465 470 475 480
   Thr
<210> 33
   <211> 1446
   <212> DNA
   <213> micromonospora carbonacea subspecies aurantiaca
<400> 33
   atgacctccg cagcgcacca ttccccgcat ccggcgaagg ccgacgccct gatggacgac 60
   gcccacgccg acatcggggc cgatgccgag gccgacggtc gacggctcga ccgggccgcc 120
   ctgcggcggg tcgccgggct gtcgaccgag agggccgacg tcacggaggt cgagtaccgg 180
   caggtgcggc tggagcgcgt cgtcctggtc ggcgtgtgga cctcgggcac cgccgacgag    240
   gccgaacggt ccctcgccga gctggcggca ctcgccgaga ccgcgggagc cgtggtgctc    300
   gacggggtga tccagcgccg cgaccggccc gacccggcga cgtacatcgg ctccggcaag    360
   gcgcgggagt tgcgggacat cgtccaggag gtgggggccg acacggtgat ctgcgacggt    420
   gagctgagcc cggcccaact ggtacgcctc gaagaggtcg tcgacgccaa ggtggtggac    480
   cgcaccgcgc tgatcctcga catcttcgcc cagcacgcca cgtcccgcga ggggaaggcg    540
   caggtggccc tggcacagat gcaatacatg ctgccgcggc tgcgcggctg gggccagtcg     600
   ctctcccggc agatgggcgg aggtgccggc ggcggtggca tggccacccg ggggcccggc     660
   gagaccaaga tcgagaccga ccggcggcgc atccacgaga ggatggcccg gctccgacgg    720
   gagatcgcgg agatgaagtc cggccgcgaa ctcaagcgcc gcgatcggcg gcgcaacagc    780
   gtcccgtcgg tcgcgatcgc cggttacacc aacgccggca agtcctcgct gctcaaccgg    840
   ctcactggcg cgagcgtgct ggtgcagaac gcgctgttcg ccaccctcga cccgacggtg    900
   cgccgggcca ccaccccgag cgggcgcagc tacacgatca ccgacaccgt cggattcgtc    960
   cggcacctgc cgcaccacct ggtggaggcg ttccgctcca ccctggaaga ggtggccgag    1020
   gccgacctcc tgctgcacgt ggtggacggc gcccaccccg ccccgctgga gcagctcgcc    1080
   tcggtgcgcg cggtcatccg ggacgtggac gcggcgggag tgcccgaact cgtcgtgatc    1140
   aacaaggccg acgccgccac cccggccgcc ctggccgcgt tggcggaggc cgagccgcac    1200
   cacgtcgtcg tctcggcccg caccggtcag ggcatcgaca cgcttcggca gttgctggag    1260
   gccgcgctgc cgcaccggga ggtccgggtc gacgtcctga tcccgtacgt cgcgggcagc    1320
   ctcgtggccc gggtgcacgc cgacggcgag gtgctggccg aggagcacac ggccgacggc    1380
   accctgctgc aggcgcgggt ggcccccgac ctggctgccg agctcagcgc gtacgccagg    1440
   acgtga                                                               1446
<210> 34
   <211> 408
   <212> PRT
   <213> micromonospora carbonacea subspecies aurantiaca
<400> 34
   Met Lys Arg Asp Leu Gly Asp Leu Ala Leu Phe Gly Gly His Ala Ser
   1               5                   10                  15
   Phe Leu Gln Gln Ile His Val Gly Arg Pro Asn Arg Ile Asp Arg Ala
               20                  25                  30
   Arg Leu Phe Asp Arg Leu Ser Trp Ala Leu Asp Asn Glu Trp Leu Thr
   35 40 45
   Asn Asn Gly Pro Leu Ala Arg Glu Phe Glu Glu Arg Val Ala Asp Met
   50 55 60
   Val Gly Val Gly Asn Cys Val Ala Thr Cys Asn Ala Thr Val Ala Leu
   65 70 75 80
   Gln Leu Leu Ala His Ala Thr Glu Leu Thr Gly Glu Val Ile Met Pro
   85 90 95
   Ser Leu Thr Phe Ala Ala Thr Ala His Ala Val Arg Trp Leu Gly Leu
   100 105 110
   Glu Pro Val Phe Cys Asp Ile Asp Pro Arg Thr Gly Cys Leu Asp His
   115 120 125
   Val Ala Val Ala Ala Ala Ile Thr Pro Arg Thr Ser Ala Val Phe Gly
   130 135 140
   Val His Leu Trp Gly Arg Pro Cys Asp Val Asn Ala Leu Glu Lys Val
   145 150 155 160
   Thr Ala Asp Ala Gly Leu Arg Leu Phe Phe Asp Ala Ala His Ala Ile
   165 170 175
   Gly Cys Thr Ser Gln Gly Arg Pro Val Gly Arg Phe Gly His Ala Glu
   180 185 190
   Val Phe Ser Phe His Ala Thr Lys Val Val Asn Ala Phe Glu Gly Gly
   195 200 205
   Ala Ile Val Thr Asp Asp Asp Asp Leu Ala His Arg Val Arg Ser Leu
   210 215 220
   Ala Asn Phe Gly Phe Gly Leu His Ser Pro Ser Ala Ala Gly Gly Thr
   225 230 235 240
   Asn Ala Lys Met Ser Glu Ala Ser Ala Ala Met Gly Leu Thr Ser Leu
   245 250 255
   Asp Ala Phe Pro Glu Val Ala Arg His Asn Gln Ala Asn Tyr Glu Gln
   260 265 270
   Tyr Cys Gly Glu Leu Ala Arg Ile Pro Gly Leu Ser Val Ile Asp Phe
   275 280 285
   Ala Pro Asp Glu Arg His Asn Tyr Gln Tyr Val Ile Val Glu Ile Asp
   290 295 300
   Pro Asp Val Thr Gly Leu His Arg Asp Leu Leu Val Asp Leu Leu Arg
   305 310 315 320
   Ala Glu Asn Val Val Ala Gln Arg Tyr Phe Ser Pro Ala Cys His Gln
   325 330 335
   Leu Glu Pro Tyr Arg Ser Arg Gln Gln Phe Gln Leu Pro His Thr Glu
   340 345 350
   Arg Leu Ser Ala Arg Val Leu Ala Leu Pro Thr Gly Ser Ala Ile Ser
   355 360 365
   Arg Glu Asp Ile Arg Arg Val Cys Asn Ile Val Arg Leu Ala Val Ser
   370 375 380
   Arg Gly Phe Glu Leu Thr Ala Arg Trp Gln Gln Gln Pro Gly Pro Asp
   385 390 395 400
   Gly Gln Ser Val Val Ala Pro Gly
   405
<210> 35
   <211> 1227
   <212> DNA
   <213> micromonospora carbonacea subspecies aurantiaca
<400> 35
   atgaagcgag atctcgggga tctggcactc ttcggaggac acgccagctt cctccagcag 60
   atccacgtcg ggcgccccaa ccggatcgat cgggccaggc tgttcgaccg gctgtcctgg 120
   gcgctcgaca acgagtggtt gaccaacaac gggccgctgg cacgggagtt cgaggagcgg 180
   gtcgccgaca tggtcggggt cggcaactgc gtggcgacgt gcaacgccac ggtggccctc 240
   cagctgctcg cgcacgccac cgagctgacc ggtgaggtga tcatgccatc gctcaccttc 300
   gccgcgaccg cacacgcggt gcgctggctc gggctggagc cggtcttctg cgacatcgac 360
   ccgcgcaccg gatgcctcga ccacgtggcg gtcgccgcgg ccatcacgcc gcgcacgtcg 420
   gcggtcttcg gcgtccacct ctggggccgc ccctgcgacg tcaacgcgct ggagaaggtg 480
   accgccgacg cgggcctgcg cctgttcttc gacgccgccc acgccatcgg gtgcacctca 540
   cagggccgcc cggtggggcg gttcggccac gccgaggtgt tcagcttcca cgcgacgaag     600
   gtcgtcaacg ccttcgaggg cggggcgatc gtcaccgacg acgacgacct cgcccaccgc     660
   gtccgctccc tggcgaactt cggcttcggc ctgcacagcc ccagcgcggc cggcggcacc 720
   aacgcgaaga tgagcgaggc gtccgccgcc atggggctca cctcgctcga cgcgttcccc 780
   gaggtggccc gccacaacca ggccaactac gagcagtact gcggtgagct ggcccggatt 840
   cccggcctca gcgtgatcga cttcgccccc gacgagcggc acaactacca gtacgtgatc 900
   gtcgagatcg acccggacgt caccgggttg caccgcgacc tgctcgtcga cctgctccgg 960
   gccgagaacg tcgtggcgca gcgctacttc tcgccggcct gtcaccaatt ggagccctac 1020
   cggtcccggc agcagttcca gctgccgcac accgagcggc tctcggcgcg cgtcctggcg 1080
   ctgccgaccg gctccgccat ctcccgggaa gacatccgca gggtgtgcaa catcgtgcgg 1140
   ttggcggtct cccggggatt cgaattgacc gctcggtggc agcagcagcc cgggcccgac 1200
   ggacagagcg tggtggcacc cggttga 1227
<210> 36
   <211> 488
   <212> PRT
   <213> micromonospora carbonacea subspecies aurantiaca
<400> 36
   Val Gly Gly Pro Val Thr Met Glu Ile Ser Ala Ser Asn Pro Val Ala
   1 5 10 15
   Thr Cys Ala Val Pro Gly Ser Asp Pro Thr Ala Ala Ala Arg Val Leu
   20 25 30
   Tyr Asp Glu Val Ala Gly Ser Gly Ile Val Pro Pro Ala Glu Ile Gly
   35 40 45
   Ala Ala Ala Gln Gly Leu Val Ala Leu Ala Arg Ile Tyr Gly Thr Thr
   50 55 60
   Pro Phe Leu Pro Leu Glu Gln Ala Arg Arg Glu Ile Gly Leu Asp Arg
   65 70 75 80
   Ala Gly Phe Gly Arg Leu Leu Asp Leu Phe Ala Arg Ile Pro Gly Leu
   85 90 95
   Arg Thr Ala Val Glu Asn Gly Pro Ser Gly Arg Tyr Trp Thr Asn Thr
   100 105 110
   Val Leu Gly Leu Glu Arg Ala Gly Val Phe Asp Ala Val Leu Asp Arg
   115 120 125
   Arg Pro Ala Phe Pro His Leu Val Gly Leu Tyr Pro Gly Pro Thr Cys
   130 135 140
   Met Phe Arg Cys His Phe Cys Val Arg Val Thr Gly Ala Arg Tyr Gln
   145 150 155 160
   Ala Ser Ala Leu Asp Asp Gly Asn Ala Met Phe Ala Ser Val Ile Asp
   165 170 175
   Glu Val Pro Ala His Asn Arg Asp Ala Val Tyr Val Ser Gly Gly Leu
   180 185 190
   Glu Pro Leu Thr Asn Pro Gly Leu Gly Ala Leu Val Ser Arg Ala Ala
   195 200 205
   Glu Arg Gly Phe Arg Ile Ile Leu Tyr Thr Asn Ser Phe Ala Leu Thr
   210 215 220
   Glu Gln Lys Leu Lys Gly Glu Arg Gly Leu Trp Ser Leu His Ala Ile
   225 230 235 240
   Arg Thr Ser Leu Tyr Gly Leu Asn Asp Glu Glu Tyr Arg Ala Thr Thr
   245 250 255
   Gly Lys Gln Gly Ala Phe Thr Arg Val Arg Ala Asn Leu Thr Arg Phe
   260 265 270
   Gln Gln Leu Arg Ala Glu Arg Gly Glu Pro Val Arg Leu Gly Leu Ser
   275 280 285
   Tyr Ile Val Leu Pro Gly Arg Ala Gly Arg Leu Ser Ala Leu Ile Asp
   290 295 300
   Phe Val Ala Glu Leu Asn Glu Ala Ala Pro Asp Arg Pro Leu Asp Tyr
   305 310 315 320
   Ile Asn Leu Arg Glu Asp Tyr Ser Gly Arg Pro Asp Gly Lys Leu Ser
   325 330 335
   Leu Asp Glu Arg Ala Glu Leu Gln Ala Glu Leu His Arg Phe Arg Glu
   340 345 350
   Arg Ala Met Gln Arg Thr Pro Thr Leu His Ile Asp Tyr Gly Tyr Ala
   355 360 365
   Leu His Ser Leu Met Thr Gly Ser Asp Val Glu Leu Val Arg Ile Arg
   370 375 380
   Pro Glu Thr Met Arg Pro Ala Ala His Pro Gln Val Ser Val Gln Val
   385 390 395 400
   Asp Ile Leu Gly Asp Val Tyr Leu Tyr Arg Glu Ala Ala Phe Pro Gly
   405 410 415
   Leu Ala Gly Ala Asp Arg Tyr Arg Ile Gly Thr Val Ser Pro Gly Thr
   420 425 430
   Thr Leu Ala Gln Val Val Glu Thr Phe Val Thr Ser Gly Gly Ser Val
   435 440 445
   Val Ala Lys Pro Gly Asp Glu Tyr Phe Leu Asp Gly Phe Asp Gln Ala
   450 455 460
   Val Thr Ala Arg Leu Asn Gln Met Glu Thr Asp Val Ala Asp Gly Trp
   465 470 475 480
   Gly Asp Arg Arg Gly Phe Leu Arg
   485
<210> 37
   <211> 1467
   <212> DNA
   <213> micromonospora carbonacea subspecies aurantiaca
<400> 37
   gtgggagggc ccgtgaccat ggagatctcc gcctcgaatc ccgtggcgac ctgcgctgtc    60
   cccggcagcg acccgaccgc ggcggcgcgc gtgctgtacg acgaggtcgc cgggtcagga   120
   atcgtgccgc cggcagagat cggggccgcc gcccaggggt tggtggcatt ggcacgcatc   180
   tacgggacca caccttttct gccgcttgag caggcccgcc gcgaaatcgg cctggaccgg   240
   gccgggttcg ggcggctgct ggacctgttc gcccggattc ccgggttgcg caccgcagtg   300
   gagaacggac cgtccggtcg ctactggacc aacacggtgc tcggcctcga aagggccggc   360
   gtcttcgacg ccgtgctcga ccggaggccg gcgtttccgc atctcgtcgg gctctacccg   420
   ggccccacgt gcatgttccg ctgtcacttc tgcgtaaggg tcaccggggc ccgctaccag   480
   gcctcggcgc tggacgacgg gaacgccatg ttcgcctctg tcatcgacga ggtccccgcg   540
   cacaaccgcg acgcggtgta cgtctccggt ggcctcgagc cactcaccaa ccccgggctc   600
   ggtgcactgg tcagccgggc ggccgagcgg ggatttcgga tcatcctcta caccaactcg   660
   ttcgccctca cggagcagaa gctcaagggt gagcggggat tgtggagcct gcacgccatc   720
   cgcacgtcgc tgtacgggtt gaacgacgag gaataccggg cgaccaccgg caagcagggg   780
   gccttcaccc gggtacgggc gaacctcacg cggttccagc agctgcgtgc cgagcggggc   840
   gagccggtgc ggctcggcct cagctacatc gtcctgcccg gccgcgccgg gcggctgagc   900
   gcgctgatcg acttcgtcgc cgagctcaac gaggcggcac cggaccgccc gctggactac   960
   atcaacctgc gggaggacta cagcgggcgg ccggacggga agctctccct ggacgagcgc  1020
   gccgagctcc aggccgagct gcaccggttc cgggagaggg caatgcagcg gacgccgacc  1080
   ctgcacatcg actacggcta cgccctgcac agcctgatga cgggaagcga cgtggagctc  1140
   gtgcgtatcc ggccggagac gatgcgccct gcggcccacc cgcaggtgtc ggtgcaggtg  1200
   gatatcctcg gtgatgtcta cctctatcgg gaggcggcgt ttccgggcct ggccggtgcc  1260
   gaccgctatc gcatcggcac ggtatctccc ggcacgacgt tggcgcaggt ggtggagacg  1320
   ttcgtgacca gcggcggatc ggtggtcgcg aagcctggcg acgaatactt cctggacgga  1380
   ttcgaccagg cggtgaccgc gcggctgaac cagatggaga ccgacgtcgc cgatggctgg  1440
   ggagaccgac ggggtttcct ccgctga                                       1467
<210> 38
   <211> 277
   <212> PRT
   <213> micromonospora carbonacea subspecies aurantiaca
<400> 38
   Met Pro Tyr Ile Gln His Ala Gly Arg His Glu Phe Gly Gln Asn Phe
   1               5                   10                  15
   Leu Val Asp Arg Ser Val Ile Asp Asp Phe Val Glu Leu Val Ala Arg
               20                  25                  30
   Thr Asp Gly Pro Ile Val Glu Ile Gly Ala Gly Asp Gly Ala Leu Thr
           35                  40                  45
   Leu Pro Leu Ser Arg Gln Gly Arg Glu Leu Thr Ala Val Glu Ile Asp
       50                  55                  60
   Ser Lys Arg Ser Lys Arg Leu Ser Arg Gln Thr Pro Asp Asn Val Thr
   65                  70                  75                  80
   Val Val Cys Ala Asp Val Leu Ser Phe Arg Phe Pro Gln His Pro His
                   85                  90                  95
   Val Val Val Gly Asn Ile Pro Phe His Val Thr Thr Pro Ile Val Arg
               100                 105                 110
   Ala Leu Leu Ala Ala Asp His Trp His Thr Ala Val Leu Leu Val Gln
           115                 120                 125
   Trp Glu Val Ala Arg Arg Arg Ala Gly Val Gly Gly Ala Thr Leu Leu
       130                 135                 140
   Thr Ala Ser Trp Trp Pro Trp Tyr Asp Phe Glu Leu His Ser Arg Val
   145                 150                 155                 160
   Pro Ala Arg Ala Phe Arg Pro Val Pro Ser Val Asp Gly Gly Leu Phe
                   165                 170                 175
   Ser Met Val Arg Arg Gly Thr Pro Leu Val Asp Asp Arg Arg Gly Tyr
               180                 185                 190
   Gln Glu Phe Val Arg Leu Val Phe Thr Gly Lys Gly His Gly Leu Pro
           195                 200                 205
   Glu Ile Leu Gln Arg Thr Gly Arg Ile Ala Arg Lys Asp Gln Gln Asp
       210                 215                 220
   Trp Gln Arg Ala Asn Arg Val Gly Pro Gln His Leu Pro Lys Asp Leu
   225                 230                 235                 240
   Thr Ala His Gln Trp Ala Ser Leu Trp His Leu Val Ala Pro Ala Arg
                   245                 250                 255
   Pro Ala Gly Pro Arg Arg Pro Ala Pro Arg Arg Pro Gly Ser Pro Ala
               260                 265                 270
   Ser Ala Arg Arg Arg
           275
<210> 39
   <211> 834
   <212> DNA
   <213> micromonospora carbonacea subspecies aurantiaca
<400> 39
   atgccctaca tccagcacgc cgggcgacat gaattcggcc agaatttcct ggtcgaccgc    60
   tcggtgatcg acgatttcgt cgaactcgtc gcccggaccg acggccctat cgtggagatc   120
   ggcgccggcg acggtgcgct gaccctaccc ctgagccggc agggaaggga gttgaccgca   180
   gtggagatcg actccaagcg ttccaagcgg ctcagccggc agacacccga caacgtcacc   240
   gtggtctgcg cggatgtcct gagcttccgg ttcccccagc atccgcacgt ggtcgtcggg   300
   aacatcccct tccacgtgac cacccccatc gtgcgggctc tcctcgccgc ggaccactgg   360
   cacacggcgg tgctgctggt gcagtgggag gtggcccgca ggcgggccgg cgtcggcggc   420
   gcgacgctgc tgaccgcgag ctggtggccc tggtacgact tcgaactgca ctcccgggtt   480
   ccggcccgcg ccttccggcc tgtcccttcc gtcgacggcg ggctgttctc catggtccgt   540
   cgcgggaccc cgctggtcga cgaccggagg ggttaccagg aattcgtccg gctggtgttc   600
   accggcaagg ggcacggatt gccggagatc cttcagcgga ccgggcggat cgcccgcaag   660
   gaccagcagg actggcaacg ggccaaccgg gtggggccgc agcacctgcc caaggacctg   720
   accgcccacc agtgggcctc cctgtggcac ctggtggcac ccgcccggcc ggccggcccc   780
   cgccgtccgg caccgcgccg gccaggaagc cccgcttcgg cgcgccggcg ctga         834

## Description

### FIELD OF INVENTION:

The present invention relates to nucleic acid molecules that encode proteins that direct the synthesis of macrolides, in particular the 16-member macrolide rosaramicin. The present invention also is directed to the use of nucleic acids and proteins to produce compounds exhibiting antibiotic activity based on the rosaramicin structure.

### BACKGROUND:

Rosaramicin is a 16-member macrolide antibiotic. Macrolides consitute a group of antibiotics mainly active against Gram-positive bacteria. They have clinical applications in the treatment of bacterial infections. Macrolides compounds are structurally characterized by a macrolide lactone ring to which one or several deoxy-sugars moieties are attached.

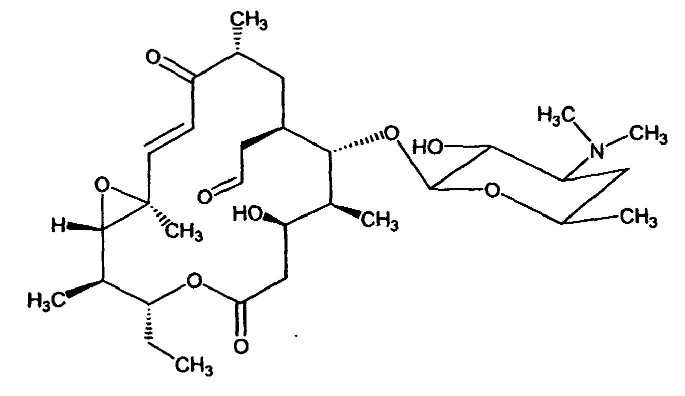

The carbohydrate ligands and macrolide lactone ring serve as molecular recognition elements critical for biological activity. Variations in the sugar composition of a macrolide or in the structure of the macrolide lactone ring may vary the biological activity of the molecule. Elucidation of gene clusters involved in the biosynthesis of rosaramicin expands the repertoire of genes and proteins useful to macrolides via combinatorial biosynthesis.

The increasing number of microbial strains that have acquired resistance to the currently available antibiotic compounds is recognized as a dangerous threat to public health. The genes and proteins involved in the biosynthesis of rosaramicin may be used to generate new unnatural compounds having desirable biological activity. The genes and proteins from the rosaramicin locus may also be used as probes to identify new rosaramicin-like natural products.

The genome of many microorganisms contains multiple natural product biosynthetic loci that are not normally expressed in nature or under conventional experimental conditions. For example, twenty-five secondary metabolic gene clusters in the genome of the actinomycete *Streptomyces avermitilis* were identified by whole genome shotgun sequencing of the genome despite the fact that the organism was known to produce only two antimicrobial natural products (Osura *et al.* PNAS, vol. 98, no. 21 12215-12220). Furthermore, Database entry EMBL SF08223 describes Streptomyces fradiae cytochrome P-450, dTDP-glucose synthase, dTDP-glucose dehydratase, thioesterase, TylCVI (tylCVI),and TylR (tylR) genes, and complete CDS.

Database entry EMBL SFTYLORF describes Streptomyces fradiae tylMlll (orf 1*), tylmll (orf2*), tylMl (orf3*) and ccr (orf4*) genes, which are tylosin biosynthetic genes from the tylLM region.

Database entry SWALL Q9ZBK1 describes the protein sequence of crotonyl CoA reductase of the model actinomycete Streptomyces coelicor A3(2).

In addition, US-patent US-A-4234690 describes rosaramicin as a new broad spectrum antibiotic which is described together with methods for its production via the cultivation under controlled conditions of Micromonosporia rosaria, a new species of Micromonospora and with methods for purifying and using the same. US-patent US-A-4234690 further describes that the 2'-monoesters and 3,2'-diesters and their addition salts of rosaramicin also possess antibiotic activity. An important new source of antimicrobial compounds lies in the products of cryptic biosynthetic loci. It is desirable to discover and characterize a biosynthetic locus producing an antimicrobial product and present in the genome of organisms not known to product the antimicrobial product of the locus.

### SUMMARY OF THE INVENTION:

*Micromonospora carbonacea* is known to produce the antimicrobial orthosomycin natural product eveminomicin. *Micromonospora carbonacea* was not previously reported to produce other natural products. We have surprisingly discovered, in the *Micromonospora carbonacea* genome, a type I polyketide biosynthetic gene cluster directed to the production of a rosaramicin-type polyketide.

The invention provides polynucleotides and polypeptides useful in the production and engineering of macrolides. In one embodiment, the polynucleotide molecules are selected from the contiguous DNA sequence SEQ ID NO: 1. Other embodiments of the polynucleotides and polypeptides are provided in the accompanying sequence listing. SEQ ID NOS: 3, 5, 7, 9, 11, 13, 15, 17, 19, 21, 23, 25, 27, 29, 31, 33, 35, 37, 39 provide nucleic acids responsible for biosynthesis of the 16-member macrolide rosaramicin. SEQ ID NOS: 2, 4, 6, 8, 10, 12, 14, 16, 18, 20, 22, 24, 26, 28, 30, 32, 34, 36, 38 provide amino acid sequences for proteins responsible for biosynthesis of the16-member macrolide rosaramicin. Certain embodiments of the invention specifically exclude one or more of open reading frames of the rosaramicin biosynthetic locus, most notably any one or more of ORFs 3, 11, 13, 16, 17 and 18 (SEQ ID NOS: 7, 23, 27, 33, 35 and 37) and the corresponding gene products (SEQ ID NOS: 6, 22, 26, 32, 34 and 36) deduced therefrom, although other ORFs and polypeptides listed in the sequence listing can be excluded from certain embodiments without departing from the scope of the invention.

The polynucleotides and polypeptides of the invention provide the machinery for producing novel compounds based on the structure of rosaramicin. The invention allows direct manipulation of rosaramicin and related chemical structures via chemical engineering of the enzymes involved in the biosynthesis of rosaramicin, modifications which may not be presently possible by chemical methodology because of the complexity of the structures. The invention can also be used to introduce "chemical handles" into normally inert positions that permit subsequence chemical modifications. Several general approaches to achieve the development of novel macrolides are facilitated by the methods and compositions of the present invention. For example, tylosin is structurally related to rosaramicin but, unlike rosaramicin, it does not contain an epoxide. Accordingly, genes and proteins disclosed herein may be used to enzymatically create a tylosin derivative that contains an epoxide modification.

Various macrolide structures can be generated by genetic manipulation of the rosaramicin gene cluster or use of various genes from the rosaramicin gene cluster in accordance with the methods of the invention. The invention can be used to generate a focused library of analogs around a macrolide lead candidate to fine-tune the compound for optimal properties. Genetic engineering methods of the invention can be directed to modify positions of the molecule previously inert to chemical modifications. Known techniques allow one to manipulate a known macrolide gene cluster either to produce the macrolide compound synthesized by that gene cluster at higher levels than occur in nature or in hosts that otherwise do not produce the macrolide. Known techniques allow one to produce molecules that are structurally related to, but distinct from, the macrolide compounds produced from known macrolide gene clusters. Cloning, analysis, and manipulation by recombinant DNA technology of genes that encode rosaramicin gene products can be performed according to known techniques.

Thus, in a first aspect the invention provides a nucleic acid comprising a sequence selected from the group consisting of SEQ ID NO:1; the sequences complementary to SEQ ID NO: 1; fragments comprising at least 100, 200, 300, 500; 1000, 2000 or more consecutive nucleotides of SEQ ID NO: 1; and fragments comprising at least 100, 200, 300, 500, 1000, 2000 or more consecutive nucleotides of the sequences complementary to SEQ ID NO: 1. Preferred embodiments of this aspect include nucleic acids capable of hybridizing to the above sequences under conditions of moderate or high stringency; nucleic acid comprising at least 100, 200, 300, 500, 1000, 2000 or more consecutive bases of the above sequences; and nucleic acid having at least 70%, 75%, 80%, 85%, 90%, 95%, 97% or 99% homology to the above sequences as determined by analysis with BLASTN version 2.0 with the default parameters. The aforementioned fragments, hybridizing nucleic acids and homologues are capable of directing the synthesis of a rosaramicin compound or analogue.

Further embodiments of this aspect of the invention include a nucleic acid comprising a sequence selected from the group consisting of SEQ ID NOS: 3, 5, 7, 9, 11, 13, 15, 17, 19, 21, 23, 25, 27, 29, 31, 33, 35, 37, 39 and the sequences complementary thereto; a nucleic acid comprising at least 50, 75, 100, 200, 500, 800 or more consecutive bases of a sequence selected from the group consisting of SEQ ID NOS: 3, 5, 7, 9, 11, 13, 15, 17, 19, 21, 23, 25, 27, 29, 31, 33, 35, 37, 39 and the sequences complementary thereto; and a nucleic acid capable of hybridizing to the above listed nucleic acids under conditions of moderate or high stringency, and nucleic acid having at least 70%, 75%, 80%, 85%, 90%, 95%, 97% or 99% homology to the nucleic acid of SEQ ID NOS: 3, 5, 7, 9, 11, 13, 15, 17, 19, 21, 23, 25, 27, 29, 31, 33, 35, 37, 39 as determined by analysis with BLASTN version 2.0 with the default parameters, wherein said homologues are capable of directing the synthesis of a rosaramicin compound or analogue.

In a second embodiment, the invention provides a polypeptide comprising a sequence selected from the group consisting of SEQ ID NOS: 2, 4, 6, 8, 10, 12, 14, 16, 18, 20, 22, 24, 26, 28, 30, 32, 34, 36, 38; a polypeptide comprising at least 50, 75, 100, 200, 300 or more consecutive amino acids of the polypeptides of SEQ ID NOS: 2, 4, 6, 8, 10, 12, 14, 16, 18, 20, 22, 24, 26, 28, 30, 32, 34, 36, 38; and a polypeptide having at least 70%, 75%, 80%, 85%, 90%, 95%, 97%, or 99% homology to the polypeptide of SEQ ID NOS: 2, 4, 6, 8, 10, 12, 14, 16, 18, 20, 22, 24, 26, 28, 30, 32, 34, 36, 38 as determined by analysis with BLASTP version 2.2.2 with the default parameters, wherein the polypeptide is capable of directing the synthesis of rosaramicin. In a further aspect, the invention provides a polypeptide comprising one or two or three or five or more or the above polypeptide sequences.

The invention also provides recombinant DNA expression vectors containing the above nucleic acids. The polynucleotides and the methods of the invention enable one skilled in the art to create recombinant host cells with the ability to produce macrolides. Thus, the invention provides a method of preparing a macrolide compound, said method comprising transforming a heterologous host cell with a recombinant DNA vector that encodes at least one of the above nucleic acids, and culturing said host cell under conditions such that a macrolide is produced. In one aspect, the method is practiced with a *Streptomyces* host cell. In another aspect, the macrolide produced is rosoramicin. In another aspect, the macrolide produced is a compound related in structure to rosaramicin. The invention also provides a method for producing a rosaramicin compound by culturing *Micromonospora carbonacea* under conditions allowing for expression of its endogenous rosaramicin biosynthetic locus.

The invention also encompasses a method of invention for detecting by traditional hybridization, putative macrolide gene clusters or macrolide-producing microorganisms using compositions of the invention. In one embodiment, a polypeptide encoding one or more of the polyketide synthase proteins (SEQ ID NOS: 10, 12, 14, 16 and 18) or fragments thereof are used as probes to detect putative macrolide gene clusters by hybridization.

### BRIEF DESCRIPTION OF THE DRAWINGS

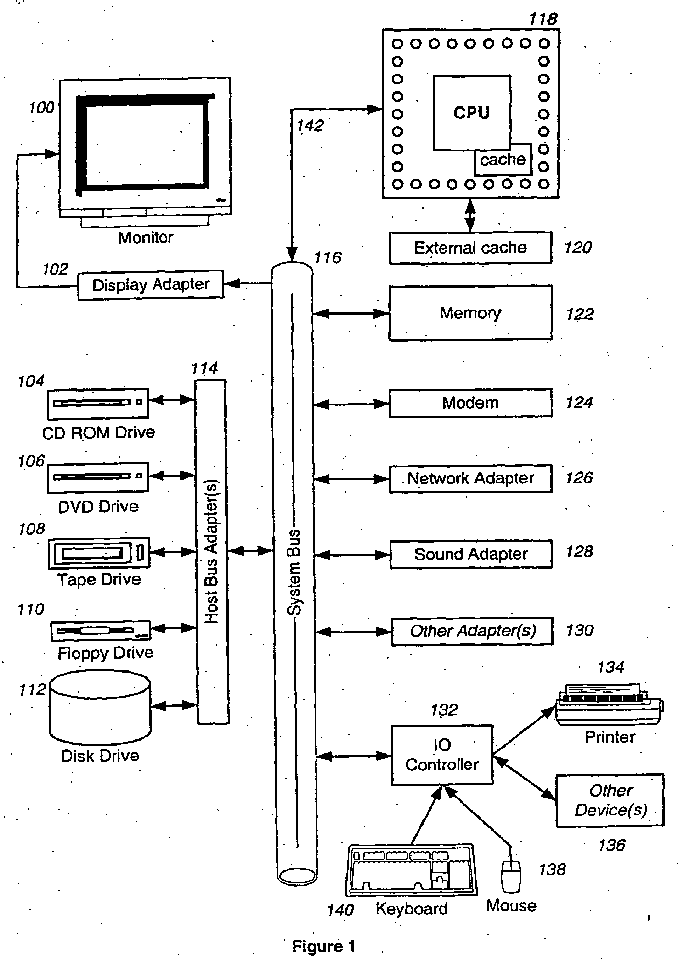

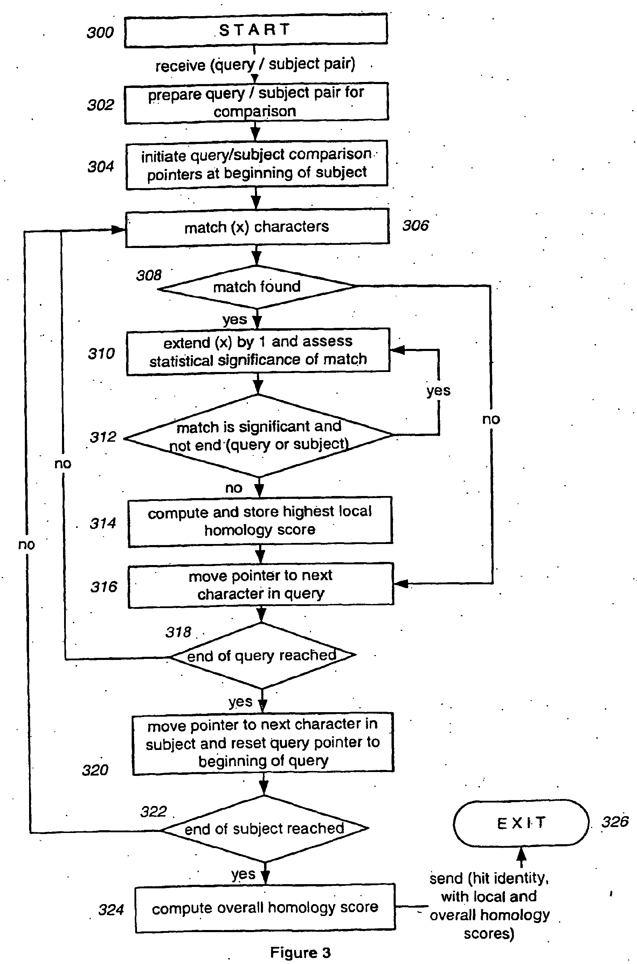

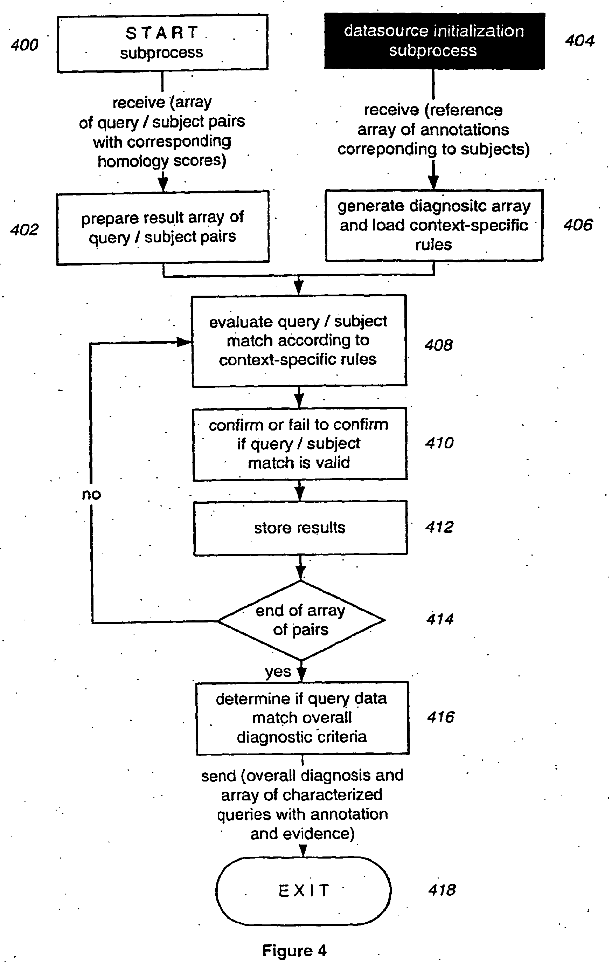

The present invention will be further understood from the following description with reference to the following figures, wherein Figure 1 to 4 do not form part of the application:
Figure 1 is a block diagram of a computer system which implements and executes software tools for the purpose of comparing a query to a subject, wherein the subject is selected from the reference sequences of the invention.

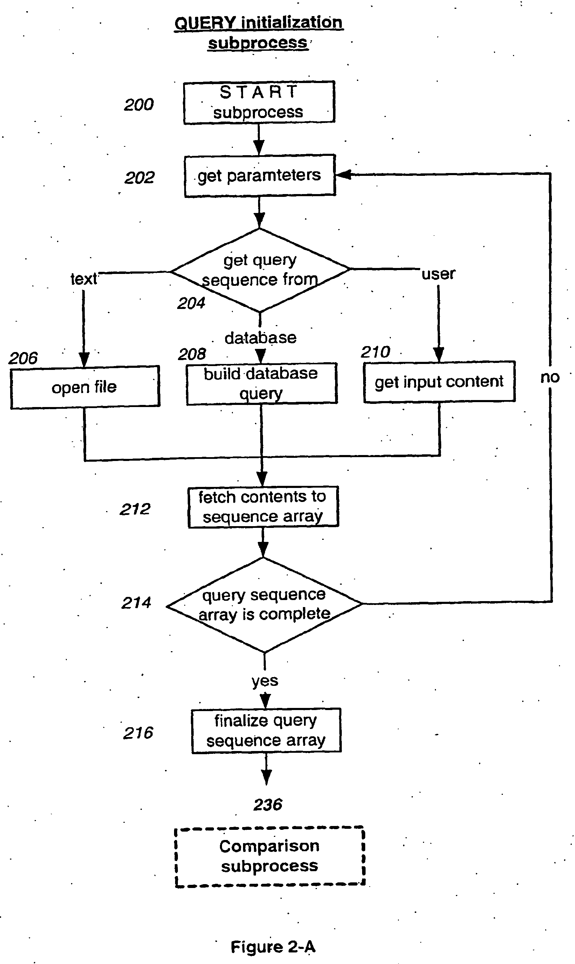

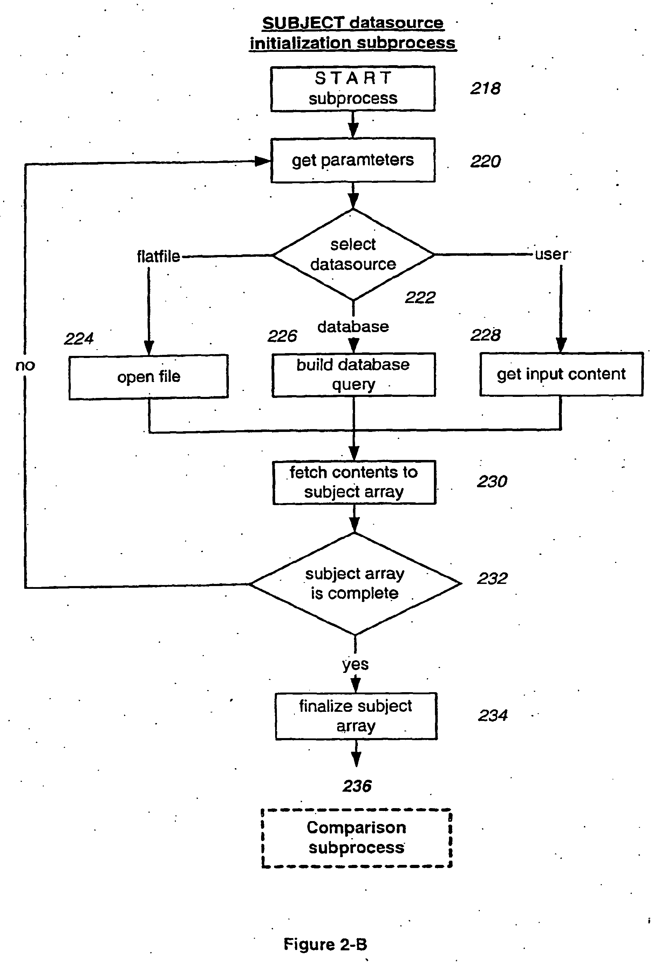

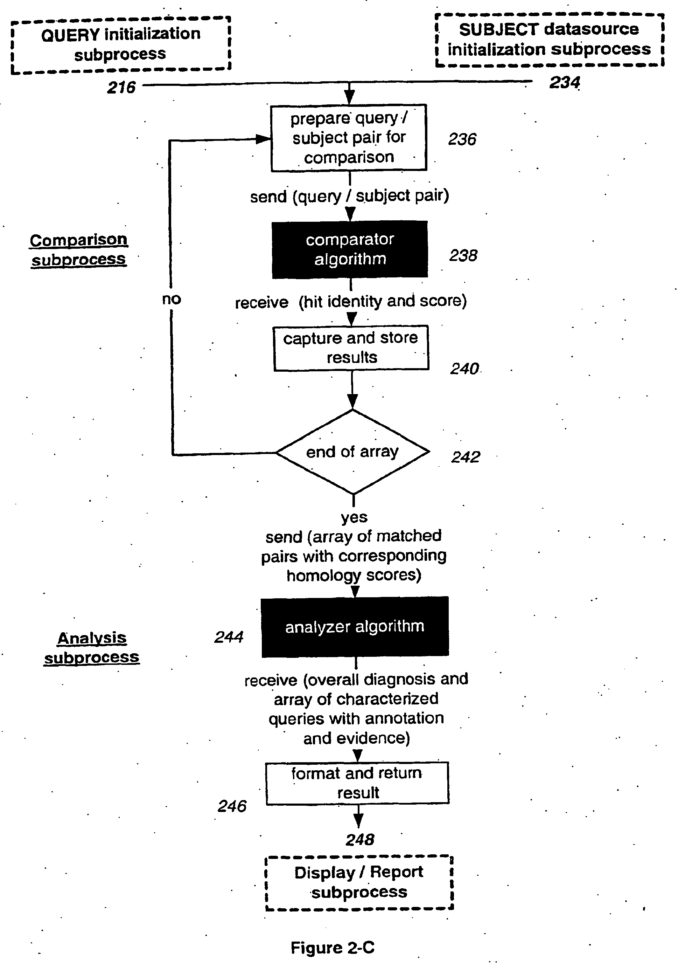

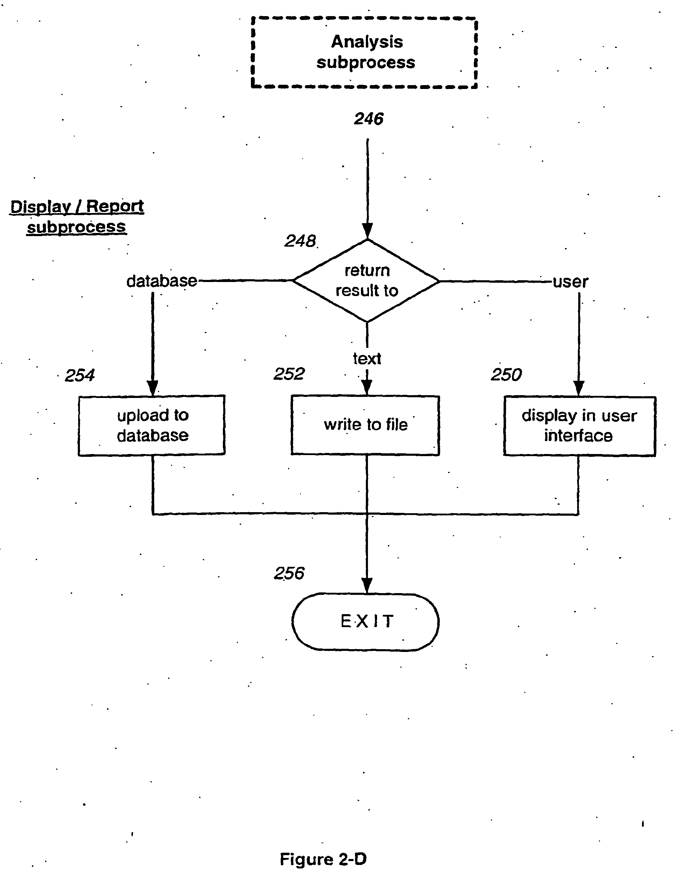

Figures 2A, 2B, 2C and 2D are flow diagrams of a sequence comparison software that can be employed for the purpose of comparing a query to a subject, wherein the subject is selected from the reference sequences of the invention, wherein Figure 2A is the query initialization subprocess of the sequence comparison software, Figure 2B is the subject datasource initialization subprocess of the sequence comparison software, Figure 2C illustrates the comparison subprocess and the analysis subprocess of the sequence comparison software, and Figure 2D is the Display/Report subprocess of the sequence comparison software.
Figure 3 is a flow diagram of the comparator algorithm (238) of Figure 2C which is one embodiment of a comparator algorithm that can be used for pairwise determination of similarity between a query/subject pair.
Figure 4 is a flow diagram of the analyzer algorithm (244) of Figure 2C which is one embodiment of an analyzer algorithm that can be used to assign identity to a query sequence, based on similarity to a subject sequence, where the subject sequence is a reference sequence of the invention.
Figure 5 is a graphical depiction of the rosaramicin biosynthetic locus showing, at the top of the figure, the regions covered by the three deposited cosmid clones D10CK, D10CF and D10CJ; a scale in kilobase pairs; the positioning of the open reading frames on a continuous black line representing the continuous DNA sequence (SEQ ID NO: 1); and the relative position and orientation of 19 ORFs referred to by number at the bottom of figure.
Figure 6 illustrates the construction of the rosaramicin backbone by the Type 1 polyketide synthase enzymes (PKS) in the rosaramicin biosynthetic locus.
Figure 7 illustrates a mechanism for the biosynthesis of rosaramicin.
Figures 8A and 8B represent a Clustal amino acid alignment of the eight ketosynthase (KS) domains found in the rosaramicin PKS enzyme complex. Key residues are highlighted.
Figures 9A and 9B represent a Clustal amino acid alignment of the eight acyl transferase (AT) domains in the rosaramicin PKS enzyme complex. Key residues are highlighted. Regions important in substrate recognition are indicated by "s" above the alignment.
Figure 10 represents a Clustal amino acid alignment of the 3 DH domains in the rosaramicin PKS enzyme complex. Key residues are highlighted.
Figure 11 represents a Clustal amino acid alignment comparing the single enoyl reductase (ER) domain in the rosaramicin PKS enzyme complex to a prototypical ER domain of the erythromycin PKS, *i.e.* 6-deoxyerythronolide B synthase (DEBS), key residues are highlighted.
Figure 12 represents a Clustal amino acid alignment of the 7 KR domains in the rosaramicin PKS enzyme complex. Key residues are highlighted.
Figure 13 represents a Clustal amino acid alignment of the 8 ACP domains in the rosaramicin PKS enzyme complex. The key active site serine residue is highlighted.
Figure 14 represents a Clustal amino acid alignment comparing the single thioesterase (Te) domain in the rosaramicin PKS enzyme complex to a prototypical Te domain of the erythromycin PKS, DEBS.
Figure 15 represents a Clustal amino acid alignment that demonstrates the overall high degree of homology between the second AT domain of ORF7 with two other ethylmalonyl-CoA-specific AT domains from the tylosin and niddamycin PKS complexes.

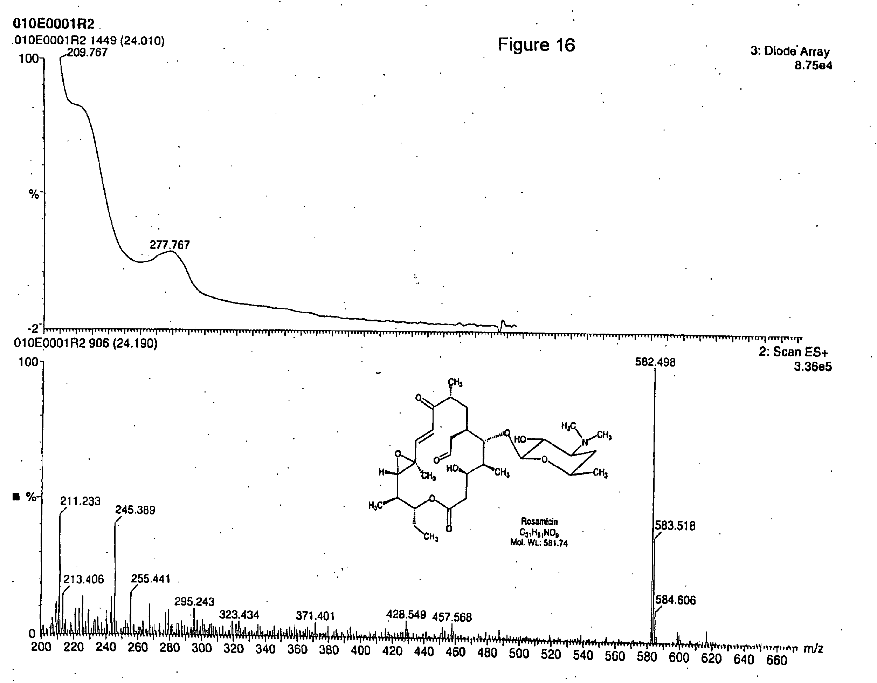

Figure 16 is a LCMS graph showing the production of a compound of the molecular weight of rosaramicin.

### DETAILED DESCRIPTION OF THE INVENTION:

Throughout the description and the figures, the biosynthetic locus for rosaramicin from *Micromonospora carbonacea* is sometimes referred to as ROSA. The ORFs in ROSA are assigned a putative function sometimes referred to throughout the description and figures by reference to a four-letter designation, as indicated in Table I.

**Table 1**

| | | |
|---|---|---|
| **Families** | **ORF#** | **Function** |
| ABCC | 1 | ABC transporter; contains repeated domain |
| DATF | 17 | dehydratase/aminotransferase; SMAT family (secondary metabolism aminotransferase); transaminase |
| GTFA | 11 | glycosyl transferase |
| MTFA | 12 | methyltransferase, SAM-dependent; N,N-dimethyltransferases |
| MTRA | 19 | resistance methyltransferase; 23S ribosomal |
| NBPA | 16 | unknown, nucleotide (ATP/GTP) binding protein; may be involved in regulated proteolysis |
| OXRB | 10 | oxidoreductase; similar to NDP-hexose-3,4-isomerases (tautomerase) |
| OXRC | 3, 4 | oxidoreductase; cytP450 monooxygenase, hydroxylase; oxygen-binding site motif: LLxAGx(D,E); heme-binding pocket motif: GxGxHxCxGxxLxR, the cysteine is invariable and coordinates the heme |
| OXRH | 13 | oxidoreductase, NAD(P)-dependent; similar to crotonyl CoA reductases (CCR); similarity to some quinone oxidoreductases, zinc-containing alcohol dehydrogenases |
| PKSH | 5-9 | polyketide synthase, type I |
| REGM | 15 | regulator; similar to TyIR global activator of the tylosin locus and the carbomycin AcyB2 positive regulator |
| REGS | 14 | regulator, may be positive regulator; similar to spiramycin SrmR, which specifically activates the production of spiramycin |
| SURA | 18 | sugar reductase; iron-sulfur (4Fe-4S) protein; may be involved in 1,2-migration of the amino group from C4 to C3 via the Schiff's base intermediate |
| TESA | 2 | thioesterase |

The terms "macrolide producer" and "macrolide-producing organism" refer to a microorganism that carries the genetic information necessary to produce a macrolide compound, whether or not the organism is known to produce a macrolide compound. The terms "rosaramicin producer" and "rosaramicin-producing organism" refer to a microorganism that carries the genetic information necessary to produce a rosaramicin compound, whether or not the organism is known to produce a rosaramicin product. The terms apply equally to organisms in which the genetic information to produce the macrolide or rosaramicin compound is found in the organism as it exists in its natural environment, and to organisms in which the genetic information is introduced by recombinant techniques. For the sake of particularity, specific organisms contemplated herein include organisms of the family *Micromonosporaceae,* of which preferred genera include *Micromonospora, Actinoplanes* and *Dactylosporangium;* the family *Streptomycetaceae,* of which preferred genera include *Streptomyces* and *Kitasatospora;* the family *Pseudonocardiaceae,* of which preferred genera are *Amycolatopsis* and *Saccharopolyspora;* and the family *Actinosynnemataceae,* of which preferred genera include *Saccharothrix* and *Actinosynnema;* however the terms are intended to encompass all organisms containing genetic information necessary to produce a macrolide compound.

The term rosaramicin biosynthetic gene product refers to any enzyme or polypeptide involved in the biosynthesis of rosaramicin. The term "rosaramicin" is intended to encompass the compounds sometimes referred to as 4'-deoxycirramycin A1, rosamicin, izenamicin A1, juvenimicin A3, 6108A3, M 4365A2, Sch 14947, antibiotic 6108A3, antibiotic M 4365A2 and antibiotic Sch 14947. For the sake of particularity, the rosaramicin biosynthetic pathway is associated with *Micromonospora carbonacea.* However, it should be understood that this term encompasses rosaramicin biosynthetic enzymes (and genes encoding such enzymes) isolated from any microorganism of the genus *Micromonospora* or *Streptomyces,* and furthermore that these genes may have novel homologues in related actinomycete microorganisms or non-actinomycete microorganisms that fall within the scope of the invention. Representative rosaramicin biosynthetic gene products include the polypeptides listed in SEQ ID NOS: 2, 4, 6, 8, 10, 12, 14, 16, 18, 20, 22, 24, 26, 28, 30, 32, 34, 36, 38 or homologues thereof, wherein the homologues are capable of directing the synthesis of a rosaramicin compound or analogue.

The term "isolated" means that the material is removed from its original environment, e.g. the natural environment if it is naturally-occurring. For example, a naturally-occurring polynucleotide or polypeptide present in a living organism is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The term "purified" does not require absolute purity; rather, it is intended as a relative definition. Individual nucleic acids obtained from a library have been conventionally purified to electrophoretic homogeneity. The purified nucleic acids of the present invention have been purified from the remainder of the genomic DNA in the organism by at least 10⁴ to 10⁶ fold. However, the term "purified" also includes nucleic acids which have been purified from the remainder of the genomic DNA or from other sequences in a library or other environment by at least one order of magnitude, preferably two or three orders of magnitude, and more preferably four or five orders of magnitude.

"Recombinant" means that the nucleic acid is adjacent to "backbone" nucleic acid to which it is not adjacent in its natural environment. "Enriched" nucleic acids represent 5% or more of the number of nucleic acid inserts in a population of nucleic acid backbone molecules. "Backbone" molecules include nucleic acids such as expression vectors, self-replicating nucleic acids, viruses, integrating nucleic acids, and other vectors or nucleic acids used to maintain or manipulate a nucleic acid of interest. Preferably, the enriched nucleic acids represent 15% or more, more preferably 50% or more, and most preferably 90% or more, of the number of nucleic acid inserts in the population of recombinant backbone molecules.

"Recombinant" polypeptides or proteins refer to polypeptides or proteins produced by recombinant DNA techniques, i.e. produced from cells transformed by an exogenous DNA construct encoding the desired polypeptide or protein. "Synthetic" polypeptides or proteins are those prepared by chemical synthesis.

The term "gene" means the segment of DNA involved in producing a polypeptide chain; it includes regions preceding and following the coding region (leader and trailer) as well as, where applicable, intervening regions (introns) between individual coding segments (exons).

A DNA or nucleotide "coding sequence" or "sequence encoding" a particular polypeptide or protein, is a DNA sequence which is transcribed and translated into a polypeptide or protein when placed under the control of appropriate regulatory sequences.

"Oligonucleotide" refers to a nucleic acid, generally of at least 10, preferably 15 and more preferably at least 20 nucleotides, preferably no more than 100 nucleotides, that are hybridizable to a genomic DNA molecule, a cDNA molecule, or an mRNA molecule encoding a gene, mRNA, cDNA or other nucleic acid of interest.

A promoter sequence is "operably linked to" a coding sequence recognized by RNA polymerase which initiates transcription at the promoter and transcribes the coding sequence into mRNA.

"Plasmids" are designated herein by a lower case p preceded or followed by capital letters and/or numbers. The starting plasmids herein are commercially available, publicly available on an unrestricted basis, or can be constructed from available plasmids in accord with published procedures. In addition, equivalent plasmids to those described herein are known in the art and will be apparent to the skilled artisan.

"Digestion" of DNA refers to enzymatic cleavage of the DNA with a restriction enzyme that acts only at certain sequences in the DNA. The various restriction enzymes used herein are commercially available and their reaction conditions, cofactors and other requirements were used as would be known to the ordinary skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular enzymes are specified by the manufacturer. Incubation times of about 1 hour at 37°C are ordinarily used, but may vary in accordance with the supplier's instructions. After digestion the gel electrophoresis may be performed to isolate the desired fragment.

We have now discovered the genes and proteins involved in the biosynthesis of the 16-member macrolide rosaramicin. Nucleic acid sequences encoding proteins involved in the biosynthesis of rosaramicin are provided in the accompanying sequence listing as SEQ ID NOS: 3, 5, 7, 9, 11, 13, 15, 17, 19, 21, 23, 25, 27, 29, 31, 33, 35, 37, 39. Polypeptides involved in the biosynthesis of rosaramicin are provided in the accompanying sequence listing as SEQ ID NOS: 2, 4, 6, 8, 10, 12, 14, 16, 18, 20, 22, 24, 26, 28, 30, 32, 34, 36, 38.

One aspect of the present invention is a nucleic acid comprising one of the sequences of SEQ ID NOS: 3, 5, 7, 9, 11, 13, 15, 17, 19, 21, 23, 25, 27, 29, 31, 33, 35, 37, 39, the sequences complementary thereto, or a fragment comprising at least 100, 200, 300, 400, 500, 600, 700, 800 or more consecutive bases of one of the sequences of SEQ ID NOS: 3, 5, 7, 9, 11, 13, 15, 17, 19, 21, 23, 25, 27, 29, 31, 33, 35, 37, 39 or the sequences complementary thereto, wherein the fragments are preferably capable of directing the synthesis of a rosaramicin compound or analogue. The nucleic acids may comprise DNA, including cDNA, genomic DNA, and synthetic DNA. The DNA may be double stranded or single stranded, and if single stranded may be the coding (sense) or non-coding (anti-sense) strand. Alternatively, the nucleic acids may comprise RNA.

As discussed in more detail below, the nucleic acids of one of SEQ ID NOS: 3, 5, 7, 9, 11, 13, 15, 17, 19, 21, 23, 25, 27, 29, 31, 33, 35, 37, 39 may be used to prepare one of the polypeptides of SEQ ID NOS: 2, 4, 6, 8, 10, 12, 14, 16, 18, 20, 22, 24, 26, 28, 30, 32, 34, 36, 38 respectively or fragments comprising at least 50, 75, 100, 200, 300, 500 or more consecutive amino acids of one of the polypeptides of SEQ ID NO: 2, 4, 6, 8, 10, 12, 14, 16, 18, 20, 22, 24, 26, 28, 30, 32, 34, 36, 38, wherein the fragments are preferably capable of directing the synthesis of a rosaramicin compound or analogue.

Accordingly, another aspect of the present invention is a nucleic acid which encodes one of the polypeptides of SEQ ID NOS: 2, 4, 6, 8, 10, 12, 14, 16, 18, 20, 22, 24, 26, 28, 30, 32, 34, 36, 38 or fragments comprising at least 50, 75, 100, 150, 200, 300 or more consecutive amino acids of one of the polypeptides of SEQ ID NOS: 2, 4, 6, 8, 10, 12, 14, 16, 18, 20, 22, 24, 26, 28, 30, 32, 34, 36, 38. The coding sequences of these nucleic acids may be identical to one of the coding sequences of one of the nucleic acids of SEQ ID NOS: 3, 5, 7, 9, 11, 13, 15, 17, 19, 21, 23, 25, 27, 29, 31, 33, 35, 37, 39 or a fragment thereof or may be different coding sequences which encode one of the polypeptides of SEQ ID NOS: 2, 4, 6, 8, 10, 12, 14, 16, 18, 20, 22, 24, 26, 28, 30, 32, 34, 36, 38 or fragments comprising at least 50, 75, 100, 150, 200, 300 consecutive amino acids of one of the polypeptides of SEQ ID NOS: 2, 4, 6, 8, 10, 12, 14, 16, 18, 20, 22, 24, 26, 28, 30, 32, 34, 36, 38 as a result of the redundancy or degeneracy of the genetic code. The genetic code is well known to those of skill in the art and can be obtained, for example, from Stryer, *Biochemistry,* 3^{rd} edition, W. H. Freeman & Co., New York. The aforementioned fragments are preferably capable of directing the synthesis of a rosaramicin compound or analogue.

The nucleic acid which encodes one of the polypeptides of SEQ ID NOS: 2, 4, 6, 8, 10, 12, 14, 16, 18, 20, 22, 24, 26, 28, 30, 32, 34, 36, 38, may include, but is not limited to: (1) only the coding sequences of one of SEQ ID NOS: 3, 5, 7, 9, 11, 13, 15, 17, 19, 21, 23, 25, 27, 29, 31, 33, 35, 37, 39; (2) the coding sequences of SEQ ID NOS: 3, 5, 7, 9, 11, 13, 15, 17, 19, 21, 23, 25, 27, 29, 31, 33, 35, 37, 39 and additional coding sequences, such as leader sequences or proprotein; and (3) the coding sequences of SEQ ID NOS: 3, 5, 7, 9, 11, 13, 15, 17, 19, 21, 23, 25, 27, 29, 31, 33, 35, 37, 39 and non-coding sequences, such as introns or non-coding sequences 5' and/or 3' of the coding sequence. Thus, as used herein, the term "polynucleotide encoding a polypeptide" encompasses a polynucleotide that includes only coding sequence for the polypeptide as well as a polynucleotide that includes additional coding and/or non-coding sequence.

The invention relates to polynucleotides based on SEQ ID NOS: 3, 5, 7, 9, 11, 13, 15, 17, 19, 21, 23, 25, 27, 29, 31, 33, 35, 37, 39 but having polynucleotide changes that are "silent", for example changes which do not alter the amino acid sequence encoded by the polynucleotides of SEQ ID NOS: 3, 5, 7, 9, 11, 13, 15, 17, 19, 21, 23, 25, 27, 29, 31, 33, 35, 37, 39. The invention also relates to polynucleotides which have nucleotide changes which result in amino acid substitutions, additions, deletions, fusions and truncations of the polypeptides of SEQ ID NOS: 2, 4, 6, 8, 10, 12, 14, 16, 18, 20, 22, 24, 26, 28, 30, 32, 34, 36, 38, wherein the polypeptides are capable of directing the synthesis of a rosaramicin compound or analogue. Such nucleotide changes may be introduced using techniques such as site directed mutagenesis, random chemical mutagenesis, exonuclease III deletion, and other recombinant DNA techniques.

The nucleic acids of SEQ ID NOS: 3, 5, 7, 9, 11, 13, 15, 17, 19, 21, 23, 25, 27, 29, 31, 33, 35, 37, 39, the sequences complementary thereto, or a fragment comprising at least 10, 15, 20, 25, 30, 35, 40, 50, 75, 100, 150, 200, 300, 400 or 500 consecutive bases of one of the sequence of SEQ ID NOS: 3, 5, 7, 9, 11, 13, 15, 17, 19, 21, 23, 25, 27, 29, 31, 33, 35, 37, 39, or the sequences complementary thereto may be used as probes to identify and isolate DNAs encoding the polypeptides of SEQ ID NOS: 2, 4, 6, 8, 10, 12, 14, 16, 18, 20, 22, 24, 26, 28, 30, 32, 34, 36, 38 respectively. In such procedures, a genomic DNA library is constructed from a sample microorganism or a sample containing a microorganism capable of producing a macrolide. The genomic DNA library is then contacted with a probe comprising a coding sequence or a fragment of the coding sequence, encoding one of the polypeptides of SEQ ID NOS: 2, 4, 6, 8, 10, 12, 14, 16, 18, 20, 22, 24, 26, 28, 30, 32, 34, 36, 38, or a fragment thereof under conditions which permit the probe to specifically hybridize to sequences complementary thereto. In a preferred embodiment, the probe is an oligonucleotide of about 10 to about 30 nucleotides in length designed based on a nucleic acid of SEQ ID NOS: 3, 5, 7, 9, 11, 13, 15, 17, 19, 21, 23, 25, 27, 29, 31, 33, 35, 37, 39. Genomic DNA clones which hybridize to the probe are then detected and isolated. Procedures for preparing and identifying DNA clones of interest are disclosed in Ausubel *et al.,* Current Protocols in Molecular Biology, John Wiley 503 Sons, Inc. 1997; and Sambrook *et al.,* Molecular Cloning: A Laboratory Manual 2d Ed., Cold Spring Harbor Laboratory Press, 1989. In another embodiment, the probe is a restriction fragment or a PCR amplified nucleic acid derived from SEQ ID NOS: 3, 5, 7, 9, 11, 13, 15, 17, 19, 21, 23, 25, 27, 29, 31, 33, 35, 37, 39. The aforementioned fragments are preferably capable of directing the synthesis of a rosaramicin compound or analogue.

The nucleic acids of SEQ ID NOS: 3, 5, 7, 9, 11, 13, 15, 17, 19, 21, 23, 25, 27, 29, 31, 33, 35, 37, 39, the sequences complementary thereto, or a fragment comprising at least 10, 15, 20, 25, 30, 35, 40, 50, 75, 100, 150, 200, 300, 400 or 500 consecutive bases of one of the sequences of SEQ ID NOS: 3, 5, 7, 9, 11, 13, 15, 17, 19, 21, 23, 25, 27, 29, 31, 33, 35, 37, 39, or the sequences complementary thereto, wherein the fragments are preferably capable of directing the synthesis of a rosaramicin compound or analogue, may be used as probes to identify and isolate related nucleic acids. In some embodiments, the related nucleic acids may be genomic DNAs (or cDNAs) from potential macrolide producers. In such procedures, a nucleic acid sample containing nucleic acids from a potential macrolide-producer or rosaramicin-producer is contacted with the probe under conditions that permit the probe to specifically hybridize to related sequences. The nucleic acid sample may be a genomic DNA (or cDNA) library from the potential macrolide-producer. Hybridization of the probe to nucleic acids is then detected using any of the methods described above.

Hybridization may be carried out under conditions of low stringency, moderate stringency or high stringency. As an example of nucleic acid hybridization, a polymer membrane containing immobilized denatured nucleic acids is first prehybridized for 30 minutes at 45 °C in a solution consisting of 0.9 M NaCl, 50 mM NaH₂PO₄, pH 7.0, 5.0 mM Na₂EDTA, 0.5% SDS, 10X Denhardt's, and 0.5 mg/ml polyriboadenylic acid. Approximately 2 x 10⁷ cpm (specific activity 4-9 x 10⁸ cpm/ug) of ³²P end-labeled oligonucleotide probe are then added to the solution. After 12-16 hours of incubation, the membrane is washed for 30 minutes at room temperature in 1X SET (150 mM NaCl, 20 mM Tris hydrochloride, pH 7.8, 1 mM Na₂EDTA) containing 0.5% SDS, followed by a 30 minute wash in fresh 1X SET at Tm-10°C for the oligonucleotide probe where Tm is the melting temperature. The membrane is then exposed to autoradiographic film for detection of hybridization signals.

By varying the stringency of the hybridization conditions used to identify nucleic acids, such as genomic DNAs or cDNAs, which hybridize to the detectable probe, nucleic acids having different levels of homology to the probe can be identified and isolated. Stringency may be varied by conducting the hybridization at varying temperatures below the melting temperatures of the probes. The melting temperature of the probe may be calculated using the following formulas:
For oligonucleotide probes between 14 and 70 nucleotides in length the melting temperature (Tm) in degrees Celcius may be calculated using the formula: Tm=81.5+16.6(log [Na+]) + 0.41 (fraction G+C)-(600/N) where N is the length of the oligonucleotide.

If the hybridization is carried out in a solution containing formamide, the melting temperature may be calculated using the equation Tm=81.5+16.6(log [Na+]) + 0.41 (fraction G + C)-(0.63% formamide)-(600/N) where N is the length of the probe.

Prehybridization may be carried out in 6X SSC, 5X Denhardt's reagent, 0.5% SDS, 0.1 mg/ml denatured fragmented salmon sperm DNA or 6X SSC, 5X Denhardt's reagent, 0.5% SDS, 0.1 mg/ml denatured fragmented salmon sperm DNA, 50% formamide. The composition of the SSC and Denhardt's solutions are listed in Sambrook et al., *supra.*

Hybridization is conducted by adding the detectable probe to the hybridization solutions listed above. Where the probe comprises double stranded DNA, it is denatured by incubating at elevated temperatures and quickly cooling before addition to the hybridization solution. It may also be desirable to similarly denature single stranded probes to eliminate or diminish formation of secondary structures or oligomerization. The filter is contacted with the hybridization solution for a sufficient period of time to allow the probe to hybridize to cDNAs or genomic DNAs containing sequences complementary thereto or homologous thereto. For probes over 200 nucleotides in length, the hybridization may be carried out at 15-25 °C below the Tm. For shorter probes, such as oligonucleotide probes, the hybridization may be conducted at 5-10 °C below the Tm. Preferably, the hybridization is conducted in 6X SSC, for shorter probes. Preferably, the hybridization is conducted in 50% formamide containing solutions, for longer probes.

All the foregoing hybridizations would be considered to be examples of hybridization performed under conditions of high stringency.

Following hybridization, the filter is washed for at least 15 minutes in 2X SSC, 0.1% SDS at room temperature or higher, depending on the desired stringency. The filter is then washed with 0.1X SSC, 0.5% SDS at room temperature (again) for 30 minutes to 1 hour.

Nucleic acids which have hybridized to the probe are identified by conventional autoradiography and non-radioactive detection methods.

The above procedure may be modified to identify nucleic acids having decreasing levels of homology to the probe sequence. For example, to obtain nucleic acids of decreasing homology to the detectable probe, less stringent conditions may be used. For example, the hybridization temperature may be decreased in increments of 5 °C from 68 °C to 42 °C in a hybridization buffer having a Na+ concentration of approximately 1M. Following hybridization, the filter may be washed with 2X SSC, 0.5% SDS at the temperature of hybridization. These conditions are considered to be "moderate stringency" conditions above 50°C and "low stringency" conditions below 50°C. A specific example of "moderate stringency" hybridization conditions is when the above hybridization is conducted at 55°C. A specific example of "low stringency" hybridization conditions is when the above hybridization is conducted at 45°C.

Alternatively, the hybridization may be carried out in buffers, such as 6X SSC, containing formamide at a temperature of 42 °C. In this case, the concentration of formamide in the hybridization buffer may be reduced in 5% increments from 50% to 0% to identify clones having decreasing levels of homology to the probe. Following hybridization, the filter may be washed with 6X SSC, 0.5% SDS at 50 °C. These conditions are considered to be "moderate stringency" conditions above 25% formamide and "low stringency" conditions below 25% formamide. A specific example of "moderate stringency" hybridization conditions is when the above hybridization is conducted at 30% formamide. A specific example of "low stringency" hybridization conditions is when the above hybridization is conducted at 10% formamide.

Nucleic acids which have hybridized to the probe are identified by conventional autoradiography and non-radioactive detection methods.

For example, the preceding methods may be used to isolate nucleic acids having a sequence with at least 97%, at least 95%, at least 90%, at least 85%, at least 80%, or at least 70% homology to a nucleic acid sequence selected from the group consisting of the sequences of SEQ ID NOS: 3, 5, 7, 9, 11, 13, 15, 17, 19, 21, 23, 25, 27, 29, 31, 33, 35, 37, 39, fragments comprising at least 10, 15, 20, 25, 30, 35, 40, 50, 75, 100, 150, 200, 300, 400, or 500 consecutive bases thereof, and the sequences complementary thereto, wherein the homologues and fragments are capable of directing the synthesis of a rosaramicin compound or analogue. Homology may be measured using BLASTN version 2.0 with the default parameters. For example, the homologous polynucleotides may have a coding sequence that is a naturally occurring allelic variant of one of the coding sequences described herein. Such allelic variant may have a substitution, deletion or addition of one or more nucleotides when compared to the nucleic acids of SEQ ID NOS: 3, 5, 7, 9, 11, 13, 15, 17, 19, 21, 23, 25, 27, 29, 31, 33, 35, 37, 39, or the sequences complementary thereto.

Additionally, the above procedures may be used to isolate nucleic acids which encode polypeptides having at least 99%, 95%, at least 90%, at least 85%, at least 80%, or at least 70% homology to a polypeptide having the sequence of one of SEQ ID NOS: 2, 4, 6, 8, 10, 12, 14, 16, 18, 20, 22, 24, 26, 28, 30, 32, 34, 36, 38, or fragments comprising at least 50, 75, 100, 150, 200, 300 consecutive amino acids thereof as determined using the BLASTP version 2.2.2 algorithm with default parameters, wherein the homologues and fragments are capable of directing the synthesis of a rosaramicin compound or analogue.

Another aspect of the present invention is a polypeptide comprising the sequence of one of SEQ ID NOS: 2, 4, 6, 8, 10, 12, 14, 16, 18, 20, 22, 24, 26, 28, 30, 32, 34, 36, 38 or fragments comprising at least 50, 75, 100, 150, 200 or 300 consecutive amino acids thereof, wherein the fragments are preferably capable of directing the synthesis of a rosaramicin compound or analogue. As discussed herein, such polypeptides may be obtained by inserting a nucleic acid encoding the polypeptide into a vector such that the coding sequence is operably linked to a sequence capable of driving the expression of the encoded polypeptide in a suitable host cell. For example, the expression vector may comprise a promoter, a ribosome binding site for translation initiation and a transcription terminator. The vector may also include appropriate sequences for modulating expression levels, an origin of replication and a selectable marker.

Promoters suitable for expressing the polypeptide or fragment thereof in bacteria include the *E.coli lac* or *trp* promoters, the lacl promoter, the lacZ promoter, the T3 promoter, the T7 promoter, the gpt promoter, the lambda P_{R} promoter, the lambda P_{L} promoter, promoters from operons encoding glycolytic enzymes such as 3-phosphoglycerate kinase (PGK), and the acid phosphatase promoter. Fungal promoters include the α factor promoter. Eukaryotic promoters include the CMV immediate early promoter, the HSV thymidine kinase promoter, heat shock promoters, the early and late SV40 promoter, LTRs from retroviruses, and the mouse metallothionein-I promoter. Other promoters known to control expression of genes in prokaryotic or eukaryotic cells or their viruses may also be used.

Mammalian expression vectors may also comprise an origin of replication, any necessary ribosome binding sites, a polyadenylation site, splice donors and acceptor sites, transcriptional termination sequences, and 5' flanking nontranscribed sequences. In some embodiments, DNA sequences derived from the SV40 splice and polyadenylation sites may be used to provide the required nontranscribed genetic elements.

Vectors for expressing the polypeptide or fragment thereof in eukaryotic cells may also contain enhancers to increase expression levels. Enhancers are cis-acting elements of DNA, usually from about 10 to about 300 bp in length that act on a promoter to increase its transcription. Examples include the SV40 enhancer on the late side of the replication origin bp 100 to 270, the cytomegalovirus early promoter enhancer, the polyoma enhancer on the late side of the replication origin, and the adenovirus enhancers.

In addition, the expression vectors preferably contain one or more selectable marker genes to permit selection of host cells containing the vector. Examples of selectable markers that may be used include genes encoding dihydrofolate reductase or genes conferring neomycin resistance for eukaryotic cell culture, genes conferring tetracyctine or ampicillin resistance in *E. coli,* and the *S. cerevisiae* TRP1 gene.

In some embodiments, the nucleic acid encoding one of the polypeptides of SEQ ID NOS: 2, 4, 6, 8, 10, 12, 14, 16, 18, 20, 22, 24, 26, 28, 30, 32, 34, 36, 38, or fragments comprising at least 50, 75, 100, 150, 200 or 300 consecutive amino acids thereof, wherein the fragments are preferably capable of directing the synthesis of a rosaramicin compound or analogue, is assembled in appropriate phase with a leader sequence capable of directing secretion of the translated polypeptides or fragments thereof. Optionally, the nucleic acid can encode a fusion polypeptide in which one of the polypeptide of SEQ ID NOS: 2, 4, 6, 8, 10, 12, 14, 16, 18, 20, 22, 24, 26, 28, 30, 32, 34, 36, 38 or fragments comprising at least 5, 10, 15, 20, 25, 30, 35, 40, 50, 75, 100, or 150 consecutive amino acids thereof, wherein the fragments are preferably capable of directing the synthesis of a rosaramicin compound or analogue, is fused to heterologous peptides or polypeptides, such as N-terminal identification peptides which impart desired characteristics such as increased stability or simplified purification or detection.

The appropriate DNA sequence may be inserted into the vector by a variety of procedures. In general, the DNA sequence is ligated to the desired position in the vector following digestion of the insert and the vector with appropriate restriction endonucleases. Alternatively, appropriate restriction enzyme sites can be engineered into a DNA sequence by PCR. A variety of cloning techniques are disclosed in Ausbel et al. Current Protocols in Molecular Biology, John Wiley 503 Sons, Inc. 1997 and Sambrook et al., Molecular Cloning: A Laboratory Manual 2d Ed., Cold Spring Harbour Laboratory Press, 1989. Such procedures and others are deemed to be within the scope of those skilled in the art.

The vector may be, for example, in the form of a plasmid, a viral particle, or a phage. Other vectors include derivatives of chromosomal, nonchromosomal and synthetic DNA sequences, viruses, bacterial plasmids, phage DNA, baculovirus, yeast plasmids, vectors derived from combinations of plasmids and phage DNA, viral DNA such as vaccinia, adenovirus, fowl pox virus, and pseudorabies. A variety of cloning and expression vectors for use with prokaryotic and eukaryotic hosts are described by Sambrook, et al., Molecular Cloning: A Laboratory Manual, Second Edition, Cold Spring Harbor, N.Y., (1989).

Particular bacterial vectors which may be used include the commercially available plasmids comprising genetic elements of the well known cloning vector pBR322 (ATCC 37017), pKK223-3 (Pharmacia Fine Chemicals, Uppsala, Sweden), pGEM1 (Promega Biotec, Madison, WI, USA) pQE70, pQE60, pQE-9 (Qiagen), pD10, phiX174, pBluescript II KS, pNH8A, pNH16a, pNH18A, pNH46A (Stratagene), ptrc99a, pKK223-3, pKK233-3, pDR540, pRIT5 (Pharmacia), pKK232-8 and pCM7. Particular eukaryotic vectors include pSV2CAT, pOG44, pXT1, pSG (Stratagene) pSVK3, pBPV, pMSG, and pSVL (Pharmacia). However, any other vector may be used as long as it is replicable and stable in the host cell.

The host cell may be any of the host cells familiar to those skilled in the art, including prokaryotic cells or eukaryotic cells. As representative examples of appropriate hosts, there may be mentioned: bacteria cells, such as *E. coli*, *Streptomyces, Bacillus subtilis, Salmonella typhimurium* and various species within the genera *Pseudomonas, Streptomyces,* and *Staphylococcus,* fungal cells, such as yeast, insect cells such as *Drosophila S2* and *Spodoptera Sf9*, animal cells such as CHO, COS or Bowes melanoma, and adenoviruses. The selection of an appropriate host is within the abilities of those skilled in the art.

The vector may be introduced into the host cells using any of a variety of techniques, including electroporation transformation, transfection, transduction, viral infection, gene guns, or Ti-mediated gene transfer. Where appropriate, the engineered host cells can be cultured in conventional nutrient media modified as appropriate for activating promoters, selecting transformants or amplifying the genes of the present invention. Following transformation of a suitable host strain and growth of the host strain to an appropriate cell density, the selected promoter may be induced by appropriate means (e.g., temperature shift or chemical induction) and the cells may be cultured for an additional period to allow them to produce the desired polypeptide or fragment thereof.

Cells are typically harvested by centrifugation, disrupted by physical or chemical means, and the resulting crude extract is retained for further purification. Microbial cells employed for expression of proteins can be disrupted by any convenient method, including freeze-thaw cycling, sonication, mechanical disruption, or use of cell lysing agents. Such methods are well known to those skilled in the art. The expressed polypeptide or fragment thereof can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the polypeptide. If desired, high performance liquid chromatography (HPLC) can be employed for final purification steps.

Various mammalian cell culture systems can also be employed to express recombinant protein. Examples of mammalian expression systems include the COS-7 lines of monkey kidney fibroblasts (described by Gluzman, Cell, 23:175(1981)), and other cell lines capable of expressing proteins from a compatible vector, such as the C127, 3T3, CHO, HeLa and BHK cell lines.

The constructs in host cells can be used in a conventional manner to produce the gene product encoded by the recombinant sequence. Depending upon the host employed in a recombinant production procedure, the polypeptide produced by host cells containing the vector may be glycosylated or may be non-glycosylated. Polypeptides of the invention may or may not also include an initial methionine amino acid residue.

Alternatively, the polypeptides of SEQ ID NOS: 2, 4, 6, 8, 10, 12, 14, 16, 18, 20, 22, 24, 26, 28, 30, 32, 34, 36, 38, or fragments comprising at least 50, 75, 100, 150, 200 or 300 consecutive amino acids thereof, wherein the fragments are preferably capable of directing the synthesis of a rosaramicin compound or analogue, can be synthetically produced by conventional peptide synthesizers. In other embodiments, fragments or portions of the polynucleotides may be employed for producing the corresponding full-length polypeptide by peptide synthesis; therefore, the fragments may be employed as intermediates for producing the full-length polypeptides.

Cell-free translation systems can also be employed to produce one of the polypeptides of SEQ ID NOS: 2, 4, 6, 8, 10, 12, 14, 16, 18, 20, 22, 24, 26, 28, 30, 32, 34, 36, 38, or fragments comprising at least 50, 75, 100, 150, 200 or 300 consecutive amino acids thereof, wherein the fragments are preferably capable of directing the synthesis of a rosaramicin compound or analogue, using mRNAs transcribed from a DNA construct comprising a promoter operably linked to a nucleic acid encoding the polypeptide or fragment thereof. In some embodiments, the DNA construct may be linearized prior to conducting an *in vitro* transcription reaction. The transcribed mRNA is then incubated with an appropriate cell-free translation extract, such as a rabbit reticulocyte extract, to produce the desired polypeptide or fragment thereof.

The application also describes variants of the polypeptides of SEQ ID NOS: 2, 4, 6, 8, 10, 12, 14, 16, 18, 20, 22, 24, 26, 28, 30, 32, 34, 36, 38, or fragments comprising at least 50, 75, 100, 150, 200 or 300 consecutive amino acids thereof. The term "variant" includes derivatives or analogs of these polypeptides. In particular, the variants may differ in amino acid sequence from the polypeptides of SEQ ID NOS: 2, 4, 6, 8, 10, 12, 14, 16, 18, 20, 22, 24, 26, 28, 30, 32, 34, 36, 38, by one or more substitutions, additions, deletions, fusions and truncations, which may be present in any combination.

The variants may be naturally occurring or created *in vitro.* In particular, such variants may be created using genetic engineering techniques such as site directed mutagenesis, random chemical mutagenesis, Exonuclease III deletion procedures, and standard cloning techniques. Alternatively, such variants, fragments, analogs, or derivatives may be created using chemical synthesis or modification procedures.

Other methods of making variants are also familiar to those skilled in the art. These include procedures in which nucleic acid sequences obtained from natural isolates are modified to generate nucleic acids that encode polypeptides having characteristics which enhance their value in industrial or laboratory applications. In such procedures, a large number of variant sequences having one or more nucleotide differences with respect to the sequence obtained from the natural isolate are generated and characterized. Preferably, these nucleotide differences result in amino acid changes with respect to the polypeptides encoded by the nucleic acids from the natural isolates.

For example, variants may be created using error prone PCR. In error prone PCR, DNA amplification is performed under conditions where the fidelity of the DNA polymerase is low, such that a high rate of point mutation is obtained along the entire length of the PCR product. Error prone PCR is described in Leung, D.W., *et al.,* Technique, 1:11-15 (1989) and Caldwell, R. C. & Joyce G.F., PCR Methods Applic., 2:28-33 (1992). Variants may also be created using site directed mutagenesis to generate site-specific mutations in any cloned DNA segment of interest. Oligonucleotide mutagenesis is described in Reidhaar-Olson, J.F. & Sauer, R.T., *et al*., Science, 241:53-57 (1988). Variants may also be created using directed evolution strategies such as those described in US patent nos. 6,361,974 and 6,372,497. The variants of the polypeptides of SEQ ID NOS: 2, 4, 6, 8, 10, 12, 14, 16, 18, 20, 22, 24, 26, 28, 30, 32, 34, 36, 38, may be (i) variants in which one or more of the amino acid residues of the polypeptides of SEQ ID NOS: 2, 4, 6, 8, 10, 12, 14, 16, 18, 20, 22, 24, 26, 28, 30, 32, 34, 36, 38, are substituted with a conserved or non-conserved amino acid residue (preferably a conserved amino acid residue) and such substituted amino acid residue may or may not be one encoded by the genetic code.

Conservative substitutions are those that substitute a given amino acid in a polypeptide by another amino acid of like characteristics. Typically seen as conservative substitutions are the following replacements: replacements of an aliphatic amino acid such as Ala, Val, Leu and Ile with another aliphatic amino acid; replacement of a Ser with a Thr or vice versa; replacement of an acidic residue such as Asp or Glu with another acidic residue; replacement of a residue bearing an amide group, such as Asn or Gln, with another residue bearing an amide group; exchange of a basic residue such as Lys or Arg with another basic residue; and replacement of an aromatic residue such as Phe or Tyr with another aromatic residue.

Other variants are those in which one or more of the amino acid residues of the polypeptides of SEQ ID NOS: 2, 4, 6, 8, 10, 12, 14, 16, 18, 20, 22, 24, 26, 28, 30, 32, 34, 36, 38 includes a substituent group.

Still other variants are those in which the polypeptide is associated with another compound, such as a compound to increase the half-life of the polypeptide (for example, polyethylene glycol).

Additional variants are those in which additional amino acids are fused to the polypeptide, such as leader sequence, a secretory sequence, a proprotein sequence or a sequence which facilitates purification, enrichment, or stabilization of the polypeptide.

In some embodiments, the fragments retain the same biological function or activity as the polypeptides of SEQ ID NOS: 2, 4, 6, 8. 10, 12, 14, 16, 18, 20, 22, 24, 26, 28, 30, 32, 34, 36, 38. In other embodiments, the fragment includes a fused heterologous sequence which facilitates purification, enrichment, detection, stabilization or secretion of the polypeptide that can be enzymatically cleaved, in whole or in part, away from the fragment, derivative or analogue.

Another aspect of the present invention are polypeptides or fragments thereof which have at least 70%, at least 80%, at least 85%, at least 90%, or more than 95% homology to one of the polypeptides of SEQ ID NOS: 2, 4, 6, 8, 10, 12, 14, 16, 18, 20, 22, 24, 26, 28, 30, 32, 34, 36, 38, or a fragment comprising at least 50, 75, 100, 150, 200 or 300 consecutive amino acids thereof, wherein said fragments are capable of directing the synthesis of a rosaramicin compound or analogue. Homology may be determined using a program, such as BLASTP version 2.2.2 with the default parameters, which aligns the polypeptides or fragments being compared and determines the extent of amino acid identity or similarity between them. It will be appreciated that amino acid "homology" includes conservative substitutions such as those described above.

The polypeptides or fragments having homology to one of the polypeptides of SEQ ID NOS: 2, 4, 6, 8, 10, 12, 14, 16, 18, 20, 22, 24, 26, 28, 30, 32, 34, 36, 38, or a fragment comprising at least 50, 75, 100, 150, 200 or 300 consecutive amino acids thereof, wherein said fragments are preferably capable of directing the synthesis of a rosaramicin compound or analogue, may be obtained by isolating the nucleic acids encoding them using the techniques described above.

Alternatively, the homologous polypeptides or fragments may be obtained through biochemical enrichment or purification procedures. The sequence of potentially homologous polypeptides or fragments may be determined by proteolytic digestion, gel electrophoresis and/or microsequencing. The sequence of the prospective homologous polypeptide or fragment can be compared to one of the polypeptides of SEQ ID NOS: 2, 4, 6, 8, 10, 12, 14, 16, 18, 20, 22, 24, 26, 28, 30, 32, 34, 36, 38, or a fragment comprising at least 5, 10, 15, 20, 25, 30, 35, 40, 50, 75, 100, or 150 consecutive amino acids thereof using a program such as BLASTP version 2.2.2 with the default parameters.

The polypeptides of SEQ ID NOS: 2, 4, 6, 8, 10, 12, 14, 16, 18, 20, 22, 24, 26, 28, 30, 32, 34, 36, 38, or fragments thereof comprising at least 40, 50, 75, 100, 150, 200 or 300 consecutive amino acids thereof, wherein said fragments are preferably capable of directing the synthesis of a rosaramicin compound or analogue, may be used in a variety of applications. For example, the polypeptides or fragments thereof may be used to catalyze certain biochemical reactions. In particular, the polypeptides of the TESA family, namely SEQ ID NO: 4 or fragments thereof; the PKSH family, namely SEQ ID NOS: 10, 12, 14, 16, 18 or fragments thereof; the OXRH family, namely SEQ ID NO: 26 or fragments thereof may be used in any combination, *in vitro* or *in vivo,* to direct or enhance the synthesis or modification of a polyketide, polyketide substructure, or precursor thereof. Polypeptides of the MTFA family, namely SEQ ID NO: 24 or fragments thereof may be used, *in vitro* or *in vivo,* to catalyze methylation reactions that modify compounds that are either endogenously produced by the host, supplemented to the growth medium, or are added to a cell-free, purified or enriched preparation of MTFA polypeptide. Polypeptides of the OXRC family, namely SEQ ID NOS: 6, 8 or fragments thereof; the OXRB family, namely SEQ ID NO: 20 or fragments thereof; the OXRH family, namely SEQ ID NO: 26 or fragments thereof may be used, *in vitro* or *in vivo,* to catalyze oxidation reactions that modify compounds that are either endogenously produced by the host, supplemented to the growth medium, or are added to a cell-free, purified or enriched preparation of said polypeptide. Polypeptides of the NBPA family, namely SEQ ID NO: 32 or fragments thereof; the OXRB family, namely SEQ ID NO: 20 or fragments thereof; the DATF family, namely SEQ ID NO: 34 or fragments thereof; the SURA family, namely SEQ ID NO: 36 or fragments thereof; the MTFA family, namely SEQ ID NO: 24 or fragments thereof; the GTFA family, namely SEQ ID NO: 22 or fragments thereof may be used, *in vitro* or *in vivo,* to catalyze biochemical reactions involved in activating, modifying, or transferring sugar moieties. Polypeptides of the ABCC family, namely SEQ ID NO: 2 or fragments thereof; the MTRA family, namely SEQ ID NO: 38 or fragments thereof may be used to confer to microorganisms or eukaryotic cells resistance to polyketides, macrolides, rosaramicin, or compounds related to rosaramicin. Polypeptides of the REGS family, namely SEQ ID NO: 28 or fragments thereof; the REGM family, namely SEQ ID NO: 30 or fragments thereof may be used to increase the yield of polyketides, macrolides, rosaramicin, or compounds related to rosaramicin in either naturally producing organisms or heterologously producing recombinant organisms.

The polypeptides of SEQ ID NOS: 2, 4, 6, 8, 10, 12, 14, 16, 18, 20, 22, 24, 26, 28, 30, 32, 34, 36, 38, or fragments thereof comprising at least 5, 10, 15, 20, 25, 30, 35, 40, 50, 75, 100, or 150 consecutive amino acids thereof, wherein the fragments are preferably capable of directing the synthesis of a rosaramicin compound or analogue, may also be used to generate antibodies which bind specifically to the polypeptides or fragments. The antibodies generated from SEQ ID NOS: 2, 4, 6, 8, 10, 12, 14, 16, 18, 20, 22, 24, 26, 28, 30, 32, 34, 36, 38 may be used to determine whether a biological sample contains *Micromonospora carbonacea* or a related microorganism.

In such procedures, a biological sample is contacted with an antibody capable of specifically binding to one of the polypeptides of SEQ ID NOS: 2, 4, 6, 8, 10, 12, 14, 16, 18, 20, 22, 24, 26, 28, 30, 32, 34, 36, 38, or fragments comprising at least 5, 10, 15, 20, 25, 30, 35, 40, 50, 75, 100, or 150 consecutive amino acids thereof, wherein the fragments are preferably capable of directing the synthesis of a rosaramicin compound or analogue. The ability of the biological sample to bind to the antibody is then determined. For example, binding may be determined by labeling the antibody with a detectable label such as a fluorescent agent, an enzymatic label, or a radioisotope. Alternatively, binding of the antibody to the sample may be detected using a secondary antibody having such a detectable label thereon. A variety of assay protocols which may be used to detect the presence of an rosaramicin-producer or of *Micromonospora carbonacea* or of polypeptides related to SEQ ID NOS: 2, 4, 6, 8, 10, 12, 14, 16, 18, 20, 22, 24, 26, 28, 30, 32, 34, 36, 38, in a sample are familiar to those skilled in the art. Particular assays include ELISA assays, sandwich assays, radioimmunoassays, and Western Blots. Alternatively, antibodies generated from SEQ ID NOS: 2, 4, 6, 8, 10, 12, 14, 16, 18, 20, 22, 24, 26, 28, 30, 32, 34, 36, 38, may be used to determine whether a biological sample contains related polypeptides that may be involved in the biosynthesis of natural products of the rosaramicin class or other macrolides.

Polyclonal antibodies generated against the polypeptides of SEQ ID NOS: 2, 4, 6, 8, 10, 12, 14, 16, 18, 20, 22, 24, 26, 28, 30, 32, 34, 36, 38, or fragments comprising at least 5, 10, 15, 20, 25, 30, 35, 40, 50, 75, 100, or 150 consecutive amino acids thereof, wherein the fragments are preferably capable of directing the synthesis of a rosaramicin compound or analogue, can be obtained by direct injection of the polypeptides into an animal or by administering the polypeptides to an animal, preferably a nonhuman. The antibody so obtained will then bind the polypeptide itself. In this manner, even a sequence encoding only a fragment of the polypeptide can be used to generate antibodies that may bind to the whole native polypeptide. Such antibodies can then be used to isolate the polypeptide from cells expressing that polypeptide.

For preparation of monoclonal antibodies, any technique which provides antibodies produced by continuous cell line cultures can be used. Examples include the hybridoma technique (Kholer and Milstein, 1975, Nature, 256:495-497), the trioma technique, the human B-cell hybridoma technique (Kozbor et al., 1983, Immunology Today 4:72), and the EBV-hybridoma technique (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to the polypeptides of SEQ ID NOS: 2, 4, 6, 8, 10, 12, 14, 16, 18, 20, 22, 24, 26, 28, 30, 32, 34, 36, 38, or fragments comprising at least 5, 10, 15, 20, 25, 30, 35, 40, 50, 75, 100, or 150 consecutive amino acids thereof, wherein the fragments are preferably capable of directing the synthesis of a rosaramicin compound or analogue. Alternatively, transgenic mice may be used to express humanized antibodies to these polypeptides or fragments thereof.

Antibodies generated against the polypeptides of SEQ ID NOS: 2, 4, 6, 8, 10, 12, 14, 16, 18, 20, 22, 24, 26, 28, 30, 32, 34, 36, 38, or fragments comprising at least 5, 10, 15, 20, 25, 30, 35, 40, 50, 75, 100, or 150 consecutive amino acids thereof, wherein the fragments are preferably capable of directing the synthesis of a rosaramicin compound or analogue, may be used in screening for similar polypeptides from a sample containing organisms or cell-free extracts thereof. In such techniques, polypeptides from the sample is contacted with the antibodies and those polypeptides which specifically bind the antibody are detected. Any of the procedures described above may be used to detect antibody binding. One such screening assay is described in "Methods for measuring Cellulase Activities", Methods in Enzymology, Vol 160, pp. 87-116.

As used herein, the term "nucleic acid codes of SEQ ID NOS: 3, 5, 7, 9, 11, 13, 15, 17, 19, 21, 23, 25, 27, 29, 31, 33, 35, 37, 39" encompass the nucleotide sequences of SEQ ID NOS: 3, 5, 7, 9, 11, 13, 15, 17, 19, 21, 23, 25, 27, 29, 31, 33, 35, 37, 39, fragments of SEQ ID NOS: 3, 5, 7, 9, 11, 13, 15, 17, 19, 21, 23, 25, 27, 29, 31, 33, 35, 37, 39, nucleotide sequences homologous to SEQ ID NOS: 3, 5, 7, 9, 11, 13, 15, 17, 19, 21, 23, 25, 27, 29, 31, 33, 35, 37, 39, or homologous to fragments of SEQ ID NOS: 3, 5, 7, 9, 11, 13, 15, 17, 19, 21, 23, 25, 27, 29, 31, 33, 35, 37, 39, and sequences complementary to all of the preceding sequences. The fragments include portions of SEQ ID NOS: 3, 5, 7, 9, 11, 13, 15, 17, 19, 21, 23, 25, 27, 29, 31, 33, 35, 37, 39, comprising at least 10, 15, 20, 25, 30, 35, 40, 50, 75, 100, 150, 200, 300, 400 or 500 consecutive nucleotides of SEQ ID NOS: 3, 5, 7, 9, 11, 13, 15, 17, 19, 21, 23, 25, 27, 29, 31, 33, 35, 37, 39. Preferably, the fragments are novel fragments. The aforementioned fragments are capable of directing the synthesis of a rosaramicin compound or analogue. Homologous sequences and fragments of SEQ ID NOS: 3, 5, 7, 9, 11, 13, 15, 17, 19, 21, 23, 25, 27, 29, 31, 33, 35, 37, 39 refer to a sequence having at least 99%, 98%, 97%, 96%, 95%, 90%, 80%, 75% or 70% identity to these sequences. Homology may be determined using any of the computer programs and parameters described herein, including BLASTN and TBLASTX with the default parameters. Homologous sequences also include RNA sequences in which uridines replace the thymines in the nucleic acid codes of SEQ ID NOS: 3, 5, 7, 9, 11, 13, 15, 17, 19, 21, 23, 25, 27, 29, 31, 33, 35, 37, 39. The aforementioned homologues and fragments are capable of directing the synthesis of a rosaramicin compound or analogue.

The homologous sequences may be obtained using any of the procedures described herein or may result from the correction of a sequencing error. It will be appreciated that the nucleic acid codes of SEQ ID NOS: 3, 5, 7, 9, 11, 13, 15, 17, 19, 21, 23, 25, 27, 29, 31, 33, 35, 37, 39 can be represented in the traditional single character format in which G, A, T and C denote the guanine, adenine, thymine and cytosine bases of the deoxyribonucleic acid (DNA) sequence respectively, or in which G, A, U and C denote the guanine, adenine, uracil and cytosine bases of the ribonucleic acid (RNA) sequence (see the inside back cover of Stryer, *Biochemistry,* 3^{rd} edition, W. H. Freeman & Co., New York) or in any other format which records the identity of the nucleotides in a sequence.

"Polypeptide codes of SEQ ID NOS: 2, 4, 6, 8, 10, 12, 14, 16, 18, 20, 22, 24, 26, 28, 30, 32, 34, 36, 38" encompass the polypeptide sequences of SEQ ID NOS: 2, 4, 6, 8, 10, 12, 14, 16, 18, 20, 22, 24, 26, 28, 30, 32, 34, 36, 38 which are encoded by the nucleic acid sequences of SEQ ID NOS: 3, 5, 7, 9, 11, 13, 15, 17, 19, 21, 23, 25, 27, 29, 31, 33, 35, 37, 39, polypeptide sequences homologous to the polypeptides of SEQ ID NOS: 2, 4, 6, 8, 10, 12, 14, 16, 18, 20, 22, 24, 26, 28, 30, 32, 34, 36, 38, or fragments of any of the preceding sequences. Homologous polypeptide sequences refer to a polypeptide sequence having at least 99%, 98%, 97%, 96%, 95%, 90%, 85%, 80%, 75% or 70% identity to one of the polypeptide sequences of SEQ ID NOS: 2, 4, 6, 8, 10, 12, 14, 16, 18, 20, 22, 24, 26, 28, 30, 32, 34, 36, 38. Polypeptide sequence homology may be determined using any of the computer programs and parameters described herein, including BLASTP version 2.2.1 with the default parameters or with any user-specified parameters. The homologous sequences may be obtained using any of the procedures described herein or may result from the correction of a sequencing error. The polypeptide fragments comprise at least 5, 10, 15, 20, 25, 30, 35, 40, 50, 75, 100 or 150 consecutive amino acids of the polypeptides of SEQ ID NOS: 2, 4, 6, 8, 10, 12, 14, 16, 18, 20, 22, 24, 26, 28, 30, 32, 34, 36, 38. Preferably the fragments are novel fragments. The aforementioned homologues and fragments are capable of directing the synthesis of a rosaramicin compound or analogue. It will be appreciated that the polypeptide codes of the SEQ ID NOS: 2, 4, 6, 8, 10, 12, 14, 16, 18, 20, 22, 24, 26, 28, 30, 32, 34, 36, 38 can be represented in the traditional single character format or three letter format (see the inside back cover of Stryer, *Biochemistry,* 3^{rd} edition, W.H. Freeman & Co., New York) or in any other format which relates the identity of the polypeptides in a sequence.

Many computer programs and databases may be used as sequence comparers, identifiers or sources of query nucleotide sequences or query polypeptide sequences to be compared to one or more of the nucleic acid codes of SEQ ID NOS: 3, 5, 7, 9, 11, 13, 15, 17, 19, 21, 23, 25, 27, 29, 31, 33, 35, 37, 39, and one or more of the polypeptide codes of SEQ ID NOS: 2, 4, 6, 8, 10, 12, 14, 16, 18, 20, 22, 24, 26, 28, 30, 32, 34, 36, 38.

The following list is intended not to limit the invention but to provide guidance to programs and databases useful with one or more of the nucleic acid codes of SEQ ID NOS: 3, 5, 7, 9, 11, 13, 15, 17, 19, 21, 23, 25, 27, 29, 31, 33, 35, 37, 39, and the polypeptide codes of SEQ ID NOS: 2, 4, 6, 8, 10, 12, 14, 16, 18, 20, 22, 24, 26, 28, 30, 32, 34, 36, 38. The program and databases which may be used include, but are not limited to: MacPattem (EMBL), DiscoveryBase (Molecular Applications Group), GeneMine (Molecular Applications Group) Look (Molecular Applications Group), MacLook (Molecular Applications Group), BLAST and BLAST2 (NCBI), BLASTN and BLASTX (Altschul *et al., J. Mol. Biol.* 215:403 (1990)), FASTA (Person and Lipman, *Proc. Nalt. Acad. Sci. USA,* 85:2444 (1988)), FASTDB (Brutlag *et al. Comp. App. Biosci*. 6-237-245, 1990), Catalyst (Molecular Simulations Inc.), Catalyst/SHAPE (Molecular Simulations Inc.), Cerius².DBAccess (Molecular Simulations Inc.), HypoGen (Molecular Simulations Inc.), Insight II (Molecular Simulations Inc.), Discover (Molecular Simulations Inc.), CHARMm (Molecular Simulations Inc.), Felix (Molecular Simulations Inc.), DelPhi (Molecular Simulations Inc.), QuanteMM (Molecular Simulations Inc.), Homology (Molecular Simulations Inc.), Modeler (Molecular Simulations Inc.), ISIS (Molecular Simulations Inc.), Quanta/Protein Design (Molecular Simulations Inc.), WetLab (Molecular Simulations Inc.), WetLab Diversity Explorer (Molecular Simulations Inc.), Gene Explorer (Molecular Simulations Inc.), SeqFold (Molecular Simulations Inc.), the MDL Available Chemicals Directory database, the MDL Drug Data Report data base, the Comprehensive Medicinal Chemistry database, Derwents' World Drug Index database, the BioByteMasterFile database, the Genbank database, and the Gensyqn database. Many other programs and databases would be apparent to one of skill in the art given the present disclosure.

The present invention will be further described with reference to the following examples; however, it is to be understood that the present invention is not limited to such examples.

### EXAMPLE 1: Identification and sequencing of a rosaramicin biosynthetic locus in Micromonospora carbonacea var. aurantiaca NRRL 2997

*Micromonospora carbonacea* var. *aurantiaca* NRRL 2997 was obtained from the Agricultural Research Service collection (National Center for Agricultural Utilization Research, 1815 N. University Street, Peoria, Illinois 61604) and cultured using standard microbiological techniques (Kieser *et al.,* supra). This organism was propagated on oatmeal agar medium at 28 degrees Celsius for several days. For isolation of high molecular weight genomic DNA, cell mass from three freshly grown, near confluent 100 mm petri dishes was used. The cell mass was collected by gentle scraping with a plastic spatula. Residual agar medium was removed by repeated washes with STE buffer (75 mM NaCl; 20 mM Tris-HCl, pH 8.0; 25 mM EDTA). High molecular weight DNA was isolated by established protocols (Kieser *et al.* supra) and its integrity was verified by field inversion gel electrophoresis (FIGE) using the preset program number 6 of the FIGE MAPPER™ power supply (BIORAD). This high molecular weight genomic DNA served for the preparation of a small size fragment genomic sampling library (GSL), as well as a large size fragment cluster identification library (CIL). Both libraries contained randomly generated M. *carbonacea* genomic DNA fragments and, therefore, are representative of the entire genome of this organism.

For the generation of the GSL library, genomic DNA was randomly sheared by sonication. DNA fragments having a size range between 1.5 and 3 kb were fractionated on a agarose gel and isolated using standard molecular biology techniques (Sambrook et al., *supra*). The ends of the obtained DNA fragments were repaired using T4 DNA polymerase (Roche) as described by the supplier. This enzyme creates DNA fragments with blunt ends that can be subsequently cloned into an appropriate vector. The repaired DNA fragments were subcloned into a derivative of pBluescript SK+ vector (Stratagene) which does not allow transcription of cloned DNA fragments. This vector was selected as it contains a convenient polylinker region surrounded by sequences corresponding to universal sequencing primers such as T3, T7, SK, and KS (Stratagene). The unique *EcoRV* restriction site found in the polylinker region was used as it allows insertion of blunt-end DNA fragments. Ligation of the inserts, use of the ligation products to transform E. *coli* DH10B (Invitrogen) host and selection for recombinant clones were performed as previously described (Sambrook et al., *supra).* Plasmid DNA carrying the M. *carbonacea* genomic DNA fragments was extracted by the alkaline lysis method (Sambrook et al., *supra)* and the insert size of 1.5 to 3 kb was confirmed by electrophoresis on agarose gels. Using this procedure, a library of small size random genomic DNA fragments is generated that covers the entire genome of the studied microorganism. The number of individual clones that can be generated is infinite but only a small number is further analyzed to sample the microorganism's genome.

A CIL library was constructed from the M. *carbonacea* high molecular weight genomic DNA using the SuperCos-1 cosmid vector (Stratagene™). The cosmid arms were prepared as specified by the manufacturer. The high molecular weight DNA was subjected to partial digestion at 37 degrees Celsius with approximately one unit of *Sau*3Al restriction enzyme (New England Biolabs) per 100 micrograms of DNA in the buffer supplied by the manufacturer. This procedure generates random fragments of DNA ranging from the initial undigested size of the DNA to short fragments of which the length is dependent upon the frequency of the enzyme DNA recognition site in the genome and the extent of the DNA digestion by the enzyme. At various timepoints, aliquots of the digestion were transferred to new microfuge tubes and the enzyme was inactivated by adding a final concentration of 10 mM EDTA and 0.1 % SDS. Aliquots judged by FIGE analysis to contain a significant fraction of DNA in the desired size range (30-50kb) were pooled, extracted with phenol/chloroform (1:1 vol:vol), and pelletted by ethanol precipitation. The 5' ends of *Sau*3Al DNA fragments were dephosphorylated using alkaline phosphatase (Roche) according to the manufacturer's specifications at 37 degrees Celcius for 30 min. The phosphatase was heat inactivated at 70 degrees Celcius for 10 min and the DNA was extracted with phenol/chloroform (1:1 vol:vol), pelletted by ethanol precipitation, and resuspended in sterile water. The dephosphorylated *Sau*3AI DNA fragments were then ligated overnight at room temperature to the SuperCos-1 cosmid arms in a reaction containing approximately four-fold molar excess SuperCos-1 cosmid arms. The ligation products were packaged using Gigapack® III XL packaging extracts (Stratagene™) according to the manufacturer's specifications. The CIL library consisted of 864 isolated cosmid clones in E. *coli* DH10B (Invitrogen). These clones were picked and inoculated into nine 96-well microtiter plates containing LB broth (per liter of water: 10.0 g NaCl; 10.0 g tryptone; 5.0 g yeast extract) which were grown overnight and then adjusted to contain a final concentration of 25% glycerol. These microtiter plates were stored at - 80 degrees Celcius and served as glycerol stocks of the CIL library. Duplicate microtiter plates were arrayed onto nylon membranes as follows. Cultures grown on microtiter plates were concentrated by pelleting and resuspending in a small volume of LB broth. A 3 X 3 grid (96-pin) was arrayed onto nylon membranes. These membranes representing the complete CIL library were then layered onto LB agar and incubated ovenight at 37 degrees Celcius to allow the colonies to grow. The membranes were layered onto filter paper pre-soaked with 0.5 N NaOH/1.5 M NaCl for 10 min to denature the DNA and then neutralized by transferring onto filter paper pre-soaked with 0.5 M Tris (pH 8)/1.5 M NaCl for 10 min. Cell debris was gently scraped off with a plastic spatula and the DNA was crosslinked onto the membranes by UV irradiation using a GS GENE LINKER™ UV Chamber (BIORAD). Considering an average size of 8 Mb for an actinomycete genome and an average size of 35 kb of genomic insert in the CIL library, this library represents roughly a 4-fold coverage of the microorganism's entire genome.

The GSL library was analyzed by sequence determination of the cloned genomic DNA inserts. The universal primers KS or T7, referred to as forward (F) primers, were used to initiate polymerization of labeled DNA. Extension of at least 700 bp from the priming site can be routinely achieved using the TF, BDT v2.0 sequencing kit as specified by the supplier (Applied Biosystems). Sequence analysis of the small genomic DNA fragments (Genomic Sequence Tags, GSTs) was performed using a 3700 ABI capillary electrophoresis DNA sequencer (Applied Biosystems). The average length of the DNA sequence reads was ~700 bp. Further analysis of the obtained GSTs was performed by sequence homology comparison to various protein sequence databases. The DNA sequences of the obtained GSTs were translated into amino acid sequences and compared to the National Center for Biotechnology information (NCBI) nonredundant protein database and the proprietary Ecopia natural product biosynthetic gene Decipher™ database using previously described algorithms (Altschul et al., *supra*). Sequence similarity with known proteins of defined function in the database enables one to make predictions on the function of the partial protein that is encoded by the translated GST.

A total of 437 M. *carbonacea* GSTs were generated using the forward sequencing primer and analyzed by sequence comparison using the Blast algorithm (Altschul et al., *supra*). Sequence alignments displaying an E value of at least e-5 were considered as significantly homologous and retained for further evaluation. GSTs showing similarity to a gene of interest can be at this point selected and used to identify larger segments of genomic DNA from the CIL library that include the gene(s) of interest. Polyketide natural products are often synthesized by type I polyketide synthases (PKSs). Several forward GST reads were identified as portions of PKS genes. For example, one such GST encoded an internal portion of a PKS acyl transferase (AT) domain in the antisense orientation relative to the sequencing primer. The GSL clone from which this GST was obtained was also sequenced using the reverse sequencing primer and was found to encode the N-terminal portion of a PKS ketosynthase (KS) domain in the sense orientation relative to the sequencing primer. Based on the sequence of the forward read of this GSL clone, a 20mer oligonucleotide was designed for use as a probe to identify and isolate CIL clones which harbored the sequences of interest.

Hybridization oligonucleotide probes were radiolabeled with P³² using T4 polynucleotide kinase (New England Biolabs) in 15 microliter reactions containing 5 picomoles of oligonucleotide and 6.6 picomoles of [-γ-P³²]ATP in the kinase reaction buffer supplied by the manufacturer. After 1 hour at 37 degrees Celcius, the kinase reaction was terminated by the addition of EDTA to a final concentration of 5 mM. The specific activity of the radiolabeled oligonucleotide probes was estimated using a Model 3 Geiger counter (Ludlum Measurements Inc., Sweetwater, Texas) with a built-in integrator feature. The radiolabeled oligonucleotide probes were heat-denatured by incubation at 85 degrees Celcius for 10 minutes and quick-cooled in an ice bath immediately prior to use.

The CIL library membranes were pretreated by incubation for at least 2 hours at 42 degrees Celcius in Prehyb Solution (6X SSC; 20mM NaH₂PO₄; 5X Denhardt's; 0.4% SDS; 0.1 mg/ml sonicated, denatured salmon sperm DNA) using a hybridization oven with gentle rotation. The membranes were then placed in Hyb Solution (6X SSC; 20mM NaH₂PO₄; 0.4% SDS; 0.1 mg/ml sonicated, denatured salmon sperm DNA) containing 1X10⁶ cpm/ml of radiolabeled oligonucleotide probe and incubated overnight at 42 degrees Celcius using a hybridization oven with gentle rotation. The next day, the membranes were washed with Wash Buffer (6X SSC, 0.1% SDS) for 45 minutes each at 46, 48, and 50 degrees Celcius using a hybridization oven with gentle rotation. The membranes were then exposed to X-ray film to visualize and identify the positive cosmid clones. Positive clones were identified, cosmid DNA was extracted from 30 ml cultures using the alkaline lysis method (Sambrook et al., *supra)* and the inserts were entirely sequenced using a shotgun sequencing approach (Fleischmann et al., *Science,* 269:496-512).

Sequencing reads were assembled using the Phred-Phrap™ algorithm (University of Washington, Seattle, USA) recreating the entire DNA sequence of the cosmid insert. Reiterations of hybridizations of the CIL library with probes derived from the ends of the original cosmid allow indefinite extension of sequence information on both sides of the original cosmid sequence until the complete sought-after gene cluster is obtained. Three overlapping cosmid clones that were either directly identified by the original oligonucleotide probe (derived from the GSL clone) or by probes derived from the ends of the original cosmids have been completely sequenced to provide over 60 Kb of genetic information. Subsequently, the forward and reverse reads of the GSL clone from which the original oligonucleotide probe was derived were mapped to a region of the rosaramicin biosynthetic locus that encodes a portion of the PKS gene identified herein as ORF 7, more specifically nucleotides encoding amino acids 1531 to 2416 approximately. This corresponds to a GSL clone with an insert size of approximately 2.6kb, in good agreement with the selected size range of 1.5- 3kb described above. The sequence of these cosmids and analysis of the proteins encoded by them undoubtedly demonstrated that the gene cluster obtained was indeed responsible for the production of a glycosylated macrolide consistent with the known structure of rosaramicin, which was not previously reported to be produced by *M. carbonacea* var *aurantiaca* NRRL 2997.

### Example 2: Genes and proteins involved in biosynthesis of rosaramicin

The rosaramicin locus includes the 60196 base pairs provided in SEQ ID NO: 1 and contains the 19 ORFs provided SEQ ID NOS: 3, 5, 7, 9, 11, 13, 15, 17, 19, 21, 23, 25, 27, 29, 31, 33, 35, 37, 39. More than 19 kilobases of DNA sequence were analyzed on each side of the rosaramicin locus and these regions contain primary metabolic genes. The accompanying sequence listing provides the nucleotide sequence of the 19 ORFs regulating the biosynthesis of rosaramicin and the corresponding deduced polypeptides, wherein ORF 1 (SEQ ID NO: 3) represents the polynucleotide drawn from residues 1 to 1683 (sense strand) of SEQ ID NO: 1; ORF 2 (SEQ ID NO: 5) represents the polynucleotide drawn from residues 2522 to 1728 (antisense strand) of SEQ ID NO: 1; ORF 3 (SEQ ID NO: 7) represents the polynucleotide drawn from residues 3861 to 2629 (antisense strand) of SEQ ID NO: 1; ORF 4 (SEQ ID NO: 9) represents the polynucleotide drawn from residues 4365 to 5573 (sense strand) of SEQ ID NO: 1; ORF 5 (SEQ ID NO: 11) represents the polynucleotide drawn from residues 5702 to 19117 (sense strand) of SEQ ID NO: 1; ORF 6 (SEQ ID NO: 13) represents the polynucleotide drawn from residues 19144 to 24921 (sense strand) of SEQ ID NO: 1; ORF 7 (SEQ ID NO: 15) represents the polynucleotide drawn from residues 24993 to 36230 (sense strand) of SEQ ID NO: 1; ORF 8 (SEQ ID NO: 17) represents the polynucleotide drawn from residues 36292 to 41016 (sense strand) of SEQ ID NO: 1; ORF 9 (SEQ ID NO: 19) represents the polynucleotide drawn from residues 41049 to 46403 (sense strand) of SEQ ID NO: 1; ORF 10 (SEQ ID NO: 21) represents the polynucleotide drawn from residues 46400 to 47794 (sense strand) of SEQ ID NO: 1; ORF 11 (SEQ ID NO: 23) represents the polynucleotide drawn from residues 47794 to 49083 (sense strand) of SEQ ID NO: 1; ORF 12 (SEQ ID NO: 25) represents the polynucleotide drawn from residues 49092 to 49814 (sense strand) of SEQ ID NO: 1; ORF 13 (SEQ ID NO: 27) represents the polynucleotide drawn from residues 49868 to 51226 (sense strand) of SEQ ID NO: 1; ORF 14 (SEQ ID NO: 29) represents the polynucleotide drawn from residues 51506 to 53416 (sense strand) of SEQ ID NO: 1; ORF 15 (SEQ ID NO: 31) represents the polynucleotide drawn from residues 54569 to 53358 (antisense strand) of SEQ ID NO: 1; ORF 16 (SEQ ID NO: 33) represents the polynucleotide drawn from residues 54897 to 56342 (sense strand) of SEQ ID NO: 33; ORF 17 (SEQ ID NO: 35) represents the polynucleotide drawn from residues 56408 to 57634 (sense strand) of SEQ ID NO: 1; ORF 18 (SEQ ID NO: 37) represents the polynucleotide drawn from residues 57657 to 59123 (sense strand) of SEQ ID NO: 1; ORF 19 (SEQ ID NO: 39) represents the polynucleotide drawn from residues 59363 to 60196 (sense strand) of SEQ ID NO: 1.

Some open reading frames listed herein initiate with non-standard initiation codons (e.g. GTG - Valine or CTG - Leucine) rather than the standard initiation codon ATG, namely ORFs 1, 6, 7, 10, 14 and 18. All ORFs are listed with the appropriate M, V or L amino acids at the amino-terminal position to indicate the specificity of the first codon of the ORF. It is expected, however, that in all cases the biosynthesized protein will contain a methionine residue, and more specifically a formylmethionine residue, at the amino terminal position, in keeping with the widely accepted principle that protein synthesis in bacteria initiates with methionine (formylmethionine) even when the encoding gene specifies a non-standard initiation codon (e.g. Stryer, Biochemistry 3^{rd} edition, 1998, W.H. Freeman and Co., New York, pp. 752-754).

Three deposits, namely E. *coli* DH10B (D10CK) strain, E. *coli* DH10B (D10CF) strain and E. *coli* DH10B (D10CJ) strain each harbouring a cosmid clone of a partial biosynthetic locus for rosaramicin from *Micromonospora carbonacea* subsp. *aurantiaca* have been deposited with the International Depositary Authority of Canada, Bureau of Microbiology, Health Canada, 1015 Arlington Street, Winnipeg, Manitoba, Canada R3E 3R2 on July 10, 2002 and were assigned deposit accession number IDAC 100702-1, 100702-2 and 100702-3 respectively. The E. *coli* strain deposits are referred to herein as "the deposited strains".

The cosmids harbored in the deposited strains comprise a complete biosynthetic locus for rosaramicin. The sequence of the polynucleotides comprised in the deposited strains, as well as the amino acid sequence of any polypeptide encoded thereby are controlling in the event of any conflict with any description of sequences herein.

The deposit of the deposited strains has been made under the terms of the Budapest Treaty on the International Recognition of the Deposit of Micro-organisms for Purposes of Patent Procedure. The deposited strains will be irrevocably and without restriction or condition released to the public upon the issuance of a patent. The deposited strains are provided merely as convenience to those skilled in the art and are not an admission that a deposit is required for enablement. A license may be required to make, use or sell the deposited strains, and compounds derived therefrom, and no such license is hereby granted.

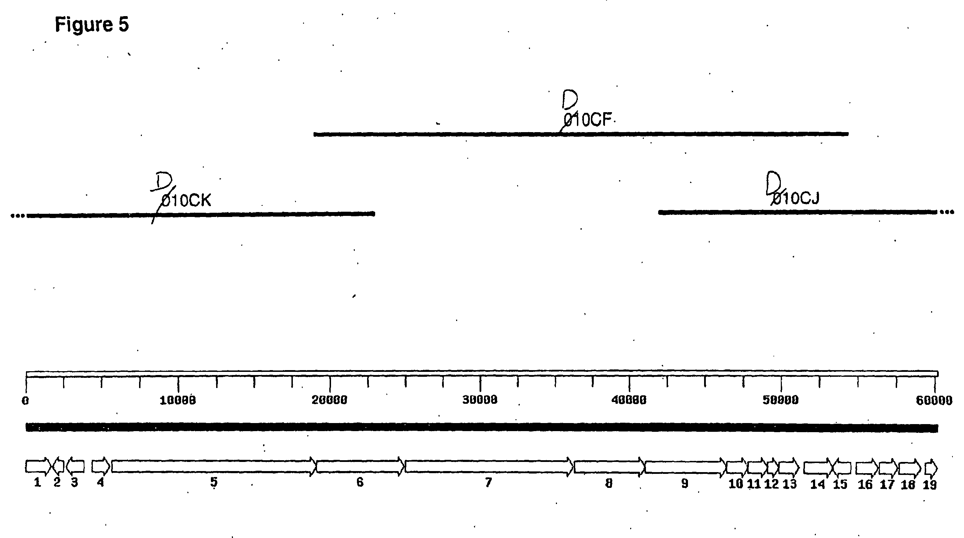

The order and relative position of the 19 open reading frames and the corresponding polypeptides of the biosynthetic locus for rosaramicin are provided in Figure 5. The arrows represent the orientatation of the ORFs of the rosaramicin biosynthetic locus. The top line in Figure 5 provides a scale in kilobase pairs. The black bars depict the part of the locus covered by each of the deposited cosmids D10CK, D10CF and D10CJ.

In order to identify the function of the genes in the rosaramicin locus, SEQ ID NOS: 2, 4, 6, 8, 10, 12, 14, 16, 18, 20, 22, 24, 26, 28, 30, 32, 34, 36, 38 were compared, using the BLASTP version 2.2.1 algorithm with the default parameters, to sequences in the National Center for Biotechnology Information (NCBI) nonredundant protein database and the DECIPHER™ database of microbial genes, pathways and natural products (Ecopia BioSciences Inc. St.-Laurent, QC, Canada).

The accession numbers of the top GenBank hits of this BLAST analysis are presented in Table 2 along with the corresponding E value. The E value relates the expected number of chance alignments with an alignment score at least equal to the observed alignment score. An E value of 0.00 indicates a perfect homolog or nearly perfect homolog. The E values are calculated as described in Altschul *et al.* J. Mol. Biol., October 5; 215(3) 403-10. The E value assists in the determination of whether two sequences display sufficient similarity to justify an inference of homology.

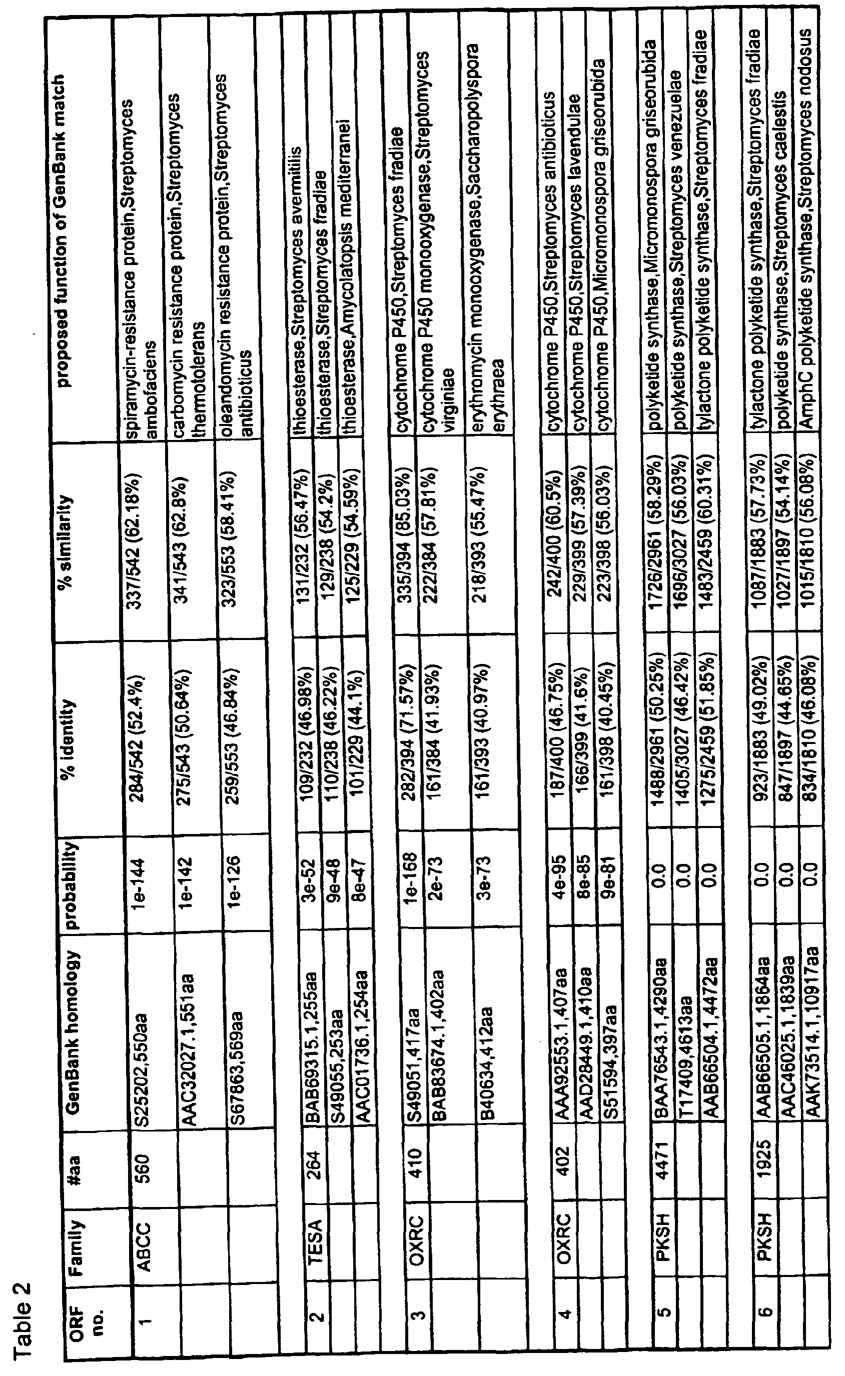

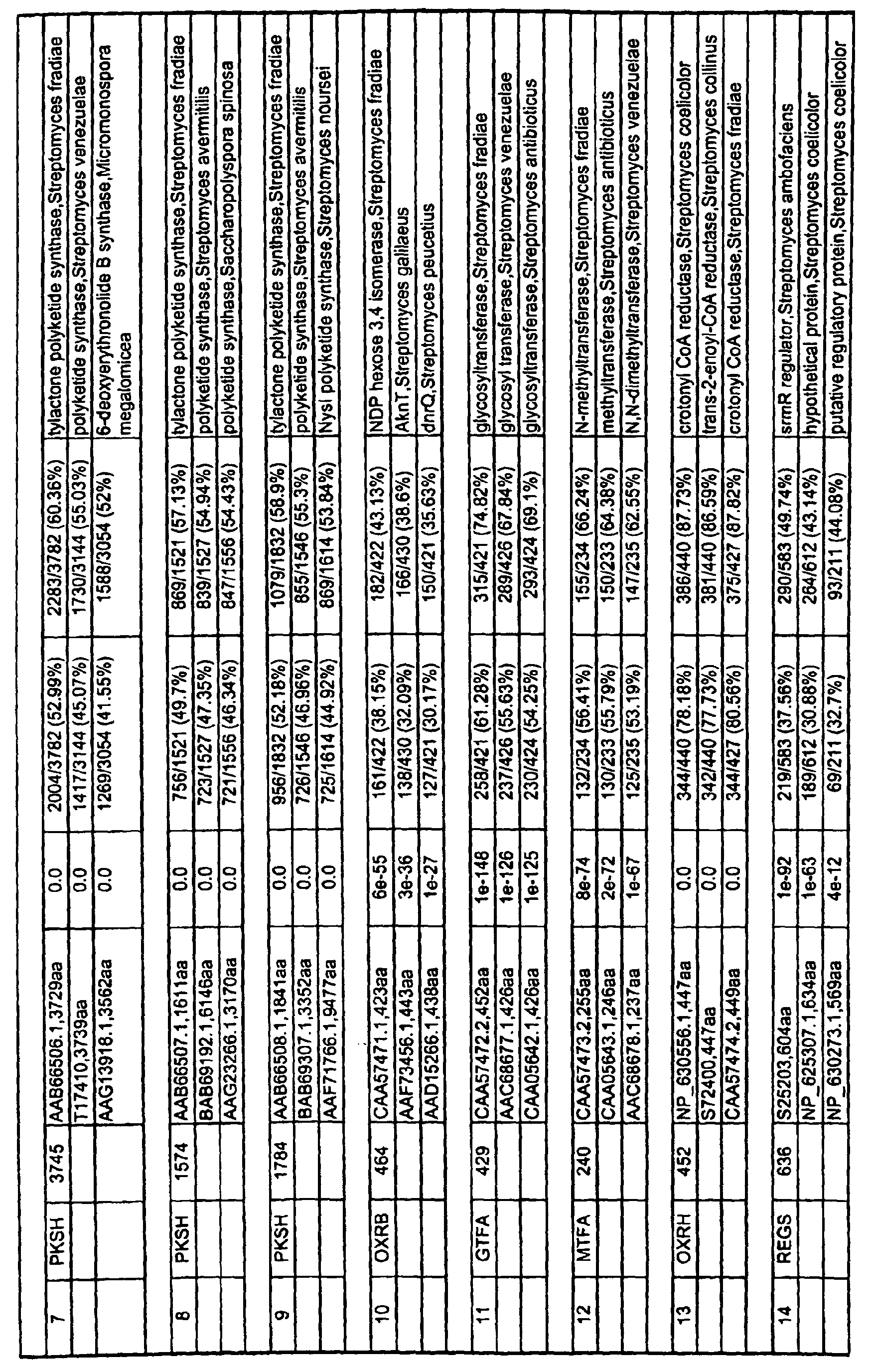

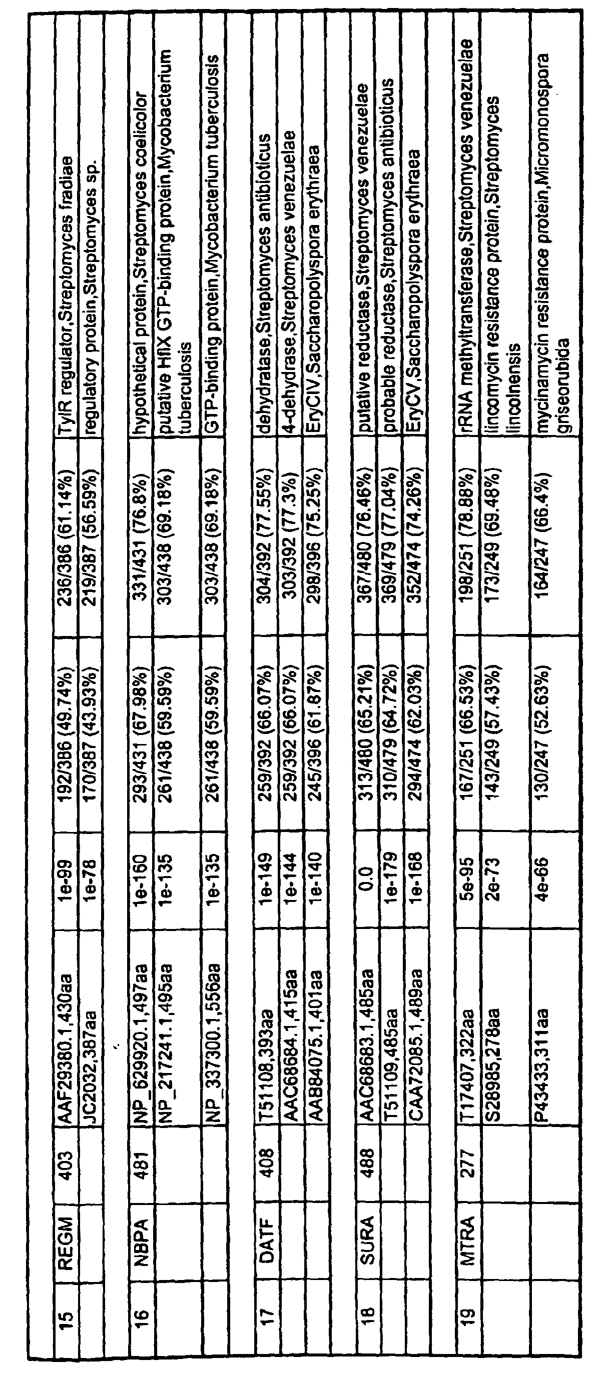

### Example 3: Formation of rosaramicin

The chemical structure of rosaramicin is a 16-membered macrolide having an epoxide, an aldehyde and a deoxyamino sugar. The rosaramicin locus includes five polyketide synthase (PKS) Type I genes. ORF 5 represents a PKS Type I gene having a domain arrangement of KS-AT-ACP-KS-AT-KR-ACP-KS-AT-DH-KR-ACP. ORF 6 represents a PKS Type 1 gene having a domain arrangement of KS-AT-DH-KR-ACP. ORF 7 represents a PKS Type I gene having a domain arrangement of KS-AT-KR-ACP-KS-AT-DH-ER-KR-ACP. ORF 8 represents a PKS Type I gene having a domain arrangement of KS-AT-KR-ACP. ORF 9 represents a PKS Type I gene having a domain arrangement of KS-AT-KR-ACP-Te.

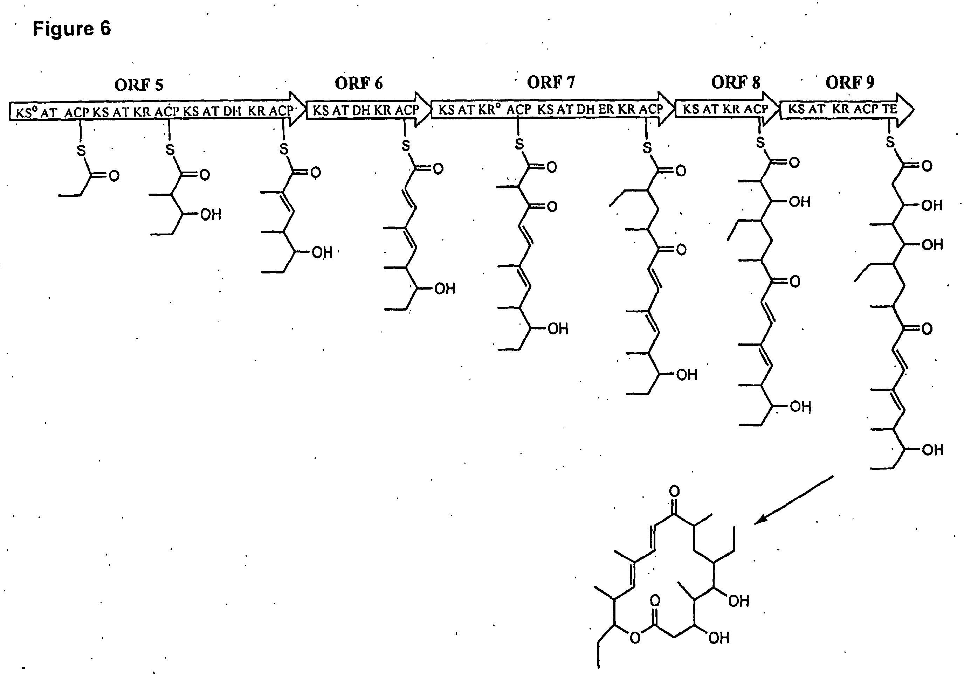

While not intending to be limited to any particular mode of action or biosynthetic scheme, the gene products of the invention can explain the synthesis of rosaramicin. ORFs 5, 6, 7, 8, and 9 constitute a polyketide synthase system that assembles the core polyketide precursor of rosaramicin. Figure 6 highlights schematically the series of reactions catalyzed by this polyketide synthase system based on the correlation between the deduced domain architecture and the polyketide core of rosaramicin. Type I PKS domains and the reactions they carry out are well known to those skilled in the art and well documented in the literature, see for example, Hopwood (1997) Chem. Rev. Vol 97 pp. 2465-2497.

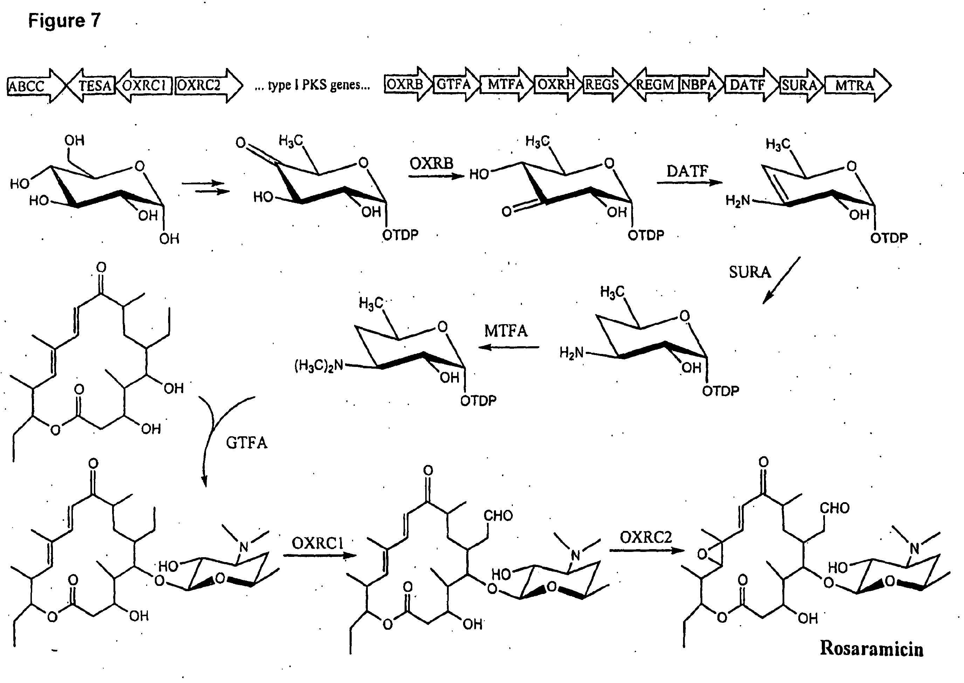

Figure 7 depicts a proposed biochemical pathway involving the OXRB, DATF, SURA, MTFA gene products for the formation of the deoxyamino sugar. This sugar is transferred to the core polyketide precursor of rosaramicin by the GTFA gene product. Also depicted in Figure 7 are the oxidation reactions carried out by two cytochrome P450 monooxygenases OXRC1 and OXRC2, referring to ORFs 3 and 4, respectively. OXRC1 is expected to catalyze the formation of an aldehyde while OXRC2 is expected to catalyze the formation of an epoxide. While Figure 7 proposes one scheme in regard to timing of the glycosylation and oxidation reactions catalyzed by the GTFA, OXRC1 and OXRC2, the invention does not reside in the actual timing and order of the reactions, which may be different then that depicted in Figure 7.

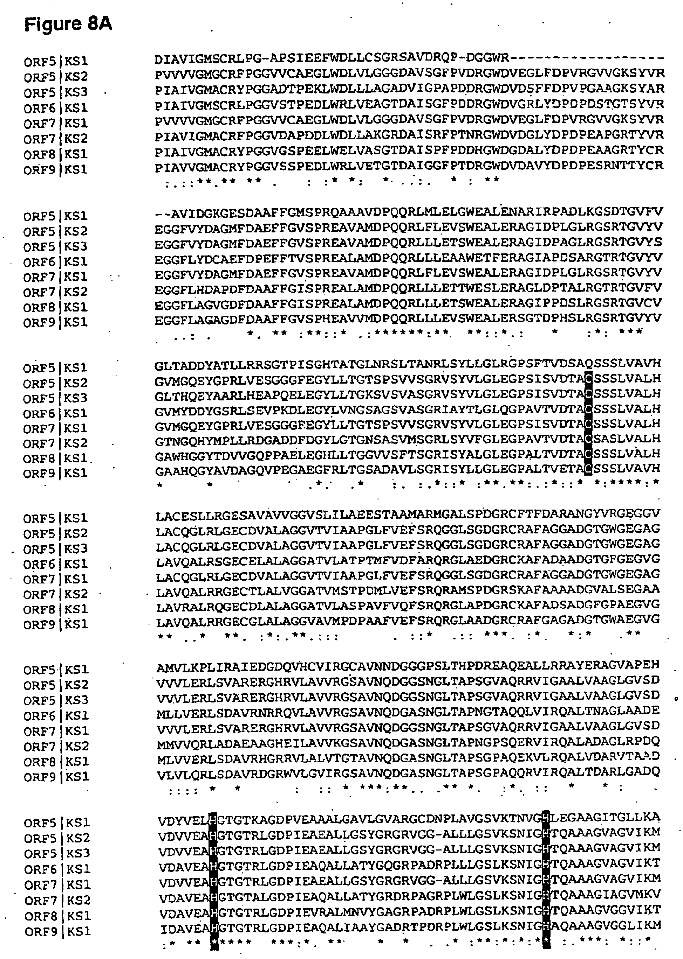

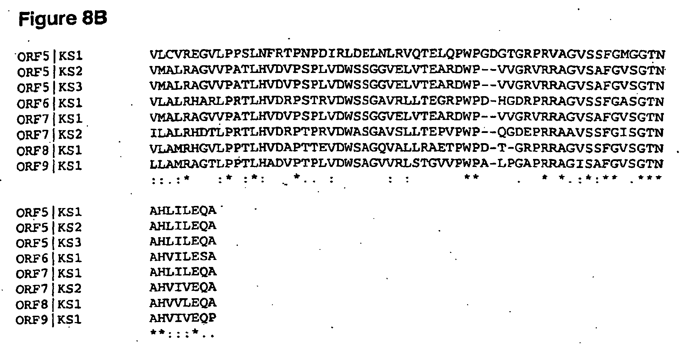

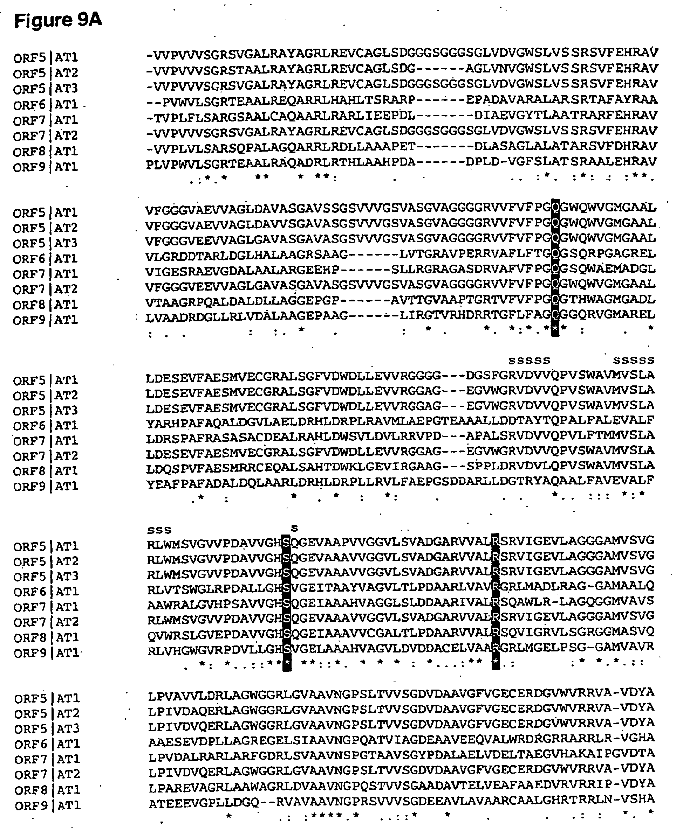

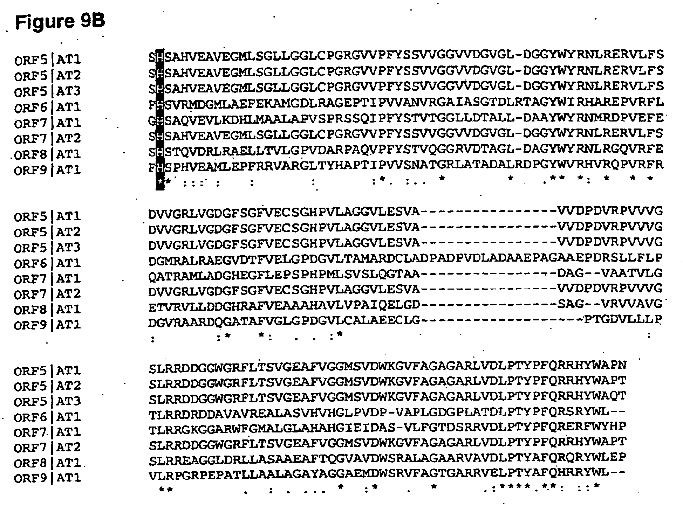

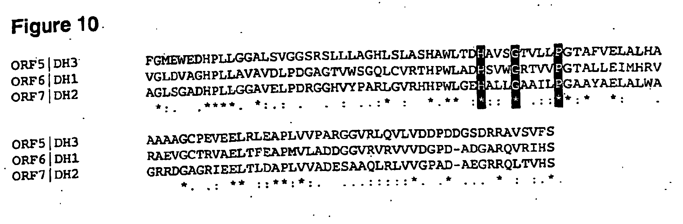

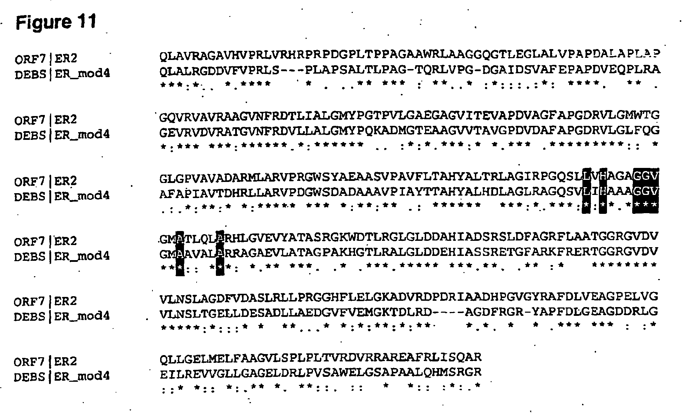

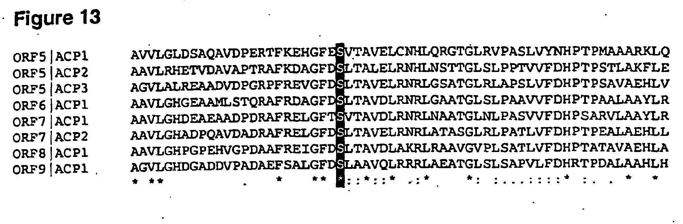

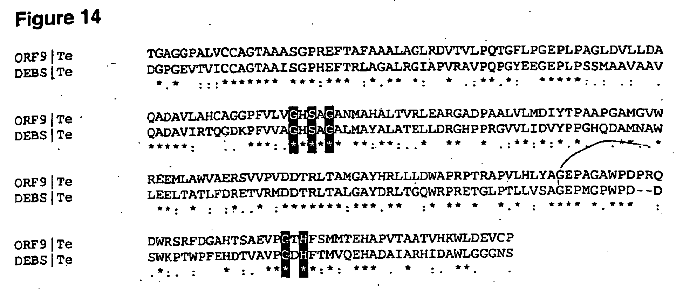

Figures 8 to 10 are amino acid alignments comparing the rosaramicin PKS domains. The domains which occur only once in the rosaramicin PKS, namely the enoylreductase (ER) and thioesterase (Te) domains, are compared to prototypical domains from the erythromycin PKS system (DEBS). Where applicable, key active site residues and motifs for the various polyketide synthase domains as described in Kakavas et al. (1997) J. Bacteriol. Vol 179 pp. 7515-7522 are indicated in Figures 8 to 14. In each of the clustal alignments a line above the alignement is used to mark strongly conserved positions. In addition, three characters, namely * (asterisk), : (colon) and . (period) are used, wherein "*" indicates positions which have a single, fully conserved residue; ":" indicates that one of the following strong groups is fully conserved: STA, NEQK, NHQK, NDEQ, QHRK, MILV, MILF, HY, and FYW; and "." Indicates that one of the following weaker groups is fully conserved: CSA, ATV, SAG, STNK, STPA, SGND, SNDEQK, NDEQHK, NEQHRK, FVLIM, and HFY.

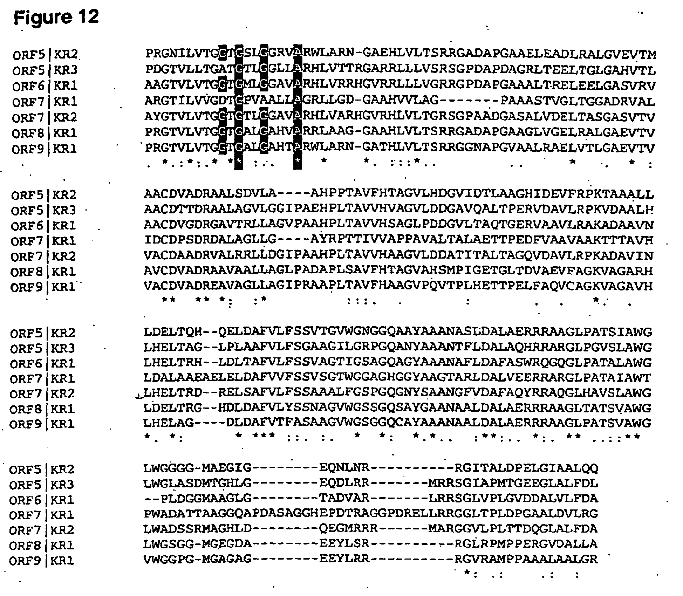

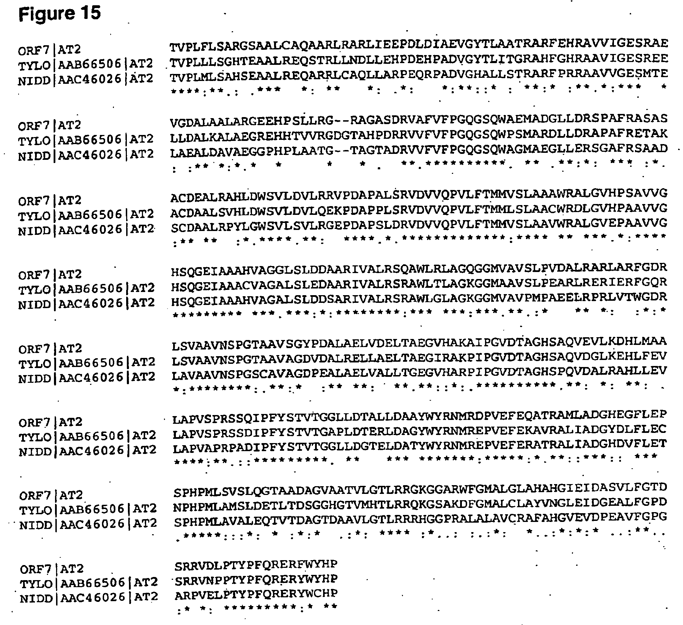

Of particular relevance with respect to PKS domain function, the KS domain in the loading module (ORF5|KS1) contains a Gln (Q) in place of the active site Cys (C) residue (Figure 8) and that the KR domain of the first module of ORF7 (ORF7|KR1) contains several amino acid substitutions in the key cofactor-binding motif (Figure 12). Figure 15 shows the high degree of overall homology between ethylmalonyl-CoA-specific AT domains from the tylosin PKS (TYLO) and the niddamycin PKS (NIDD) and the second AT domain of rosaramicin ORF 7. This high degree of homology is indicative of their shared substrate specificity.

REGS and REGM are involved in regulation of gene expression. ABCC, a membrane transport protein and MTRA, a rRNA methyltransferase, are involved in resistance to and/or export of rosaramicin. The TESA gene product represents a freestanding thioesterase enzyme that is expected to play a "proofreading" role in the assembly of the rosaramicin core polyketide precursor. The OXRH gene product represents a crotonyl CoA reductase that is involved in the formation of the acyl-CoA precursor used by the loading module of ORF 5 and/or the second module of ORF 7. The step involving crotonyl CoA reductase, *ie*. the OXRH gene product, is expected to be a rate-limiting step in the biosynthesis of rosaramicin (Stassi D.L. *et al., Proc Natl Acad Sci* 95(13), 7305-9, June 23, 1998) and it is expected that increasing the levels of the OXRH enzyme will have a beneficial effect on the yield of rosaramicin. The NBPA gene product is a nucleotide binding protein (i.e., contains a GTP/ATP binding motif) and is expected to activate a sugar by tethering it to a nucleotide, usually TTP. Therefore, the NBPA gene product is expected to be involved in the first step in the pathway leading to the formation of the deoxyamino sugar of rosaramicin.

### Example 4: Fermentation of Micronomospora carbonacea, aurantiaca and detection of rosaramicin:

*Micromonospora carbonacea aurantiaca* NRRL 2997 was cultured on a 30ml media A plate (glucose 1.0%, dextrin 4.0%. sucrose 1.5%, casein enzymatic hydrolysate 1.0%, MgSO₄ 0.1%, CaCO₃ 0.2%, and agar 2.2g/100ml) at 30°C for 14 days. The cells and agar were added to 25 ml of 95 % ethanol and incubated at room temperature for 2h under agitation. The ethanol phase was collected and the extraction step was repeated under the same conditions. The ethanol was evaporated from the pooled extracts and the residue was freeze-dried. The residue was then resuspended in 1.0 ml of water.

SPE of extracts: The C-18 solid phase column (Burdick & Jackson) was conditioned before use by sequential washing with 3ml of distilled water, 3 ml of methanol, and finally 3 ml of distilled water. The residue previously resuspended in 1.0 ml of water was loaded on the conditioned solid phase extraction system (SPE). Following passage of the sample though the SPE column washes were performed first, with 5 ml of water to remove polar materials, and then with 70% acetone and 30% methanol to elute a secondary metabolite-containing fraction which was then freeze-dried. This organic fraction was dissolved in 300ul of 50% acetonitrile―distilled water.

Chemical analysis: Chemical analysis of the organic fraction from the SPE column was performed by HPLC-ES-MS (Waters, ZQ systems). The extracts (50.0 ul) were separated on a C18 symmetry analytical column (2.1X150mm) with HPLC 2690 system (Waters) using a 60-min linear gradient from 30% acetonitrile-5mM ammonium acetate to 95% acetonitrile-5mM ammonium acetate at a flow rate of 150µl min⁻¹. UV and visible light absorption spectra (220 to 500nm) were acquired with a PDA (Waters) by using the column effluents prior to their analysis by ES-MS. The electrospray source was switched between positive ion mode and negative ion mode at 0.3 s intervals to acquire both positive and negative ion spectra. The cone voltage was 25.0 V. The capillary was maintained at 3.0 V. The source temperature was kept at 100°C. The desolvation temperature was kept at 400°C and the desolvation gas flow was 479 litre.h⁻¹. The data collection and analysis were performed with MassLynx V3.5 program (Waters).

Figure 8 is a HPLC-ES-MS analysis of rosaramicin showing a UV spectra at a retention time of 24.4 minutes and a MS spectra showing a molecular ion consistent with rosaramicin at retention time 24.4 minutes (mass of 582.57 [M+H]⁺).

It is further to be understood that all sizes and all molecular weight or mass values are approximate, and are provided for description.

## Claims

1. A nucleic acid comprising a nucleic acid sequence selected from the group consisting of:
(a) a nucleic acid of SEQ ID NOS: 3, 5, 9, 11, 13, 15, 17, 19, 21, 23, 25, 29, 31, 33, 35, 37, 39;
(b) a nucleic acid encoding a polypeptide of SEQ ID NOS: 2, 4, 8, 10, 12, 14, 16, 18, 20, 22, 24, 28, 30, 32, 34, 36, 38;
(c) a nucleic acid having at least 75% homology to a nucleic acid of (a) or (b) as determined by analysis with BLASTN version 2.0 with the default parameters and which can substitute for the nucleic acid of (a) or (b) to direct the synthesis of a rosaramicin compound or analogue;
(d) a nucleic acid capable of hybridizing under stringent conditions to a nucleic acid of (a), (b) or (c) and can substitute for the nucleic acid to which it specifically hybridizes to direct the synthesis of a rosaramicin compound or analogue;
(e) a nucleic acid comprising the sequence of at least two or at least three nucleic acids of (a) to (d);
(f) a nucleic acid comprising a nucleic acid that hybridizes to any one of rosaramicin open reading frames (ORFs) 1 to 19 (SEQ ID NOS: 3, 5, 7, 9, 11, 13, 15, 17, 19, 21, 23, 25, 27, 29, 31, 33, 35, 37, 39) and can substitute for the ORF to which it specifically hybridizes to direct the synthesis of a rosaramicin compound or analogue;
(g) a nucleic acid that hybridizes to any one of rosaramicin ORFs 1, 2, 4, 5, 6, 7, 8, 9, 10, 12, 14 or 15 (SEQ ID NOS: 3, 5, 9, 11, 13, 15, 17, 19, 21, 25, 29, and 31) and can substitute for the ORF to which it specifically hybridizes to direct the synthesis of a rosaramicin compound or analogue;
(h) a nucleic acid of any one of (a) to (g) that hybridizes under stringent conditions to a nucleic acid encoding a polypeptide selected from the group comprising SEQ ID NOS: 2, 4, 6, 8, 10, 12, 14, 16, 18, 20 and can substitute for the nucleic acid to which it specifically hybridizes to direct the synthesis of a rosaramicin compound or analogue;
(i) the nucleic acid of any one of (a) to (g) that hybridizes under stringent conditions to a nucleic acid encoding a polypeptide selected from the group consisting of SEQ ID NOS: 22, 24, 26, 28, 30, 32, 34, 36, 38 and can substitute for the nucleic acid to which it specifically hybridizes to direct the synthesis of a rosaramicin compound or analogue;
(j) a nucleic acid of SEQ ID NOS: 7 and 27 encoding a polypeptide of SEQ ID NOS: 6 and 26;
(k) a nucleic acid having at least 85% identity to a nucleic acid of (j) as determined by BLASTN Version 2.0 with the default parameters and which can substitute for the nucleic acid of (j) to direct the synthesis of a rosaramicin compound or analogue; and
(I) a nucleic acid complementary to a nucleic acid of (a) to (k).

2. A gene cluster to direct the synthesis of a rosaramicin compound or analogue comprising ORFs encoding the polyketide synthase system of SEQ ID NOs: 10, 12, 14, 16 and 18 sufficient to assemble a core polyketide precursor for rosaramicin.

3. The gene cluster of claim 2, further comprising ORFs encoding one or more of SEQ ID NOs.: 2, 4, 6, 8, 20, 22, 24, 26, 28, 30, 32, 34, 36, and 38.

4. A prokaryotic host cell containing the gene cluster of claim 2 or 3.

5. The gene cluster of claim 2 or claim 3 wherein the gene cluster contains a nucleic acid of any one of rosaramicin ORFs 1 to 19 (SEQ ID NOS: 3, 5, 7, 9, 11, 13, 15, 17, 19, 21, 23, 25, 27, 29, 31, 33, 35, 37, 39) present in the E. coli strains DH10B having accession nos. IDAC 100702-1, 100702-2 and 100702-3.

6. A polypeptide comprising a polypeptide sequence selected from any one of:
(a) a polypeptide of any one of SEQ ID NOS: 2, 4, 6, 8, 10, 12, 14, 16, 18, 20, 22, 24, 26, 28, 30, 32, 34, 36, 38; and
(b) a polypeptide which is at least 75% identical in amino acid sequence to a polypeptide of any one of SEQ ID NOS: 2, 4, 6, 8, 10, 12, 14, 16, 18, 20, 22, 24, 28, 30, 32, 34, 36, 38 as determined by analysis with BLASTP with the default parameters and which can substitute for the polypeptide of SEQ ID NO: 2, 4, 6, 8, 10, 12, 14, 16, 18, 20, 22, 24, 26, 28, 30, 32, 34, 36 or 38 to direct the synthesis of a rosaramicin compound or analogue.

7. The polypeptide of claim 6 wherein the polypeptide sequence is selected from any one of:
(a) a polypeptide of any one of rosaramicin ORFs 1, 2, 4, 5, 6, 7, 8, 9, 10, 12, 14 or 15 (SEQ ID NOS: 2, 4, 8, 10, 12, 14, 16, 18, 20, 24, 28 and 30); and
(b) a polypeptide which is at least 75% identical in amino acid sequence to a polypeptide of any one of rosaramicin ORFs 1, 2, 4, 5, 6, 7, 8, 9, 10, 12, 14 or 15 (SEQ ID NOS: 2, 4, 8, 10, 12, 14, 16, 18, 20, 24, 28 and 30) as determined by analysis with BLASTP with the default parameters and which can substitute for the polypeptide of SEQ ID NO: 2, 4, 8, 10, 12, 14, 16, 18, 20, 24, 28 or 30 to direct the synthesis of a rosaramicin compound or analogue.

8. A polypeptide comprising at least two, at least three or at least five or more polypeptides of claim 6 or 7.

9. A ketosynthase domain for the production of rosaramicin or a rosaramicin analog comprising a polypeptide selected from the group consisting of:
(a) residues 43-448 of SEQ ID NO: 10 and a polypeptide having at least 73% identity to residues 43-448 of SEQ ID NO: 10;
(b) residues 1055-1479 of SEQ ID NO: 10 and a polypeptide having 76% identity to residues 1055-1479 of SEQ ID NO: 10;
(c) residues 2557-2981 of SEQ ID NO: 10 and a polypeptide having at least 76% identity to residues 2557-2981 of SEQ ID NO: 10;
(d) residues 36-462 of SEQ ID NO: 12 and a polypeptide having at least 76% identity to residues 36-462 of SEQ ID NO: 12;
(e) residues 39-463 of SEQ ID NO: 14 and a polypeptide having at least 76% identity to residues 39-463 of SEQ ID NO: 14;
(f) residues 1591-2016 of SEQ ID NO: 14 and a polypeptide having at least 81% identity to residues 1591-2016 of SEQ ID NO: 14;
(g) residues 35-460 of SEQ ID NO: 16 and a polypeptide having at least 75% identity to residues 35-460 of SEQ ID NO: 16;
(h) residues 36-462 of SEQ ID NO: 18 and a polypeptide having at least 77% identity to residues 36-462 of SEQ ID NO: 18.

10. An acyl transferase domain for the production of rosaramicin or a rosaramicin analog, comprising a polypeptide selected from the group consisting of:
(a) residues 467-920 of SEQ ID NO: 10 and a polypeptide having at least 58% identity to residues 461-920. of SEQ ID NO: 10;
(b) residues 1502-1949 of SEQ ID NO: 10 and a polypeptide having at least 57% identity to residues 1502-1949 of SEQ ID NO: 10;
(c) residues 3016-3469 of SEQ ID NO: 10 and a polypeptide having at least 57% identity to residues 3016-3469 of SEQ ID NO: 10;
(d) residues 512-968 of SEQ ID NO: 12 and a polypeptide having at least 61% identity to the acyl transferase domain of residues 512-968 of SEQ ID NO: 12;
(e) residues 496-949 of SEQ ID NO: 14 and a polypeptide having at least 57% identity to residues 496-949 of SEQ ID NO: 14;
(f) residues 2032-2469 of SEQ ID NO: 14 and a polypeptide having at least 69% identity to residues 2032-2469 of SEQ 10 NO:14;
(g) residues 485-923 of SEQ ID NO: 16 and a polypeptide having at least 56% identity to residues 485-923 of SEQ ID NO: 16; and
(h) residues 478-915 of SEQ ID NO: 18 and a polypeptide having at least 62% identity to residues 478-915 of SEQ ID NO: 18.

11. An acyl carrier protein for the production of rosaramicin or a rosaramicin analog, comprising a polypeptide selected from the group consisting of:
(a) residues 971-1032 of SEQ ID NO: 10 and a polypeptide having at least 59% identity to residues 971-1032;
(b) residues 2473-2534 of SEQ ID NO: 10 and a polypeptide having 68% identity to residues 2473-2534 of SEQ ID NO: 10;
(c) residues 4323-4384 of SEQ ID NO: 10 and a polypeptide having at least 72% identity to residues 4323-4384 of SEQ ID NO: 10;
(d) residues 1767-1828 of SEQ ID NO: 12 and a polypeptide having 75% identity to residues 1767-1828 of SEQ ID NO: 12;
(e) residues 1500-1561 of SEQ ID NO: 14 and a polypeptide having at least 64% identity to residues 1500-1561 of SEQ ID NO: 14;
(f) residues 3592-3653 of SEQ ID NO: 14 and a polypeptide having at least 78% identity to residues 3592-3653 of SEQ ID NO: 14;
(g) residues 1434-1495 of SEQ ID NO: 16 and a polypeptide having at least 64% identity to residues 1434-1495 of SEQ ID NO: 16; and
(h) residues 1431-1492 of SEQ ID NO: 18 and a polypeptide having at least 67% identity to residues 1431-1492 of SEQ ID NO: 18.

12. A dehydratase domain for the production of rosaramicin or a rosaramicin analog, selected from the group consisting of
(a) residues 3482-3589 of SEQ ID NO: 10 and a polypeptide having at least 63% identity to residues 3482-3589 of SEQ ID NO: 10;
(b) residues 980-1086 of SEQ ID NO: 12 and a polypeptide having at least 60% identity to residues 980-1086 of SEQ ID NO: 12; and
(c) residues 2479-2585 of SEQ ID NO: 14 and a polypeptide having at least 62% identity to residues 2479-2585 of SEQ ID NO: 14.

13. A ketoreductase domain for the production of rosaramicin or a rosaramicin analog, comprising a polypeptide selected from the group consisting of:
(a) residues 2185-2392 of SEQ ID NO: 10 and a polypeptide having at least 64% identity to residues 2185-2392 of SEQ ID NO: 10;
(b) residues 4010-4226 of SEQ ID NO: 10 and a polypeptide having at least 72% identity to residues 4010-4226 of SEQ ID NO: 10;
(c) residues 1474-1688 of SEQ ID NO: 12 and a polypeptide having at least 65% identity to residues 1474-1688 of SEQ ID NO: 12;
(d) residues 1196-1417 of SEQ ID NO: 14 and a polypeptide having at least 52% identity to residues 1196-1417 of SEQ ID NO: 14;
(e) residues 3296-3513 of SEQ ID NO: 14 and a polypeptide having at least 67% identity to residues 3296-3513 of SEQ ID NO: 14;
(f) residues 1146-1357 of SEQ ID NO: 16 and a polypeptide having at least 70% identity to residues 1146-1357 of SEQ ID NO: 16; and
(g) residues 1147-1356 of SEQ ID NO: 18 and a polypeptide having at least 69% identity to residues 1147-1356 of SEQ ID NO: 18.

14. An enoyl reductase domain for the production of rosaramicin or a rosaramicin analog, comprising a polypeptide selected from the group consisting of residues 2943-3291 of SEQ ID NO: 14, and a polypeptide having at least 72% identity to residues 2943-3291 of SEQ ID NO: 14.

15. A thioesterase domain for the production of rosaramicin or a rosaramicin analog, comprising residues 1558-1780 of SEQ ID NO: 18 and a polypeptide having at least 65% identity to residues 1558-1780 of SEQ ID NO: 18.

16. An expression vector comprising a nucleic acid of claim 1.

17. A host cell transformed with an expression vector of claim 16.

18. The host cell of claim 17, wherein the cell is transformed with an exogenous nucleic acid comprising a gene cluster encoding polypeptides sufficient to direct the assembly of a rosaramicin compound or analogue.

19. A method of chemically modifying a biological molecule that is a substrate for a polypeptide encoded by a rosaramicin biosynthesis gene cluster, said method comprising contacting the biological molecule with a polypeptide of claim 6 or 7, wherein said polypeptide chemically modifies said biological molecule.

20. The method of chemically modifying a biological molecule that is a substrate for a polypeptide encoded by a rosaramicin biosynthesis gene cluster, said method comprising contacting the biological molecule with at least two different polypeptides of claim 6.

21. An antibody capable of specifically binding to a polypeptide having a sequence selected from the group consisting of SEQ ID NOS: 2, 4, 6, 8, 10, 12, 14, 16, 18, 20, 22, 24, 26, 28, 30, 32, 34, 36, 38.

22. A method of making a polypeptide having a sequence selected from the group consisting of SEQ ID NOS: 2, 4, 6, 8, 10, 12, 14, 16, 18, 20, 22, 24, 26, 28, 30, 32, 34, 36, 38 comprising introducing a nucleic acid encoding said polypeptide, said nucleic acid being operably linked to a promoter, into a host cell.

23. A method of making a rosaramicin compound or analog comprising the step of providing a bacterium containing a gene cluster with sufficient genes to produce a rosaramicin compound or analog and culturing the bacterium under conditions allowing for expression of the sufficient genes to produce a rosaramicin compound, wherein the gene cluster contains at least one nucleic acid of claim 1.

24. A method of making a rosaramicin compound or analog comprising culturing a *Micromonospora carbonacea* bacterium under conditions allowing for expression of rosaramicin ORFs 1 to 19 (SEQ ID NOS: 3, 5, 7, 9, 11, 13, 15, 17, 19, 21, 23, 25, 27, 29, 31, 33, 35, 37, 39) present in the E. coli strains DH10B having accession nos. IDAC 100702-1, 100702-2 and 100702-3.

## Patentansprüche

1. Nucleinsäure, umfassend eine Nucleinsäuresequenz ausgewählt aus der Gruppe bestehend aus:
(a) einer Nucleinsäure von SEQ ID NOs: 3, 5, 9, 11, 13, 15, 17, 19, 21, 23, 25, 29, 31, 33, 35, 37, 39;
(b) einer Nucleinsäure, die ein Polypeptid von SEQ ID NOs: 2, 4, 8, 10, 12, 14, 16, 18, 20, 22, 24, 28, 30, 32, 34, 36, 38 codiert;
(c) einer Nucleinsäure, die mindestens 75% Homologie zu einer Nucleinsäure aus (a) oder (b) aufweist, wie durch eine Analyse mit BLASTN, Version 2.0 mit den Standardparametern bestimmt, und die die Nucleinsäure aus (a) oder (b) ersetzen kann, um die Synthese einer Rosaramicinverbindung oder eines Rosaramicinanalogs zu steuern;
(d) einer Nucleinsäure, die in der Lage ist, unter stringenten Bedingungen mit einer Nucleinsäure aus (a), (b) oder (c) zu hybridisieren, und die die Nucleinsäure ersetzen kann, mit der sie spezifisch hybridisiert, um die Synthese einer Rosaramicinverbindung oder eines Rosaramicinanalogs zu steuern;
(e) einer Nucleinsäure, umfassend die Sequenz von mindestens zwei oder mindestens drei Nucleinsäuren aus (a) bis (d);
(f) einer Nucleinsäure, umfassend eine Nucleinsäure, die mit einem der offenen Rosaramicin-Leserahmen (ORFs) 1 bis 19 (SEQ ID NOs: 3, 5, 7, 9, 11, 13, 15, 17, 19, 21, 23, 25, 27, 29, 31, 33, 35, 37, 39) hybridisiert und die den ORF ersetzen kann, mit dem sie spezifisch hybridisiert, um die Synthese einer Rosaramicinverbindung oder eines Rosaramicinanalogs zu steuern;
(g) einer Nucleinsäure, die mit einem der Rosaramicin-ORFs 1, 2, 4, 5, 6, 7, 8, 9, 10, 12, 14 oder 15 (SEQ ID NOs: 3, 5, 9, 11, 13, 15, 17, 19, 21, 25, 29 und 31) hybridisiert und die den ORF ersetzen kann, mit dem sie spezifisch hybridisiert, um die Synthese einer Rosaramicinverbindung oder eines Rosaramicinanalogs zu steuern;
(h) einer Nucleinsäure aus (a) bis (g), die unter stringenten Bedingungen mit einer Nucleinsäure hybridisiert, die ein Polypeptid codiert, ausgewählt aus der Gruppe umfassend SEQ ID NOs: 2, 4, 6, 8, 10, 12, 14, 16, 18, 20, und die die Nucleinsäure ersetzen kann, mit der sie spezifisch hybridisiert, um die Synthese einer Rosaramicinverbindung oder eines Rosaramicinanalogs zu steuern;
(i) einer Nucleinsäure aus (a) bis (g), die unter stringenten Bedingungen mit einer Nucleinsäure hybridisiert, die ein Polypeptid codiert, ausgewählt aus der Gruppe bestehend aus SEQ ID NOs: 22, 24, 26, 28, 30, 32, 34, 36, 38, und die die Nucleinsäure ersetzen kann, mit der sie spezifisch hybridisiert, um die Synthese einer Rosaramicinverbindung oder eines Rosaramicinanalogs zu steuern;
(j) einer Nucleinsäure der SEQ ID NOs: 7 und 27, die ein Polypeptid der SEQ ID NOs: 6 und 26 codiert;
(k) einer Nucleinsäure, die mindestens 85% Identität zu einer Nucleinsäure aus (j) aufweist, wie durch eine Analyse mit BLASTN, Version 2.0 mit den Standardparametern bestimmt, und die die Nucleinsäure aus (a) oder (b) ersetzen kann, um die Synthese einer Rosaramicinverbindung oder eines Rosaramicinanalogs zu steuern; und
(I) einer Nucleinsäure, die zu einer Nucleinsäure aus (a) bis (k) komplementär ist.

2. Gencluster um die Synthese einer Rosaramicinverbindung oder eines Rosaramicinanalogs zu steuern, umfassend ORFs, die das Polyketidsynthasesystem von SEQ ID NOs: 10, 12, 14, 16 und 18 codieren, das zur Anordnung eines Kernpolyketidvorläufers für Rosaramicin ausreicht.

3. Gencluster nach Anspruch 2, weiterhin umfassend ORFs, die eine oder mehrere der SEQ ID NOs: 2, 4, 6, 8, 20, 22, 24, 26, 28, 30, 32, 34, 36 und 38 codieren.

4. Prokaryontische Wirtszelle, umfassend einen Gencluster nach Anspruch 2 oder 3.

5. Gencluster nach Anspruch 2 oder 3, wobei der Gencluster eine Nucleinsäure eines der Rosaramicin-ORFs 1 bis 19 (SEQ ID NOs: 3, 5, 7, 9, 11, 13, 15, 17, 19, 21, 23, 25, 27, 29, 31, 33, 35, 37, 39) enthält, die in den E. coli-Stämmen DH1OB mit den Hinterlegungsnummern IDAC 100702-1, 100702-2 und 100702-3 vorliegen.

6. Polypeptid, umfassend eine Polypeptidsequenz, ausgewählt aus:
(a) einem Polypeptid von einer der SEQ ID NOs: 2, 4, 6, 8, 10, 12, 14, 16, 18, 20, 22, 24, 26, 28, 30, 32, 34, 36, 38; und
(b) einem Polypeptid, das bezüglich seiner Aminosäuresequenz zu mindestens 75% identisch ist mit einem Polypeptid von einer der SEQ ID NOs: 2, 4, 6, 8, 10, 12, 14, 16, 18, 20, 22, 24, 28, 30, 32, 34, 36, 38, wie durch eine Analyse mit BLASTP mit den Standardparametern bestimmt, und das das Polypeptid der SEQ ID NOs: 2, 4, 6, 8, 10, 12, 14, 16, 18, 20, 22, 24, 26, 28, 30, 32, 34, 36 oder 38 ersetzen kann, um die Synthese einer Rosaramicinverbindung oder eines Rosaramicinanalogs zu steuern.

7. Polypeptid gemäß Anspruch 6, wobei die Polypeptidsequenz ausgewählt ist aus einem der folgenden:
(a) einem Polypeptid aus einem der Rosaramicin-ORFs 1, 2, 4, 5, 6, 7, 8, 9, 10, 12, 14 oder 15 (SEQ ID NOs: 2, 4, 8, 10, 12, 14, 16, 18, 20, 24, 28 und 30); und
(b) einem Polypeptid, das bezüglich seiner Aminosäuresequenz zu mindestens 75% identisch ist mit einem Polypeptid einer der Rosaramicin-ORFs 1, 2, 4, 5, 6, 7, 8, 9, 10, 12, 14 oder 15 (SEQ ID NOs: 2, 4, 8, 10, 12, 14, 16, 18, 20, 24, 28 oder 30), wie durch eine Analyse mit BLASTP mit den Standardparametern bestimmt, und das das Polypeptid der SEQ ID NOs: 2, 4, 8, 10, 12, 14, 16, 18, 20, 24, 28 oder 30 ersetzen kann, um die Synthese einer Rosaramicinverbindung oder eines Rosaramicinanalogs zu steuern.

8. Polypeptid, umfassend mindestens zwei, mindestens drei oder mindestens fünf oder mehr Polypeptide nach Anspruch 6 oder 7.

9. Ketosynthasedomäne zur Herstellung von Rosaramicin oder eines Rosaramicinanalogs, umfassend ein Polypeptid ausgewählt aus der Gruppe bestehend aus:
(a) den Resten 43-448 von SEQ ID NO:10 und einem Polypeptid, das zu mindestens 73% identisch ist mit den Resten 43-448 von SEQ ID NO:10;
(b) den Resten 1055-1479 von SEQ ID NO:10 und einem Polypeptid, das zu mindestens 76% identisch ist mit den Resten 1055-1479 von SEQ ID NO:10;
(c) den Resten 2557-2981 von SEQ ID NO:10 und einem Polypeptid, das zu mindestens 76% identisch ist mit den Resten 2557-2981 von SEQ ID NO:10;
(d) den Resten 36-462 von SEQ ID NO:12 und einem Polypeptid, das zu mindestens 76% identisch ist mit den Resten 36-462 von SEQ ID NO:12;
(e) den Resten 39-463 von SEQ ID NO:14 und einem Polypeptid, das zu mindestens 76% identisch ist mit den Resten 39-463 von SEQ ID NO:14;
(f) den Resten 1591-2016 von SEQ ID NO:14 und einem Polypeptid, das zu mindestens 81% identisch ist mit den Resten 1591-2016 von SEQ ID NO:14;
(g) den Resten 35-460 von SEQ ID NO:16 und einem Polypeptid, das zu mindestens 75% identisch ist mit den Resten 35-460 von SEQ ID NO:16;
(h) den Resten 36-462 von SEQ ID NO:18 und einem Polypeptid, das zu mindestens 77% identisch ist mit den Resten 36-462 von SEQ ID NO:18.

10. Acyltransferasedomäne zur Herstellung von Rosaramicin oder eines Rosaramicinanalogs, umfassend ein Polypeptid ausgewählt aus der Gruppe bestehend aus:
(a) den Resten 467-920 von SEQ ID NO:10 und einem Polypeptid, das zu mindestens 58% identisch ist mit den Resten 467-920 von SEQ ID NO:10;
(b) den Resten 1502-1949 von SEQ ID NO:10 und einem Polypeptid, das zu mindestens 57% identisch ist mit den Resten 1502-1949 von SEQ ID NO:10;
(c) den Resten 3016-3469 von SEQ ID NO:10 und einem Polypeptid, das zu mindestens 57% identisch ist mit den Resten 3016-3469 von SEQ ID NO:10;
(d) den Resten 512-968 von SEQ ID NO:12 und einem Polypeptid, das zu mindestens 61 % identisch ist mit der Acyltransferasedomäne der Reste 512-968 von SEQ ID NO:12;
(e) den Resten 496-949 von SEQ ID NO:14 und einem Polypeptid, das zu mindestens 57% identisch ist mit den Resten 496-949 von SEQ ID NO:14;
(f) den Resten 2032-2469 von SEQ ID NO:14 und einem Polypeptid, das zu mindestens 69% identisch ist mit den Resten 2032-2469 von SEQ ID NO:14;
(g) den Resten 485-923 von SEQ ID NO:16 und einem Polypeptid, das zu mindestens 56% identisch ist mit den Resten 485-923 von SEQ ID NO:16; und
(h) den Resten 478-915 von SEQ ID NO:18 und einem Polypeptid, das zu mindestens 62% identisch ist mit den Resten 478-915 von SEQ ID NO:18.

11. Acylträgerprotein zur Herstellung von Rosaramicin oder eines Rosaramicinanalogs, umfassend ein Polypeptid, ausgewählt aus der Gruppe bestehend aus:
(a) den Resten 971-1032 von SEQ ID NO:10 und einem Polypeptid, das zu mindestens 59% identisch ist mit den Resten 971-1032;
(b) den Resten 2473-2534 von SEQ ID NO:10 und einem Polypeptid, das zu mindestens 68% identisch ist mit den Resten 2473-2534 von SEQ ID NO:10;
(c) den Resten 4323-4384 von SEQ ID NO:10 und einem Polypeptid, das zu mindestens 72% identisch ist mit den Resten 4323-4384 von SEQ ID NO:10;
(d) den Resten 1767-1828 von SEQ ID NO:12 und einem Polypeptid, das zu mindestens 75% identisch ist mit den Resten 1767-1828 von SEQ ID NO:12;
(e) den Resten 1500-1561 von SEQ ID NO:14 und einem Polypeptid, das zu mindestens 64% identisch ist mit den Resten 1500-1561 von SEQ ID NO:14;
(f) den Resten 3592-3653 von SEQ ID NO:14 und einem Polypeptid, das zu mindestens 78% identisch ist mit den Resten 3592-3653 von SEQ ID NO:14;
(g) den Resten 1434-1495 von SEQ ID NO:16 und einem Polypeptid, das zu mindestens 64% identisch ist mit den Resten 1434-1495 von SEQ ID NO:16; und
(h) den Resten 1431-1492 von SEQ ID NO:18 und einem Polypeptid, das zu mindestens 67% identisch ist mit den Resten 1431-1492 von SEQ ID NO:18.

12. Dehydratasedomäne zur Herstellung von Rosaramicin oder eines Rosaramicinanalogs, ausgewählt aus der Gruppe bestehend aus:
(a) den Resten 3482-3589 von SEQ ID NO:10 und einem Polypeptid, das zu mindestens 63% identisch ist mit den Resten 3482-3589 von SEQ ID NO:10;
(b) den Resten 980-1086 von SEQ ID NO:12 und einem Polypeptid, das zu mindestens 60% identisch ist mit den Resten 980-1086 von SEQ ID NO:12; und
(c) den Resten 2479-2585 von SEQ ID NO:14 und einem Polypeptid, das zu mindestens 62% identisch ist mit den Resten 2479-2585 von SEQ ID NO:14.

13. Ketoreduktasedomäne zur Herstellung von Rosaramicin oder eines Rosaramicinanalogs, umfassend ein Polypeptid, ausgewählt aus der Gruppe bestehend aus:
(a) den Resten 2185-2392 von SEQ ID NO:10 und einem Polypeptid, das zu mindestens 64% identisch ist mit den Resten 2185-2392 von SEQ ID NO:10;
(b) den Resten 4010-4226 von SEQ ID NO:10 und einem Polypeptid, das zu mindestens 72% identisch ist mit den Resten 4010-4226 von SEQ ID NO:10;
(c) den Resten 1474-1688 von SEQ ID NO:12 und einem Polypeptid, das zu mindestens 65% identisch ist mit den Resten 1474-1688 von SEQ ID NO:12;
(d) den Resten 1196-1417 von SEQ ID NO:14 und einem Polypeptid, das zu mindestens 52% identisch ist mit den Resten 1196-1417 von SEQ ID NO:14;
(e) den Resten 3296-3513 von SEQ ID NO:14 und einem Polypeptid, das zu mindestens 67% identisch ist mit den Resten 3296-3513 von SEQ ID NO:14;
(f) den Resten 1146-1357 von SEQ ID NO:16 und einem Polypeptid, das zu mindestens 70% identisch ist mit den Resten 1146-1357 von SEQ ID NO:16; und
(g) den Resten 1147-1356 von SEQ ID NO:18 und einem Polypeptid, das zu mindestens 69% identisch ist mit den Resten 1147-1356 von SEQ ID NO:18.

14. Enoylreduktasedomäne zur Herstellung von Rosaramicin oder eines Rosaramicinanalogs, umfassend ein Polypeptid, ausgewählt aus der Gruppe bestehend aus den Resten 2943-3291 von SEQ ID NO:14 und einem Polypeptid, das zu mindestens 72% identisch ist mit den Resten 2943-3291 von SEQ ID NO:14.

15. Thioesterasedomäne zur Herstellung von Rosaramicin oder eines Rosaramicinanalogs, umfassend die Reste 1558-1780 von SEQ ID NO:18 und ein Polypeptid, das zu mindestens 65% identisch ist mit den Resten 1558-1780 von SEQ ID NO:18.

16. Expressionsvektor umfassend eine Nucleinsäure nach Anspruch 1.

17. Wirtszelle, transformiert mit einem Expressionsvektor nach Anspruch 16.

18. Wirtszelle nach Anspruch 17, wobei die Zelle mit einer exogenen Nucleinsäure transformiert wird, die einen Gencluster umfasst, der Polypeptide codiert, die ausreichen, um die Anordnung einer Rosaramicinverbindung oder eines Rosaramicinanalogs zu steuern.

19. Verfahren zum chemischen Modifizieren eines biologischen Moleküls, das ein Substrat für ein Polypeptid ist, das von einem Rosaramicinbiosynthese-Gencluster codiert wird, wobei das Verfahren das Inkontaktbringen des biologischen Moleküls mit einem Polypeptid nach Anspruch 6 oder 7 umfasst, wobei das Polypeptid das biologische Molekül chemisch modifiziert.

20. Verfahren zum chemischen Modifizieren eines biologischen Moleküls, das ein Substrat für ein Polypeptid ist, das von einem Rosaramicinbiosynthese-Gencluster codiert wird, wobei das Verfahren das Inkontaktbringen des biologischen Moleküls mit mindestens zwei Polypeptiden nach Anspruch 6 umfasst.

21. Antikörper, der in der Lage ist, spezifisch an ein Polypeptid mit einer Sequenz, ausgewählt aus der Gruppe bestehend aus SEQ ID NOs: 2, 4, 6, 8, 10, 12, 14, 16, 18, 20, 22, 24, 26, 28, 30, 32, 34, 36, 38, zu binden.

22. Verfahren zur Herstellung eines Polypeptids mit einer Sequenz, ausgewählt aus der Gruppe bestehend aus SEQ ID NOs: 2, 4, 6, 8, 10, 12, 14, 16, 18, 20, 22, 24, 26, 28, 30, 32, 34, 36, 38, umfassend das Einführen einer das Polypeptid codierenden, funktionell mit einem Promotor verknüpften Nucleinsäure in eine Wirtszelle.

23. Verfahren zur Herstellung einer Rosaramicinverbindung oder eines Rosaramicinanalogs, umfassend die Schritte des Bereitstellens eines Bakteriums, das einen Gencluster mit ausreichend Genen enthält, um eine Rosaramicinverbindung oder ein Rosaramicinanalog herzustellen, und Züchten des Bakteriums unter Bedingungen, die die Expression der ausreichenden Gene ermöglicht, um eine Rosaramicinverbindung herzustellen, wobei der Gencluster mindestens eine Nucleinsäure nach Anspruch 1 enthält.

24. Verfahren zur Herstellung einer Rosaramicinverbindung oder eines Rosaramicinanalogs, umfassend das Züchten eines *Micromonospora carbonacea*-Bakteriums unter Bedingungen, die die Expression von Rosaramicin-ORFs 1 bis 19 (SEQ ID NOs: 3, 5, 7, 9, 11, 13, 15, 17, 19, 21, 23, 25, 27, 29, 31, 33, 35, 37, 39) erlauben, die in den E. coli-Stämmen DH10B mit den Hinterlegungsnummern IDAC 100702-1, 100702-2 und 100702-3 vorhanden sind.

## Revendications

1. Acide nucléique comprenant une séquence d'acide nucléique choisie dans le groupe constitué de:
(a) un acide nucléique de SEQ ID NOS: 3, 5, 9, 11, 13, 15, 17, 19, 21, 23, 25, 29, 31, 33, 35, 37, 39;
(b) un acide nucléique codant un polypeptide de SEQ ID NOS: 2, 4, 8, 10, 12, 14, 16, 18, 20, 22, 24, 28, 30, 32, 34, 36, 38;
(c) un acide nucléique ayant au moins 75% d'homologie avec un acide nucléique de (a) ou (b) telle que déterminée par analyse avec BLASTN version 2.0 avec les paramètres par défaut et qui peut être substitué à l'acide nucléique de (a) ou (b) pour diriger la synthèse d'un composé rosaramicine ou d'un analogue de rosaramicine;
(d) un acide nucléique capable de s'hybrider dans des conditions stringentes à un acide nucléique de (a), (b) ou (c) et qui peut être substitué à l'acide nucléique auquel il s'hybride spécifiquement pour diriger la synthèse d'un composé rosaramicine ou d'un analogue de rosaramicine;
(e) un acide nucléique comprenant la séquence d'au moins deux ou d'au moins trois acides nucléiques de (a) à (d);
(f) un acide nucléique comprenant un acide nucléique qui s'hybride à l'un quelconque des cadres ouverts de lecture (ORFs) 1 à 19 de la rosaramicine (SEQ ID NOS: 3, 5, 7, 9, 11, 13, 15, 17, 19, 21, 23, 25, 27, 29, 31, 33, 35, 37, 39) et qui peut être substitué à l'ORF auquel il s'hybride spécifiquement pour diriger la synthèse d'un composé rosaramicine ou d'un analogue de rosaramicine;
(g) un acide nucléique qui s'hybride à l'un quelconque des ORFs 1, 2, 4, 5, 6, 7, 8, 9, 10, 12, 14 ou 15 de la rosaramicine (SEQ ID NOS: 3, 5, 9, 11, 13, 15, 17, 19, 21, 25, 29 et 31) et qui peut être substitué à l'ORF auquel il s'hybride spécifiquement pour diriger la synthèse d'un composé rosaramicine ou d'un analogue de rosaramicine;
(h) un acide nucléique de l'un quelconque de (a) à (g) qui s'hybride dans des conditions stringentes à un acide nucléique codant un polypeptide choisi dans le groupe comprenant SEQ ID NOS: 2, 4, 6, 8, 10, 12, 14, 16, 18, 20 et qui peut être substitué à l'acide nucléique auquel il s'hybride spécifiquement pour diriger la synthèse d'un composé rosaramicine ou d'un analogue de rosaramicine;
(i) un acide nucléique de l'un quelconque de (a) à (g) qui s'hybride dans des conditions stringentes à un acide nucléique codant un polypeptide choisi dans le groupe constitué de SEQ ID NOS: 22, 24, 26, 28, 30, 32, 34, 36, 38 et qui peut être substitué à l'acide nucléique auquel il s'hybride spécifiquement pour diriger la synthèse d'un composé rosaramicine ou d'un analogue de rosaramicine ;
(j) un acide nucléique de SEQ NOS: 7 et 27 codant un polypeptide de SEQ ID NOS: 6 et 26;
(k) un acide nucléique ayant au moins 85% d'identité avec un acide nucléique de (j) telle que déterminée par BLASTN version 2.0 avec les paramètres par défaut et qui peut être substitué à l'acide nucléique de (j) pour diriger la synthèse d'un composé rosaramicine ou d'un analogue de rosaramicine; et
(1) un acide nucléique complémentaire d'un acide nucléique de (a) à (k).

2. Batterie de gènes pour diriger la synthèse d'un composé rosaramicine ou d'un analogue de rosaramicine comprenant des ORFs codant le système de polykétide synthase de SEQ ID NOS: 10, 12, 14, 16 et 18 suffisant pour assembler un précurseur de noyau polyketide pour une rosaramicine.

3. Batterie de gènes selon la revendication 2, comprenant en outre des ORFs codant une ou plusieurs de SEQ ID NOS: 2, 4, 6, 8, 20, 22, 24, 26, 28, 30, 32, 34, 36 et 38.

4. Cellule hôte procaryote contenant la batterie de gènes selon la revendication 2 ou 3.

5. Batterie de gènes selon la revendication 2 ou 3 dans laquelle la batterie de gènes contient un acide nucléique de l'un quelconque des ORFs 1 à 19 de la rosaramicine (SEQ ID NOS: 3, 5, 7, 9, 11, 13, 15, 17, 19, 21, 23, 25, 27, 29, 31, 33, 35, 37, 39) présent des les souches E.Coli DH10B ayant les numéros de dépôt IDAC 100702-1, 100702-2 et 100702-3.

6. Polypeptide comprenant une séquence polypeptidique choisie parmi l'un quelconque de:
(a) un polypeptide de l'une quelconque de SEQ ID NOS: 2, 4, 6, 8, 10, 12, 14, 16, 18, 20, 22, 24, 26, 28, 30, 32, 34, 36, 38; et
(b) un polypeptide qui est au moins 75% identique en séquence d'acides aminés à un polypeptide de l'une quelconque de SEQ ID NOS: 2, 4, 6, 8, 10, 12, 14, 16, 18, 20, 22, 24, 28, 30, 32, 34, 36, 38 tel que déterminé par analyse avec BLASP avec les paramètres par défaut et qui peut être substitué au polypeptide de SEQ ID NO: 2, 4, 6, 8, 10, 12, 14, 16, 18, 20, 22, 24, 26, 28, 30, 32, 34, 36 ou 38 pour diriger la synthèse d'un composé rosaramicine ou d'un analogue de rosaramicine.

7. Polypeptide selon la revendication 6 dans lequel la séquence polypeptidique est choisie parmi l'une quelconque de:
(a) un polypeptide de l'un quelconque des ORFs 1, 2, 4, 5, 6, 7, 8, 9, 10, 12, 14 ou 15 de la rosaramicine (SEQ ID NOS: 2, 4, 8, 10, 12, 14, 16, 18, 20, 24, 28 et 30); et
(b) un polypeptide qui est au moins 75% identique en séquence d'acides aminés à un polypeptide de l'un quelconque des ORFs 1, 2, 4, S, 6, 7, 8, 9, 10, 12, 14 ou 15 de la rosaramicine (SEQ ID NOS: 2, 4, 8, 10, 12, 14, 16, 18, 20, 24, 28 et 30) comme déterminé par analyse avec BLASTP avec les paramètres par défaut et qui peut être substitué au polypeptide de SEQ ID NO: 2, 4, 8, 10, 12, 14, 16, 18, 20, 24, 28 ou 30 pour diriger la synthèse d'un composé rosaramicine ou d'un analogue de rosaramicine.

8. Polypeptide comprenant au moins deux, au moins trois ou au moins cinq ou plus polypeptides selon la revendication 6 ou 7.

9. Domaine kétosynthase pour la production de rosaramicine ou d'un analogue de rosaramicine comprenant un polypeptide choisi dans le groupe constitué de:
(a) résidus 43-448 de SEQ ID NO: 10 et un polypeptide ayant au moins 73% d'identité avec les résidus 43-448 de SEQ ID NO: 10;
(b) résidus 1055-1479 de SEQ ID NO: 10 et un polypeptide ayant au moins 76% d'identité avec les résidus 1055-1479 de SEQ ID NO: 10;
(c) résidus 2557-2981 de SEQ ID NO: 10 et un polypeptide ayant au moins 76% d'identité avec les résidus 2557-2981 de SEQ ID NO: 10;
(d) résidus 36-462 de SEQ ID NO: 12 et un polypeptide ayant au moins 76% d'identité avec les résidus 36-462 de SEQ ID NO: 12;
(e) résidus 39-463 de SEQ ID NO: 14 et un polypeptide ayant au moins 76% d'identité avec les résidus 39-463 de SEQ ID NO: 14;
(f) résidus 1591-2016 de SEQ ID NO: 14 et un polypeptide ayant au moins 81% d'identité avec les résidus 1591-2016 de SEQ ID NO: 14;
(g) résidus 35-460 de SEQ ID NO: 16 et un polypeptide ayant au moins 75% d'identité avec les résidus 35-460 de SEQ ID NO: 16;
(h) résidus 36-462 de SEQ ID NO: 18 et un polypeptide ayant au moins 77% d'identité avec les résidus 36-462 de SEQ ID NO: 18.

10. Domaine acyle transférase pour la production de rosaramicine ou d'un analogue de rosaramicine, comprenant un polypeptide choisi dans le groupe constitué de:
(a) résidus 467-920 de SEQ ID NO: 10 et un polypeptide ayant au moins 58% d'identité avec les résidus 467-920 de SEQ ID NO: 10;
(b) résidus 1502-1949 de SEQ ID NO: 10 et un polypeptide ayant au moins 57% d'identité avec les résidus 1502-1949 de SEQ ID NO: 10;
(c) résidus 3016-3469 de SEQ ID NO: 10 et un polypeptide ayant au mois 57% d'identité avec les résidus 3016-3469 de SEQ ID NO: 10;
(d) résidus 512-968 de SEQ ID NO: 12 et un polypeptide ayant au moins 61% d'identité avec le domaine acyle transférase des résidus 512-968 de SEQ ID NO: 12;
(e) résidus 496-949 de SEQ ID NO: 14 et un polypeptide ayant au moins 57% d'identité avec les résidus 496-949 de SEQ ID NO: 14;
(f) résidus 2032-2469 de SEQ ID NO: 14 et un polypeptide ayant au moins 69% d'identité avec les résidus 2032-2469 de SEQ ID NO: 14;
(g) résidus 485-923 de SEQ ID NO: 16 et un polypeptide ayant au moins 56% d'identité avec les résidus 485-923 de SEQ ID NO: 16; et
(h) résidus 478-915 de SEQ ID NO: 18 et un polypeptide ayant au moins 62% d'identité avec les résidus 478-915 de SEQ ID NO: 18.

11. Protéine porteuse d'acyle pour la production de rosaramicine ou d'un analogue de rosaramicine, comprenant un polypeptide choisi dans le groupe constitué de:
(a) résidus 971-1032 de SEQ ID NO: 10 et un polypeptide ayant au moins 59% d'identité avec les résidus 971-1032;
(b) résidus 2473-2534 de SEQ ID NO: 10 et un polypeptide ayant 68% d'identité avec les résidus 2473-2534 de SEQ ID NO: 10;
(c) résidus 4323-4384 de SEQ ID NO: 10 et un polypeptide ayant au moins 72% d'identité avec les résidus 4323-4384 de SEQ ID NO: 10;
(d) résidus 1767-1828 de SEQ ID NO: 12 et un polypeptide ayant au moins 75% d'identité avec les résidus 1767-1828 de SEQ ID NO: 12;
(e) résidus 1500-1561 de SEQ ID NO: 14 et un polypeptide ayant au moins 64% d'identité avec les résidus 1500-1561 de SEQ ID NO: 14;
(f) résidus 3592-3653 de SEQ ID NO: 14 et un polypeptide ayant au moins 78% d'identité avec les résidus 3592-3653 de SEQ ID NO: 14;
(g) résidus 1434-1495 de SEQ ID NO: 16 et un polypeptide ayant au moins 64% d'identité avec les résidus 1434-1495 de SEQ ID NO: 16; et
(h) résidus 1431-1492 de SEQ ID NO: 18 et un polypeptide ayant au moins 67% d'identité avec les résidus 1431-1492 de SEQ ID NO: 18.

12. Domaine déshydratase pour la production de rosaramicine ou d'un analogue de rosaramicine, choisi dans le groupe constitué de:
(a) résidus 3482-3589 de SEQ ID NO: 10 et un polypeptide ayant au moins 63% d'identité avec les résidus 3482-3589 de SEQ ID NO: 10;
(b) résidus 980-1086 de SEQ ID NO: 12 et un polypeptide ayant au moins 60% d'identité avec les résidus 980-1086 de SEQ ID NO: 12; et
(c) résidus 2479-2585 de SEQ ID NO: 14 et un polypeptide ayant au moins 62% d'identité avec les résidus 2479-2585 de SEQ ID NO: 14.

13. Domaine kétoréductase pour la production de rosaramicine ou d'un analogue de rosaramicine, comprenant un polypeptide choisi dans le groupe constitué de:
(a) résidus 2185-2392 de SEQ ID NO: 10 et un polypeptide ayant au moins 64% d'identité avec les résidus 2185-2392 de SEQ ID NO: 10;
(b) résidus 4010-4226 de SEQ ID NO: 10 et un polypeptide ayant au moins 72% d'identité avec les résidus 4010-4226 de SEQ ID NO: 10;
(c) résidus 1474-1688 de SEQ ID NO: 12 et un polypeptide ayant au moins 65% d'identité avec les résidus 1474-1688 de SEQ ID NO: 12;
(d) résidus 1196-1417 de SEQ ID NO: 14 et un polypeptide ayant au moins 52% d'identité avec les résidus 1196-1417 de SEQ ID NO: 14;
(e) résidus 3296-3513 de SEQ ID NO: 14 et un polypeptide ayant au moins 67% d'identité avec les résidus 3296-3513 de SEQ ID NO: 14;
(f) résidus 1146-1357 de SEQ ID NO: 16 et un polypeptide ayant au moins 70% d'identité avec les résidus 1146-1357 de SEQ ID NO: 16; et
(g) résidus 1147-1356 de SEQ ID NO: 18 et un polypeptide ayant au moins 69% d'identité avec les résidus 1147-1356 de SEQ ID NO: 18.

14. Domaine énoyle réductase pour la production de rosaramicine ou d'un analogue de rosaramicine, comprenant un polypeptide choisi dans le groupe constitué des résidus 2943-3291 de SEQ ID NO:14, et un polypeptide ayant au moins 72% d'identité avec les résidus 2943-3291 de SEQ ID NO: 14.

15. Domaine thioestérase pour la production de rosaramicine ou d'un analogue de rosaramicine, comprenant les résidus 1558-1780 de SEQ ID NO: 18 et un polypeptide ayant au moins 65% d'identité avec les résidus 1558-1780 de SEQ ID NO: 18.

16. Vecteur d'expression comprenant un acide nucléique selon la revendication 1.

17. Cellule hôte transformée avec un vecteur d'expression selon la revendication 16.

18. Cellule hôte selon la revendication 17, dans laquelle la cellule est transformée avec un acide nucléique exogène comprenant une batterie de gènes codant des polypeptides suffisants pour diriger l'assemblage d'un composé rosaramicine ou d'un analogue de rosaramicine.

19. Procédé pour modifier chimiquement une molécule biologique qui est un substrat d'un polypeptide codé par une batterie de gènes de biosynthèse de la rosaramicine, ledit procédé comprenant la mise en contact de la molécule biologique avec un polypeptide selon la revendication 6 ou 7, dans lequel ledit polypeptide modifie chimiquement ladite molécule biologique.

20. Procédé pour modifier chimiquement une molécule biologique qui est un substrat d'un polypeptide codé par une batterie de gènes de biosynthèse de rosaramicine, ledit procédé comprenant la mise en contact de la molécule biologique avec au moins deux polypeptides différents selon la revendication 6.

21. Anticorps capable de se lier spécifiquement à un polypeptide ayant une séquence choisie dans le groupe constitué de SEQ ID NOS: 2, 4, 6, 8, 10, 12, 14, 16, 18, 20, 22, 24, 26, 28, 30, 32, 34, 36, 38.

22. Procédé pour fabriquer un polypeptide ayant une séquence choisie dans le groupe constitué de SEQ ID NOS: 2, 4, 6, 8, 10, 12, 14, 16, 18, 20, 22, 24, 26, 28, 30, 32, 34, 36, 38 comprenant l'introduction d'un acide nucléique codant ledit polypeptide, ledit acide nucléique étant lié de manière opérationnelle à un promoteur, dans une cellule hôte.

23. Procédé pour fabriquer un composé rosaramicine ou d'un analogue de rosaramicine comprenant l'étape de fourniture d'une bactérie contenant une batterie de gènes avec les gènes suffisants pour produire un composé rosaramicine ou d'un analogue de rosaramicine et de culture de la bactérie dans des conditions permettant l'expression des gènes suffisants pour produire un composé rosaramicine, dans lequel ladite batterie de gènes contient au moins un acide nucléique selon la revendication 1.

24. Procédé pour fabriquer un composé rosaramicine ou d'un analogue de rosaramicine comprenant la culture d'une bactérie *Micromonospora carbonacea* dans des conditions permettant l'expression des ORFs 1 à 19 de la rosaramicine (SEQ ID NOS: 3, 5, 7, 9, 11, 13, 15, 17, 19, 21, 23, 25, 27, 29, 31, 33, 35, 37, 39) présentes dans les souches E. coli DH10B ayant les numéros de dépôt IDAC 100702-1, 100702-2 et 100702-3.
